(19) Europäisches Patentamt
European Patent Office
Office européen des brevets

(11) EP 1 730 198 B1

(12) EUROPEAN PATENT SPECIFICATION

(45) Date of publication and mention of the grant of the patent:
05.05.2010 Bulletin 2010/18

(21) Application number: 05724507.8

(22) Date of filing: 02.03.2005

(51) Int Cl.:
C07K 19/00 (2006.01)   C12N 9/76 (2006.01)
A61K 38/48 (2006.01)   C12N 15/52 (2006.01)
C12N 15/62 (2006.01)   C12N 5/10 (2006.01)
C12N 1/20 (2006.01)   C12N 1/16 (2006.01)

(86) International application number:
PCT/US2005/006976

(87) International publication number:
WO 2005/085430 (15.09.2005 Gazette 2005/37)

(54) **ADZYMES AND USES THEREOF**

ADZYME UND DEREN VERWENDUNGEN

CONSTRUCTIONS DE PROTEINES APPELEES  ADZYMES  ET LEURS UTILISATIONS

(84) Designated Contracting States:
AT BE BG CH CY CZ DE DK EE ES FI FR GB GR HU IE IS IT LI LT LU MC NL PL PT RO SE SI SK TR

(30) Priority: 02.03.2004  US 792498

(43) Date of publication of application:
13.12.2006   Bulletin 2006/50

(73) Proprietor: **Bristol-Myers Squibb Company Princeton NJ 08543-4000 (US)**

(72) Inventors:
• **AFEYAN, Noubar, B.**
  **Lexington, MA 02421 (US)**
• **LEE, Frank, D.**
  **Chestnut Hill, MA 02467 (US)**
• **DAS GUPTA, Ruchira**
  **Auburndale, MA 02466 (US)**
• **BAYNES, Brian**
  **Apt.1, Somerville, MA 02144 (US)**
• **CAMPHAUSEN, Ray**
  **Waltham, MA 02452 (US)**
• **WONG Gordon  G.**
  **Brookline, MA 02445 (US)**

(74) Representative: **Reitstötter - Kinzebach Patentanwälte Sternwartstrasse 4 81679 München (DE)**

(56) References cited:
WO-A-97/20038         WO-A-02/097042
US-A1- 2004 081 647

• NYARUHUCHA CORNELIO N M ET AL: "Identification and expression of the cDNA-encoding human mesotrypsin(ogen), an isoform of trypsin with inhibitor resistance" JOURNAL OF BIOLOGICAL CHEMISTRY, vol. 272, no. 16, 1997, pages 10573-10578, XP002356307 ISSN: 0021-9258
• SZMOLA RICHÁRD ET AL: "Human mesotrypsin is a unique digestive protease specialized for the degradation of trypsin inhibitors" JOURNAL OF BIOLOGICAL CHEMISTRY, AMERICAN SOCIETY OF BIOLOCHEMICAL BIOLOGISTS, BIRMINGHAM,, US, vol. 278, no. 49, 5 December 2003 (2003-12-05), pages 48580-48589, XP002342456 ISSN: 0021-9258

## Description

### Background of the Invention

This invention relates to synthetic protein constructs useful in modulating a variety of targeted molecules *in situ.* In particular aspects, it relates to a family of constructs employing linked molecular parts which target and modulate the activity of a biomolecule catalytically to induce a therapeutic effect.

Many diseases are caused by or associated with biomolecules, either free in solution in body fluids or exposed to extracellular body fluids such as membrane-bound proteins and polysaccharides, such as cytokines or growth factors, and it is widely recognized that it is possible to develop therapies for such diseases by modulating the activity of the biomolecule.

For example, overproduction of TNF-$\alpha$ and/or TNF-$\beta$ is closely linked to the development of many diseases, including septic shock, adult respiratory distress syndrome, rheumatoid arthritis, selective autoimmune disorders, graft-host disease following bone marrow transplantation and cachexia. Other diseases associated with excessive TNF-$\alpha$ and/or TNF-$\beta$ production include hemorrhagic shock, asthma and post-renal dialysis syndrome. The multiplicity of actions of TNF-$\alpha$ and TNF-$\beta$ can be ascribed to the fact that TNF-$\alpha$ and/or TNF-$\beta$ actions result in activation of multiple signal transduction pathways, kinases, transcription factors, as well as an unusually large array of cellular genes. (Walajtys-Rode, Elizbieta, Kosmos (Warsaw), 44, 451-464, 1995, C.A. 124:199735a, 1995). TNF$\alpha$ has also been linked to the development of autoimmune disorders.

Current therapies for combating the foregoing disorders include the administration of a binding agent, such as an antibody or soluble receptor, that binds to and thereby inhibits a targeted biomolecule that causes or is associated with the disease. However, there are many drawbacks associated with this approach. For example, binding agents, by their very nature, can only inhibit the biomolecules(s) to which they are bound, and can neither catalytically inactivate a series of biomolecules nor chemically alter the bound biomolecules(s). It is probably for these reasons that relatively large doses of binding agents are often needed to achieve therapeutic effectiveness, exposing the subject to dangerous and often toxic side-effects. Moreover, production of such large quantities of antibodies and other binding agents is expensive.

WO 02/097042 describes fusion proteins which comprise (i) an apoptosis inducing protein portion, which may be a protease, and (ii) a cell targeting portion.

The identification and expression of the cDNA-encoding human mesotrypsin(ogen), an isoform of trypsin(ogen) with inhibitor resistance, is described in Cornelio N.M. Nyaruhucha, Makoto Kito, and Shin-Ichi Fukuoka, The Journal of Biological Chemistry, Vol. 272, 10573-10578, 1997.

Targeted therapeutic agents with greater effectiveness than traditional binding agent therapeutics would be a desirable improvement.

### Summary of the Invention

The present invention relates to fusion proteins comprising:

(a) a mesotrypsin catalytic domain that catalyzes the proteolysis of a target substrate; and

(b) a targeting moiety that binds to the target substrate.

In certain aspects, the fusion proteins of the invention are a new class of engineered protein constructs, referred to herein as "adzymes", as well as methods and compositions related to the use and production of adzymes. In general, adzymes are chimeric protein constructs that join one or more catalytic domains with one or more targeting moieties (or "addresses"). The catalytic domains and the targeting moieties need not be separate entities. In certain embodiments, the targeting moieties / addresses are inserted within the catalytic domains. A catalytic domain of an adzyme has an enzymatically active site that catalyzes a reaction converting a pre-selected substrate (the "target" or "targeted substrate") into one or more products, such as by cleavage, chemical modifications (transformations) or isomerization. Such products may have an altered activity relative to the substrate, optionally having an increased or decreased activity or an activity that is qualitatively different.

The invention is partially based on the unexpected discovery that, when designing adzymes, certain kinetic properties of the final adzyme can be altered to achieve a balance between optimal selectivity and optimal adzyme potency. More specifically, it is determined that as the enzyme or catalytic domain of an adzyme becomes more potent, the overall adzyme quickly loses its selectivity against a panel of different substrates, thus compromising the overall usefulness of the adzyme. On the other hand, if maximal selectivity is to be achieved without regard to potency, the potency can quickly approach that of a stoichiometric binder, e.g., the address domain or targeting moiety, and again compromise the overall usefulness of the adzyme. Therefore, there is a trade-off between the potency and selectivity of an adzyme. The optimal balance is achieved when the catalytic efficiency of the enzyme domain ($k_{cat}^{ES} / K_M^{ES}$) is equal to $k_{off}^{AS} / [S]_{eff}$. Such balance can be most efficiently achieved by adjusting $[S]_{eff}$, such as by adjusting the length of the linker between the catalytic domain and the targeting moiety.

The adzyme of the instant invention comprises the catalytic domain of mesotrypsin, or its functional fragments, derivatives, variants, or stabilizing mutants with longer half life and/or increased potency. Thus the invention provides an adzyme comprising: (a) mesotrypsin catalytic domain that catalyzes the proteolysis of a target substrate; and (b) a targeting moiety that binds to the target substrate. In one embodiment, the mesotrypsin catalytic domain is positioned N-terminal to the address domain. In one embodiment, the mesotrypsin catalytic domain comprises a polypeptide having an amino acid sequence that is at least about 70%, 80%, 85%, 90%, or about 97% or more identical to an amino acid sequence of a human mesotrypsin polypeptide. In one embodiment, the mesotrypsin catalytic domain comprises an amino acid sequence of SEQ ID NO: 45. In one embodiment, the mesotrypsin catalytic domain comprises an amino acid sequence that differs from SEQ ID NO: 45 only in one or more conservative amino acid changes.

An additional preferred adzyme of the instant invention comprises a targeting moiety that is a polypeptide scaffold, preferably an immunoglobulin-like scaffold, that has been engineered to bind to the targeted substrate. Examples of such targeting moieties include those that are based on the fibronectin type III domain.

Thus, in certain aspects, the invention provides adzymes comprising a mesotrypsin catalytic domain and a targeting moiety, wherein the catalytic domain catalyzes a chemical reaction converting a substrate into one or more products, and wherein the targeting moiety reversibly binds to an address site that is either on the substrate or in functional proximity with the substrate. Preferably, the targeting moiety binds reversibly to the address site. Optionally, said targeting moiety and said catalytic domain are heterologous with respect to each other. Generally, said targeting moiety, when provided separately, binds to the substrate, and said catalytic domain, when provided separately, catalyzes the chemical reaction converting said substrate to one or more products.

In certain embodiments, a catalytic domain and a targeting domain of the adzyme are joined by a polypeptide linker to form a fusion protein. A fusion protein may be generated in a variety of ways, including chemical coupling and cotranslation. In a preferred embodiment, the fusion protein is a cotranslational fusion protein encoded by a recombinant nucleic acid. In certain embodiments the linker for the fusion protein is an unstructured peptide. Optionally, the linker includes one or more repeats of Ser$_4$Gly (SEQ ID NO: 41), SerGly$_4$ (SEQ ID NO: 42), Gly$_4$Ser (SEQ ID NO: 43), GlySer$_4$ (SEQ ID NO: 44), or GS. In certain embodiments, the linker selected to provide steric geometry between said catalytic domain and said targeting moiety such that said adzyme is more effective against the substrate than either the catalytic domain or targeting moiety alone. For example, the linker may be selected such that the adzyme is more potent than said catalytic domain or targeting moiety with respect to the reaction with said substrate. The linker may be selected such that the targeting moiety presents the substrate to the enzymatic domain at an effective concentration at least 5 fold greater than would be present in the absence of the targeting moiety. Optionally, the catalytic domain (e.g the mesotrypsin catalytic domains of the invention) may be directly linked to the address / targeting domain (e.g., a target binding immunoglobulin-like protein scaffold) with no intervening linker. In other embodiments, the catalytic domain (e.g. the mesotrypsin catalytic domains of the invention) may be linked to the address / targeting domain (e.g., a target binding immunoglobulin-like protein scaffold) by an unstructured peptide, which peptide may be at least about 10, 20, 30, 40, 50, 60, 70, 80, 90, or about 100 or more residues in length. In one embodiment, the unstructured peptide comprises a plurality of Glycine and Serine amino acids, and has a length of between 15 and 50 amino acids.

In one embodiment, the fusion protein is a cotranslational fusion protein encoded by a recombinant nucleic acid. In one embodiment,

In certain embodiments, the adzyme is an immunoglobulin fusion, wherein the catalytic domain and the targeting moiety are joined, in a geometry consistent with effectiveness against substrate, to at least a portion of an immunoglobulin comprising a constant domain of an immunoglobulin. For example, the adzyme may comprise a first fusion protein and a second fusion protein, wherein the first fusion protein comprises a constant portion of an immunoglobulin heavy chain and a catalytic domain, and wherein the second fusion protein comprises a constant portion of an immunoglobulin heavy chain and a targeting domain that reversibly binds with an address site on or in functional proximity to the substrate. Preferably the immunoglobulin portions are Fc portions that dimerize by disulfide bonds.

In certain embodiments, an adzyme is designed so as to have one or more desirable properties, with respect to the reaction with said substrate. In many instances, such properties will be significant for achieving the desired effect of the adzyme on the substrate. For example, an adzyme may have a potency at least 2 times greater than the potency of catalytic domain or the targeting moiety alone, and preferably at least 3, 5, 10, 20 or more times greater than the potency of the catalytic domain or targeting moiety alone.

In one embodiment, the potency of the adzyme is at least 5 times greater than a mesotrypsin catalytic domain or the targeting moiety alone.

An adzyme may have a $k_{on}$ of $10^3$ M$^{-1}$s$^{-1}$ or greater, and optionally a $k_{on}$ of $10^4$ M$^{-1}$s$^{-1}$, $10^5$ M$^{-1}$s$^{-1}$, $10^6$ M$^{-1}$s$^{-1}$, $10^7$ M$^{-1}$s$^{-1}$ or greater. An adzyme may have a $k_{cat}$ of 0.1 sec$^{-1}$ or greater, and optionally a $k_{cat}$ of 1 sec$^{-1}$, 10 sec$^{-1}$, 50

sec$^{-1}$ or greater. An adzyme may have a K$_D$ that is at least 5, 10, 25, 50 or 100 or more fold less than the $K_M$ of the catalytic domain. An adzyme may have a $k_{off}$ of 10$^{-4}$ sec$^{-1}$ or greater, and optionally a $k_{off}$ of 10$^{-3}$ sec$^{-1}$, 10$^{-2}$ sec$^{-1}$, or greater. An adzyme may have a catalytic efficiency that is at least 5 fold greater than the catalytic efficiency of the catalytic domain alone, and optionally a catalytic efficiency that is at least 10 fold, 20 fold, 50 fold or 100 fold greater than that of the catalytic domain.

In one embodiment, the catalytic efficiency is at least 10 fold greater than that of a mesotrypsin catalytic domain alone.

An adzyme of the invention may have a $K_M$ at least 5 fold, 10 fold, 20 fold, 50 fold, or 100 fold less than the $K_M$ of the catalytic domain alone. An adzyme may have an effective substrate concentration that is at least 5 fold, 10 fold, 20 fold, 50 fold or 100 fold greater than the actual substrate concentration. An adzyme may have an optimal balance between selectivity and potency, such that the $K_{cat}^{ES}$ / $K_M^{ES}$ is equal to $k_{off}^{AS}$ / $[S]_{eff}$, and both equals $k_{on}^{AS}[S]_o$ / $[S]_{eff}$. Preferably, the $k_{cat}^{ES}$ / $K_M^{ES}$ ratio is no more than 10-fold different (more or less), or 5-fold, 3-fold, 2-fold, 100%, 50%, 20%, 5% or 1% different from the ratio of $k_{off}^{AS}$ /$[S]_{eff}$. For example, when $k_{on}$ is 10$^6$ M$^{-1}$s$^{-1}$ and $[S]_0$ is 10$^{-12}$ M (pM), the adzyme has a $k_{off}^{AS}$ of about 10$^{-6}$ s$^{-1}$ ($k_{off}^{AS}$ = $k_{on}$ × $[S]_0$ = 10$^{-6}$ s$^{-1}$), and/or a $k_{cat}^{ES}$ / $K_M^{ES}$ of about 10$^{-3}$ M$^{-1}$ s$^{-1}$. In certain preferred embodiments, an adzyme will be designed so as to combine two or more of the above described properties.

In certain preferred embodiments, the mesotrypsin catalytic domain, when active, cleaves at least one peptide bond of a polypeptide substrate. In general it will be desirable to design the adzyme such that it is at least partially resistant to cleavage by the mesotrypsin catalytic domain. The protease domain may be generated as a zymogen (an inactive form) and then activated prior to use. The adzyme may be purified from a cell culture in the presence of a reversible protease inhibitor, and such inhibitor may be included in any subsequent processing or storage activities.

In the case of adzymes disclosed herein having a mesotrypsin-based catalytic domain, a targeting moiety may include essentially any molecule or assembly of molecules that binds to the address site (e.g., on the substrate in the case of direct adzymes or on a molecule that occurs in functional proximity to the substrate, in the case of proximity adzymes). In many embodiments, a targeting moiety will comprise a polypeptide or polypeptide complex, and particularly an antibody or polypeptide(s) including an antigen binding site of an antibody. For example, a targeting moiety may include a monoclonal antibody, an Fab and F(ab)$_2$, an scFv, a heavy chain variable region and a light chain variable region. Optionally, the targeting moiety is an artificial protein or peptide sequence engineered to bind to the substrate. In certain embodiments, the targeting moiety is a polyanionic or polycationic binding agent. Optionally, the targeting moiety is an oligonucleotide, a polysaccharide or a lectin. In certain embodiments, the substrate is a receptor, and the targeting moiety includes a ligand (or binding portion thereof) that binds to the receptor. In certain embodiments, the substrate is a ligand of a receptor, and the targeting moiety includes a ligand binding portion of the receptor, particularly a soluble ligand binding portion.

An adzyme may be used to target essentially any amenable substrate in a variety of technological applications, including therapeutic uses, industrial uses, environmental uses and uses in microfabrications. In a preferred embodiment, an adzyme substrate is from a mammal, such as a rodent, a non-human primate or a human. In a preferred embodiment, the substrate is endogenous to a human patient. In certain embodiments, the substrate is a biomolecule produced by a cell, such as a polypeptide, a polysaccharide, a nucleic acid, a lipid, or a small molecule. In certain embodiments, the substrate is a diffusible extracellular molecule, and preferably an extracellular signaling molecule that may act on an extracellular or intracellular receptor to triggers receptor-mediated cellular signaling. Optionally, the extracellular signaling molecule is an extracellular polypeptide signaling molecule, such as an inflammatory cytokine. In a preferred embodiment, the substrate is an interleukin-1 (e.g., IL-1α, IL-1β) or TNF-α. In certain embodiments, the substrate is a polypeptide hormone, a growth factor and/or a cytokine, especially an inflammatory cytokine. Optionally, the adzyme acts to reduce a pro-inflammatory activity of a substrate. A substrate may be selected from among the following: four-helix bundle factors, EGF-like factors, insulin-like factors, β-trefoil factors and cysteine knot factors. In certain embodiments, the substrate is a receptor, particularly a receptor with some portion exposed to the extracellular surface. Optionally, the substrate is a unique receptor subunit of a heteromeric receptor complex. In certain embodiments, the substrate is a biomolecules that is a component of a biomolecular accretion, such as an amyloid deposit or an atherosclerotic plaque. In certain embodiments, the substrate is an intracellular biomolecule, and in such instances, it may be desirable to use an adzyme that is able to enter the targeted cells, such as an adzyme that further comprises a transcytosis moiety that promotes transcytosis of the adzyme into the cell. In certain embodiments, the substrate is a biomolecule produced by a pathogen, such as a protozoan, a fungus, a bacterium or a virus. The substrate may be a prion protein. In a preferred embodiment, the substrate is endogenous to a human patient. In such an embodiment, the adzyme is preferably effective against the substrate in the presence of physiological levels of an abundant human serum protein, such as, serum albumins or an abundant globin.

In one aspect, the invention provides an adzyme for enzymatically altering a substrate, the adzyme comprising: a mesotrypsin catalytic domain that catalyzes a chemical reaction converting said substrate to one or more products, and a targeting moiety that reversibly binds with an address site on said substrate or with an address site on a second molecule that occurs in functional proximity to the substrate, wherein said targeting moiety and said catalytic domain are heterologous with respect to each other, said targeting moiety, when provided separately, binds to the substrate, said mesotrypsin catalytic domain, when provided separately, catalyzes the chemical reaction converting said substrate to one or more products, and said adzyme has one or more desirable properties, with respect to the reaction with said substrate.

For example, in this aspect, the adzyme may have a potency at least 2 times greater than the mesotrypsin catalytic domain or the targeting moiety alone, and preferably at least 3, 5, 10, 20 or more times greater than the potency of the mesotrypsin catalytic domain or targeting moiety alone. The adzyme may have a $k_{on}$ of $10^3$ M$^{-1}$s$^{-1}$ or greater, and optionally a $k_{on}$ of $10^4$ M$^{-1}$s$^{-1}$, $10^5$ M$^{-1}$s$^{-1}$, $10^6$ M$^{-1}$s$^{-1}$, $10^7$ M$^{-1}$s$^{-1}$ or greater. The adzyme may have a $k_{cat}$ of 0.1 sec$^{-1}$ or greater, and optionally a $k_{cat}$ of 1 sec$^{-1}$, 10 sec$^{-1}$, 50 sec$^{-1}$ or greater. The adzyme may have a $K_D$ that is at least 5, 10, 25, 50 or 100 or more fold less than the $K_M$ of the catalytic domain. The adzyme may have a $k_{off}$ of $10^{-4}$ sec$^{-1}$ or greater, and optionally a $k_{off}$ of $10^{-3}$ sec$^{-1}$, $k_{off}$ of $10^{-2}$ sec$^{-1}$, or greater. The adzyme may have a catalytic efficiency that is at least 5 fold greater than the catalytic efficiency of the mesotrypsin catalytic domain alone, and optionally a catalytic efficiency that is at least 10 fold, 20 fold, 50 fold or 100 fold greater than that of the catalytic domain. The adzyme may have a $K_M$ at least 5 fold, 10 fold, 20 fold, 50 fold, or 100 fold less than the $K_M$ of the mesotrypsin catalytic domain alone. The adzyme may have an effective substrate concentration that is at least 5 fold, 10 fold, 20 fold, 50 fold or 100 fold greater than the actual substrate concentration. An adzyme may have an optimal balance between selectivity and potency, such that the $k_{cat}^{ES} / K_M^{ES}$ is equal to $k_{off}^{AS} / [S]_{eff}$, and both equals $k_{on}^{AS}[S]_o / [S]_{eff}$. Preferably; the $k_{cat}^{ES} / K_M^{ES}$ ratio is no more than 10-fold different (more or less), or 5-fold, 3-fold, 2-fold, 100%, 50%, 20%, 5% or 1% different from the ratio of $k_{off}^{AS} / [S]_{eff}$. For example, when $k_{on}$ is $10^6$ M$^{-1}$s$^{-1}$ and $[S]_0$ is $10^{-12}$ M (pM), the adzyme has a $k_{off}^{AS}$ of about $10^{-6}$ s$^{-1}$ ($k_{off}^{AS} = k_{on} \times [S]_0 = 10^{-6}$ s$^{-1}$), and/or a $k_{cat}^{ES} / K_M^{ES}$ of about $10^{-3}$ M$^{-1}$ s$^{-1}$. In certain preferred embodiments, the adzyme will be designed so as to combine two or more of the above described properties.

In certain embodiments of an adzyme having one or more of such properties, the adzyme is an immunoglobulin fusion, wherein the mesotrypsin catalytic domain and the targeting moiety are joined, in a geometry consistent with effectiveness against substrate, to at least a portion of an immunoglobulin comprising a constant domain of an immunoglobulin. For example, the adzyme may comprise a first fusion protein and a second fusion protein, wherein the first fusion protein comprises a constant portion of an immunoglobulin heavy chain and a catalytic domain, and wherein the second fusion protein comprises a constant portion of an immunoglobulin heavy chain and a targeting domain that reversibly binds with an address site on or in functional proximity to the substrate. Preferably the immunoglobulin portions are Fc portions that dimerize by disulfide bonds.

In certain embodiments of an adzyme having one or more of the properties described above with respect to the reaction with the substrate, the substrate is a biomolecule produced by a cell, such as a polypeptide, a polysaccharide, a nucleic acid, a lipid, or a small molecule. In certain embodiments, the substrate is a diffusible extracellular molecule, and preferably an extracellular signaling molecule that may act on an extracellular or intracellular receptor to triggers receptor-mediated cellular signaling. Optionally, the extracellular signaling molecule is an extracellular polypeptide signaling molecule, such as an inflammatory cytokine. In a preferred embodiment, the substrate is an interleukin-1 (e.g., IL-1$\alpha$, IL-1$\beta$) or a TNF- $\alpha$. In certain embodiments, the substrate is a polypeptide hormone, a growth factor and/or a cytokine, especially an inflammatory cytokine. Optionally, the adzyme acts to reduces a pro-inflammatory activity of a substrate. A substrate may be selected from is selected from the group consisting of four-helix bundle factors, EGF-like factors, insulin-like factors, $\beta$-trefoil factors and cysteine knot factors. In certain embodiments, the substrate is a receptor, particularly a receptor with some portion exposed to the extracellular surface. Optionally, the substrate is a unique receptor subunit of a heteromeric receptor complex. In certain embodiments, the biomolecule is a component of a biomolecular accretion, such as an amyloid deposit or an atherosclerotic plaque. In certain embodiments, the substrate is an intracellular biomolecule, and in such instances, it may be desirable to use an adzyme that is able to enter the targeted cells, such as an adzyme that further comprises a transcytosis moiety that promotes transcytosis of the adzyme into the cell. In certain embodiments, the substrate is a biomolecule produced by a pathogen, such as a protozoan, a fungus, a bacterium or a virus. The substrate may be a prion protein. In a preferred embodiment, the substrate is endogenous to a human patient. In such an embodiment, the adzyme is preferably effective against the substrate in the presence of physiological levels of an abundant human serum protein, such as, serum albumins or an abundant globin.

In certain aspects, the invention provides adzyme preparations for use in a desired application, such as a therapeutic use, an industrial use, an environmental use or in a microfabrication. Such preparations may be termed adzyme preparations. In certain embodiments, the invention provides an adzyme preparation for therapeutic use in a human patient, the preparation comprising any adzyme disclosed herein. Optionally, the preparation further comprising a pharmaceutically effective carrier. Optionally, the adzyme preparation is formulated such that autocatalytic modification of the adzyme is inhibited. As the adzyme comprises a catalytic domain that is a protease, in certain embodiments, the preparation comprises a reversible inhibitor of said protease, preferably a reversible inhibitor that is safe for administration to a human patient. Optionally, an adzyme preparation for therapeutic use is substantially pyrogen free. An adzyme preparation may be packaged along with instructions for use. For example, an adzyme preparation for therapeutic use may be packaged with instructions for administration to a patient.

Thus one aspect of the invention provides a pharmaceutical preparation comprising the mesotrypsin-based adzyme of the instant invention, and a pharmaceutically effective carrier. In one embodiment, the pharmaceutical preparation is formulated such that autocatalytic proteolysis of the adzyme is inhibited. In one embodiment, the pharmaceutical preparation further comprises a reversible inhibitor of the mesotrypsin protease domain. In one embodiment, the reversible inhibitor is safe for administration to a human patient.

In certain aspects, the invention provides methods for making a medicament for use in treating a disorder that is associated with an activity of the substrate of any adzyme disclosed herein, the method comprising formulating the adzyme for administration to a patient, preferably a human patient. In certain embodiments, the invention provides a method of making a medicament for use in treating an inflammatory or allergic disorder, the method comprising formulating an adzyme for administration to a human patient in need thereof, wherein the substrate of the adzyme is an inflammatory cytokine.

In certain aspects, the invention relates to the adzyme for use in methods of treating a disorder that is associated with an activity of the substrate of an adzyme, the method comprising administering a therapeutically effective dose of an adzyme to a human patient in need thereof. In certain embodiments, an adzyme may be for use in a method of treating an inflammatory of allergic disorder, the method comprising administering a therapeutically effective dose of an adzyme to a human patient in need thereof, wherein the substrate of the adzyme is an inflammatory cytokine.

In certain aspects, the invention provides an adzyme for inhibiting the activity of a substrate polypeptide, the adzyme being an immunoglobulin fusion complex comprising: a first fusion protein bound to a second fusion protein, wherein the first fusion protein comprises a constant portion of an immunoglobulin heavy chain and a mesotrypsin catalytic domain that catalyzes the proteolytic cleavage of at least one peptide bond of the substrate polypeptide, and wherein the second fusion protein comprises a constant portion of an immunoglobulin heavy chain and a targeting domain that binds with an address site on said substrate polypeptide, wherein said targeting domain and said protease domain are discrete and heterologous with respect to each other.

In certain aspects, the invention provides a stabilized mesotrypsin comprising one or more amino acid sequence changes at potential autoproteolytic sites, wherein said changes do not substantially diminish the catalytic activity of said mesotrypsin. In one embodiment, the potential autoproteolytic sites comprise Arg or Lys. In one embodiment, the potential autoproteolytic sites comprise one or more of: K98, R122, K159 and K193. In one embodiment, the changes comprise one or more of: K98Q, R122H, K159Q and K193A. In one embodiment, the apparent activity of the stabilized mesotrypsin is at least about 3-fold higher than wild-type mesotrypsin. In one embodiment, the stabilized mesotrypsin retains substantially the same activity over a period of at least about 2 days, preferably about 3, 4, 5, 6, 10 or more days. In certain aspects, the invention provides a vector comprising a polynucleotide encodingany of the subject stabilized mesotrypsins. In certain aspects, the invention provides a host cell comprising any of the stabilized mesotrypsin, any of the polynucleotide encoding the stabilized mesotrypsin, or any vector comprising a polynucleotide encodingany of the subject stabilized mesotrypsins. Another aspect of the invention provides any adzyme comprising a catalytic domain derived from the stabilized mesotrypsin of the invention.

In certain aspects, the invention provides nucleic acids encoding any of the various polypeptide portions of the adzyme described herein (include the mesotrypsin catalytic domains, the linkers if present, and/or the address domains), and particularly recombinant nucleic acids encoding a fusion protein adzyme. Such nucleic acids may be incorporated into an expression vector wherein the expression vector directs expression of the adzyme in a suitable host cell. The invention further provides cells comprising such nucleic acids and vectors. In certain embodiments, the invention provides cells comprising a first nucleic acid comprising a first coding sequence and a second nucleic acid comprising a second coding sequence, wherein the first coding sequence encodes a first fusion protein comprising an immunoglobulin heavy chain and a catalytic domain, and wherein the second coding sequence encodes a second fusion protein comprising an immunoglobulin heavy chain and a targeting domain. Preferably, such as cell, in appropriate culture conditions, secretes an adzyme comprising an Fc fusion protein construct that is a dimer of the first fusion protein and the second fusion protein.

In certain aspects, the invention provides methods for manufacturing a subject mesotrypsin (including the stabilizing mutant) or adzyme. Such methods may include expression of polypeptide components in cells. Such methods may include chemical joining of various adzyme components or various mesotrypsins. In one embodiment, a method comprises culturing a cell having an expression vector for producing a fusion protein adzyme in conditions that cause the cell to produce the adzyme encoded by the expression vector; and purifying the adzyme to substantial purity. In one embodiments, a method comprises culturing a cell designed to produce an immunoglobulin fusion (or mesotrypsins) in conditions that cause the cell to produce the adzyme (or the mesotrypsins) encoded by the expression vector; and purifying the adzyme or mesotrypsin to substantial purity. In certain embodiments, purifying a polypeptide to substantial purity includes the use of a reversible inhibitor that inhibits autocatalytic activity of the catalytic domain, and particularly, wherein the catalytic domain of the adzyme is a protease domain, and wherein purifying the polypeptide to substantial purity includes the use of a reversible protease inhibitor that inhibits the protease activity of the catalytic domain.

In certain aspects, the subject adzyme can be designed to modify a target so as to increase its immunogenicity, resulting in an immune response, either cellular or humoral or both, directed at epitopes that also exist in the native proteins. In this way, an adzyme can be used to break tolerance with a "self" antigen, such as an antigen expressed on a tumor cell or a growth factor inhibitor. In other instances, the adzyme can be used to enhance the immunogenicity of a foreign antigen, such as that of a pathogen (bacteria, virus, parasite, etc.).

In further aspects, the invention provides methods for designing and producing adzymes with desirable properties, and methods for operating a business that involves designing and selling adzymes with desirable properties, such as therapeutically effective adzymes.

The embodiments and practices of the present invention, other embodiments, and their features and characteristics, will be apparent from the description, figures and claims that follow. In addition, all embodiments of the invention, whether described under the same or different aspects of the invention, can be combined with one another whenever appropriate.

## Brief Description of the Drawings

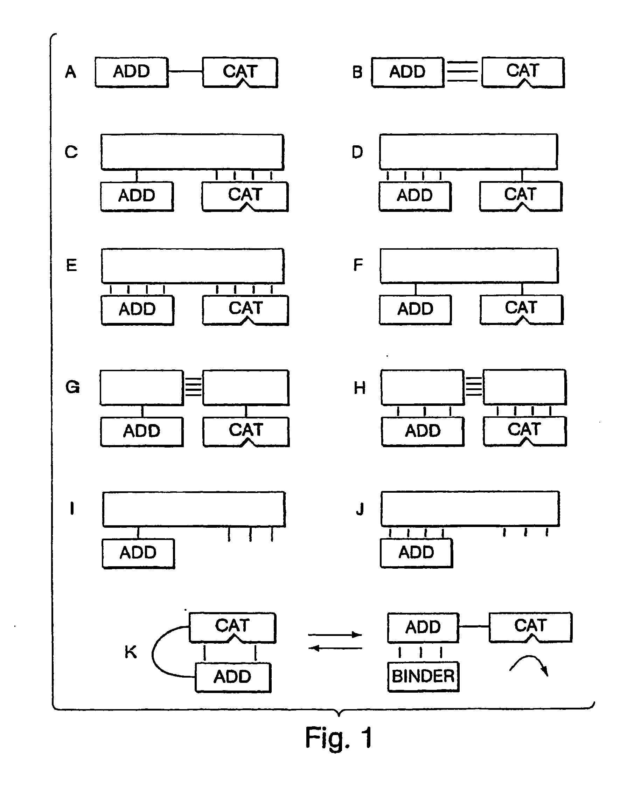

*Figures 1A-1J* are schematic representations of the structure of a series of different exemplary constructs embodying the invention. The boxes represent moieties having binding or catalytic properties, and can be embodied as true protein domains, *i.e.*, bonded sequences of amino acids forming structures characterized by folding of the peptide chain into alpha helices, beta pleated sheets, random coils, etc., to form separate binding surfaces or enzymatically active sites, and including catalytic moieties (CAT), address moieties (ADD), and protein domains serving to associate these parts together in various operative configurations. Lines connecting boxes represent a covalent bond linking together amino acid sequence defining the respective functional regions, or linkers comprising, for example, a flexible linear linker such as a string of peptide bonded amino acids or a poly(ethylene glycol) chain. Lines between boxes represent non covalent, reversible attachments wherein the parts are held together by a combination of forces such as hydrogen bonding, hydrophobic-hydrophobic interaction, opposite charge matching, etc., for example, ligand-receptor interactions.

*Figure 1K* is a schematic diagram illustrating the basic concept of a contingent adzyme.

*Figures 2A-2J* are cartoons illustrating various exemplary embodiments of adzyme constructs at various types of targeted biomolecules in position to initiate an enzymatic reaction on the substrate site of the target. The address is designated as AD, the catalytic domain as CD.

*Figures 3A-3G* are cartoons illustrating various exemplary embodiments of contingent adzyme constructs in the absence of and in the vicinity of their respective intended targeted biomolecules.

*Figure 4* is a cartoon illustrating components of a pre thrombin scFvαHA adzyme.

*Figure 5* is electrophoretic analysis of purified model adzyme.

*Figure 6* is Western blot analysis of model adzyme activated using Factor Xa.

*Figure 7* shows proteolytic activity of thrombin and model adzyme before and after activation on standard thrombin tripeptide substrate.

*Figure 8* shows that enhanced adzyme activity is driven by the presence of an address domain.

*Figure 9* shows that enhanced adzyme activity requires cotranslational linkage of the domains.

*Figure 10* shows proteolytic inactivation of TNFα cytotoxicity.

*Figure 11* shows that soluble TNFα receptor p55 address domain binds TNFα.

*Figure 12* is a representative expression of several adzyme constrcuts as analyzed by Western blotting with anti-myc antibody. Lane 1: trypsinogen expressed in the absence of stabilizing benzamidine, Lane 2: trypsinogen, Lane 3: trypsinogen-0aa-sp55, Lane 4: trypsinogen-20aa-sp55; Lane 5: trypsinogen-3aa-sp55, Lane 6: sp55. Material in lanes 2 through 6 was expressed in the presence of 1 mM benzamidine.

*Figure 13* shows a snapshot of representative experiments where the fluorescence detected at the end of 2 hours of incubation is compared for the different recombinant adzymes and other control proteins.

*Figure 14* shows normalization of trypsin activities.

*Figure 15* shows detection of TNFα binding of adzymes by ELISA.

*Figure 16* shows kinetic model results comparing the performance of an adzyme, an address, and an enzyme.

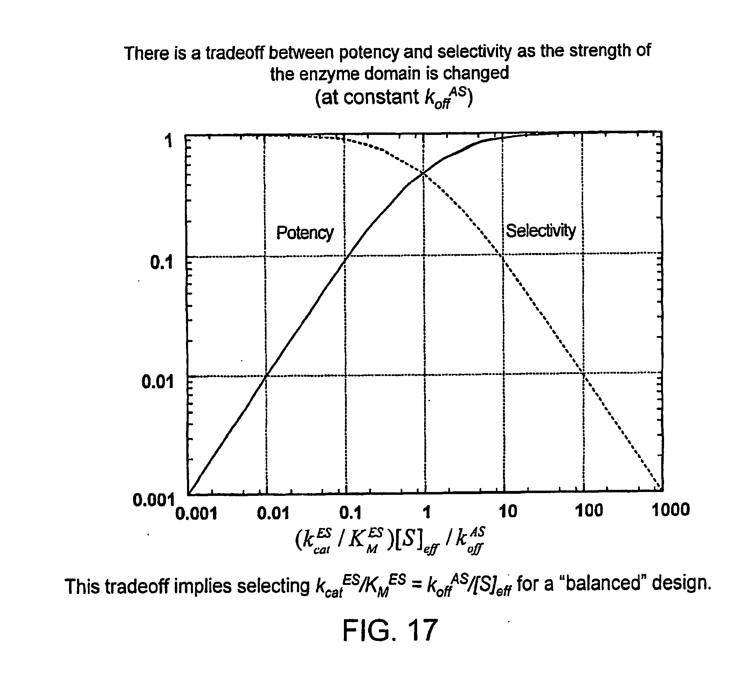

*Figure 17* shows kinetic model results indicating that there is a trade-off between potency and selectivity when the strength of the enzyme domain is changed.

*Figure* 18 shows that a molar excess of mesotrypsin is needed to inactivate TNF in the L929 bioassay.

*Figure 19* shows largely equivalent proteolytic activities of enzyme and adzyme towards the synthetic peptide t-GPR-AMC, which fits into the active site of the protease.

*Figure 20* demonstrates that adzyme is more selective than enzyme.

*Figure 21* demonstrates that adzyme is more potent than the stoichiometric binder.

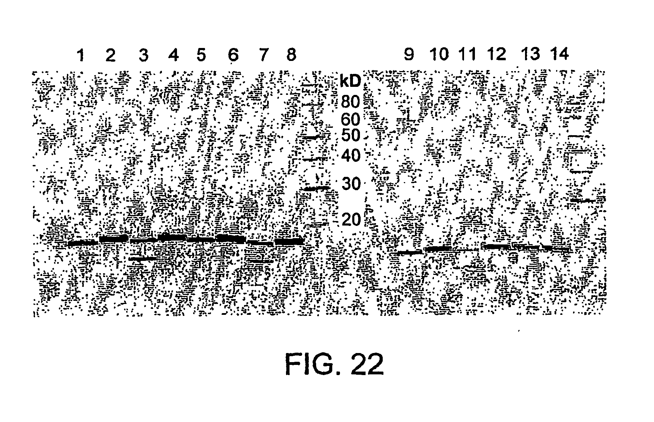

*Figure 22* shows cleavage of TNF by different concentrations of adzymes, but not appreciatably by the corresponding enzyme mesotrypsin. 15 μL of overnight digestion reactions were electrophoresed under denaturing non-reducing conditions on a 10-20% Tris glycine SDS gel, transferred to nitrocellulose, and then blotted with anti-TNF antibody (Abcam 9348 ) at 1:1000. Lane 1: Mesotrypsin 86 nM + 100 nM TNF; Lane 2: Mesotrypsinogen 86 nM + 100 nM TNF; Lane 3: Mesotypsin_35aa _p55_2.6 86 nM + 100 nM TNF; Lane 4: Mesotrypsinogen_35aa_p55_2.6 86 nM + 100 nM TNF; Lane 5: Mesotrypsin 43 nM + 100 nM TNF; Lane 6: Mesotrypsinogen 43 nM + 100 nM TNF; Lane 7: Mesotrypsin_35aa_p55_2.6 43 nM + 100 nM TNF; Lane 8: Mesotrypsinogen_35aa_p55_2.6 43 nM + 100 nM TNF; Lane 9: Mesotrypsin 22 nM + 100 nM TNF; Lane 10: Mesotrypsinogen 22 nM + 100 nM TNF; Lane 11: Mesotrypsin_35aa_p55_2.6 22 nM + 100 nM TNF; Lane 12: Mesotrypsinogen_35aa_p55_2.6 22 nM + 100 nM TNF; Lane 13: 100 nM TNF; Lane 14: 100 nM TNF + EK.

*Figure 23* Panels (a) and (b) shows the bioactivity of TNF after overnight digestions, as assayed using the viability of L929 cells.

*Figure 24* illustrates the requirement of activation for meso_Sc7 to demonstrate adzyme behavior, i.e. inactivation of TNF at the substoichiometric ratio of 1:10. The loss of bioactive TNF observed in Figure 23 cannot arise from neutralization of TNF by the address domain alone since neither unactivated adzyme or sc7 have any effect on TNF.

*Figure 25* shows the effect of linker length (0 - 60 amino acids) on adzyme activity.

*Figure 26* shows the coding sequence of mesotrypsin with the stabilizing mutations.

*Figure 27* demonstrates that the stable adzyme with the stabilizing mutations (solid line) exhibits significantly superior properties as shown by the greater than 2 log inactivation of TNF.

## Detailed Description of the Invention

### I. Overview

The invention provides a new class of engineered protein constructs, referred to herein as "adzymes", as well as methods and compositions related to the use and production of adzymes. Adzymes are chimeric protein constructs that join one or more catalytic domains with one or more targeting moieties (or "addresses"). The catalytic domains and the targeting moieties need not be separate entities. In certain embodiments, the targeting moieties /addresses are inserted within the catalytic domains. A catalytic domain of an adzyme has an enzymatically active site that catalyzes a reaction converting a pre-selected substrate (the "target" or "targeted substrate") into one or more products, such as by cleavage, chemical modifications (transformations) or isomerization. Generally, the catalytic domain is selected such that one or more of the product(s) of the adzyme-mediated reaction have a qualitatively or quantitatively different activity relative to the selected substrate. Merely to illustrate, the adzyme may alter such functional characteristics of a selected substrate as affinity, potency, selectivity, solubility, immunogenicity, half-life, clearance (such as by renal or hepatic function), biodistribution or other pharmacokinetic properties. In certain instances, the product of an adzyme-mediated reaction is itself an antagonist of an activity of the selected substrate.

According to the invention, the catalytic domain is that of a mesotrypsin of the invention, including its functional fragments, derivatives, variants, homologs, and stabilizing mutants thereof.

In a preferred embodiment, the target moiety is a polypeptide scaffold protein, preferably one having an immunoglobulin-like fold, that binds to the targeted substrate. An example of a suitable scaffold is the FN3 domain (particularly the 10th FN3 domain of fibronectin) which has an immunoglobulin-like fold that presents at least three loop portions that may be combinatorially modified to generate a diverse spectrum of target binding activities. Although the mesotrypsin catalytic domain and the polypeptide scaffold targeting moiety are independent embodiments, adzymes having a mesotrypsin catalytic domain and a polypeptide scaffold targeting moiety are contemplated.

The targeting moiety (or "address") is a moiety capable of recognizing and reversibly binding to a pre-determined "address binding sits" (also herein "address site"), such as, for example, a soluble or membrane-bound biomolecules, or a component of a biomolecular accretion (*e.g.*, a plaque or other insoluble protein-containing aggregate). In certain types of adzymes (termed "direct adzymes"), the targeting moiety binds to the target molecule. In certain types of adzymes (termed "proximity adzymes") the targeting moiety binds to a molecule that tends to occur in functional proximity to the target. The term "moiety" should be understood as including single molecules or portions thereof (*e.g.*, a polypeptide or sugar that binds to the address binding site), as well as combinations of molecules (*e.g.*, an antibody that binds to an address binding site).

In an adzyme, at least one targeting moiety is operatively associated with at least one catalytic domain. An adzyme may be a single polypeptide chain (*e.g.*, a fusion protein) or an assembly of polypeptide chains and/or other molecules that are joined through covalent or non-covalent bonds. Regardless of how the constituent portions of an adzyme are associated, at least one targeting moiety and one catalytic domain should be operatively associated. The term "operatively associated", as used herein to describe the relationship between a catalytic domain and a targeting moiety, means that the effectiveness of the associated catalytic domain and targeting moiety in chemically altering or otherwise affecting the activity of the pre-selected substrate is greater than the effectiveness of either the targeting moiety or the catalytic domain alone, and also greater than the effectiveness of both the targeting moiety and the catalytic domain when provided in combination but not in association with each other (*e.g.*, where the target is simultaneously contacted with both a discrete catalytic domain and a discrete targeting moiety). As described below, the adzyme may include other components as well, such as linkers, moieties that influence stability or biodistribution, and the like.

In certain embodiments, adzymes may contain separate catalytic domain(s) and address domain(s) connected by linkers, or otherwise operatively associated by other means (see below). Preferably, the catalytic domain and the address domain are heterologous proteins not naturally associated with each other.

In certain other embodiments, adzymes may be constructed in which the address domain(s) is inserted within the catalytic domain of an enzyme.

Novel adzymes based upon the mesotrypsin catalytic domain but directed towards other targets, particularly heterologous targets, may be constructed using recombinant DNA methods by linking various target-specific address domains to the mesotrypsin catalytic domain. More specifically, using any of many art-recognized sequence alignment programs, such as DNAStar's MegaAlign, multiple mesotrypsin homologs can be aligned. The alignment may be used to design stabilizing mutants (see below).

Address domains suitable for Adzymes of this form may be constrained and non-constrained peptides, scFvs, Fabs, soluble receptors, soluble cytokines and growth factors, and other protein scaffolds that have been pre-selected for their ability to bind to the target of interest. Insertion of address domains into the catalytic domain may be further facilitated by including polypeptide linkers (e.g., (GGGGS)n, (GS)n) at the N- and/or C-terminus of the address domain, ensuring that the address domains could fold correctly and are optimally disposed for engagement of the targets.

The effectiveness of an adzyme relative to its constituent parts may be assessed in a variety of ways. For example, effectiveness may be assessed in terms of potency of the adzyme, as compared to its component parts, to affect a biological activity of the pre-selected substrate. As another example, effectiveness may be assessed in terms of a comparison of kinetic or equilibrium constants that describe the reaction between the adzyme and the pre-selected substrate to those that apply to the reaction between the component parts and the targeted substrate. In embodiments where an adzyme is intended for use in a mammal, at least one catalytic domain and at least one targeting moiety of an adzyme will be associated such that these portions are operatively associated under physiological conditions (e.g., in whole blood, serum, cell culture conditions, or phosphate buffered saline solution, pH 7). Where the adzyme is intended for other purposes (e.g., the modification of an environmental pollutant or the modification of a component of a molecular reaction), at least one catalytic domain and at least one targeting moiety of an adzyme will be associated such that these portions are operatively associated under the expected or desired reaction conditions.

Merely to illustrate, an adzyme may comprise a catalytic domain that cleaves or otherwise modifies TNF-$\alpha$, converting it into one or more products having reduced activity, no activity or antagonist activity, thereby ameliorating a disease state associated with TNF-$\alpha$, such as rheumatoid arthritis or other conditions associated with TNF-$\alpha$ activity.

While not wishing to be bound to any particular mechanism of action, it is expected that a targeting moiety will bind to the pre-selected targeted substrate (direct adzyme) or to another molecule that occurs in the same vicinity as the pre-selected targeted substrate (proximity adzyme), and thereby functions to increase the concentration of the catalytic domain at or near the targeted substrate. In this way, the adzyme is self-concentrating at or in the vicinity of a targeted substrate and has an enhanced effectiveness for reacting with and altering the activity of the targeted substrate, relative to the catalytic or binding domains alone. As a consequence to the improved effectiveness of the targeted reaction, the adzyme has a greater selectivity and/or catalytic efficiency for the targeted substrate as compared to other non-targeted (potential) substrates of the catalytic domain.

Again, while not wishing to be bound to any particular theory, for certain adzymes it is expected that a relatively fast $k_{on}$ rate for the targeted substrate will be desirable. In one embodiment, such high $k_{on}$ rate is particularly beneficial for improving potency of the adzyme. A $k_{on}$ of at least $10^3$ M$^{-1}$s$^{-1}$, preferably $10^6$ M$^{-1}$s$^{-1}$ M$^{-1}$s$^{-1}$, may be desirable. Other kinetic and performace parameters that may be useful in certain embodiments are described below.

Further, while not wishing to be bound to any particular theory, for certain adzymes, it is expected that adzymes are particularly advantageous at somewhat higher target concentrations.

In most embodiments, the modular components of an adzyme are heterologous with respect to each other, meaning that these domains are not found naturally as part of a single molecule or assembly of molecules, and accordingly, adzymes of these embodiments are not naturally occurring substances. Each of the various domains and moieties that are present in an adzyme may themselves be a naturally occurring protein or protein fragment, or other naturally occurring biomolecule (*e.g.*, a sugar, lipid or non-proteinaceous factor), or an engineered or wholly synthetic molecule.

In most embodiments, a catalytic domain will comprise a polypeptide having enzymatic activity. In certain preferred embodiments, a targeting moiety will comprise a polypeptide. In general, at least one catalytic domain and at least one targeting moiety of the adzyme are selected from amongst "modular" entities, i.e., able to function as a catalyst or binding agent independently. To exemplify, an adzyme may be a single fusion protein comprising (1) a catalytic domain that comprises a polypeptide and has enzymatic activity and (2) an targeting domain that comprises a polypeptide and binds to an address binding site, and, optionally, (3) a polypeptide linker configured such that the catalytic domain and targeting domain are operatively associated. As another example, an adzyme may be a type of immunoglobulin fusion construct, wherein a first fusion protein comprises a catalytic domain fused to a first Fc chain and a second fusion protein comprises a targeting domain fused to a second Fc chain, and wherein the first and second Fc chains are associated in such a way as to cause the catalytic domain and the targeting domain to be operatively associated.

Within the broad category of adzymes, various subcategories or classes of adzymes may be identified. As noted above, two such classes are termed herein "direct" adzymes and "proximity" adzymes. In a direct adzyme the targeting moiety binds to a targeted substrate. The catalytic domain acts on the same type of molecule as the targeting moiety has bound. In certain embodiments, this will require the targeting moiety to dissociate from the targeted substrate in order for the catalytic domain to alter that molecule. Depending on a variety of conditions, such as the concentration of the direct adzyme and the concentration of the targeted substrate, the catalytic domain of a direct adzyme may primarily act on the targeted substrate that is or was bound by the targeting moiety, or the direct adzyme may act on one substrate while the targeting moiety is bound to another. While not wishing to be bound to mechanism, it is generally expected that when the targeted substrate is present in relatively low concentrations (as is the case for most extracellular signaling molecules in the extracellular fluids of a multicellular organism), a direct adzyme will primarily act on the targeted substrate that is or was bound by the targeting moiety. In a proximity adzyme, the targeting moiety binds to a molecule that is not covalently part of the targeted substrate. Instead, the targeting moiety binds to a molecule that is expected to be found in functional proximity to the targeted substrate. By "functional proximity" is meant that the address binding site is present at sufficient concentration or with sufficient stability in the proximity of targeted substrates that the adzyme reacts with the targeted substrate with greater effectiveness than the catalytic domain and targeting moiety alone or in non-associated combination. While the existence of functional proximity between an address binding site and a targeted substrate is most accurately assessed in the milieu in which the adzyme is intended for use (*e.g.*, in the human body, in a contaminated soil site), an adzyme may be considered a proximity adzyme if it shows the appropriate effectiveness in a reasonable experimental system, such as a culture of cells related to the type of cells that are predicted to be targeted by the adzyme, or in a purified protein mixture where the address binding site and the adzyme are present at concentrations that fairly approximate those that are expected in the intended milieu. In certain embodiments, the targeting moiety binds to a molecule which is diffusionally constrained with respect to the targeted substrate, meaning that, for whatever reason, the targeted substrate and the address binding site are neither covalently attached nor free to diffuse apart. For example, the targeting moiety may bind one protein in a receptor complex while the catalytic domain acts on another protein in the receptor complex. As another example, the targeting moiety may bind to a protein that is lodged in cell membranes and the targeted substrate may also be lodged in or attached to cell membranes. The terms "direct adzyme" and "indirect adzyme", while distinct concepts that raise different issues in adzyme design, may not, in practice, be entirely mutually exclusive. For example, an targeting moiety may bind to both the targeted substrate and a separate molecule that occurs in functional proximity to the targeted substrate.

An additional discernible class of adzymes are the "contingent adzymes". The term "contingent adzymes" refers to adzyme constructs that are catalytically activated or upregulated in the vicinity of the targeted substrate but less active, such as by inhibition, elsewhere. Both direct and proximity adzymes can be modified to be contingent adzymes, in which the interaction of the targeting domain with its cognate partner alters the activity of the catalytic domain, such as by allosteric, competitive, or non-competitive mechanisms.

As a descriptive example, a variety of antibodies with affinity for particular targets (e.g., anti-TNF-a and anti-EGF receptor) have been used as effective therapeutic agents for certain disorders, and it is expected, in accordance with the teachings herein, that adzymes with greater potency than the antibodies alone may be designed.

In a further aspect, the present invention provides pharmaceutical compositions comprising an adzyme of the invention and a pharmaceutically acceptable carrier, as well as methods for making a medicament for use in a human by combining an adzyme with a pharmaceutically acceptable carrier.

In another aspect, the present invention provides an adzyme of the invention for use in a method for treating a subject, e.g., a human, suffering from a disease. The method includes administering (e.g., using a pharmaceutical formulation) a therapeutically, prophylactically or analgesically effective amount of an adzyme, thereby treating a subject suffering from a disease. In one embodiment, the disease is associated with a soluble molecule and the adzyme is administered to the subject in an amount effective to render the soluble molecule biologically inactive.

II. Definitions

For convenience, certain terms employed in the specification, examples, and appended claims are collected here. Unless defined otherwise, all technical and scientific terms used herein have the same meaning as commonly understood by one of ordinary skill in the art to which this invention belongs.

As used herein, the term "aptamer", when referring to a targeting moiety, encompasses an oligonucleotide that interacts with a targeted substrate or associated molecule, e.g., binds to the address site for an adzyme.

As used herein, the term "biologically inactive" as it relates to a targeted biomolecule is intended to mean that its biological function is down-regulated, e.g., suppressed or eliminated. For example, if the target is TNF$\alpha$, biological inactivation would include modifying TNF$\alpha$ such that the inflammatory response mediated by NFKB is inhibited, there is inhibition of the secretion of other pro-inflammatory cytokines, the induction of endothelial procoagulant activity of the TNF is inhibited; the binding of TNF to receptors on endothelial cells is inhibited; the induction of fibrin deposition in the tumor and tumor regression activities of the TNF are enhanced; and/or the cytotoxicity and receptor binding activities of the TNF are unaffected or enhanced on tumor cells. For example, a catalytic domain capable of methylating TNF$\alpha$ (e.g., methylating TNF$\alpha$ on [15]His as described in Yamamoto R. et al. (1989) Protein Engineering 2(7):553-8) would inactivate TNF$\alpha$.

The term "$k_{cat}$", or the "turnover number", is the number of substrates converted to product per enzyme molecule per unit of time, when E is saturated with substrate.

The term "$k_{cat}/K_M$", is an apparent second-order rate constant that is a measure of how the enzyme performs when the concentration of substrate is low (e.g., not saturating). The upper limit for $k_{cat}/K_M$ is the diffusion limit - i. e., the rate at which enzyme and substrate diffuse together. $k_{cat}/K_M$ is also known as the "catalytic efficiency" for the enzyme.

The term "catalytic efficiency", as applied to an adzyme, is the apparent second-order rate constant of the adzyme when the concentration of substrate is substantially (at least tenfold) lower than the Michaelis-Menten constant ($K_M$) for the adzyme (i.e., when $[S] << K_M$), at least with respect to those adzymes that can be reasonably modeled using Michaelis-Menten kinetic modeling theories. In the case of many simple catalytic domains taken in isolation, the catalytic efficiency may be defined as the ratio $k_{cat}/K_M$ (see above).

In most cases where Michaelis-Menten modeling applies, the catalytic efficiency will be different for the adzyme and for its component enzyme, i.e. the adzyme's catalytic efficiency is not $k_{cat}/K_M$. Both $v_{max}$ and $K_M$ are also different for the adzyme. For a case where the Michaelis-Menten pseudo-steady state analysis is valid (generally $[AE]_o << [S]_o$, wherein $[AE]_o$ is the initial adzyme concentration, $[S]_o$ is the initial substrate concentration) and substrate holdup is negligible, simple closed-form expressions for these quantities can be derived:

$$v_{max}^{AE} = \frac{k_{off}^{AS}}{k_{cat}^{ES} + k_{off}^{AS} K_m^E / [S]_{eff} + k_{off}^{AS}} v_{max}^E$$

$$K_m^{AE} = \frac{(k_{off}^{AS} K_m^E /[S]_{eff} + k_{cat}^{ES}) k_{off}^{AS}}{(k_{cat}^{ES} + k_{off}^{AS} K_m^E /[S]_{eff} + k_{off}^{AS}) k_{on}^{AS}}$$

wherein $v_{max}^{AE}$ and $v_{max}^{E}$ are the maximum velocity for the adzyme and its enzyme component, respectively; $K_M^{AE}$ and $K_M^E$ are the $K_M$ for the adzyme and its enzyme component, respectively. The superscript "AS" indicates that the kinetic constant is that of an address / targeting moiety, which is determined by independent experiments on the address; the superscript "ES" or "E" indicates that the kinetic constant is that of an enzyme / catalytic moiety, which is determined by independent experiments on the enzyme. $[S]_{eff}$ or the "effective concentration" of the targeted substrate is a geometric parameter of the adzyme with concentration units. $k_{off}$ and $k_{on}$ are kinetic constances used to describe the the binding between, for example, adzyme, and a target molecule.

The catalytic efficiency for an adzyme is:

$$\text{Catalytic Efficiency} = \frac{v_{max}^{AE}}{K_m^{AE}[AE]_o}$$

$$= \frac{k_{on}^{AS} k_{cat}^{ES}}{k_{off}^{AS} K_m^E /[S]_{eff} + k_{cat}^{ES}}$$

A "chimeric protein construct" is an assemblage comprising at least two heterologous moieties, e.g., a catalytic domain and an address that are heterologous with respect to each other, that are covalently or non-covalently associated to form a complex. A chimeric protein construct may comprise non-proteinaceous molecules.

"Differentiation" in the present context means the formation of cells expressing markers known to be associated with cells with different functional properties or cells that are more specialized and closer to becoming terminally differentiated cells incapable of further division or differentiation.

A "fusion protein" is a chimeric protein wherein at least two heterologous amino acid sequences are covalently joined through an amide backbone bond, e.g., to form one contiguous polypeptide.

As used herein, the terms "modulate" or "alter" the activity of the targeted substrate are intended to include inhibiting, stimulating, up-regulating, down-regulating, activating, inactivating, or modifying the activity of the target in any other way.

A polynucleotide sequence (DNA, RNA) is "operatively linked" to an expression control sequence when the expression control sequence controls and regulates the transcription and translation of that polynucleotide sequence. The term "operatively linked" includes having an appropriate start signal (e.g., ATG) in front of the polynucleotide sequence to be expressed, and maintaining the correct reading frame to permit expression of the polynucleotide sequence under the control of the expression control sequence, and production of the desired polypeptide encoded by the polynucleotide sequence.

The term "pharmaceutically acceptable salts" refers to physiologically and pharmaceutically acceptable salts of the compounds of the invention, i.e., salts that retain the desired biological activity of the parent compound and do not impart undesired toxicological effects thereto.

The terms "polynucleotide sequence" and "nucleotide sequence" are also used interchangeably herein.

As used herein, "protein" is a polymer consisting essentially of any of the 20 amino acids. Accordingly, a protein may include various modifications. (e.g., glycosylation, phosphorylation) or non-amino acids. Although "polypeptide" is often used in reference to relatively large polypeptides, and "peptide" is often used in reference to small polypeptides, usage of these terms in the art overlaps and is varied.

As used herein, "proliferating" and "proliferation" refer to cells undergoing mitosis.

The International Union of Biochemistry and Molecular Biology (1984) has recommended to use the term "peptidase" for the subset of peptide bond hydrolases (Subclass E.C 3.4.). The widely used term protease is synonymous with peptidase. Peptidases comprise two groups of enzymes: the endopeptidases and the exopeptidases. Endopeptidases cleave peptide bonds at points within a protein, and exopeptidases remove amino acids sequentially from either the N- or C-terminus.

The term "proteinase" is also used as a synonym for endopeptidase. Proteinases are classified according to their catalytic mechanisms. Five mechanistic classes have been recognized by the International Union of Biochemistry and Molecular Biology: serine proteinases, cysteine proteinases, aspartic proteinases, threonine proteinases, and metalloproteinases.

This classification by catalytic types has been suggested to be extended by a classification by families based on the evolutionary relationships of proteases (Rawlings, N.D. and Barrett, A.J., (1993), Biochem. J., 290, 205-218). This classification is available in the SwissProt database.

In addition to these five mechanistic classes, there is a section of the enzyme nomenclature which is allocated for proteases of unidentified catalytic mechanism. This indicates that the catalytic mechanism has not been identified, and the possibility remains that novel types of proteases do exist.

The class "serine proteinases" comprises two distinct families: the chymotrypsin family which includes the mammalian enzymes such as chymotrypsin, trypsin or elastase or kallikrein, and the substilisin family which includes the bacterial enzymes such as subtilisin. The general three-dimensional structure is different in the two families but they have the same active site geometry and catalysis proceeds via the same mechanism. The serine proteinases exhibit different substrate specificities which are related to amino acid substitutions in the various enzyme subsites (see the nomenclature of Schechter and Berger) interacting with the substrate residues. Three residues which form the catalytic triad are essential in the catalytic process: His-57, Asp-102 and Ser-195 (chymotrypsinogen numbering).

The family of "cysteine proteinases" includes the plant proteases such as papain, actinidin or bromelain, several mammalian lysosomal cathepsins, the cytosolic calpains (calcium-activated), and several parasitic proteases (e.g., Trypanosoma, Schistosoma). Papain is the archetype and the best studied member of the family. Like the serine proteinases, catalysis proceeds through the formation of a covalent intermediate and involves a cysteine and a histidine residue. The essential Cys-25 and His-159 (papain numbering) play the same role as Ser-195 and His-57 respectively. The nucleophile is a thiolate ion rather than a hydroxyl group. The thiolate ion is stabilized through the formation of an ion pair with neighboring imidazolium group of His-159. The attacking nucleophile is the thiolate-imidazolium ion pair in both steps and then a water molecule is not required.

Most of the "aspartic proteinases" belong to the pepsin family. The pepsin family includes digestive enzymes such as pepsin and chymosin as well as lysosomal cathepsins D, processing enzymes such as renin, and certain fungal proteases (penicillopepsin, rhizopuspepsin, endothiapepsin). A second family comprises viral proteinases such as the protease from the AIDS virus (HIV) also called retropepsin. In contrast to serine and cysteine proteinases, catalysis by aspartic proteinases does not involve a covalent intermediate, though a tetrahedral intermediate exists. The nucleophilic attack is achieved by two simultaneous proton transfers: one from a water molecule to the dyad of the two carboxyl groups and a second one from the dyad to the carbonyl oxygen of the substrate with the concurrent CO-NH bond cleavage. This general acid-base catalysis, which may be called a "push-pull" mechanism leads to the formation of a non-covalent neutral tetrahedral intermediate.

The "metalloproteinases" are found in bacteria, fungi as well as in higher organisms. They differ widely in their sequences and their structures but the great majority of enzymes contain a zinc (Zn) atom which is catalytically active. In some cases, zinc may be replaced by another metal such as cobalt or nickel without loss of the activity. Bacterial thermolysin has been well characterized and its crystallographic structure indicates that zinc is bound by two histidines and one glutamic acid. Many enzymes contain the sequence HEXXH, which provides two histidine ligands for the zinc whereas the third ligand is either a glutamic acid (thermolysin, neprilysin, alanyl aminopeptidase) or a histidine (astacin). Other families exhibit a distinct mode of binding of the Zn atom. The catalytic mechanism leads to the formation of a non-covalent tetrahedral intermediate after the attack of a zinc-bound water molecule on the carbonyl group of the scissile bond. This intermediate is further decomposed by transfer of the glutamic acid proton to the leaving group.

In discussing the interactions of peptides with proteinases, e.g., serine and cysteine proteinases and the like, the present application utilizes the nomenclature of Schechter and Berger [(1967) Biochem. Biophys. Res. Commun. 27:157-162)]. The individual amino acid residues of a substrate or inhibitor are designated P1, P2, etc. and the corresponding subsites of the enzyme are designated S1, S2, etc. The scissile bond of the substrate is P1-P1'.

The binding site for a peptide substrate consists of a series of "specificity subsites" across the surface of the enzyme. The term "specificity subsite" refers to a pocket or other site on the enzyme capable of interacting with a portion of a substrate for the enzyme.

"Recombinant," as used herein with respect to a protein, means that the protein is derived from the expression of a recombinant nucleic acid by, for example, a prokaryotic, eukaryotic or in vitro expression system. A recombinant nucleic acid is any non-naturally occurring nucleic acid sequence or combination of nucleic acid sequences that was generated as a result of human intervention.

The term "substrate" refers to a substrate of an enzyme which is catalytically acted on and chemically converted by the enzyme to product(s).

The term "stereoisomers" refers to compounds which have identical chemical constitution, but differ with regard to the arrangement of the atoms or groups in space. In particular, "enantiomers" refer to two stereoisomers of a compound which are non-superimposable mirror images of one another. "Diastereomers", on the other hand, refers to stereoisomers with two or more centers of asymmetry and whose molecules are not mirror images of one another. With respect to the nomenclature of a chiral center, terms "D" and "L" configuration are as defined by the IUPAC Recommendations. As to the use of the terms, diastereomer, racemate, and enantiomer will be used in their normal context to describe the stereochemistry of peptide preparations.

"Transcriptional regulatory sequence" is a generic term used throughout the specification to refer to nucleic acid sequences, such as initiation signals, enhancers, and promoters, which induce or control transcription of protein coding sequences with which they are operably linked. In some examples, transcription of a recombinant gene is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally-occurring form of a protein.

As used herein, the term "vector" refers to a nucleic acid molecule capable of transporting another nucleic acid to which it has been linked. Preferred vectors are those capable of autonomous replication and/or expression of nucleic acids to which they are linked. Vectors capable of directing the expression of genes to which they are operatively linked are referred to herein as "expression vectors".

III. Exemplary Embodiments

An adzyme comprises at least two modular moieties: a targeting moiety and a catalytic domain. With respect to altering the activity of a targeted substrate, the adzyme is more potent relative to either the catalytic domain or targeting moiety alone.

The catalytic domain will often be protein-based, though even then may include other components, such as organic ligands or co-factors, or metal ions. It comprises a catalytically active site that reacts with a substrate without itself being consumed in the reaction. A catalytic domain will generally alter one or more bonds of a substrate, e.g., breaking the bond, removing one or more atoms across the bond (including oxidizing or reducing), and/or altering the stereochemistry of an atom participating in the bond. The site of chemical modification on the targeted substrate is referred to herein as the "substrate site".

The targeting moiety recognizes and binds to a pre-determined molecule, *i.e.*, an address binding site such as on a soluble or membrane bound intracellular or extracellular targeted biomolecule, which molecule is the same as or associated with the targeted substrate. The effect in both instances is to impart "addressability" to the adzyme construct, that is, to increase the local concentration of the construct in the vicinity of the targeted substrate so as to increase the proximity of the catalytic domain to the targeted substrate and thereby increase the catalytic efficiency for that substrate.

The targeting moiety and catalytic domain may be covalently attached or associated by non-covalent means. For instance, the moieties can be covalently attached as by fusion of two protein domains, with or without intervening sequences, to form a single polypeptide chain, or through derivation of the amino or carboxy terminus, or a sidechain of a polypeptide chain. In certain preferred embodiments, the targeting moiety and catalytic domain are produced as a cotranslational fusion by expression of a single recombinant nucleic acid construct. The various moieties may also be associated by non-covalent interactions, such as between protein domains, interaction with a common cross-linking ligand, etc.

The adzyme concept can be exploited in appropriate circumstances using a recruitment approach. Here, a multispecific binder is administered. An address of the multispecific binder complexes with a binding site on or near the intended targeted biomolecule. A chaperone protein or other structure of the multispecific binder, linked to or constituting a part of the address, displays a surface which complexes with a catalytic domain such as an enzyme already present in the body, or a co-administered enzymatically active moiety. The multispecific binder thereby induces complex formation between the address and a catalytic domain. The affinity of the address for the binding site serves to increase the effective concentration of the catalytic domain in the vicinity of the targeted biomolecule.

The address and catalytic domain of an adzyme often cooperate to produce synergistic behavior. The target may be modulated, e.g., inhibited by cleavage, by a catalytic domain used alone at a potency determined by its $K_M$ and $k_{cat}$. The target may also be inhibited by binding with a molecule defining an address used alone at a potency determined by its $K_a$, acting simply as a conventional drug. The amount of modulation of the target often may be objectively measured by standard assays. Thus modulation induced independently through each mechanism often can be at least roughly quantitated. It often will be found, at least in some adzyme constructs, that an adzyme comprising an optimized combination of a catalytic domain having the same $K_M$ and $k_{cat}$, and an address having the same $K_a$ will have a potency at least 10, $10^2$, $10^3$, or even $10^4$ times the sum of the potency of the individual components (catalytic and targeting) acting alone.

Another way to express the functional improvement of the adzyme in a pharmaceutical setting, relative to the targeting moiety and/or catalytic domain alone, is that in certain preferred embodiments the adzyme will have an effective dose ($ED_{50}$) for altering the activity of the targeted substrate in vivo at least 2 times less than the catalytic domain and/or targeting moiety (e.g., if a neutralizing moiety) alone, and more preferably at least 5, 10 or even 100 times less.

In the case of embodiments in which the targeted substrate is degraded to an inactive form by the adzyme, the potency may be expressed in terms of "$HL_{50}$", e.g., the concentration of adzyme required to reduce the half-life ($T\frac{1}{2}$) in vivo of the targeted substrate by 50 percent. The more potent and selective the adzyme is, the lower the $HL_{50}$ concentration is relative to the catalytic domain alone. In certain preferred embodiments, the $HL_{50}$ of the adzyme is at least 2 times less than the catalytic domain alone, and more preferably at least 5, 10 or even 100 times less.

In certain embodiments, the adzyme has a catalytic efficiency for the catalyzed reaction with the targeted substrate of at least $10^4$ M$^{-1}$sec$^{-1}$, and even more preferably at least $10^5$ M$^{-1}$sec$^{-1}$ or even at least $10^6$ M$^{-1}$sec$^{-1}$.

In certain embodiments, the adzyme has a catalytic efficiency for the catalyzed reaction with the targeted substrate at least 5 times greater than the catalytic domain alone, and even more preferably at least 10, 50 or even 100 times greater.

In certain therapeutic applications, it will be important to balance the potency and specificity of an adzyme. A good balance of potency and specificity can be achieved through the following design criterion:

$$k_{cat}^{ES} / K_m^E = k_{off}^{AS} / [S]_{eff}$$

The above value should be about $k_{on}^{AS}[S]_o / [S]_{eff}$.

In adzyme embodiments designed with this criterion, the catalytic domain will be very weak, in some cases having a catalytic efficiency as low as 100, 10, or 1 M$^{-1}$s$^{-1}$, or even lower, such as $10^{-3}$ M$^{-1}$s$^{-1}$. Thus, adzymes designed to balance potency and specificity should be derived from weak enzyme domains. In addition, the $k_{off}^{AS}$ value is also typically extremely low, such as $10^{-6}$ s$^{-1}$, 0.5 x $10^{-6}$ s$^{-1}$, $10^{-7}$ s$^{-1}$, or even lower. To achieve this goal, the following criteria may be followed in adzyme design:

| Parameter | Guideline | Rationale |
|---|---|---|
| $k_{on}^{AS}$ | Maximize, practical limit about $10^6$ M$^{-1}$s$^{-1}$ | Increases potency and selectivity |
| $k_{off}^{AS}$ | Decrease to $k_{on}^{AS}[S]_o$ | Increases selectivity |
| $[S]_{eff}$ | Maximize, practical limit $10^{-3}$ M | Increases potency and selectivity |
| $k_{cat}^{ES} / K_M^{ES}$ | Set to $k_{off}^{AS} / [S]_{eff}$ | Balances potency and selectivity |

Theoretically, any of the four variables in the equation above can be adjusted to approach the optimal balance between potency and selectivity. However, the easiest variable that can be changed is probably $[S]_{eff}$, which is largely dictated by the length and structure of the linker between the address domain and the enzyme domain (see linker design below). Alternatively, the design of the catalytic domain itself maybe altered such that the value of $k_{cat}^{ES} / K_M^{ES}$ (or the catalytic efficiency of the catalytic domain) is changed. To lower the catalytic efficiency, for example, either random mutatgenesis or targeted mutation at or around the catalytic domain active site and/or substrate binding site can yield "sub-optimal" catalytic domains with slightly diminished $k_{cat}$ and/or increased $K_M$ values. The advantage of changing $k_{cat}^{ES} / K_M^{ES}$ is that the design can accept a serendipitously produced $k_{off}^{AS} / [S]_{eff}$ value to achieve optimal balance.

In certain embodiments, the $k_{off}$ rate of the targeting moiety will be similar for the substrate and the adzyme reaction product, and it will be desirable to optimize the $k_{off}$ rate for high substrate affinity and rapid release of the product when bound to the address. In these embodiments, the optimal $k_{off}$ rate may be 0.001 sec$^{-1}$, 0.01 sec$^{-1}$, 0.1 sec$^{-1}$, or greater, and can be approximated by:

$$k_{off,optimal}^{AS} \approx \sqrt{k_{on}^{AS} \frac{k_{cat}}{K_m^E} [S]_{eff} [S]}$$

when $[S]_{eff} << K_M^E$, wherein $K_M^E$ is the enzyme's $K_M$ (not the adzyme's). The $k_{on}^{AS}$ ($k_{on}$ of adzyme) above is the same as $k_I$ in Equation 2 below.

For a fusion protein of two domains both of which independently bind the substrate, the "effective concentration of a substrate," $[S]_{eff}$, is the quotient of the overall association equilibrium constant for the fusion protein binding to its substrate and the product of the association equilibrium constants for the two, independent address domains binding to the substrate. This definition follows Figure 1 and Equation 2 in Zhou, J. Mol. Biol. (2003) 329, 1-8. Each of the three equilibrium constants required to determine $[S]_{eff}$ can be measured via standard binding assays. In performing kinetic analysis, it is further assumed that the microscopic off rates for each domain in a fusion protein are not affected by the presence of the linker.

In certain embodiments, the adzyme has a $K_M$ for catalyzed reaction with the targeted substrate at least 5 times less than the catalytic domain alone, and even more preferably at least 10, 50 or even 100 times less.

Broadly, the adzyme may be designed to interact with any biomolecule target provided the site of enzymatic attack and the binding site for the address are solvent accessible. Thus, both the targeted biomolecule and the binder for the address may be a soluble biomolecule or a membrane-bound biomolecule. The target may be intracellular, although extracellular targets are more accessible to protein constructs and are therefore preferred.

Referring to Figure 1, schematic diagrams illustrative of various structures which can exploit the invention are set forth as Figs. 1A through 1K. In 1A, perhaps the simplest adzyme, an address (ADD) is covalently linked to a catalytic domain (CAT). Such a construct may be embodied as two separate globular protein domains attached by a flexible or rigid linker as illustrated,, or by a single globular protein wherein one portion of the molecular surface functions as the address and another as a catalytically active site. In Figure 1B, the domains are complexed, i.e., each comprises a surface that reversibly binds to a surface on its partner. In Figures 1C through 1F, the address and catalytic domains are associated via a chaperone protein, with either or both linked to the chaperone via covalent bonds such as a linker or noncovalent protein-protein complexation. In Figures 1G and 1H, each of the address and catalytic domains is linked, covalently or non covalently, to a chaperone protein domain, and the chaperone domains are noncovalently complexed together.

Figures 1I and 1J illustrate one way to exploit the recruitment embodiment of the invention. These constructs, as illustrated, comprises an address linked (covalently or non covalently) to a chaperone protein, which defines a binding surface specific for a predetermined catalytic domain, i.e., an enzyme either already present in a body fluid or one co administered with the construct. This type of construct functions by recruiting the enzyme to the vicinity of the targeted biomolecule, mediated by the affinity of the address for the target so that the fully functional adzyme is assembled *in vivo*. Of course, such enzyme recruiting constructs could also be embodied in other forms provided they have a binding surface serving as an address that binds to the binding site on or adjacent the target, and a binding surface that serves to bind specifically to an enzyme. For example, a recruitment construct may be embodied as a single globular protein, or as a globular protein defining a binding surface for a catalytic domain and a small molecule with affinity for the target linked to it through a length of biocompatible polymer.

After the enzymatic reaction is complete, the adzyme disassociates from the target (now converted to a product) and moves on to bind to and act on another molecule of the target, creating turnover. As a result of this feature of the adzymes, the potency of the drug constructs is not dependant directly on drug/target stoichiometry. This provides a significant engineering advantage and can permit avoidance of toxicity issues associated with the use of antibodies or small molecule drugs inhibiting soluble biomolecules associated with a disease.

The equations below illustrates two possible adzyme (A-E) interactions between an address (A) and its binding site on a targeted biomolecule (S), and between the adzyme's enzymatically active site (E) and the targeted substrate (S) to make product (P).

$$A\!-\!E + S \underset{k_{-1}}{\overset{k_1}{\rightleftharpoons}} (A\!-\!E\!-\!-\!-\!S) \overset{k_{cat}}{\longrightarrow} A\!-\!E + P \qquad \text{(Eq-1)}$$

$$A\!-\!E + S \underset{k_{-1}}{\overset{k_1}{\rightleftharpoons}} \overset{S}{A\!-\!E} \underset{k_{-2}}{\overset{k_2}{\rightleftharpoons}} \overset{S}{A\!-\!E} \overset{k_{cat}}{\overset{P}{\longrightarrow}} A\!-\!E \overset{k_3}{\longrightarrow} A\!-\!E + P \quad \text{(Eq-2)}$$

Reaction 1 is the normal catalytic reaction, where the address is not involved, such as might occur with a substrate that does not display a binding site for the address. In the presence of a local concentration of both the adzyme (A-E) and the biomolecule (S) the targeted substrate has an on rate $k_1$ for the enzyme pocket (E), forms a complex A-E---S with the pocket, and is converted at a rate dependent on $k_{cat}$ to product P and released.

Reaction 2 occurs when the binding site on the targeted substrate S binds to the adzyme through formation of an address: binding site interaction (with an affinity that may be higher than the E---S affinity), forming a complex S---AE with on rate $k_1$. Presuming a suitable structure of the adzyme, e.g., the length of the linker or stereochemistry of the complex and its target permits, this complex can enter an intermediate state at rate $k_2$ where the targeted substrate interacts simultaneously with the address and the enzyme pocket. In this state the targeted substrate is converted to product P at a rate governed by $k_{cat}$, and then disassociates from the adzyme at rate $k_3$.

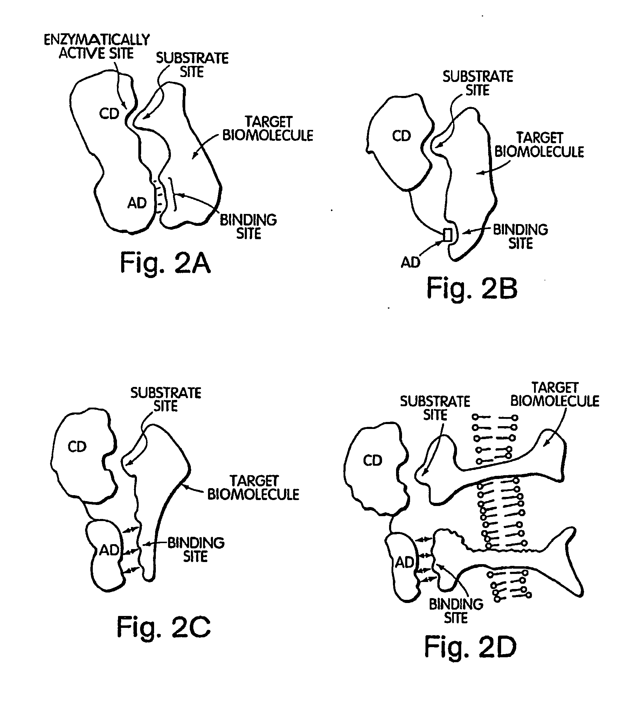

The functioning and structure of various forms of adzymes may be understood better with reference to Figures 2A-2J. *Figure 2A* depicts an adzyme in situ at a moment when it has bound to its intended biomolecule. In this case the adzyme is embodied as a single globular protein which defines a catalytic domain (CD) having an enzymatically active site and an address (AD) defined by a separate surface on the protein. The address binds reversibly with a binding site, in this case embodied as a surface on the targeted biomolecule. The targeted substrate site is vulnerable to immediate enzymatic attack by the enzymatically active site of the catalytic domain.

*Figure 2B* shows a construct similar to Figure 2A except that the address is a small molecule attached to the catalytic domain by a flexible linker that binds reversibly directly with a binding site on the intended targeted biomolecule.

*Figure 2C* is an adzyme similar to 2B in which the address and the catalytic domain are attached by a flexible leash. Binding of the address domain to the binding site, here again illustrated as a portion of the targeted biomolecule, serves effectively to increase the local concentration of the catalytic domain in the region of the target, as illustrated. The address domain and the catalytic domain may be linked via a flexible linker, or a more rigid structure (not shown) such that binding of the address domain serves to pose the catalytic domain in position to induce chemical change in its targeted biomolecule.

The adzyme of *Figure 2D* is similar to Figure 2C, except that the binding site and the targeted biomolecule are separate molecular species, here illustrated as being lodged in a membrane, such as a cell membrane. As in the embodiments of Figures 2A-2C, binding of the address domain to the recognition site of what here functions as a attractant molecule serves to effectively increase the local concentration of the catalytic domain in the region of the target. Where the concentration of two proteins on a cell is significant, especially in cases where they are known to interact in lipid rafts or the like, one molecule can be used as the binding site to attract the construct to the other molecule that will be catalytically modulated.

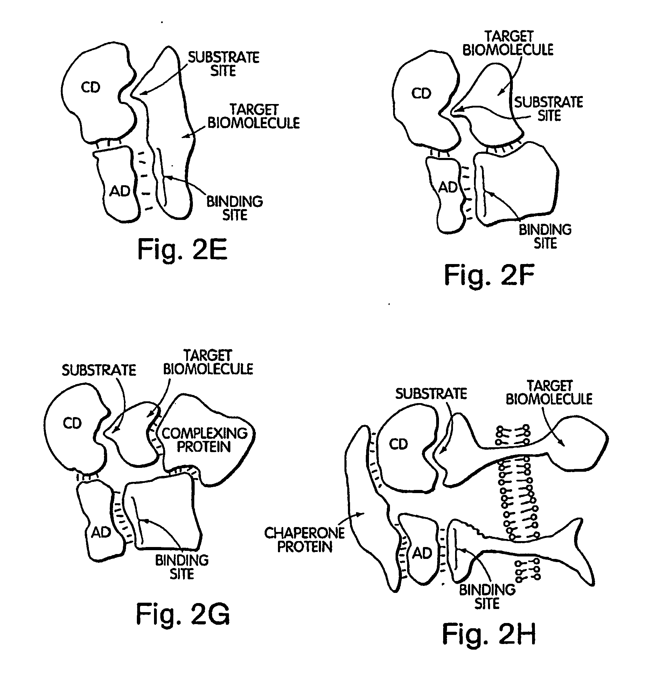

The adzyme of *Figure 2E* is similar to Figure 2C, except that the address domain and the catalytic domain are non-covalently associated directly to each other. Examples of this type of association include dimerization, optionally stabilized by disulfide linkages, hybridization of complementary nucleotides, or protein-protein complexation of the type that is ubiquitous within cells.

*Figure 2F* shows an embodiment of an adzyme similar to Figure 2E, except that the address domain is designed to bind to an attractant biomolecule separate from but complexed to the targeted biomolecule. Nevertheless, binding increases the effective concentration of the target and its substrate site in the vicinity of the catalytic domain as shown.

*Figure* 2G is the same as Figure 2F except that the targeted biomolecule is complexed with a separate protein displaying the binding site through a third, complexing protein.

*Figure 2H* illustrates an embodiment of an adzyme in which the address and the catalytic domain are non-covalently associated through a third, chaperone protein, to form an active complex. Its intended targeted biomolecule is illustrated as being embedded in a lipid bilayer, and the binding site is illustrated as residing on a separate molecule in the lipid bilayer, similar to Figure 2D. Again, binding nevertheless increases the effective concentration of the target and its substrate site in the vicinity of the catalytic domain.

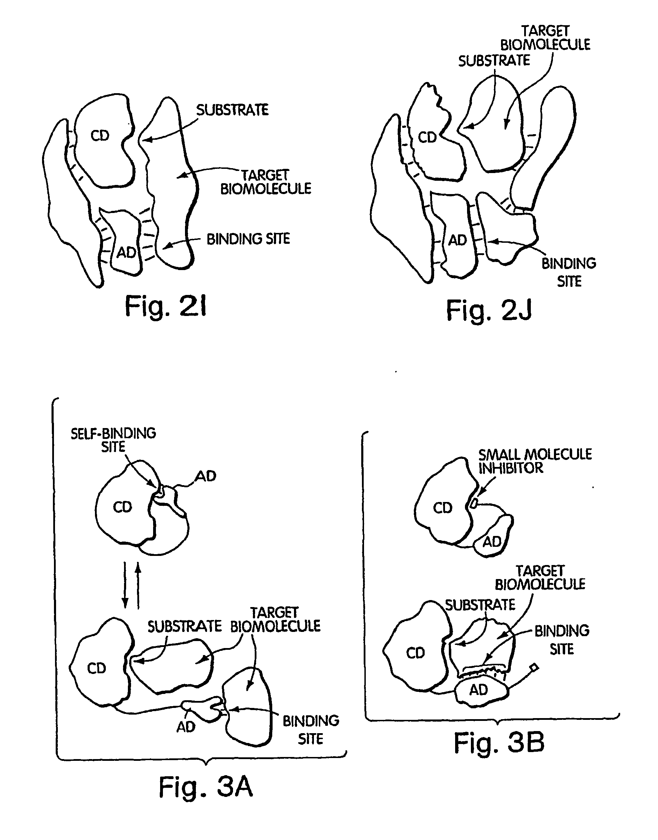

*Figure 2I* illustrates an embodiment of an adzyme similar to Figure 2H, except that the address domain binds to a binding site directly on the targeted biomolecule.

*Figure 2J* is similar to Figure 2G, except that the address domain and catalytic domain of the adzyme are held together via complexation with a chaperone protein. In all construct where the AD and CD are non covalently complexed, the surface on the address domain that binds to the catalytic domain (or a chaperone protein) may be the same or different from the one that binds to the binding site on the target or trigger molecule.

A further optional feature of adzymes is "engineered contingency," that is, creation of a family of adzymes that *become* capable of reacting with their target in the presence of the target or another triggering or attractant molecule having an affinity for the address. Figure 1K illustrates the fundamental idea behind the contingent adzyme. As illustrated, the address has an affinity for the catalytic domain and is configured so that it can bind to it and inhibit its enzymatic activity. In the presence of the target, a competition for the address ensues, freeing the catalytic domain to induce chemical change in its intended target.

Stated differently, contingent adzyme constructs are inactive (have low enzymatic activity) in the absence of a triggering molecule, but become active in the presence of the triggering molecule, e.g., the target (see Legendre D. et al. (1999) Nature Biotechnology 17:67-72; Legendre D. et al. (2002) Protein Science 11:1506-1518; Soumillion P. and Fastrez J. (2001) Current Opinion in Biotechnology 12:387-394). This type of adzyme also requires a catalytic domain and an address. However, in this case, binding of the address has the effect of freeing up the catalytic site of the catalytic domain to enhance its activity. This may be achieved in several ways, illustrated by way of example in Figures 3A through 3G, which are described in more details in the contingent adzyme section.

In addition to the address and catalytic domains, and the optional chaperone proteins, linkers and other structures defining the relationship of these parts, an adzyme may further comprise one or more fusion partners operatively linked to any of its components, e.g., N-terminal or C-terminal fusions, or added or substituted sequences in loops on protein domains. Adzymes may also include polymeric side chains, small molecules, or metal ions. These moieties may, for example, restrict the adzyme to a conformationally restricted or stable form; serve as a targeting sequence allowing the localization of the adzyme into a subcellular or extracellular compartment; assist in the purification or isolation of either the adzyme or the nucleic acids encoding it; serve to confer a desired solubility on the adzyme; or confer stability or protection from degradation to the adzyme or the nucleic acid molecule(s) encoding it (*e.g.*, resistance to proteolytic degradation). The adzyme may comprise one or any combination of the above fusion partners as needed.

The fusion partners can, for example, be (histidine)$_6$-tag, glutathione S-transferase, protein A, dihydrofolate reductase, Tag•100 epitope (EETARFQPGYRS; SEQ ID NO: 1), c-myc epitope (EQKLISEEDL; SEQ ID NO: 2), FLAG®-epitope (DYKDDDK; SEQ ID NO: 3), lacZ, CMP (calmodulin-binding peptide), HA epitope (YPYDVPDYA; SEQ ID NO: 4), protein C epitope (EDQVDPRLIDGK; SEQ ID NO: 5) or VSV epitope (YTDIEMNRLGK; SEQ ID NO: 6).

The fusion partner may also be a membrane translocation domain, *i.e.*, a peptide capable of permeating the membrane of a cell and which is used to transport attached peptides into or out of a cell *in vivo*. Membrane translocation domains that may be used include, but are not limited to, the third helix of the *antennapedia* homeodomain protein and the HIV-1 protein Tat or variants thereof. Additional membrane translocation domains are known in the art and include those described in, for example, Derossi et al., (1994) J. Biol. Chem. 269, 10444-10450; Lindgren et al., (2000) Trends Pharmacol. Sci. 21, 99-103; Ho et al., Cancer Research 61, 474-477 (2001); U.S. Patent No. 5,888,762; U.S. Patent No. 6,015,787; U.S. Patent No. 5,846,743; U.S. Patent No. 5,747,641; U.S. Patent No. 5,804,604; and Published PCT applications WO 98/52614, WO 00/29427 and WO 99/29721.

A. Exemplary Targeting Moieties

It will be appreciated that a wide range of entities can be used as targeting moieties in the subject adzymes. Fundamentally, the targeting moiety reversibly binds to a predetermined feature ("address site") associated with the targeted substrate. The targeting moiety presents one or more surfaces having chemical characteristics (e.g., hydrophobic, steric and/or ionic) which permit it to bind selectively, or relatively selectively, with the address site. In many embodiments, the address will be a modular protein (including peptide) domain which is provided in association with the catalytic domain. For example, the targeting moiety can be an antibody, or a fragment of an antibody which retains the ability to bind to the address site. Accordingly, the targeting moiety can be derived from such antibody and antibody fragments as monoclonal antibodies, including Fab and F(ab)2 fragments, single chain antibodies (scFv), diabodies, and even fragments including the variable regions of an antibody heavy or light chain that binds to the address site.

Other examples of proteins that can be suitably adapted for use in the subject adzymes including ligand binding domains of receptors, such as where the targeted substrate of the adzyme is the receptor ligand. Conversely, the targeting moiety can be a receptor ligand where the adzyme is directed to the receptor as the targeted substrate. Such ligands include both polypeptide moieties and small molecule ligands.

In a further embodiment, a targeting moiety may be derived from a polypeptide that has an immunoglobulin-like fold, such as the 10th type III domain of human fibronectin ("Fn3"). See US Pat. Nos. 6,673,901; 6,462,189. Fn3 is small (about.95 residues), monomeric, soluble and stable. It does not have disulfide bonds which permit improved stability in reducing environments. The structure may be described as a .beta.-sandwich similar to that of Ab VH domain except that Fn3 has seven beta-strands instead of nine. There are three loops on each end of Fn3; and the positions of three of these loops correspond to those of CDR1, 2 and 3 of the VH domain. The 94 amino acid Fn3 sequence is:

VSDVPRDLEVVAATPTSLLISWDAPAVTVRYYRITYGETGGNSPVQEFTVPGSKSTATISGL

KPGVDYTITGYAVTGRGDSPASSKPISINYRT

The amino acid positions of the CDR-like loops will be defined as residues 23-30 (BC Loop), 52-56 (DE Loop) and 77-87 (FG Loop). Accordingly, one or more of the CDR-like loops may be modified, and preferably randomized, to generate a library of Fn3 binding domains which may then be screened for binding to a desired address binding site.

See also PCT Publication WO0232925. Fn3 is an example of a large subfamily of the immunoglobulin superfamily (IgSF). The Fn3 family includes cell adhesion molecules, cell surface hormone and cytokine receptors, chaperoning, and carbohydrate-binding domains, all of which may also be adapted for use as binding agents. Additionally, the structure of the DNA binding domains of the transcription factor NF-kB is also closely related to the Fn3 fold and may also be adapted for use as a binding agent. Similarly, serum albumin, such as human serum albumin contains an immunoglobulin-like fold that can be adapted for use as a targeting moiety. Additional scaffolds that may be used as targeting moieties include: anticalins (lipocalin derivatives, see Pieris Proteolab AG), Affibodies (based on Protein A, see US Pat. Nos. 5831012, 6534628 and 6740734), knottins (cysteine knot structures, typically derived from protease inhibitors), tendamistat, GST P1-1/A1-1, tetranectins (plasminogen kringle 4 domain-binding protein), and trimeric binding proteins (Borean Pharma, W004039841A2).

In still other embodiments, the targeting moiety can be an engineered polypeptide sequence that was selected, e.g., synthetically evolved, based on its kinetics and selectivity for binding to the address site.

The targeting moiety can also be a polyanionic or polycatonic binding agent, such as an oligonucleotide, a polysaccharide, a polyamino peptide (such as poly-aspartate, poly-glutamate, poly-lysine or poly-arginine). In certain embodiments, such targeting moieties maintain a number of either negative or positive charges over their structure at physiological pH. The address may also be a protein nucleic acid (PNA), a lock nucleic acid (LNA) or a nucleotide sequence, such as a single strand of DNA or RNA.

The targeting moiety may also be a small molecule that has been selected based on the kinetics and selectivity it displays for binding to an address site associated with the targeted substrate.

There are a variety of well-known techniques for generating libraries of polypeptide/peptide, nucleic acid (aptamer) and small molecule moieties that can be used to identify molecules having the appropriate specificity, selectivity and binding kinetics for use in any particular adzyme. For example, such techniques as described in US Patents 6258558 titled "Method for selection of proteins using RNA-protein fusions" and 5837500 titled "Directed evolution of novel binding proteins" can be readily adapted for use in identifying peptide or polypeptide targeting moieties for use in generating the subject adzymes. Likewise, the preparation of aptamers previously described in the art can be adapted for generating appropriate targeting moieties. See, for example, Tuerk Science 249:505-510 (1990); Klug Mol Biol Reports 20:97-107 (1994); and Morris et al, PNAS 95:2902-2907 (1998), as well as U.S. Patents 5,843,701 and 5,843,653.

The address may be at least about 5, 10, 15, 20, 25, 30, 35, 40, 45, 50, 55, 60, 70, 80, 90 or 100 amino acid residues long. Ranges using a combination of any of the foregoing recited values as upper and/or lower limits are intended to be included in the present invention.

In certain preferred embodiments, the dissociation constant ($K_d$) for binding to the address site is lower (higher affinity) and/or the $K_{off}$ rate is slower when the address site is bound to the unmodified targeted substrate relative to when it is bound to the adzyme reaction product (e.g, the targeted substrate that has been acted on by the catalytic domain). That is, conversion of the targeted substrate to an adzyme reaction product reduces the affinity of the targeting moiety for the address binding site and promotes dissociation of the adzyme from the reaction product. In certain embodiments: the $K_d$ of the targeting moiety for the adzyme reaction product relative to the targeted substrate is at least 5 times greater, and even more preferably 10, 100 or even 1000 times greater; and/or the $K_{off}$ rate of the targeting moiety for the adzyme reaction product is at least 5 times faster, and even more preferably 10, 100 or even 1000 times faster relative to the $K_{off}$ rate for the targeted substrate.

In certain embodiments of direct adzymes, the address site and substrate site are overlapping in the sense that binding of the targeting moiety to the targeted substrate interferes with the ability of the catalytic domain to act on the targeted substrate site. This interference may be the result of steric occlusion, or the lack of flexibility in the adzyme and/or targeted substrate to permit both portions of the adzyme to simultaneously interact with the targeted substrate. In other embodiments, the address and substrate sites are spaced sufficiently apart, and the adzyme has sufficient steric flexibility, that dissociation of the targeting moiety is not required for the adzyme to modify the targeted substrate. In many embodiments, the adzyme will be designed such that there is functional cooperativity between the catalytic domain and targeting moiety, particularly resulting from appropriate selection of linker(s) between the two components, such that the affinity of the resulting adzyme is at least 2 times greater than the sum of the affinities of the catalytic domain and targeting moiety, and even more preferably at least 5, 10, 100 or even 500 times greater.

In some instances, the targeting moiety itself interferes with the activity of the targeted substrate. For example, the targeting moiety may be a blocking or neutralizing agent that inhibits an intrinsic activity or interaction mediated by the targeted substrate. In such cases, the adzyme with preferably be at least 5 times more potent an inhibitor, and even more preferably at least 10, 100 or even 1000 times more potent than the targeting moiety alone.

In other embodiments, the targeting moiety does not itself have any significant effect on the activity of the targeted substrate.

Where there are more than one possible substrate site of the catalytic domain on a targeted substrate, such as more than one substrate recognition sequences for a proteolytic domain, the targeting moiety can be selected to enhance the selectivity/preference of the adzyme for one of the sites. This can be accomplished, for example, by using a targeting moiety that binds to the targeted substrate in a manner that sterically interferes with the catalytic domain's ability to act at one of the sites. In other embodiments, the targeting moiety can be used to increase the concentration of the catalytic domain in the proximity of the desired substrate site.

In certain embodiments, the adzyme may include two or more address/ targeting moieties, which may be the same or different (i.e., their respective $K_d$ may be the same or different). In such embodiments, the effective $K_d$ of the adzyme for the targeted substrate may be as low as $10^{-15}$M (femtomolar), when the effective substrate concentration $[S]_{eff}$ is greater than the highest individual $K_d$ of the addresses (or targeting moieties).

In certain embodiments, the targeting moiety binds to a a targeted substrate which is soluble under the reaction conditions, such as a soluble protein. In many cases, these soluble protein substrates will be present in the reaction milieu at relatively low concentrations, such as less than 0.1 $\mu$M, and often at less than 10 nM. In such embodiments, and certain others herein, it may be desirable to select a targeting moiety which, when provided in the adzyme, results in a direct adzyme having a relative fast $k_{on}$ for binding to the targeted substrate, e.g., a $k_{on}$ of $10^3$ M$^{-1}$s$^{-1}$ or greater, e.g., at least $10^4$ M$^{-1}$s$^{-1}$, $10^5$ M$^{-1}$s$^{-1}$ or even $10^6$ M$^{-1}$s$^{-1}$.

*(i) Exemplary Targeted Biomolecules*

In certain embodiments, the subject adzymes are directed to biologically active molecules ("targeted biomolecule"), e.g., including solvent accessible extracellular and intracellular substrates, as well as extracellular or cytoplasmic portions of membrane associated substrates. These include, but are not limited to, substrates from among such classes as protein and peptide substrates, nucleic acids, lipids, small molecules including extracellular factors (such as steroids and neurotransmitters) and intracellular second messengers (such as phosphorylated inositol and cAMP). By modifying the functional performance of a targeted substrate of biological relevance, the subject adzymes can be used to alter such cellular processes as gene expression, morphology, cell adhesion, growth, proliferation, migration, differentiation and/or viability of cell.

Taregeted substrates can be modified by the adzyme so as to produce one or more products having one or more differences in biological activities relative to the targeted substrate (including, for example, the elimination of all or near all biological activity of the targeted substrate). For instance, for targeted substrates which are themselves enzymes, the subject adzymes can be used to alter the intrinsic enzymatic activity of those targeted substrates. To illustrate, an adzyme can used to inhibit such proteases as elastase (in the treatment of cystic fibrosis, acute respiratory distress syndrome, and emphysema) or matrix metalloproteases involved in metastasis. In other embodiments, the adzyme alters the ability of a targeted substrate to interact with other biological moieties, e.g., such as by altering receptor-ligand interactions, protein-protein interactions, protein-lipid interactions, protein-DNA or protein-RNA interactions to name but a few. In this respect, the adzyme can be used to increase or decrease the intrinsic activity or binding activity of the targeted substrate. Adzymes can also be used to alter the half-life or biodistribution of a targeted substrate.

In certain instances, the adzyme can be used to covert a targeted substrate into a functional antagonist of the unmodified biomolecule. Merely to illustrate, in the case of a polypeptide factor that acts through a receptor interaction, rather than generate a product that is unable to interact with the cognate receptor of the targeted substrate, the adzyme can be selected so as to alter the targeted substrate to produce a product that retains the ability to bind to the receptor but not induce the level of receptor activation possible by the unmodified targeted substrate. In this way, the adzyme inhibits the function of the polypeptide factor by (a) reducing the concentration of the polypeptide factor, and (b) generating an antagonist which reduces the effective concentration of receptor for the polypeptide factor. In preferred embodiments of this system, the product has a $K_i$ of 10$\mu$M or less for inhibiting an activity of the targeted substrate, and even more preferably has a $K_i$ less than 10$\mu$M, 100nM, 10nM or even 1 nM.

(a) Extracellular Targets

In certain embodiments, the adzyme is directed to an extracellular target, including target molecules that are typically located entirely outside of a cell and target molecules that are inserted into a cellular membrane but have a portion that is exposed to the extracellular environment. Several categories of extracellular targets are recognizable, including, for example, diffusible extracellular molecules (*e.g.*, growth factors, serum proteins, antibodies, any diffusible small molecule, extracellular nucleotides, lipids), extracellular molecules that are part of an insoluble aggregate (*e.g.*, β-amyloid protein, constituents of atherosclerotic plaques, insoluble fibrin fibers), membrane associated proteins and other membrane bound moieties (e.g., transmembrane proteins, lipids, membrane associated polysaccharides), and constituents of or associated with an organized extracellular matrix.

Accordingly, the subject adzymes can be used to alter, e.g., inhibit or potentiate, such cell-surface mediated signaling as autocrine signaling (self-signaling), paracrine signaling (between nearby cells), and/or endocrine signaling (over a long distance, usually via the bloodstream or other bodily fluid). The subject adzymes can also be used to alter juxtacrine signaling, e.g., signaling consequences of cell contact.

Various illustrative examples of different types of extracellular targets are provided in Table I, below, along with associated conditions that antagonistic adzymes may be used to treat.

**Table I: Examples of various extracellular targets and associated conditions.**

| Target | Disease/Condition |
|---|---|
| TNF receptor | Inflammation, arthritis, autoimmune thyroid disease, ischemic heart disease |
| TNF-$\alpha$ and $\beta$ | Inflammation, arthritis, autoimmune thyroid disease, ischemic heart disease |
| IL-2 receptor | Ischemic heart disease |
| Aldosterone | Cardiovascular heart disease |
| Amyloid beta-peptide [Abeta (1-42)] | Alzheimer's disease |
| Transthyretin | Alzheimer's disease |
| Erythropoietin | benign erythrocytosis |
| Prostaglandin | Neurodegeneration |
| Cholesterol | Heart disease |
| Retinoid X | hepatogastroenterological diseases |
| Apolipoprotein B-100 | Coronary heart disease |
| Homocysteine | Cardiovascular disease |
| Insulin | Diabetes |
| Apolipoprotein A1 | Heart disease |
| Apolipoprotein CII | Hyperlipidemia |
| Apolipoprotein CII | heart disease |
| Apolipoprotein E | Cardiovascular disease |
| Apolipoprotein E | Alzheimer's disease |
| CD4 | Immune response |
| CD4 receptor | Immune response/ HIV infection |
| CCR5 | Immune response/ HIV infection |
| SBR1 | HDL receptor/coronary heart disease |
| Annexin V | Clot formation, Apoptosis |
| Fibrin | Wound healing, clot formation |

Among the diffusible extracellular molecules, further subcategories are recognizable. In a preferred embodiment, a target of an adzyme is an extracellular signaling molecule, meaning a molecule that is produced by one cell with the primary effect of triggering a response in another cell. Examples of extracellular signaling molecules include most growth factors and cytokines, neurotransmitters, hormones, and prostaglandins. Many extracellular signaling molecules are actually part of a larger assemblage that carries out the signaling function; for example, TGF-$\beta$1 contains two 112 amino acid chains that are linked by a disulfide bond, and either of the two polypeptide chains may be considered to be extracellular signaling molecules that are targeted by an adzyme. Antibodies are explicitly not included in the term "extracellular signaling molecule".

In certain embodiments, an extracellular signaling molecule is a molecule that binds to an extracellular portion of a membrane bound receptor and triggers a signal transduction event in the cell. In certain embodiments, an extracellular signaling molecule is a molecule that enters a cell and binds to an intracellular receptor to trigger a signal transduction event in the cell (e.g., steroid hormones, harpin proteins of various bacterial pathogens).

In a particularly preferred embodiment, the target of an adzyme is an extracellular polypeptide signaling molecule, e.g., as may be found in biological fluid(s), such as a growth factor, cytokine, polypeptide hormone or the like. In certain preferred embodiments, the targeted substrate is a signaling molecule, particularly a polypeptide signaling molecule, present in serum or other bodily fluid at a concentration of less than 1 $\mu$M, and even more preferably less than 0.1 $\mu$M, 10 nM, 1 nM, 0.1 nM, 10 pM or even 1 pM. The catalytic domain is chosen so as to modify the signaling molecule in a manner that alters its interaction with a cognate receptor (e.g., abrograting binding or limiting receptor activation), ability to form protein complexes with other soluble factors, half-life and/or biodistribution.

In certain preferred embodiments, the adzyme alters the level of signal transduction induced by an extracellular factor. The term "signal transduction" is intended to encompass the processing of physical or chemical signals from the extracellular environment through the cell membrane and into the cell, and may occur through one or more of several mechanisms, such as activation/inactivation of enzymes (such as proteases, or enzymes which may alter phosphorylation patterns or other post-translational modifications), activation of ion channels or intracellular ion stores, effector enzyme activation via guanine nucleotide binding protein intermediates, second messenger generation (e.g., GTP hydrolysis, calcium mobilization, formation of inositol phosphates, cyclic nucleotides, sugar nucleosides or dissolved gases such as NO or $O_3$), redistribution of intracellular ions ($Ca^{+2}$, $Zn^{+2}$, $Na^+$, $K^+$), and/or direct activation (or inhibition) of a transcriptional factor. Signal transduction may result in physiological changes to the cell, such as changes in morphology, cell adhesion, chemotaxis, drug resistance, growth, proliferation, death (apoptosis or necrosis), effector function, secretion of matrix, etc.

The induction of intracellular signals by the binding of an extracellular signaling molecule, such as a soluble growth factor, to a membrane-spanning receptor is of considerable biological importance. In many cases, promotion of receptor-receptor interactions by protein factors is a key initial step in the induction of a signal transduction process. In certain preferred embodiments, the subject adzymes can be used to alter the biological function/performance of an inductive protein factor, such as a protein factor selected from one of the protein factor superfamilies known as (i) four-helix bundle factors, (ii) EGF-like factors, (iii) insulin-like factors, (iv) $\beta$-trefoil factors and (v) cysteine knot factors. Exemplary substrates within in these classes are listed in Table II.

**Table II: Growth factor structural superfamilies**

| Family | Subclass | Examples |
|---|---|---|
| Four-helix bundle | Short chain | IL-2, IL-3, IL-4, IL-5, IL-7, IL-9, IL-13, IL-15, M-CSF, GM-CSF |
| | Long chain | GH, LIF, G-CSF, IL-6, IL-12, EPO, OSM, CNTF |
| | Interferon | IFN$\beta$, IFN$\gamma$ |
| EGF-like | | EGF, TGF$\alpha$, heregulin |
| Insulin-like | | Insulin, IGF1, IGF2 |
| $\beta$-trefoil | | FGF, IL-1 |
| Cysteine knot | | NGF, PDGF, TGF$\beta$ proteins |

Examples of particular extracellular signaling molecules and conditions associated with these targets which may be treated using an appropriate adzyme are listed in Table III, below.

**Table III: Examples of Extracellular Signaling Molecules**

| Target | Disease/Condition |
|---|---|
| IL-1$\alpha$ and $\beta$ | Inflammation, Arthritis, inflammatory bowel disease |
| IL-4 | Asthma, allergic airway disease |
| IL-5 | Asthma, Allergic airway disease |
| IL-6 | Inflammation, Kaposi's sarcoma |
| IL-7 | Immune response |
| IL-8 | Inflammatory disease, Crohn's disease |
| IL-18 | Arthritis |
| IL-9 | Asthma |
| IL-10 | Colitis |
| IL-11 | Crohn's disease, Ischemic heart disease |

(continued)

| Target | Disease/Condition |
|---|---|
| TNF-α and β | Inflammation, arthritis, autoimmune thyroid disease, ischemic heart disease |
| VEGF | Cancer, Angiogenesis, Arthritis, Eales' disease |
| Aldosterone | Cardiovascular heart disease |
| Somatostatin | Grave's disease |
| Fibronectin | Ullrich's disease |
| Angiotensin | Heart disease |
| Erythropoietin | Benign erythrocytosis |
| Prostaglandins | Neurodegeneration |
| Interferon α and β | Immune response |
| Retinoid X | hepatogastroenterological diseases |
| Adrenocorticotropic Hormone | Cushing's disease |
| Hepatocyte growth factor | Cardiovascular disease, periodontal disease |
| Transforming growth factor-beta1 | Graft-versus-host disease, renal disease |
| Transforming growth factor-beta | Coeliac Disease |
| Insulin-like growth factor binding protein-1 (IGFBP-1) | macrovascular disease and hypertension in type 2 diabetes |
| VEGF-A | Paget's disease |
| Platelet-derived endothelial cell growth factor/thymidine phosphorylase (PD-ECGF/TP) | Paget's disease |
| Insulin-like growth factor I (IGF-I) | Inflammatory bowel disease |
| IGF binding protein-3 | Inflammatory bowel disease |
| Insulin | Diabetes |
| EGF | Oncogenesis, Wound healing |
| Vasoactive intestinal peptide | Inflammation |

In certain particularly preferred embodiments, the targeted substrate is an inflammatory cytokine, such as tumor necrosis factor (TNF-α), interleukin-6 (IL-6) or interleukin-1b (IL-1b), and the adzyme can be used therapeutically to reduce inflammation.

In certain other preferred embodiments, the targeted substrate is a polypeptide hormone, such as Adrenocorticotrophic Hormone, Amylin Peptide, Bombesin, Calcitonin, Cholecystokinin (CCK-8), Gastrin, Glicentin, GLP-1, GLP-2, PYY, NPY, GIP, Glucagon, Human Chorionic Gonadotrophin (α), Human Chorionic Gonadotrophin (β), Human Follicle Stimulating Hormone (β2), Human Growth Hormone, Insulin, Luteinising Hormone, Pancreatic Polypeptide, Parathyroid Hormone, Placental Lactogen, Proinsulin, Prolactin, Secretogranin II, Somatostatin, Thyroglobulin, Thyroid Stimulating Hormone, Vasoactive Intestinal Polypeptide.

Other exemplary substrates for the subject adzymes include polypeptide factors selected from the group consisting of: Granulocyte-colony stimulating factor (G-CSF), Myelomonocytic growth factor, Interleukin-3, Interleukin-7, Leukemia inhibitory factor (LIF), Oncostatin M, Ciliary neurotrophic factor (CNTF), cholinergic differentiation factor (CDF), Interleukin-4, Interleukin-13, Interleukin-16, Interleukin-17, Interferon-alpha (IFN-α), Interferon-beta (IFN-β), IFN-tau (IFN-τ), Interferon-omega (IFN-ω), Interleukin-5, Granulocyte-macrophage colony-stimulating factor (GM-CSF), Macrophage colony-stimulating factor (M-CSF), Interleukin-10, Interleukin 1-alpha (IL1-α), Interleukin 1-beta (IL1-β), Gonadotropin, Nerve Growth Factor (NGF), platelet factor 4 (PF-4), bTG, GRO, 9E3, HLA-A2, macrophage inflammatory protein 1 alpha (MIP-1α), macrophage inflammatory protein 1 beta (MIP-1β), Melanoma growth stimulating activity (MGSA), 4-1BB Ligand, ADF, Autocrine Motility Factors, B61, Betacellulin, Cardiotrophin-1, CD27 Ligand, CD30 Ligand, CD40 Ligand, CeK5 Receptor Ligand, EMAP-II, ENA-78, Eosinophil Cationic Protein, Epiregulin, Erythrocyte-derived Growth-Promoting Factor, Erythropoietin, Fas Ligand, Fibrosin, FIC, GDNF, Growth/Differentiation Factor-5, Interleukin-1 Receptor Antagonist, Interleukin-3, Interleukin-6, Interleukin-7, Interleukin-9, Interleukin-11, Interleukin-12, Interleukin-13, Interleukin-14, Interleukin-15, Lymphotactin, LT-beta, Lymphotoxin, MCP-2, MCP-3, Megapoietin, Melanoma-derived Growth Regulatory Protein, Monocyte Chemoattractant Protein-1, Macrophage Migration Inhibitory Factor, Neu Differentiation Factor, Oncostatin M, OX40 Ligand, Placenta Growth Factor, PLF, Scatter Factor, Steel Factor, TCA 3, Thrombopoietin, Vascular Endothelial Cell Growth Factor, Bone Morphogenetic Proteins, Interleukin-1 Receptor Antagonist, Monocyte Chemoattractant Protein-1, c-Kit ligand (stem cell factor), CXC chemokines, CC chemokines, lymphotactin, and C-X3-C chemokines (fractalkine / neurotactin).

In other embodiments, the adzyme is directed to a substrate associated with a cell surface, such as for altering the activity of a cell surface receptor, ion channel, transporter, adhesion molecule, lipid, or extracellular matrix molecule such as a polysaccharide or glycosaminoglycan.

In certain preferred embodiments, the targeted substrate is a cell surface receptor protein or ion channel. For instance, the adzyme can be designed to modify a ligand-binding receptor protein in a manner that alters ligand binding kinetics and/or signal transduction activity of the receptor. Receptor proteins which can be substrates for the subject adzymes include any receptor or channel which interacts with an extracellular molecule (i.e. hormone, growth factor, peptide, ion) to modulate a signal in the cell. To illustrate, the targeted substrate of the adzyme can be a site on a serpentine receptor (such as G protein coupled receptor), an enzyme-linked receptor (such as a receptor tyrosine kinase, receptor serine/threonine kinase, receptor protein tyrosine phosphatase, receptor guanylyl cyclase, or receptor nitric oxide synthase), or an ion channel (including an ion-channel-linked receptor). Exemplary receptors which can be altered by an adzyme include cytokine receptors; multisubunit immune recognition receptors (MIRR), chemokine receptors; growth factor receptors, or chemoattracttractant peptide receptors, neuropeptide receptors, light receptors, neurotransmitter receptors, and polypeptide hormone receptors, to name but a few. Further examples of cell surface receptors are provided in Table IV, along with associated conditions that may be treated by administration of an appropriately targeted adzyme.

**Table IV: Examples of Cell Surface Receptors**

| Target | Disease/Condition |
|---|---|
| IL-1 receptor | Inflammation, Arthritis, inflammatory bowel disease |
| TNF receptor | Inflammation, arthritis, autoimmune thyroid disease, ischemic heart disease |
| IL-2 receptor | Ischemic heart disease |
| EGF receptor | Cancer |
| Vascular endothelial growth factor receptor | Arthritis |
| VEGF receptor | Cancer |
| Aldosterone receptor | Cardiovascular heart disease |
| Somatostatin receptor | Grave's disease |
| Fibronectin receptor | Ullrich's disease |
| Angiotensin receptor | Heart disease |
| SBR1 | HDL receptor/coronary heart disease |

Additional examples of cell surface associated or extracellular matrix targets for the subject adzymes include cellular adhesion molecules, such as selectins, integrins and other hemidesmosomal proteins, cadherins, laminins, CD44 isoforms, proteoglycans (such as syndecans), Ig superfamily (IgCAM) proteins, catenins (such as $\alpha$, $\beta$ and $\gamma$ catenins) and cadherins (such as E-cadherin or P-cadherin), galectins, collagens, elastins, fibrins, and the like.

In certain embodiments, the adzyme acts on a Cluster of Differentiation (CD) protein, such as CD1a, CD2 (LFA-2), CD3, CD4, CD5, CD6, CD7, CD8, CD9 (Motility-Related Protein-1), CD10 (CALLA), CD11b (Mac-1), CD11b, CD13, CD14, CD15, CD16, CD18 (b2), CD19, CD20, CD21, CD22 (BL-CAM), CD23, CD25 (Interleukin-2 Receptor), CD27, CD29 (b1), CD30, CD31 (PECAM-1), CD34 (Endothelial Cell Marker), CD35, CD37, CD38, CD39, CD40, CD40L (CD154), CD41 (GPIIb/IIIa), CD42b (GPIb), CD43, CD44 (H-CAM) , CD44 Variant 3, CD44 Variant 4 , CD44 Variant 5 , CD44 Variant 6 , CD45 (Leucocyte Common Antigen) , CD45RA , CD45RB, CD45RO , CD48, CD49b (VLA-2) , CD49c (VLA-3) , CD49f (VLA-6) , CD50 (ICAM-3), CD51, CD54 (ICAM-1) , CD56 (NCAM) , CD57 , CD58 (LFA-3) , CD61 (GPIIIa) , CD61 (GPIIIa) , CD62E (E-selectin), CD62L (L-selectin) , CD62P (P-selectin) , CD63 (Melanoma Marker), CD66a (CEACAM1) , CD66e (Carcinoembryonic Antigen) , CD68 , CD69, CD71 (Transferrin Receptor) , CD72, CD74 , CDw75 , CD79a , CD81 , CD82, CD83 , CD95 (Fas) , CD99 (MIC2) , CD104 , CD105 (Endoglin) , CD106 (VCAM-1) , CD117 (c-kit Oncoprotein), CD134 (OX40) , CD137 , CD138 (Syndecan-1), CD141 (Thrombomodulin) , CD141 (Thrombomodulin) , CD143 (ACE) , CD146 (MCAM) , CD147 (EMMPRIN) , CDw150 (SLAM) , CD151 (PETA-3) , CD154 (CD40L) , CD162 , CD163 , CD166 (ALCAM), CD168 (RHAMM) , or CD179a.

In certain preferred embodiments, the adzyme substrate is a selectin, e.g., a CD62 family protein. In other preferred embodiments, the adzyme substrate is an immunoglobulin superfamily protein (IgCAM), such as a CD2 family protein, CD22, CD31, CD48, CD50, CD54, CD56, CD58, CD66a, CD83, CD106, CD146, CD147, CDw150 or CD166. In still other preferred embodiments, the adzyme substrate is an integrin, such as CD49 family, CD51, CD29, CD11b, CD18, CD41, CD61 or CD104.

Certain of the subject adzymes can be used to alter the activity of scavenger receptor class A (SR-A, CD204), scavenger receptor-BI (SR-BI) or CD36, which are cell surface proteins that mediate cell adhesion to, and endocytosis of, various native and pathologically modified substances, and participate in intracellular signaling, lipid metabolism, and host defense against bacterial pathogens.

Collagenolytic adzymes can be prepared, e.g., using collagenase catalytic domains from hydrobionts, polycollagenase-K or Fermenkol, to cause deep hydrolysis of polypeptide substrates (native or partially denatured collagen types, elastin, fibrin, hemoglobin, and casein). Such adzymes have use in both medical and cosmetological applications.

In certain embodiments, a ligand (or binding portion thereof) of a receptor or other cell surface molecule may be employed as an address moiety. In certain embodiments, the adzyme can be associated with one or more ligands effective to bind to specific cell surface proteins or matrix on the target cell, thereby facilitating sequestration of the adzyme to target cells. For instance, the adzyme can be a fusion protein that also includes the ligand. Merely to illustrate, examples of ligands suitable for use in targeting the adzymes of the present invention to specific cell types are listed in the Table V below.

**Table V. Adzymes Specific for Various Cell Types**

| *Ligand* | *Receptor* | *Cell type* |
|---|---|---|
| folate | folate receptor | epithelial carcinomas, bone marrow stem cells |
| water soluble vitamins | vitamin receptor | various cells |
| pyridoxyl phosphate | CD4 | CD4 + lymphocytes |
| apolipoproteins | LDL | liver hepatocytes, vascular endothelial cells |
| insulin | insulin receptor | |
| transferrin | transferrin receptor | endothelial cells |
| galactose | asialoglycoprotein receptor | liver hepatocytes |
| sialyl-Lewis$_x$ | E, P selectin | activated endothelial cells |
| Mac-1 | L selectin | neutrophils, leukocytes |
| VEGF | Flk-1,2 | tumor epithelial cells |
| basic FGF | FGF receptor | tumor epithelial cells |
| EGF | EGF receptor | epithelial cells |
| VCAM-1 | $a_4b_1$ integrin | vascular endothelial cells |
| ICAM-1 | $a_Lb_2$ integrin | vascular endothelial cells |
| PECAM-1/CD31 | $a_vb_3$ integrin | vascular endothelial cells, activated platelets |
| osteopontin | $a_vb_1$ integrin $a_vb_5$ integrin | endothelial cells and smooth muscle cells in atherosclerotic plaques |
| RGD sequences | $a_vb_3$ integrin | tumor endothelial cells, vascular smooth muscle cells |
| HIV GP 120/41 or GP120 | CD4 | CD4$^+$ lymphocytes |

In certain embodiments of adzymes intended to be antagonists of a receptor ligand, the adzyme will alter the receptor in a manner that reduces the level of ligand-induced signal transduction, but will not substantially impair the ability of the receptor to bind to its cognate ligand. In this manner, the adzyme antagonizes the ligand not only as a consequence to the generation of loss-of-function receptors with regard to signal transduction, but also because the otherwise inactivated receptor can act as a competitive binding agent for sequestering the ligand from still functional receptors. Alternatively, the adzyme can be selected to generate a receptor product which is constitutively active, e.g., in which case the adzyme acts may as an agonist of the receptor's inductive ligand.

In certain embodiments, the intended substrate of the adzyme will be a heteromeric receptor complex, e.g., receptor complexes involving two or more different receptor subunits. For instance, receptors for most interleukins and cytokines that regulate immune and hematopoietic systems belong to the class I cytokine receptor family. These molecules form multichain receptor complexes in order to exhibit high-affinity binding to, and mediate biological functions of, their respective cytokines. In most cases, these functional receptor complexes share common signal transducing receptor components that are also in the class I cytokine receptor family, such as the gp130 protein. Adzymes which are specifically reactive with the unique receptor subunit(s), but which do not substantially impair the function of.the common subunit, can be used to enhance the selectivity of the adzyme as an antagonist of a particular ligand.

Alternatively, adzymes that selectively inactivate the unique receptor subunits of other ligand-receptor complexes, e.g., those that compete with the formation of receptor complexes for the ligand of interest, can be agonists of ligand of interest.

In still other embodiments, an adzyme is targeted to an extracellular molecule that is part of a biomolecular accretion. A biomolecular accretion is any undesirable assemblage of biomolecules, usually one that brings together components that are not typically found in an assemblage together usually one that has grown over time by the successive addition of material. Accretions are generally large enough as to be non-diffusible (although clots are accretions that may diffuse in the circulatory system) and are generally larger than the size of a typical host cell. Biomolecular accretions will often contain dead and living cells as well as extracellular matrix. Examples of biomolecular accretions include amyloid deposits, *e.g.,* a β-amyloid peptide deposit characteristic of Alzheimer's disease or a type II diabetes amyloid deposit, a collagen deposit, a protein deposit, an atherosclerotic plaque, an undesirable fat mass, an undesirable bone mass, a blood clot, or a cyst. In certain embodiments, an adzyme is designed to target one or more extracellular molecules of a biomolecular accretion and act on such targets in such a way as to cause the partial or complete dissolution of the accretion. Examples of proteins that are often present in the amyloid deposits associated with Alzheimer's disease include amyloid β-peptide [Aβ(1-42)] and transthyretin. Protein aggregation has been linked to several human diseases, including Alzheimer's disease, Parkinson's disease, and systemic amyloidosis. Most of these diseases are associated with the formation of highly ordered and beta-sheet-rich aggregates referred to as amyloid fibrils. Fibril formation by WT transthyretin (TTR) or TTR variants has been linked to systemic amyloidosis and familial amyloid polyneuropathy, respectively. Amyloid fibril formation by $\alpha$-synuclein ($\alpha$-syn) has been linked to neurodegeneration in Parkinson's disease. Atherosclerotic plaque may contain a variety of different components. Examples of certain components include: calcified substances (e.g., hydroxyapatite), cholesterol crystals, collagen matrix, macrophage foam cells, smooth muscle cells, lipid-rich atheromatous material (particularly rich in cholesterol and esters thereof), mast cells, matrix metalloproteinases (e.g., MMP-1 collagenase, MMP-2 and -9 gelatinases). Given that atherosclerotic plaque rupture is associated with dangerous thrombotic events, it may be desirable to design an adzyme that stabilizes plaques (e.g. by targeting metalloproteinases in the plaque) or to employ a plaque-dissolving adzyme in combination with an anti-thrombotic agent, such as heparin.

Often, a biomolecular accretion combines various biomolecules that have appropriate roles in other parts of an organism; accordingly, it may be desirable to selectively target molecules that are primarily present in the accretion or to provide an adzyme with multiple different address moieties that enhance adzyme concentration in the vicinity of the accretion.

*(b) Intracellular Targets*

In certain embodiments, an adzyme may be directed against an intracellular target. Examples of intracellular targets include intracellular receptors (e.g., many steroid hormone receptors), enzymes that are overexpressed or otherwise participate in an undesirable condition, intracellular signaling proteins that participate in an undesirable condition (e.g., oncoproteins, pro-inflammatory proteins) and transcription factors.

In an exemplary embodiment, the adzyme alters a nuclear receptor. Many nuclear receptors may be viewed as ligand-dependent transcription factors. These receptors provide a direct link between extracellular signals, mainly hormones, and transcriptional responses. Their transcriptional activation function is regulated by endogenous small molecules, such as steroid hormones, vitamin D, ecdysone, retinoic acids and thyroid hormones, which pass readily through the plasma membrane and bind their receptors inside the cell. The subject adzymes can be used, for example, to alter the responsiveness of a cell to a particular hormone or other nuclear receptor ligand, such as by degrading receptor complexes to inhibit response to a hormone of interest, or degrading subunits for other receptor dimers that otherwise compete with the formation of receptor complexes for the hormone of interest (such that the adzyme is an agonist of that hormone).

Examples of certain intracellular targets are provided in Table VI, along with associated conditions that may be treated with an appropriately targeted adzyme.

**Table VI: Examples of Intracellular Targets**

| Target | Disease/Condition |
|---|---|
| aldosterone receptor | Cardiovascular heart disease |
| Erythropoietin | benign erythrocytosis |
| PPARγ | hepatogastroenterological diseases |
| Adrenocorticotropic Hormone | Cushing's disease |
| Huntingtin protein | Huntington's disease |
| estrogen receptor | Coronary heart disease, Liver disease |
| glucose-6-phosphatase | Glycogen storage disease type 1 |
| erythrocyte antioxidant enzyme | Behcet's disease |
| androgen receptor | Paget's disease |
| platelet-derived endothelial cell growth factor/thymidine phosphorylase (PD-ECGF/TP) | Paget's disease |
| Rb | Cancer |
| P16 | Cancer |
| P21 | Cancer |
| P53 | Cancer |
| HIF-1 | Cancer |
| NF-κB | Inflammatory disease |
| NF-κB | Cell Death |
| IκB | Immune response |

In embodiments involving an intracellular target, it will generally be desirable to have an adzyme that is produced within cells or designed for entry into cells. In certain embodiments, the adzyme may include one or more functionalities that promote uptake by target cells, e.g., promote the initial step of uptake from the extracellular environment. In one embodiment, a subject adzyme includes an "internalizing peptide" which drives the translocation of the adzyme across a cell membrane in order to facilitate intracellular localization. The internalizing peptide, by itself, is capable of crossing a cellular membrane by, e.g., transcytosis, at a relatively high rate. The internalizing peptide is conjugated, e.g., to an adzyme. In certain embodiments, the adzyme may be expressed from a nucleic acid that is introduced into a cell, such as a viral vector or naked or encapsulated nucleic acid vector. Nucleic acids for the intracellular productions of adzymes are described in the section entitled *"Nucleic acid compositions",* below.

In one embodiment, an internalizing peptide is derived from the *Drosophila* antepennepedia protein, or homologs thereof. The 60 amino acid long homeodomain of the homeo-protein antepennepedia has been demonstrated to translocate through biological membranes and can facilitate the translocation of heterologous peptides and organic compounds to which it is couples. See for example Derossi et al. (1994) J Biol Chem 269:10444-10450; and Perez et al. (1992) J Cell Sci 102:717-722. Recently, it has been demonstrated that fragments as small as 16 amino acids long of this protein are sufficient to drive internalization. See Derossi et al. (1996) J Biol Chem 271:18188-18193. The present invention contemplates an adzyme including at least a portion of the antepennepedia protein (or homolog thereof) sufficient to increase the transmembrane transport.

Another example of an internalizing peptide is the HIV transactivator (TAT) protein. This protein appears to be divided into four domains (Kuppuswamy et al. (1989) Nucl. Acids Res. 17:3551-3561). Purified TAT protein is taken up by cells in tissue culture (Frankel and Pabo, (1989) Cell 55:1189-1193), and peptides, such as the fragment corresponding to residues 37 -62 of TAT, are rapidly taken up by cell *in vitro* (Green and Loewenstein, (1989) Cell 55:1179-1188). The highly basic region mediates internalization and targeting of the internalizing moiety to the nucleus (Ruben et al., (1989) J. Virol. 63:1-8). Peptides or analogs that include a sequence present in the highly basic region, such as CFITKALGISYGRKKRRQRRRPPQGS (SEQ ID NO: 7), can be used in the adzyme to aid in internalization.

Another exemplary adzyme can be generated to include a sufficient portion of mastoparan (T. Higashijima et al., (1990) J. Biol. Chem. 265:14176) to increase the transmembrane transport of the adzyme.

While not wishing to be bound by any particular theory, it is noted that hydrophilic polypeptides and organic molecules may be also be physiologically transported across the membrane barriers by coupling or conjugating the polypeptide to a transportable peptide which is capable of crossing the membrane by receptor-mediated transcytosis. Suitable internalizing peptides of this type can be generated using all or a portion of, e.g., a histone, insulin, transferrin, basic albumin, prolactin and insulin-like growth factor I (IGF-I), insulin-like growth factor II (IGF-II) or other growth factors. For instance, it has been found that an insulin fragment, showing affinity for the insulin receptor on capillary cells, and being less effective than insulin in blood sugar reduction, is capable of transmembrane transport by receptor-mediated transcytosis and can therefore serve as an internalizing peptide for the subject adzyme. Preferred growth factor-derived internalizing peptides include EGF (epidermal growth factor)-derived peptides, such as CMHIESLDSYTC (SEQ ID NO: 8) and CMYIEALDKYAC (SEQ ID NO: 9); TGF-beta (transforming growth factor beta)-derived peptides; peptides derived from PDGF (platelet-derived growth factor) or PDGF-2; peptides derived from IGF-I (insulin-like growth factor) or IGF-II; and FGF (fibroblast growth factor)-derived peptides.

Another class of translocating/internalizing peptides exhibits pH-dependent membrane binding. For an internalizing peptide that assumes a helical conformation at an acidic pH, the internalizing peptide acquires the property of amphiphilicity, e.g., it has both hydrophobic and hydrophilic interfaces. More specifically, within a pH range of approximately 5.0-5.5, an internalizing peptide forms an alpha-helical, amphiphilic structure that facilitates insertion of the moiety into a target membrane. An alpha-helix-inducing acidic pH environment may be found, for example, in the low pH environment present within cellular endosomes. Such internalizing peptides can be used to facilitate transport of the subject adzyme, taken up by an endocytic mechanism, from endosomal compartments to the cytoplasm.

A preferred pH-dependent membrane-binding internalizing peptide includes a high percentage of helix-forming residues, such as glutamate, methionine, alanine and leucine. In addition, a preferred internalizing peptide sequence includes ionizable residues having pKa's within the range of pH 5-7, so that a sufficient uncharged membrane-binding domain will be present within the peptide at pH 5 to allow insertion into the target cell membrane.

A particularly preferred pH-dependent membrane-binding internalizing peptide in this regard is Xaal-Xaa2-Xaa3-EAALA(EALA)4-EALEALAA-amide (SEQ ID NO: 10), which represents a modification of the peptide sequence of Subbarao et al. (Biochemistry 26:2964, 1987). Within this peptide sequence, the first amino acid residue (Xaa1) is preferably a unique residue, such as cysteine or lysine, that facilitates chemical conjugation of the internalizing peptide to a targeting protein conjugate. Amino acid residues Xaa2-Xaa3 may be selected to modulate the affinity of the internalizing peptide for different membranes. For instance, if both residues 2 and 3 are lys or arg, the internalizing peptide will have the capacity to bind to membranes or patches of lipids having a negative surface charge. If residues 2-3 are neutral amino acids, the internalizing peptide will insert into neutral membranes.

Yet other preferred internalizing peptides include peptides of apo-lipoprotein A-1 and B; peptide toxins, such as melittin, bombolittin, delta hemolysin and the pardaxins; antibiotic peptides, such as alamethicin; peptide hormones, such as calcitonin, corticotrophin releasing factor, beta endorphin, glucagon, parathyroid hormone, pancreatic polypeptide; and peptides corresponding to signal sequences of numerous secreted proteins. In addition, exemplary internalizing peptides may be modified through attachment of substituents that enhance the alpha-helical character of the internalizing peptide at acidic pH.

Pore-forming proteins or peptides may also serve as internalizing peptides herein. Pore forming proteins or peptides may be obtained or derived from, for example, C9 complement protein, cytolytic T-cell molecules or NK-cell molecules. These moieties are capable of forming ring-like structures in membranes, thereby allowing transport of attached adzyme through the membrane and into the cell interior.

*(c) Infective or Foreign Targets*

An additional category of targets for an adzyme are targets that are associated with an infective or otherwise undesirable foreign agent, such as protists, yeasts, bacteria, viruses and prions and various complexes. In certain embodiments, an adzyme is targeted against a virulence factor that is exposed on the surface of a bacterium, such as a pilin or other adhesive protein, a flagellin, or other motility protein, a protein that facilitates bacterial cell entry into the host cell cytoplasm. In certain embodiments, an adzyme is targeted so as to disrupt a structural component of a bacterial cell wall or membrane, sufficient to cause cell lysis. In certain embodiments, an adzyme is targeted against a protein or other component of a virus that is required for viral particle viability or entry into a host cell, e.g., a protein of a viral coat or envelope. In another example, an adzyme may be targeted against a toxin, a venom, an undesirable foreign chemical or a heavy metal.

*(d) Molecules targeted by Developed Therapeutic Agents*

One novel approach to designing effective adzymes is to identify molecules that are targeted by therapeutically active agents that act by binding to the targeted molecules, such as monoclonal antibodies and soluble receptor portions. In a preferred embodiment, the target molecule is a target for a FDA-approved, commercially available therapeutic binding agent. It is expected that a molecule which can be effectively targeted by a binding agent may also be targeted by an adzyme that provides increased effectiveness relative to the binding agent.

In certain embodiments, the adzyme is an antagonist of CD52. Such adzymes can be used as part of a treatment for B cell chronic lymphocytic lymphoma (CLL). CD52 is a 21-28 kD glycoprotein expressed on the surface of normal and malignant B and T lymphocytes, NK cells, monocytes, macrophages, and tissues of the male reproductive system. Campath® (Alemtuzumab) is a recombinant DNA-derived humanized CD52 monoclonal antibody (Campath-1H). One problem associated with the use of Campath is hematologic toxicity, which tend to occur when single doses of greater than 30 mg or cumulative doses greater than 90 mg per week are administered. Thus the subject adzyme, which can be administered at a much lower dose because of its catalytic nature, is expected to be a better therapeutic alternative. The adzyme address domain may use the same monoclonal antibody or functional derivative thereof (such as a scFv derivative as in the instant application), as is generally the case in adzyme treatment of other diseases described below. A panel of proteases that are capable of efficiently digesting CD52 may be used as the catalytic domain.

In certain embodiments, the adzyme is an antagonist of TNF-alpha. Such adzymes can be used as part of a treatment for Rheumatoid arthritis, inflammatory bowel disease (IBD), including Crohn's disease and and ulcerative colitis. Human TNF-alpha is a nonglycosylated protein of 17 kDa, while murine TNF-alpha is N-glycosylated. TNF-alpha shows a wide spectrum of biological activities, and is found to be the important part of the whole IBD problem. Enbrel (etanercept; Immunex) and Remicade (infliximab; Centocor) are TNF-alpha antibodies that are used for severe cases of Rheumatoid arthritis and Crohn disease. The two drugs are very similar in mechanism, as is Humira (adalimumab; Abbott), a very recently approved TNF antibody which is much more faithful to human antibody structure. The subject adzyme, which can be administered at a much lower dose because of its catalytic nature, is expected to be a better therapeutic alternative. The adzyme address domain may use the same monoclonal antibody or functional derivative thereof (such as a scFv derivative as in the instant application). A panel of proteases that are capable of efficiently digesting TNF-alpha may be used as the catalytic domain.

In certain embodiments, the adzyme is an antagonist of the HER2/neu receptor. Such adzymes can be used as part of a treatment for metastatic breast cancer and/or recurrent or refractory ovarian or primary peritoneal carcinoma with overexpression of HER2. The HER2 (or c-erbB2) proto-oncogene encodes a transmembrane receptor protein of 185 kDa, which is structurally related to the epidermal growth factor receptor 1 (EGFR1). HER2 protein overexpression is observed in 25%-30% of primary breast cancers. HERCEPTIN (Trastuzumab) is a recombinant DNA-derived humanized monoclonal antibody that selectively binds with high affinity in a cell-based assay ($K_d$ = 5 nM) to the extracellular domain of HER2. The antibody is a humanized murine IgG1 kappa. One problem associated with the use of HERCEPTIN administration is severe hypersensitivity reactions (including anaphylaxis), infusion reactions, and pulmonary events. Thus the subject adzyme, which can be administered at a much lower dose because of its catalytic nature, is expected to be a better therapeutic alternative. The adzyme address domain may use the same monoclonal antibody or functional derivative thereof (such as a scFv derivative as in the instant application). A panel of proteases that are capable of efficiently digesting HER2 may be used as the catalytic domain.

In certain embodiments, the adzyme is an antagonist of CD33. Such adzymes can be used as part of a treatment for Acute myeloid leukemia (AML), the most common type of acute leukemia in adults. CD33 antigen is a sialic acid-dependent adhesion protein found on the surface of leukemic blasts and immature normal cells of myelomonocytic lineage, but not on normal hematopoietic stem cells. "Mylotarg" (gemtuzumab ozogamicin for Injection) is a chemotherapy agent composed of a recombinant humanized IgG4, kappa antibody conjugated with a cytotoxic antitumor antibiotic, calicheamicin, isolated from fermentation of a bacterium, Micromonospora echinospora ssp. calichensis . The antibody portion of Mylotarg binds specifically to the CD33 antigen. Side effects associated with the use of Mylotarg includes hypersensitivity reactions, including anaphylaxis, infusion reactions, pulmonary events, and hepatotoxicity. Thus the subject adzyme, which can be administered at a much lower dose because of its catalytic nature, is expected to be a better therapeutic alternative. The adzyme address domain may use the same monoclonal antibody or functional derivative thereof (such as a scFv derivative as in the instant application). A panel of proteases that are capable of efficiently digesting CD33 may be used as the catalytic domain.

In certain embodiments, the adzyme is an antagonist of CD3. Such adzymes can be used as part of a treatment for transplant rejection, such as acute renal, steroid-resistant cardiac, or steroid-resistant hepatic allograft rejection. OKT3 (or "muromonab-CD3") is a murine monoclonal antibody to the CD3 antigen of human T cells which functions as an immunosuppressant. The antibody is a biochemically purified IgG2a immunoglobulin. It is directed to the CD3 glycoprotein in the human T cell surface which is essential for T cell functions. Modulated cells, which reversibly lose the expression of the CD3 T cell receptor molecular complex but still share the CD4 and CD8 antigens, have been shown to be functionally immunoincompetent. Thus the subject adzyme, which can be administered at a much lower dose because of its catalytic nature, is expected to be a better therapeutic alternative. The adzyme address domain may use the same monoclonal antibody or functional derivative thereof (such as a scFv derivative as in the instant application). A panel of proteases that are capable of efficiently digesting CD3 may be used as the catalytic domain.

In certain embodiments, the adzyme is an antagonist of gpIIb/IIIa. Such adzymes can be used as part of a treatment for Acute myocardial infarction/unstable angina. Abciximab (ReoPro®), is the Fab fragment of the chimeric human-murine monoclonal antibody 7E3. Abciximab binds to the glycoprotein (GP) IIb/IIIa ($\alpha_{IIb}\beta_3$) receptor of human platelets and inhibits platelet aggregation. Abciximab also binds to the vitronectin ($\alpha_v\beta_3$) receptor found on platelets and vessel wall endothelial and smooth muscle cells. The subject adzyme, which can be administered at a much lower dose because of its catalytic nature, is expected to be a better therapeutic alternative. The adzyme address domain may use the same monoclonal antibody or functional derivative thereof (such as a Fab or scFv derivative as in the instant application). A panel of proteases that are capable of efficiently digesting gpIIb/IIIa may be used as the catalytic domain.

In certain embodiments, the adzyme is an antagonist of CD20. Such adzymes can be used as part of a treatment for Non-Hodgkin's lymphoma (NHL), such as CD20 positive, follicular, Non-Hodgkin's lymphoma. The CD20 antigen is found on the surface of normal and malignant B lymphocytes. The RITUXAN® (Rituximab) antibody is a genetically engineered chimeric murine/human monoclonal antibody directed against the CD20 antigen found on the surface of normal and malignant B lymphocytes. The antibody is an IgG1 kappa immunoglobulin containing murine light- and heavy-chain variable region sequences and human constant region sequences. Rituximab has a binding affinity for the CD20 antigen of approximately 8.0 nM. A second approved drug, ZEVALIN (Ibritumomab Tiuxetan), is the immunoconjugate resulting from a stable thiourea covalent bond between the monoclonal antibody Ibritumomab and the linker-chelator tiuxetan [N-[2-bis(carboxymethyl)amino]-3-(p-isothiocyanatophenyl)-propyl]-[N-[2-bis(carboxymethyl)amino]-2-(methyl)-ethyl]glycine. This linker-chelator provides a high affinity, conformationally restricted chelation site for Indium-111 or Yttrium-90. The antibody moiety of ZEVALIN is Ibritumomab, a murine IgG1 kappa monoclonal antibody directed against the CD20 antigen. A third drug, Bexxar (tositumomab and iodine-131 tositumomab), is another approved drug for the treatment of patients with CD20 positive, follicular, Non-Hodgkin's lymphoma, with and without transformation, whose disease is refractory to Rituxan and has relapsed following chemotherapy. The subject adzyme, which can be administered at a much lower dose because of its catalytic nature, is expected to be a better therapeutic alternative. The adzyme address domain may use the same monoclonal antibodies or functional derivative thereof (such as a Fab or scFv derivative as in the instant application). A panel of proteases that are capable of efficiently digesting CD20 may be used as the catalytic domain.

In certain embodiments, the adzyme is an antagonist of RSV F Protein. Such adzymes can be used as part of a treatment for RSV infection. SYNAGIS® (PALIVIZUMAB) is a humanized monoclonal antibody (IgG1k) produced by recombinant DNA technology, directed to an epitope in the A antigenic site of the F protein of respiratory syncytial virus (RSV). Palivizumab is a composite of human (95%) and murine (5%) antibody sequences. The subject adzyme, which can be administered at a much lower dose because of its catalytic nature, is expected to be a better therapeutic alternative. The adzyme address domain may use the same monoclonal antibody or functional derivative thereof (such as a Fab or scFv derivative as in the instant application). A panel of proteases that are capable of efficiently digesting RSV F protein may be used as the catalytic domain.

In certain embodiments, the adzyme is an antagonist of CD25. Such adzymes can be used as part of a treatment for transplant rejection. Zenapax® (daclizumab) is an immunosuppressive, humanized IgG1 monoclonal antibody produced by recombinant DNA technology that binds specifically to the alpha subunit (p55 alpha, CD25, or Tac subunit) of the human high affinity IL-2 receptor that is expressed on the surface of activated (but not resting) lymphocytes. The drug binds to the high affinity IL-2 receptor, thus inhibiting the binding of Tac by IL-2, and the activation of lymphocytes. Therefore, the monoclonal antibody acts as a pure binder inhibitor. The subject adzyme, which can be administered at a much lower dose because of its catalytic nature, is expected to be a better therapeutic alternative. The adzyme address domain may use the same monoclonal antibody or functional derivative thereof (such as a Fab or scFv derivative as in the instant application). A panel of proteases that are capable of efficiently digesting CD25 may be used as the catalytic domain.

In certain embodiments, the adzyme is an antagonist of IL-1. Such adzymes can be used as part of a treatment for Rheumatoid arthritis. The pathogenesis of RA is a complex process that leads to significant and chronic joint inflammation. Interleukin-1 (IL-1) is a central mediator in RA and is a critical proinflammatory cytokine that has been found to be abundant in the synovial fluid of RA patients. Kineret® (anakinra) is a recombinant, nonglycosylated form of the human interleukin 1 receptor antagonist (IL-1Ra). Kineret® differs from native human IL-1Ra in that it has the addition of a single methionine residue at its amino terminus. Kineret® blocks the biologic activity of IL-1 by competitively inhibiting IL-1 binding to the interleukin-1 type I receptor (IL-1RI), which is expressed in a wide variety of tissues and organs. Therefore, Kineret® acts as a pure binder inhibitor. The subject adzyme, which can be administered at a much lower dose because of its catalytic nature, is expected to be a better therapeutic alternative. The adzyme address domain may use the same monoclonal antibody or functional derivative thereof (such as a Fab or scFv derivative as in the instant application). A panel of proteases that are capable of efficiently digesting IL-1 may be used as the catalytic domain.

In certain embodiments, IgE (immunoglobulin E) may be the target of an adzyme. IgE is a class of antibodies that protects the host against invading parasites. IgE interacts with mast cells and eosinophils to protect the host against the invading parasite. The IgE-immune cell complex is also responsible for many allergic or hypersensitivity reactions such as hay fever, asthma, hives and anaphylaxis. There are two major types of receptor for the Fc portion of the IgE on cells. A high affinity receptor is found primarily on mast cells and basophils. A low affinity receptor is found on CD23 cells. IgE attaches to these and acts as an antigen receptor. Xolair™ is a humanized monoclonal antibody directed to the Fc portion of IgE and effective in treating asthma. An adzyme targeted to and reducing the activity of IgEs (generally by targeting the Fc portion) may be used to treat asthma. The adzyme address domain may use a monoclonal antibody or functional derivative thereof (such as a scFv derivative as in the instant application), or a soluble ligand binding portion of an IgE receptor. One or more of a panel of proteases that are capable of efficiently digesting IgE may be used as the catalytic domain.

In certain embodiments, VEGF (Vascular Endothelial Growth Factor) may be the target of an adzyme. VEGF plays a critical role in angiogenesis (the formation of new blood vessels), particularly in tumors and is also involved in the maintenance of established tumor blood vessels. VEGF is homodimeric and disulfide linked. Four human splice variants of VEGF have been identified encoding, in the mature form, polypeptide monomers of 121, 165, 189, or 206 amino acids. Two receptor tyrosine kinases (RTKs), Flt-1 and Flk-1 bind VEGF with high affinity. Avastin™ is an investigational recombinant humanized antibody to VEGF, and shows effectiveness in improving the survival of metastatic colorectal cancer patients. An adzyme targeted to and reducing the activity of VEGF may be used to treat a variety of cancers, particularly colorectal cancer. The adzyme address domain may use a monoclonal antibody or functional derivative thereof (such as a scFv derivative as in the instant application), or a soluble ligand binding portion of a VEGF receptor. One or more of a panel of proteases that are capable of efficiently digesting VEGF may be used as the catalytic domain.

In certain embodiments, EGFR (Epidermal Growth Factor Receptor) may be the target for an adzyme. EGFR is expressed in a high percentage of many cancer types, including head and neck, colorectal, pancreatic, lung, esophageal, renal cell, prostate, bladder, cervical/uterus, ovarian and breast cancers. ERBITUX™ (formerly known as IMC-C225) is a highly specific chimerized monoclonal antibody that binds to EGFR and blocks the ability of EGF to initiate receptor activation and signaling to the tumor. This blockade results in an inhibition of tumor growth by interfering with the effects of EGFR activation including tumor invasion and metastases, cell repair and angiogenesis. ERBITUX™ has been used in combination with chemotherapy and radiation in animal models of human cancers. These preclinical findings indicate that when combined with chemotherapy or radiation, ERBITUX™ treatment provides an enhanced anti-tumor effect resulting in the elimination of tumors and the long-term survival of the animals. An adzyme targeted to and reducing the presence, ligand-binding or signaling capacity of EGFR may be used to treat or prevent a variety of cancers, particularly colorectal cancer, and particularly when used in combination with one or more additional chemotherapeutic agents. The adzyme address domain may use a monoclonal antibody or functional derivative thereof (such as a scFv derivative as in the instant application), or a soluble ligand (such as EGF) for EGFR. One or more of a panel of proteases that are capable of efficiently digesting extracellular portions of EGFR may be used as the catalytic domain.

In certain embodiments, one or more alpha-4 integrins, such as beta-1 and beta-7 may be the target(s) for an adzyme. Integrins are transmembrane proteins, and the alpha-4-beta 1 (VLA-4) and alpha-4-beta-7 integrins help white blood cells, particularly T lymphocytes and eosinophils, move from through the blood vessel walls into the tissues of the body at sites of inflammation, where these cells then participate in the inflammatory process. Antegren® is a humanized monoclonal antibody that binds to and blocks both the beta-1 and beta-7 integrins, preventing the contribution of many cell types to inflammation; Antegren® shows effectiveness for treatment of Crohn's disease. An adzyme targeted to and reducing the presence or ligand-binding capacity of these integrins may be used to treat or prevent a variety of inflammatory diseases, particularly Crohn's disease. The adzyme address domain may use a monoclonal antibody or functional derivative thereof (such as a scFv derivative as in the instant application), or a soluble ligand for the targeted alpha-4 integrins. One or more of a panel of proteases that are capable of efficiently digesting extracellular portions of the targeted integrins may be used as the catalytic domain.

In certain embodiments, CCR-5 may be the target of an adzyme. The human CCR5 chemokine receptor is a member of the rhodopsin superfamily of G-linked receptors having seven hydrophobic transmembrane domains. CCR5 binds RANTES, MIP-1β and MIP-1α. Raport, C.J. et al. (1996) J. Biol. Chem. 271:17161. CCR5 facilitates infection by the macrophage-tropic HIV-1 virus, RANTES, MIP-1α and MIP-1β can suppress the infection of susceptible cells by macrophage-tropic HIV-1 isolates. Choe, H. et al. (1996) Cell 85:1135. Cocchi, F. et al. (1995) Science 270:1811. Although no CCR-5 targeted affinity agent has been approved, CCR-5 is implicated in HIV infection, and an adzyme targeted to and reducing the presence or HIV-binding capacity of CCR-5 may be used to treat or prevent asthma and other allergic reactions. The adzyme address domain may use a monoclonal antibody or functional derivative thereof (such as a scFv derivative as in the instant application), or a soluble ligand for CCR-5. One or more of a panel of proteases that are capable of efficiently digesting extracellular portions of CCR-5 may be used as the catalytic domain.

In certain embodiments, interleukin-4 may be the target of an adzyme. Human IL-4 is a pleiotropic cytokine produced by activated T cells, mast cells, and basophils. The biological effects of IL-4 are mediated by the binding of IL-4 to specific cell surface receptors. The functional high-affinity receptor for IL-4 includes a ligand binding subunit (IL-4 R) and a second subunit (β chain) that can modulate the ligand binding affinity of the receptor complex. The gamma chain of the IL-2 receptor complex may also be a functional β chain of the IL-4 receptor complex. Mature IL-4 is a 129 amino acid protein Yokota, T. et al., 1986, Proc. Natl. Acad. Sci. USA 83:5894. IL-4 activity may be measured, for example, in a cell proliferation assay employing a human factor-dependent cell line, TF-1. Kitamura et al., 1989 J. Cell Physiol. 140:323. Although no IL-4 targeted affinity agent has been approved, IL-4 is implicated in allergies and asthma, and an adzyme targeted to and reducing the activity of IL-4 may be used to treat or prevent asthma and other allergic reactions. The adzyme address domain may use a monoclonal antibody or functional derivative thereof (such as a scFv derivative as in the instant application), or a soluble ligand binding portion of an IL-4 receptor. One or more of a panel of proteases that are capable of efficiently digesting IL-4 may be used as the catalytic domain.

In certain embodiments, IL-13 may be the target of an adzyme. Although no IL-13 targeted affinity agent has been approved, IL-13 is widely recognized as a cytokine that is involved in asthma and various allergies. Mature human IL-13 is a 112 amino acid polypeptide having a sequence as described in GenBank accession no. P35225. McKenzie et al. 1993, PNAS USA 90:3735-3739. IL-13 activity may be measured, for example, in a cell proliferation assay employing a human factor-dependent cell line, TF-1. Kitamura et al., 1989 J. Cell Physiol. 140:323. An adzyme targeted to and reducing the activity of IL-13 may be used to treat or prevent asthma and other allergic reactions. The adzyme address domain may use a monoclonal antibody or functional derivative thereof (such as a scFv derivative as in the instant application), or a soluble ligand binding portion of an IL-13 receptor. One or more of a panel of proteases that are capable of efficiently digesting IL-13 may be used as the catalytic domain.

*(i) TNFα Antagonists*

In certain embodiments, the subject adzyme is a TNFα antagonist, e.g., a "TNFα antagonist adzyme". TNFα is a soluble homotrimer of 17 kD protein subunits. A membrane-bound 26 kD precursor form of TNFα also exists. The pleiotropic activities of the potent proinflammatory cytokine TNF are mediated by two structurally related, but functionally distinct, receptors, p55 and p75, that are coexpressed on most cell types. To exert its biological activity, TNFα (a homotrimeric molecule) must bind to at least 2 cell surface receptors, causing cross-linking and cell signaling. The majority of biologic responses classically attributed to TNFα are mediated by p55. In contrast, p75 has been proposed to function as both a TNF antagonist by neutralizing TNFα and as a TNFα agonist by facilitating the interaction between TNFα and p55 at the cell surface. The roles of p55 and p75 in mediating and modulating the activity of TNFα in vivo have been examined in mice genetically deficient in these receptors. Selective deficits in several host defense and inflammatory responses are observed in mice lacking p55 or both p55 and p75, but not in mice lacking p75. In these models, the activity of p55 is not impaired by the absence of p75, arguing against a physiologic role for p75 as an essential element of p55-mediated signaling. In contrast, exacerbated pulmonary inflammation and dramatically increased endotoxin induced serum TNFα levels in mice lacking p75 suggest a dominant role for p75 in suppressing TNFα-mediated inflammatory responses.

The p55 receptor (also termed TNF-R55, TNF-RI, or TNFRα) is a 55 kd glycoprotein shown to transduce signals resulting in cytotoxic, antiviral, and proliferative activities of TNFα. The p75 receptor (also termed TNF-R75, TNF-RII, or TNFRα) is a 75 kDa glycoprotein that has also been shown to transduce cytotoxic and proliferative signals as well as signals resulting in the secretion of GM-CSF. The extracellular domains of the two receptors have 28% homology and have in common a set of four subdomains defined by numerous conserved cysteine residues. The p75 receptor differs, however, by having a region adjacent to the transmembrane domain that is rich in proline residues and contains sites for O-linked glycosylation. Interestingly, the cytoplasmic domains of the two receptors share no apparent homology which is consistent with observations that they can transduce different signals to the interior of the cell.

To further illustrate, a TNFα antagonist adzyme can be directed to TNFα, e.g., in biological fluids, by way of one or more TNFα targeting moieties. Exemplary TNFα targeting moieties include, but are not limited to, the extracellular domains of TNFα receptors (or appropriate portions thereof), anti-TNFα antibodies or antigen binding fragments thereof, or peptides or small molecules that (selectively) bind TNFα.

In certain preferred embodiments, the targeting moiety is derived from the extracellular ligand binding domain of the p75 or p55 receptor, e.g., a portion sufficient to specifically bind to TNF-α. For instance, the targeting moiety can include a ligand binding fragment of p75, such as from Leu23 - Asp257 of the human p75 protein (Swiss-Prot Accession P20333) or a ligand binding fragment of p55, such as from Ile22 - Thr211 of the human p55 protein (Swiss-Prot Accession P19438). In certain embodiments, the targeting moiety of the subject adzymes can be generated from Onercept (a fully human soluble fragment of p55) or Etanercept (Enbrel®, a dimeric construct in which two p75 extracellular fragments are linked to the Fc portion of human IgG1).

In other preferred embodiments, the targeting moiety is derived from an antibody that binds to TNFα, or an antigen binding domain thereof. For instance, the subject adzymes can generated using the monoclonal anti-TNFα antibody is infliximab (Remicade®), or the variable domains of one or both of the heavy and light chains thereof, such as the Fv fragment. Infliximab is a chimeric human/mouse monoclonal anti-TNFα antibody composed of the constant regions of human (Hu) IgG1κ, coupled to the Fv region of a high-affinity neutralizing murine anti-HuTNFa antibody. Likewise, the subject adzyme can including a targeting moiety derived from the human anti-TNF antibody D2E7, also known as adalumimab.

In still other embodiments, the TNFα targeting moiety is a peptide. For instance, Guo et al. (2002) Di Yi Jun Yi Da Xue Xue Bao. 22(7):597 describes the screening of TNFα-binding peptides by phage display. That reference teaches a number of short peptides that could be used to generate TNFα-targeted adzymes. Merely to illustrate, the TNFα targeting moiety can be a peptide having the sequence ALWHWWH (SEQ ID NO: 11) or (T/S)WLHWWA (SEQ ID NO: 12).

The ability of any particular adzyme to act alter the activity of TNFα can be assayed using any of a variety of cell-based and cell-free assay systems well known in the art. Exemplary assays include, but are not limited to, L929 assay, endothelial procoagulation assays, tumor fibrin deposition assays, cytotoxicity assay, tumor regression assays, receptor binding assays, arthritic index assays in mouse model systems, and the like. In certain preferred embodiments of TNFα antagonist adzymes, their biological activities will include one or more of: inhibition of TNF-α cytotoxicity in L929 cells; blocking of prostaglandin E2 production and expression of cell-associated IL1 by human dermal fibroblasts; blocking of TNF-α binding to the promonocytic cell line U937; blocking of TNF-α induced respiratory burst in human neutrophils; blocking of TNF-stimulated neutrophil lucigenin-dependent chemiluminescence response and superoxide formation; significantly reducing the priming ability of TNF-α for a response to the chemotactic peptide fMLP; blocking of class I antigen expression in the human Colo 205 tumor cell line; affecting TNF-α synergism with HLA-DR antigen expression induced by IFN-γ (yet preferably having no effect on IFN-γ activity).

In certain embodiments, the TNFα antagonist adzyme will modify the substrate TNFα protein in a manner that produces a product that is itself an antagonist of TNFα. For instance, the adzyme can include a catalytic domain that cleaves a site in the TNFα polypeptide to produce a product that retains the ability to bind, for example, to the p55 receptor but with a greatly reduced ability to activate the receptor (e.g., has an impaired ability to induce a cytotoxic response) so as to be an antagonist of native TNFα. For instance, the cleavage product may retain the ability to interact with native TNFα to form stable mixed trimers that bind to receptors but are incapable of activating receptor signaling. To further illustrate, the sites within the human TNF-α molecule that can be targeted for cleavage by an adzyme may be located at or near residues 29 to 34, 71-73, 86, and 143-146. For instance, a catalytic domain having a trypsin-like specificity can be used in an adzyme that selectively cleaves Arg$^{44}$ of human TNFα. Likewise, an adzyme including the catalytic domain of granzyme B can be used to target Asp$^{143}$. Residues within these regions are believed to be important for TNF-α cytotoxic activity. Adzyme cleavage products having the combination of antagonist activity and reduced cytotoxicity can be identified by the screening assays described above.

The subject TNFα antagonist adzymes can be used to treat various TNF-associated disorders, e.g., disorders or diseases that are associated with, result from, and/or occur in response to, elevated levels of TNFα. Such disorders may be associated with episodic or chronic elevated levels of TNFα activity and/or with local or systemic increases in TNFα activity. Such disorders include, but are not limited to, inflammatory diseases, such as arthritis and inflammatory bowel disease, and congestive heart failure.

TNFα causes pro-inflammatory actions which result in tissue injury, such as degradation of cartilage and bone, induction of adhesion molecules, inducing procoagulant activity on vascular endothelial cells, increasing the adherence of neutrophils and lymphocytes, and stimulating the release of platelet activating factor from macrophages, neutrophils and vascular endothelial cells. In certain preferred embodiments, the TNFα antagonist adzyme reduces the inflammatory activity of TNFα.

Recent evidence also associates TNFα with infections, immune disorders, neoplastic pathologies, autoimmune pathologies and graft-versus-host pathologies. For instance, TNFα is understood to play a central role in gram-negative sepsis and endotoxic shock, including fever, malaise, anorexia, and cachexia. Endotoxin strongly activates monocyte/ macrophage production and secretion of TNFα and other cytokines (Kombluth et al., J. Immunol. 137:2585-2591 (1986)). Circulating TNFα levels increase in patients suffering from gram-negative sepsis. Thus, the subject TNFα antagonist adzymes may used as part of a treatment protocol for inflammatory diseases, autoimmune diseases, viral, bacterial and parasitic infections, malignancies, and neurogenerative diseases, such as for therapy in rheumatoid arthritis and Crohn's disease.

There is evidence that TNFα is also involved in cachexia in cancer, infectious pathology, and other catabolic states. Accordingly, the TNFα antagonist adzymes can also be used to reduce muscle wasting associated with such disorders, or any other in which cachexia is an issue in patient management.

Accordingly, the present invention provides an adzyme of the present invention for use in methods in which the subject adzymes can be used as part of treatments for modulating or reducing the severity of at least one immune related disease, in a cell, tissue, organ, animal, or patient including, but not limited to, at least one of rheumatoid arthritis, juvenile rheumatoid arthritis, systemic onset juvenile rheumatoid arthritis, psoriatic arthritis, ankylosing spondilitis, gastric ulcer, seronegative arthropathics, osteoarthritis, inflammatory bowel disease, ulcerative colitis, systemic lupus erythematosis, antiphospholipid syndrome, iridocyclitis/uveitis/optic neuritis, idiopathic pulmonary fibrosis, systemic vasculitis/wegener's granulomatosis, sarcoidosis, orchitis/vasectomy reversal procedures, allergic/atopic diseases, asthma, allergic rhinitis, eczema, allergic contact dermatitis, allergic conjunctivitis, hypersensitivity pneumonitis, transplants, organ transplant rejection, graft-versus-host disease, systemic inflammatory response syndrome, sepsis syndrome, gram positive sepsis, gram negative sepsis, culture negative sepsis, fungal sepsis, neutropenic fever, urosepsis, meningococcemia, trauma/hemonhage, bums, ionizing radiation exposure, acute pancreatitis, adult respiratory distress syndrome, rheumatoid arthritis, alcohol-induced hepatitis, chronic inflammatory pathologies, sarcoidosis, Crohn's pathology, sickle cell anemia, diabetes, nephrosis, atopic diseases, hypersensity reactions, allergic rhinitis, hay fever, perermial rhinitis, conjunctivitis, endometriosis, asthma, urticaria, systemic anaphalaxis, dermatitis, pernicious anemia, hemolytic disesease, thrombocytopenia, graft rejection of any organ or tissue, kidney translplant rejection, heart transplant rejection, liver transplant rejection, pancreas transplant rejection, lung transplant rejection, bone marrow transplant (BMT) rejection, skin allograft rejection, cartilage transplant rejection, bone graft rejection, small bowel transplant rejection, fetal thymus implant rejection, parathyroid transplant rejection, xenograft rejection of any organ or tissue, allograft rejection, anti-receptor hypersensitivity reactions, Graves disease, Raynoud's disease, type B insulin-resistant diabetes, asthma, myasthenia gravis, antibody-meditated cytotoxicity, type III hypersensitivity reactions, systemic lupus erythematosus, POEMS syndrome (polyneuropathy, organomegaly, endocrinopathy, monoclonal gammopathy, and skin changes syndrome), polyneuropathy, organomegaly, endocrinopathy, monoclonal gammopathy, skin changes syndrome, antiphospholipid syndrome, pemphigus, scleroderma, mixed connective tissue disease, idiopathic Addison's disease, diabetes mellitus, chronic active hepatitis, primary billiary cirrhosis, vitiligo, vasculitis, post-MI cardiotomy syndrome, type IV hypersensitivity , contact dermatitis, hypersensitivity pneumonitis, allograft rejection, granulomas due to intracellular organisms, drug sensitivity, metabolic/idiopathic, Wilson's disease, hemachromatosis, alpha-1-antitrypsin deficiency, diabetic retinopathy; Hashimoto's thyroiditis, osteoporosis, hypothalamic-pituitary-adrenal axis evaluation, primary biliary cirrhosis, thyroiditis, encephalomyclitis, cachexia, cystic fibrosis, neonatal chronic lung disease, chronic obstructive pulmonary disease (COPD), familial hemaophagocytic lymphohistiocytosis, dermatologic conditions, psoriasis, alopecia, nephrotic syndrome, nephritis, glomerular nephritis, acute renal failure, hemodialysis, uremia, toxicity, preeclampsia, okt3 therapy, anti-cd3 therapy, cytokine therapy, chemotherapy, radiation therapy (e.g., including but not limited to asthenia, anemia, cachexia, and the like), chronic salicylate intoxication, and the like.

In one embodiment, a TNFα adzyme is for use to treat hypergastrinemia, such as Helicobacter Pylori-induced gastritis.

The present invention also provides the subject TNFα antagonist adzymes for use in modulating or treating at least one cardiovascular disease in a cell, tissue, organ, animal, or patient, including, but not limited to at least one of cardiac stun syndrome, myocardial infarction, congestive heart failure, stroke, ischemic stroke, hemorrhage, arteriosclerosis, atherosclerosis, restenosis, diabetic ateriosclerotic disease, hypertension, arterial hypertension, renovascular hypertension, syncope, shock, syphilis of the cardiovascular system, heart failure, cor pulmonale, primary pulmonary hypertension, cardiac arrhythmias, atrial ectopic beats, atrial flutter, atrial fibrillation (sustained or paroxysmal), post perfusion syndrome, cardiopulmonary bypass inflammation response, chaotic or multifocal atrial tachycardia, regular narrow QRS tachycardia, specific arrythmias, ventricular fibrillation, His bundle arrhythmias, atrioventricular block, bundle branch block, myocardial ischemic disorders, coronary artery disease, angina pectoris, myocardial infarction, cardiomyopathy, dilated congestive cardiomyopathy, restrictive cardiomyopathy, valvular heart diseases, endocarditis, pericardial disease, cardiac tumors, aordic and peripheral aneuryisms, aortic dissection, inflammation of the aorta, occulsion of the abdominal aorta and its branches, peripheral vascular disorders, occulsive arterial disorders, peripheral atherlosclerotic disease, thromboangitis obliterans, functional peripheral arterial disorders, Raynaud's phenomenon and disease, acrocyanosis, erythromelalgia, venous diseases, venous thrombosis, varicose veins, arteriovenous fistula, lymphedema, lipedema, unstable angina, reperfusion injury, post pump syndrome, ischemia-reperfusion injury, and the like.

The present invention also provides the subject TNFα antagonist adzymes for use in modulating or treating at least one infectious disease in a cell, tissue, organ, animal or patient, including, but not limited to, at least one of: acute or chronic bacterial infection, acute and chronic parasitic or infectious processes, including bacterial, viral and fungal infections, HIV infection/HIV neuropathy, meningitis, hepatitis (A, B or C, or the like), septic arthritis, peritonitis, pneumonia, epiglottitis, B. coli infection, hemolytic uremic syndrome/thrombolytic thrombocytopenic purpura, malaria, dengue hemorrhagic fever, leishmaniasis, leprosy, toxic shock syndrome, streptococcal myositis, gas gangrene, mycobacterium tuberculosis, mycobacterium avium intracellulare, pneumocystis carinii pneumonia, pelvic inflammatory disease, orchitis/epidydimitis, legionella, lyme disease, influenza a, epstein-barr virus, vital-associated hemaphagocytic syndrome, vital encephalitis/aseptic meningitis, and the like.

The present invention also provides the adzymes of the invention for use in modulating or treating at least one malignant disease in a. cell, tissue, organ, animal or patient, including, but not limited to, at least one of: leukemia, acute leukemia, acute lymphoblastic leukemia (ALL), B-cell, T-cell or FAB ALL, acute myeloid leukemia (AML), chromic myelocytic leukemia (CML), chronic lymphocytic leukemia (CLL), hairy cell leukemia, myelodyplastic syndrome (MDS), a lymphoma, Hodgkin's disease, a malignamt lymphoma, non-hodgkin's lymphoma, Burkitt's lymphoma, multiple myeloma, Kaposi's sarcoma, colorectal carcinoma, pancreatic carcinoma, nasopharyngeal carcinoma, malignant histiocytosis, paraneoplastic syndrome/hypercalcemia of malignancy, solid tumors, adenocarcinomas, sarcomas, malignant melanoma, hemangioma, metastatic disease, cancer related bone resorption, cancer related bone pain, and the like.

The present invention also provides TNFα antagonist adzymes for use in modulating or treating at least one neurologic disease in a cell, tissue, organ, animal or patient, including, but not limited to, at least one of: neurodegenerative diseases, multiple sclerosis, migraine headache, AIDS dementia complex, demylelinating diseases, such as multiple sclerosis and acute transverse myelitis; extrapyramidal and cerebellar disorders' such as lesions of the corticospinal system; disorders of the basal ganglia or cerebellar disorders; hyperkinetic movement disorders such as Huntington's Chorea and senile chorea; drug-induced movement disorders, such as those induced by drugs which block CNS dopamine receptors; hypokinetic movement disorders, such as Parkinson's disease; Progressive supranucleo Palsy; structural lesions of the cerebellum; spinocerebellar degenerations, such as spinal ataxia, Friedreich's ataxia, cerebellar cortical degenerations, multiple systems degenerations (Mencel, Dejerine-Thomas, Shi-Drager, and Machado-Joseph); systemic disorders (Refsum's disease, abetalipoprotemia, ataxia, telangiectasia, and mitochondrial multi.system disorder); demyelinating core disorders, such as multiple sclerosis, acute transverse myelitis; and disorders of the motor unit' such as neurogenic muscular atrophies (anterior hom cell degeneration, such as amyotrophic lateral sclerosis, infantile spinal muscular atrophy and juvenile spinal muscular atrophy); Alzheimer's disease; Down's Syndrome in middle age; Diffuse Lewy body disease; Senile Dementia of Lewy body type; Wemicke-Korsakoff syndrome; chronic alcoholism; Creutzfeldt-Jakob disease; Subacute sclerosing panencephalitis, Hallerrorden-Spatz disease; and Dementia pugilistica, and the like.

The TNFα antagonist adzymes can be administered before, concurrently, and/or after (referred to herein as "concomitantly with") other drugs, such as at least one selected from an antirheumatic (e.g., methotrexate, auranofin, aurothioglucose, azathioprine, etanercept, gold sodium thiomalate, hydroxychloroquine sulfate, leflunomide, sulfasalzine), a muscle relaxant, a narcotic, a non-steroid anti-inflammatory drug (NSAID), an analgesic, an anesthetic, a sedative, a local anethetic, a neuromuscular blocker, an antimicrobial (e.g., aminoglycoside, an antifungal, an antiparasitic, an antiviral, a carbapenem, cephalosporin, a flurorquinolone, a macrolide, a penicillin, a sulfonamide, a tetracycline, another antimicrobial), an antipsoriatic, a corticosteriod, an anabolic steroid, a diabetes related agent, a mineral, a nutritional, a thyroid agent, a vitamin, a calcium related hormone, an antidiarrheal, an antitussive, an antiemetic, an antiulcer, a laxative, an anticoagulant, an erythropieitin (e.g., epoetin alpha), a filgrastim (e.g., G-CSF, Neupogen), a sargramostim (GM-CSF, Leukine), an immunization, an immunoglobulin, an immunosuppressive (e.g., basiliximab, cyclosporine, daclizumab), a growth hormone, a hormone replacement drug, an estrogen receptor modulator, a mydriatic, a cycloplegic, an alkylating agent, an antimetabolite, a mitotic inhibitor, a radiopharmaceutical, an antidepressant, antimanic agent, an antipsychotic, an anxiolytic, a hypnotic, a sympathomimetic, a stimulant, donepezil, tacrine, an asthma medication, a beta agonist, an inhaled steroid, a leukotriene inhibitor, a methylxanthine, a cromolyn, an epinephrine or analog, domase alpha (Pulmozyme), a cytokine or a cytokine antagonist.

The subject adzymes can also be administered concomitantly with compounds that prevent and/or inhibit TNF receptor signaling, such as mitogen activated protein (MAP) kinase inhibitors; compounds which block and/or inhibit membrane TNF cleavage, such as metalloproteinase inhibitors; compounds which block and/or inhibit TNF activity, such as angiotensin converting enzyme (ACE) inhibitors (e.g., captopril); and compounds which block and/or inhibit TNF production and/or synthesis, such as MAP kinase inhibitors.

*(ii) IL-1b antagonists*

In certain embodiments, the subject adzyme is an Interleukin-1 antagonist, e.g., an "IL-1 antagonist adzyme". Interleukin-1 is a multi-functional proinflammatory cytokine that mediates innate and adaptive immune responses in multiple cell types. It is believed to play a role in numerous diseases including arthritis, asthma/allergy, osteoporosis, and stroke (for review, see Dinarello (1998) Int. Rev. Immunol. 16, 457-499). The IL-1 family actually consists of two proteins with similar biological activity, IL-1α and IL-1β, as well as a nonsignaling ligand termed the IL-1 receptor antagonist (IL-1ra). All three proteins exhibit a similar tertiary structure comprised of 12β strands that make up a barrel-shaped β-trefoil with pseudo-3-fold symmetry. IL-1 β is thought to be the primary circulating cytokine that mediates the systemic effects of IL-1.

IL-1 exerts its biological action by binding and activating the membrane-associated IL1R-I. A second receptor, termed the IL-1R accessory protein (AcP), is not involved in direct ligand binding but is required for IL-1 signal transduction by complexing with IL-1 and the II1R-I. IL1R-I and AcP both contain extracellular portions with three Ig-like domains and cytoplasmic portions containing conserved signaling motifs. A third IL-1 receptor exists termed the type II IL-1R (IL1R-II) that has a extracellular structure similar to that of IL1R-I and AcP but that contains a truncated cytoplasmic tail incapable of signaling. This receptor acts as a decoy by binding IL-1 with high affinity and neutralizing its activity. IL1R-II can also be proteolytically cleaved, which releases the extracellular domain from the cell surface. This creates a soluble form of the receptor (sIL1R-II) that possesses high affinity for IL-1β, but only low affinity for IL-1α, and virtually no affinity for IL-1ra.

In certain preferred embodiments, the subject adzymes are IL-1 antagonist adzyme that act on IL-1, particularly IL-1β, present in biological fluids. Exemplary EL-1 targeting moieties that be adapted for use in such adzymes include, but are not limited to, the extracellular domains of IL-1 receptors or appropriate portions thereof, IL1R-II or a portion thereof, anti-IL-1 antibodies or antigen binding fragments thereof, or peptides or small molecules that (selectively) bind IL-1.

In certain preferred embodiments, the targeting moiety is derived from IL1R-II, e.g., a portion sufficient to specifically bind to Il-1β. For instance, the targeting moiety can include a ligand binding domain from IL1R-II from the human IL1R-II protein (GI Accession 640248, PRI Accession 2IRT_A).

The inhibitory activity of an IL-1 antagonist adzyme can be assayed using any of a variety of cell-based and cell-free assay systems well known in the art. For instance, IL-1 antagonist adzymes can be identified using the mixed lymphocyte response (MLR) and phytohemagglutinin A (PHA) assay, which is useful for identifying immune suppressive molecules in vitro that can be used for treating graft-versus-host disease. The results obtained from these assays are generally predictive of their in vivo effectiveness.

Another assay that be used to assess the adzyme is with respect to inhibition of immune responsiveness involves the mitogenic stimulation of lymphocytes with mitogenic substances of plant origin. The most widely used plant molecule is PHA. Although PHA stimulates DNA synthesis non-specifically in a large number of lymphocytes, unlike true antigenic stimulation which causes mitogenesis of sub-populations of lymphocytes, the susceptibility of a patient's lymphocytes to PHA stimulation has been shown to correlate with the overall immune responsiveness of the patient.

Thus, it will be appreciated as to both the mixed lymphocyte and PHA assay that they are valuable for identifying immune suppressive IL-1 antagonist adzymes.

In addition to the above immunosuppressive assays, a secondary mixed lymphocyte reaction assay may also be used. The secondary mixed lymphocyte assays differs from the primary mixed lymphocyte reaction assays in that they employ many more primed responder cells that are responsive to the primary stimulating cells. The presence of such responsive cells is a reflection of immunological memory in an ongoing immunological response. The protocol for carrying out a secondary mixed lymphocyte assay involves performing a primary lymphocyte assay as described above, and recovering viable cells about 9-10 days after the primary mixed lymphocyte reaction exhibits little or no cell proliferation. Generally between 10% to 50% of the original input cells are recovered in viable condition. These cells are then used in the secondary mixed lymphocyte reaction.

The subject adzymes can also be assessed for their ability to block IL-1 mediated cytokine production. Assays for cytokine production and/or proliferation of spleen cells, lymph node cells or thymocytes are well known in the art.

In still other embodiments, the subject adzymes can be assessed for their effect on proliferation and differentiation of hematopoietic and lymphopoietic cells.

In certain embodiments, the IL-1 antagonist adzyme will modify the substrate IL-1β protein in a manner that produces a product that is itself an antagonist of IL1β. For instance, the adzyme can include a catalytic domain that cleaves a site in the IL-1β polypeptide to produce a product that retains the ability to bind, for example, to the IL1 receptor but with a greatly reduced ability to activate the receptor so as to be an antagonist of native Il-1β. To further illustrate, the Arg$^{127}$ residue of human IL-1β can be targeted for cleavage by an adzyme having a catalytic domain with trypsin-like specificity. Mutation of Arg$^{127}$ has been demonstrated to reduce the bioactivity of IL-1β greatly while only having slight effect on receptor binding affinity.

The IL-1 mediated diseases which may be treated or prevented by the IL-1 antagonist adzymes of this invention include, but are not limited to, inflammatory diseases, autoimmune diseases, proliferative disorders, infectious diseases, and degenerative diseases. The apoptosis-mediated diseases which may be treated or prevented by the IL-1 antagonist adzymes of this invention include degenerative diseases.

Inflammatory diseases which may be treated or prevented include, but are not limited to osteoarthritis, acute pancreatitis, chronic pancreatitis, asthma, and adult respiratory distress syndrome. Preferably the inflammatory disease is osteoarthritis or acute pancreatitis.

Autoimmune diseases which may be treated or prevented include, but are not limited to, glomeralonephritis, rheumatoid arthritis, systemic lupus erythematosus, scleroderma, chronic thyroiditis, Graves' disease, autoimmune gastritis, insulin-dependent diabetes mellitus (Type I), autoimmune hemolytic anemia, autoimmune neutropenia, thrombocytopenia, chronic active hepatitis, myasthenia gravis, multiple sclerosis, inflammatory bowel disease, Crohn's disease, psoriasis, and graft vs. host disease. Preferably the autoimmune disease is rheumatoid arthritis, inflammatory bowel disease, Crohn's disease, or psoriasis,

Destructive bone disorders which may be treated or prevented include, but are not limited to, osteoporosis and multiple myeloma-related bone disorder.

Proliferative diseases which may be treated or prevented include, but are not limited to, acute myelogenous leukemia, chronic myelogenous leukemia, metastatic melanoma, Kaposi's sarcoma, and multiple myeloma.

Infectious diseases which may be treated or prevented include, but are not limited to, sepsis, septic shock, and Shigellosis.

The IL-1-mediated degenerative or necrotic diseases which may be treated or prevented by the IL-1 antagonist adzymes of this invention include, but are not limited to, Alzheimer's disease, Parkinson's disease, cerebral ischemia, and myocardial ischemia. Preferably, the degenerative disease is Alzheimer's disease.

The apoptosis-mediated degenerative diseases which may be treated or prevented by the IL-1 antagonist adzymes of this invention include, but are not limited to, Alzheimer's disease, Parkinson's disease, cerebral ischemia, myocardial ischemia, spinal muscular atrophy, multiple sclerosis, AIDS-related encephalitis, HIV-related encephalitis, aging, alopecia, and neurological damage due to stroke.

*(e) Biomolecular Targets in Non-Therapeutic Contexts*

Adzymes may be used in a number of non-medical applications, including but are not limited to, agriculture, environmental protection, food etc., and such adzymes will be targeted accordingly.

Adzymes may be used to upgrade nutritional quality and removing anti-nutritional factors from feed components, such as barley- and wheat-based feeds. Targets for such adzymes may include gluten meal, fiber, prions (e.g., PrP, the causative agent for bovine spongiform encephalopathy), dioxin, pesticides, herbicides, starches, lipids, cellulose, pectin, certain sugars (e.g., lactose, maltose) and polysaccharides.

Adzyme may be used in industrial processes such as waste processing, textile manufacture or paper production, or essentially any other process that employs an enzyme, where the enzyme can be replaced by an adzyme with improved effectiveness. Examples of targets for such applications include cellulose, hemicellulose, pectin, lignin, starch, peroxides, phosphates and nitrates.

Adzyme may be used in detergents or other cleaning agents, providing targeted elimination of selected soils or stains. Targets for such adzymes may include chlorophyll, hemoglobin, heme groups, hydrocarbons, avidin, ovalbumin, and various pigments and dyes.

Adzymes may be used for the cleanup of various environmental contaminants, such oil, pesticides, herbicides and waste products from chemical manufacture. Targets for such adzymes include hydrocarbons, halogenated hydrocarbons (particularly halogenated hydrocarbons containing aromatic moieties), cyanides, carbon monoxide, nitrous oxides, heavy metals, organometallic compounds, organophosphates and carbamates.

B. Exemplary Catalytic Domains

The instant invention provides a mesotrypsin catalytic domain, and various functional fragments and derivatives thereof. Such wild-type or modified / mutated / engineered functional fragments and derivatives may either stand alone or act in the context of adzyme.

As used herein, the term "catalytic domain" includes any mesotrypsin moiety capable of acting on a target to induce a chemical change, thereby modulate its activity, i.e., any mesotrypsin moiety capable of catalyzing a reaction within a target. The mesotrypsin catalytic domain may be a naturally occurring mesotrypsin (from any and all species), a catalytically active fragment thereof, or an engineered enzyme, *e.g.*, a protein engineered to have an additional or enhanced enzymatic activity, such as a mesotrypsin designed to contain amino acid sequence changes that result in addition or enhancement of one or more desirable functions of the wild-type enzyme (e.g., enhanced stability). A catalytic domain, at minimum, needs to comprise only the arrangement of amino acids that are effective to induce the desired chemical change in the target. They may be N- terminal or C- terminal truncated versions of natural enzymes, mutated versions, zymogens, or complete globular domains.

The mesotrypsin catalytic domain may comprise an enzymatically active site that alone is promiscuous, binding with a vulnerable site it recognizes on many different biomolecules, and may have relatively poor reaction kinetics. Both of these features are normally antithetical to sound drug development, but often are desireable in adzyme constructs, where the address specifies preference for the desired targeted biomolecule, and its binding properties often dominate kinetics, i.e., assure preferential collision between the catalytically active site and the target.

The mesotrypsin catalytic domain also may be a protein that modifies the target so that it is recognized and acted upon by another enzyme (*e.g.*, an enzyme that is already present in a subject). In another embodiment, the mesotrypsin catalytic domain may be a moiety that alters the structure of the target so that its activity is inhibited or upregulated. Many naturally occurring enzymes activate other enzymes, and these can be exploited in accordance with the invention.

Many mesotrypsins from difference species / organisms have been identified and can be found in public databases, *e.g.*, SwissProt, PIR, PRF, or the database maintained by the National Centers for Biotechnology Information (NCBI).

The human mesotrypsin cDNA and protein sequences (NCBI RefSeq Accession No. NM_002771 & NP_002762, respectively) are reproduced below:

```
MNPFLILAFV GAAVAVPFDD DDKIVGGYTC EENSLPYQVS LNSGSHFCGG

SLISEQWVVS AAHCYKTRIQ VRLGEHNIKV LEGNEQFINA AKIIRHPKYN

RDTLDNDIML IKLSSPAVIN ARVSTISLPT APPAAGTECL ISGWGNTLSF

GADYPDELKC LDAPVLTQAE CKASYPGKIT NSMFCVGFLE GGKDSCQRDS

GGPVVCNGQL QGVVSWGHGC AWKNRPGVYT KVYNYVDWIK DTIAANS

(SEQ ID NO: 45)
```

Mesotypsin is a trypsinogen, which is a member of the trypsin family of serine proteases. This enzyme is expressed in the brain and pancreas and is resistant to common trypsin inhibitors. It is active on peptide linkages involving the carboxyl group of lysine or arginine. Additional transcript variants for this gene have been described, but their full length sequences have not been determined.

The corresponding cDNA sequence is described below:

```
  1 ACACTCTACC ACCATGAATC CATTCCTGAT CCTTGCCTTT GTGGGAGCTG CTGTTGCTGT

 61 CCCCTTTGAC GATGATGACA AGATTGTTGG GGGCTACACC TGTGAGGAGA ATTCTCTCCC

121 CTACCAGGTG TCCCTGAATT CTGGCTCCCA CTTCTGCGGT GGCTCCCTCA TCAGCGAACA

181 GTGGGTGGTA TCAGCAGCTC ACTGCTACAA GACCCGCATC CAGGTGAGAC TGGGAGAGCA

241 CAACATCAAA GTCCTGGAGG GGAATGAGCA GTTCATCAAT GCGGCCAAGA TCATCCGCCA

301 CCCTAAATAC AACAGGGACA CTCTGGACAA TGACATCATG CTGATCAAAC TCTCCTCACC

361 TGCCGTCATC AATGCCCGCG TGTCCACCAT CTCTCTGCCC ACCGCCCCTC CAGCTGCTGG

421 CACTGAGTGC CTCATCTCCG GCTGGGGCAA CACTCTGAGC TTTGGTGCTG ACTACCCAGA

481 CGAGCTGAAG TGCCTGGATG CTCCGGTGCT GACCCAGGCT GAGTGTAAAG CCTCCTACCC

541 TGGAAAGATT ACCAACAGCA TGTTCTGTGT GGGCTTCCTT GAGGGAGGCA AGGATTCCTG

601 CCAGCGTGAC TCTGGTGGCC CTGTGGTCTG CAACGGACAG CTCCAAGGAG TTGTCTCCTG

661 GGGCCATGGC TGTGCCTGGA AGAACAGGCC TGGAGTCTAC ACCAAGGTCT ACAACTATGT

721 GGACTGGATT AAGGACACCA TCGCTGCCAA CAGCTAAAGC CCCCGGTCCC TCTGCAGTCT

781 CTATACCAAT AAAGTGGCCC TGCTCTC  (SEQ ID NO: 46)
```

Certain human mesotrypsin variant sequences are described below with the following public database accession numbers (the associated sequences are incorporated herein by reference): CAI39655.1; CAA33527.1; AAH30238.1; CAB58178.1; S33496; CAH69873.1; I38363; AAL14243.1; AAC80208.1; 1H4W_A; and CAA50484.1. All human variants are generally about 95 to about 100% identical to the human query sequence.

Certain primate mesotrypsin or related sequences (such as those from *Macaca mulatta* (rhesus monkey) or *Pan troglodytes* (chimpanzee)) are generally about 90% identical to the human query sequence. They can be retrieved from, for example, public (or private) database using the human mesotrypsin sequence and any sequence comparison algorithms, such as the NCBI BLAST.

Similarly, certain other mammalian mesotrypsins, such as those from rat, mouse, dog, or bovine, are generally about 75-80% identical to the human query sequence. And other vertebrate homologs are generally about 65-70% identical to the human query sequence.

Thus the mesotrypsin of the instant invention includes naturally occuring mesotrypsins, functional fragments and variant sequences thereof that are at least about 65%, 70%, 75%, 80%, 85%, 90%, 95%, 97%, 99% or more identical to the human query sequence described above. The functional fragments may be determined by their ability to digest a mesotrypsin substrate, such as TNF, using any of the assays described herein.

The term "corresponds to" is used herein to mean that a polynucleotide sequence is homologous (i.e., is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

The following terms are used to describe the sequence relationships between two or more polynucleotides: "reference sequence", "comparison window", "sequence identity", "percentage of sequence identity", and "substantial identity". A "reference sequence" or "query sequence" is a defined sequence used as a basis for a sequence comparision; a reference sequence may be a subset of a larger sequence, for example, as a segment of a full-length cDNA or gene sequence given in a sequence listing, such as a human mesotrypsin polypeptide or polynucleotide sequence described above, or may comprise a complete cDNA or gene sequence. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window", as used herein, refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman (1981) Adv. Appl. Math. 2: 482, by the homology alignment algorithm of Needleman and Wunsch (1970) J. Mol. Biol. 48: 443, by the search for similarity method of Pearson and Lipman (1988) Proc. Natl. Acad. Sci. (U.S.A.) 85: 2444, by computerized implementations of these algorithms (GAP, BESTFIT, FASTA, and TFASTA in the Wisconsin Genetics Software Package Release 7.0, Genetics Computer Group, 575 Science Dr., Madison, Wis. or by inspection, and the best alignment (i.e., resulting in the highest percentage of homology over the comparison window) generated by the various methods is selected. The term "sequence identity" means that two polynucleotide sequences are identical (i.e., on a nucleotide-by-nucleotide basis) over the window of comparison. The term "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparision (i.e., the window size), and multiplying the result by 100 to yield the percentage of sequence identity. The terms "substantial identity" as used herein denotes a characteristic of a polynucleotide sequence, wherein the polynucleotide comprises a sequence that has at least 85 percent sequence identity, preferably at least 90 to 95 percent sequence identity, more usually at least 99 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison.

As applied to polypeptides, the term "substantial identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity or more (e.g., 99 percent sequence identity). Preferably, residue positions which are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagine-glutamine.

The term "native protein" and "full-length protein" as used herein refers to a a naturally-occurring polypeptide corresponding to the deduced amino acid sequence of a cognate full-length cDNA.

The term "fragment" as used herein refers to a polypeptide that has an amino-terminal and/or carboxy-terminal deletion, but where the remaining amino acid sequence is identical to the corresponding positions in the wild-type sequence deduced from a full-length cDNA sequence. Fragments that substantially retains (at least about 20%, 30%, 50%, 70%, 80%, 90%) the wild-type protein function is a functional fragment.

The term "cognate" or "homolog" as used herein refers to a gene sequence that is evolutionarily and functionally related between species. For example but not limitation, in the human genome, the human mesotrypsin gene is the cognate / homolog gene to the mouse mesotrypsin gene, since the sequences and structures of these two genes indicate that they are highly homologous and both genes encode a protein which functions similarly. Thus, the cognate murine gene to the human gene encodes an expressed protein which has the greatest degree of sequence identity to the human mesotrypsin protein, and which may exhibit an expression pattern similar to that of the human protein. Preferred cognate / homolog mesotrypsin genes in primates, mammalian, and other vertebrates are described above.

In addition to mesotrypsin polypeptides consisting only of naturally-occuring amino acids, mesotrypsin peptidomimetics are also provided. Peptide analogs are commonly used in the pharmaceutical industry as non-peptide drugs with properties analogous to those of the template peptide. These types of non-peptide compound are termed "peptide mimetics" or "peptidomimetics" (Fauchere, J. (1986) Adv. Drug Res. 15: 29; Veber and Freidinger (1985) TINS p.392; and Evans et al. (1987) J. Med. Chem 30: 1229) and are usually developed with the aid of computerized molecular modeling. Peptide mimetics that are structurally similar to therapeutically useful peptides may be used to produce an equivalent therapeutic or prophylactic effect. Generally, peptidomimetics are structurally similar to a paradigm polypeptide (i.e., a polypeptide that has a biological or pharmacological activity), such as human mesotrypsin, but have one or more peptide linkages optionally replaced by a linkage selected from the group consisting of $-CH_2-NH--$, $--CH_2-S--$, $--CH_2 --CH2$, $--CH=CH--$ (cis and trans), $--COCH_2 --$, $--CH(OH)CH_2 --$, and $--CH_2 SO--$, by methods known in the art and further described in the following references: Spatola, A.F. in "Chemistry and Biochemistry of Amino Acids, Peptides, and Proteins," B. Weinstein, eds., Marcel Dekker, New York, p. 267 (1983); Szatola, A. F., Vega Data (March 1983), Vol. 1, Issue 3, "Peptide Backbone Modifications" (general review); Morley, J. S., Trends Pharm Sci (1980) pp. 463-468 (general review); Hudson, D. et al., Int J Pent Prot Res (1979) 14:177-185 (--CH2 NH--, CH2 CH2 --); Spatola, A. F. et al., Life Sci (1986) 38:1243-1249 (--CH2 - S); Hann, M. M., J Chem Soc Perkin Trans I (1982) 307-314 (--CH--CH--, cis and trans); Almquist, R. G. et al., J Med Chem (1980) 23:1392-1398 (--COCH2 --); Jennings-White, C. et al., Tetrahedron Lett (1982) 23:2533 (--COCH2 --); Szelke, M. et al., European Appln. EP 45665 (1982) CA: 97:39405 (1982) (--CH(OH) CH2 --); Holladay, M. W. et al., Tetrahedron Lett (1983) 24:4401-4404 (--C(OH)CH2 --); and Hruby, V. J., Life Sci (1982) 31:189-199 (-CH2 --S--). A particularly preferred non-peptide linkage is --CH22 NH--. Such peptide mimetics may have significant advantages over polypeptide embodiments, including, for example: more economical production, greater chemical stability, enhanced pharmacological properties (half-life, absorption, potency, efficacy, etc.), altered specificity (e.g., a broad-spectrum of biological activities), reduced antigenicity, and others. Labeling of peptidomimetics usually involves covalent attachment of one or more labels, directly or through a spacer (e.g., an amide group), to non-interfering position(s) on the peptidomimetic that are predicted by quantitative structure-activity data and/or molecular modeling. Such non-interfering positions generally are positions that do not form direct contacts with the macromolecules(s) (e.g., immunoglobulin superfamily molecules) to which the peptidomimetic binds to produce the therapeutic effect. Derivitization (e.g., labelling) of peptidomimetics should not substantially interfere with the desired biological or pharmacological activity of the peptidomimetic. Peptidomimetics of mesotrypsin may be used as competitive or noncompetitive agonists or antagonists of wild-type function.

Systematic substitution of one or more amino acids of a consensus sequence with a D-amino acid of the same type (e.g., D-lysine in place of L-lysine) may be used to generate more stable peptides. In addition, constrained peptides (including cyclized peptides) comprising a consensus sequence or a substantially identical consensus sequence variation may be generated by methods known in the art (Rizo and Gierasch (1992) Ann. Rev. Biochem. 61: 387); for example, by adding internal cysteine residues capable of forming intramolecular disulfide bridges which cyclize the peptide.

The amino acid sequences of mesotrypsin polypeptides identified herein will enable those of skill in the art to produce polypeptides corresponding to any of the mesotrypsin peptide sequences and sequence variants thereof. Such polypeptides may be produced in prokaryotic or eukaryotic host cells by expression of polynucleotides encoding a mesotrypsin peptide sequence, frequently as part of a larger polypeptide. Alternatively, such peptides may be synthesized by chemical methods. Methods for expression of heterologous proteins in recombinant hosts, chemical synthesis of polypeptides, and in vitro translation are well known in the art and are described further in Maniatis et al., Molecular Cloning: A Laboratory Manual (1989), 2nd Ed., Cold Spring Harbor, N.Y.; Berger and Kimmel, Methods in Enzymology, Volume 152, Guide to Molecular Cloning Techniques (1987), Academic Press, Inc., San Diego, Calif.; Merrifield, J. (1969) J. Am. Chem. Soc. 91: 501; Chaiken I. M. (1981) CRC Crit. Rev. Biochem. 11: 255; Kaiser et al.(1989) Science 243: 187; Merrifield, B. (1986) Science 232: 342; Kent, S. B. H. (1988) Ann. Rev. Biochem. 57: 957; and Offord, R. E. (1980) Semisynthetic Proteins, Wiley Publishing).

A "reversed" or "retro" peptide sequence as disclosed herein refers to that part of an overall sequence of covalently-bonded amino acid residues (or analogs or mimetics thereof) wherein the normal carboxyl-to amino direction of peptide bond formation in the amino acid backbone has been reversed such that, reading in the conventional left-to-right direction, the amino portion of the peptide bond precedes (rather than follows) the carbonyl portion. See, generally, Goodman, M. and Chorev, M. Accounts of Chem. Res. 1979, 12, 423.

The reversed orientation peptides described herein include (a) those wherein one or more amino-terminal residues are converted to a reversed ("rev") orientation (thus yielding a second "carboxyl terminus" at the left-most portion of the molecule), and (b) those wherein one or more carboxyl-terminal residues are converted to a reversed ("rev") orientation (yielding a second "amino terminus" at the right-most portion of the molecule). A peptide (amide) bond cannot be formed at the interface between a normal orientation residue and a reverse orientation residue.

Therefore, certain reversed peptide compounds of the invention can be formed by utilizing an appropriate amino acid mimetic moiety to link the two adjacent portions of the sequences depicted above utilizing a reversed peptide (reversed amide) bond. In case (a) above, a central residue of a diketo compound may conveniently be utilized to link structures with two amide bonds to achieve a peptidomimetic structure. In case (b) above, a central residue of a diamino compound will likewise be useful to link structures with two amide bonds to form a peptidomimetic structure.

The reversed direction of bonding in such compounds will generally, in addition, require inversion of the enantiomeric configuration of the reversed amino acid residues in order to maintain a spatial orientation of side chains that is similar to that of the non-reversed peptide. The configuration of amino acids in the reversed portion of the peptides is preferably (D), and the configuration of the non-reversed portion is preferably (L). Opposite or mixed configurations are acceptable when appropriate to optimize a binding activity.

The nucleic acid sequences encoding any of the above polypeptides are also within the scope of the invention.

The invention also contemplates any polynucleotides that hybridize under high stringency conditions to any of the nucleic acid sequences encoding any of the above polypeptides, and which polynucleotide sequences encode a polypeptide that substantially retain the function of mesotrypsin. A nucleic acid molecule is "hybridizable" to another nucleic acid molecule, such as a cDNA, genomic DNA, or RNA, when a single stranded form of the nucleic acid molecule can anneal to the other micleic acid molecule under the appropriate conditions of temperature and solution ionic strength (see Sambrook et al. Molecular Cloning: A Laboratory Manual, Second Edition (1989) Cold Spring Harbor Laboratory Press, Cold Spring Harbor, N.Y.). The conditions of temperature and ionic strength determine the "stringency" of the hybridization. For preliminary screening for homologous nucleic acids, low stringency hybridization conditions, corresponding to a $T_m$ (melting temperature) of 55°C can be used, e.g., 5xSSC, 0.1% SDS, 0.25% milk, and no formamide; or 30% formamide, 5xSSC, 0.5% SDS).

Unless specified, the term "standard hybridization conditions" refers to a $T_m$ of about 55°C, and utilizes conditions as set forth above. In a preferred embodiment, the $T_m$ is 60°C; in a more preferred embodiment, the $T_m$ is 65°C. In a specific embodiment, "high stringency" refers to hybridization and/or washing conditions at 68°C in 0.2xSSC, or at 42°C in 50% formamide, 4xSSC, or under conditions that afford levels of hybridization equivalent to those observed under either of these two conditions.

Further, high resolution three dimensional structural coordinates for many enzymes can be found in the database maintained by the Research Collaboratory for Structural Bioinformatics (RCSB). The unresolved structures of proteins often can be predicted using a technique known as threading. Threading algorithms are described in the literature and can be found in Alexandrov N.N., et al., (1998) Bioinformatics 14:206-11, Labesse G, et al. (1997) Proteins 1:38-42, Xu, Y. et al. (1999). Protein Eng., 12: 899-907, Russel A. J., et al. (2002) Proteins 47:496-505, and Reva, B., et al. (2002) Proteins 47:180-93.

In a preferred embodiment, the catalytic domain is an engineered mesotrypsin with enhanced stability.

C. Generating Chimeric Adzymes

The mesotrypsin catalytic moiety can be linked to the targeting moiety in a number of ways including by cotranslation from a recombinant nucleic acid (*e.g.*, fusion proteins) or, in less preferred embodiments, chemical coupling.

*(i) Generated as Recombinant Fusion Proteins*

The adzymes of this invention can be constructed as a fusion protein, containing the catalytic moiety and the targeting moiety as one contiguous polypeptide chain. In preparing the fusion protein, a fusion gene is constructed comprising DNA encoding the sequences for the targeting moiety, the catalytic moiety, and optionally, a peptide linker sequence to span the two fragments. To make this fusion protein, an entire enzyme can be cloned and expressed as part of the protein, or alternatively, a suitable fragment containing the catalytic moiety can be used. Likewise, the entire cloned coding sequence of a targeting moiety such as a receptor or antibody, or alternatively, a fragment of the molecule capable of binding the surface component of the pathogen can be used. The use of recombinant DNA techniques to create a fusion gene, with the translational product being the desired fusion protein, is well known in the art Both the coding sequence of a gene and its regulatory regions can be redesigned to change the functional properties of the protein product, the amount of protein made, or the cell type in which the protein is produced. The coding sequence of a gene can be extensively altered--for example, by fusing part of it to the coding sequence of a different gene to produce a novel hybrid gene that encodes a fusion protein. Examples of methods for producing fusion proteins are described in PCT applications PCT/US87/02968, PCT/US89/03587 and PCT/US90/07335, as well as Traunecker et al. (1989) Nature 339:68.

Signal peptides facilitate secretion of proteins from cells. An exemplary signal peptide is the amino terminal 25 amino acids of the leader sequence of murine interleukin-7 (IL-7; Namen et al., Nature 333:571; 1988). Other signal peptides may also be employed furthermore, certain nucleotides in the IL-7 leader sequence can be altered without altering the amino acid sequence. Additionally, amino acid changes that do not affect the ability of the IL-7 sequence to act as a leader sequence can be made. A signal peptide may be added to the fusion adzyme target domain or catalytic domain, such that when these domains are synthesized by cells from transfected nucleic acids, the secreted adzyme target and catalytic domains will oligomerize to form mature adzymes to act on extracellular targets, such as cytokines.

In some instances it may be necessary to introduce a polypeptide linker region between portions of the chimeric protein derived from different proteins. This linker can facilitate enhanced flexibility of the fusion protein allowing various portions to freely and (optionally) simultaneously interact with a target by reducing steric hindrance between the portions, as well as allowing appropriate folding of each portion to occur. The linker can be of natural origin, such as a sequence determined to exist in random coil between two domains of a protein. Alternatively, the linker can be of synthetic origin. For instance, one or more repeats of $Ser_4Gly$ (SEQ ID NO: 41), $SerGly_4$ (SEQ ID NO: 42), $Gly_4Ser$ (SEQ ID NO: 43), $GlySer_4$ (SEQ ID NO: 44), or GS can be used as synthetic unstructured linkers. Linkers of this type are described in Huston et al. (1988) PNAS 85:4879; and U.S. Patent Nos. 5,091,513 and 5,258,498. Naturally occurring unstructured linkers of human origin are preferred as they reduce the risk of immunogenicity.

The length and composition of the linker connecting the address and the catalytic domain may be optimized. While it is widely appreciated that short linkers can introduce steric hindrance that can be detrimental, it may often be overlooked that very long linkers suffer from negative entropic effects, in that conformational entropy is further decreased upon binding of the substrate by the tethered enzyme when longer linkers are used. The linker geometry should be determined to optimize adzyme activity. For example, Zhou (J. Mol. Biol. 329: 1-8, 2003) describes in detail a quantitative theory for enhancing affinity for a first molecule by linking a second and a third molecule (such as two scFvs), each of which has affinity for the first molecule. The predicted affinity enhancement is found to be actually approached by a bi-specific antibody against hen egg lysozyme consisting of scFv fragments of D1.3 and HyHEL-10. The wide applicability of the theory is demonstrated by diverse examples of protein-protein interactions constrained by flexible linkers, and the theory provides a general framework for understanding protein-protein interactions constrained by flexible linkers.

In the simplest case of the theory, the linker is flexible such that its only effect is to provide a leash constraining the distances between the two antibody fragments. Then it was shown:

$$Ceff = p(d0) \qquad\qquad (Eq.\ a)$$

where p(r) is the probability density for the end-to-end vector of the flexible linker with L residues to have a distance r, and $d_0$ is the actual end-to-end distance when the linked fragments are bound to the antigen. A flexible peptide linker consisting of L residues can be modeled as a worm-like chain, such that:

$$p(r) = (3/4l_pl_c)^{3/2}\ exp(-3r^2/4l_pl_c)(1-5l_p/4l_c + 2r^2/l_c^2-33r^4/80l_pl_c^3-79l_p^2/160l_c^2-329r_2l_p/120l_c^3+6799r^4/1600l_c^4-3441r^6/2800l_pl_c^5+1089r^8/12800l_p^2l_c^6) \qquad (Eq.\ b)$$

where b=3.8 Å is the nearest $C_\alpha$-$C_\alpha$ distance, and $l_c$=bL and $1_p$=3 Å are the contour length and persistence length, respectively, of the peptide linker. Typically $p(d_0)$ is in the millimolar range or higher, and hence the linking strategy is expected to result in significant affinity enhancement, since the association constants of antibody fragments are much greater than $10^3$ $M^{-1}$. Equation (a) has been found to predict well the affinity enhancements of linking DNA-binding domains (Zhou, Biochemistry 40, pp. 15069-15073, 2001).

Based on this theoritic model, Figure 2 of Zhou describes the relationship of L and $p(d_0)$ at several given $d_0$ values, such as 10 Å, 2O A, 30 Å, 40 Å, 50 Å, and 60 Å. This linker theory incorporates two important realistic aspects. First, in the bound state, the end-to-end distance of the linker is kept at around a specific value (do) determined by the structure of the bound complex. Second, in the unbound state, the distribution p(r) of the end-to-end distance is not uniform but is what is appropriate for a semi-flexible polymer chain, such as a polypeptide chain. For entropic reasons, a polymer chain very rarely samples conformations with end-to-end distances approaching either zero or the full contour length $1_c$, thus p(r) has a maximum at an intermediate value of r. At a given end-to-end distance $d_0$, there is also a value of $l_c$ (or L) at which $p(d_0)$ is maximal (see Figure 2 of Zhou). Therefore, the chain length of a peptide linker can be optimized to achieve maximal affinity enhancement.

In the context of the adzyme linker design, once the address and the catalytic domain is chosen, molecular model of the target - adzyme avid complex may be obtained. $d_0$, the distance between the point where the linker connects to the address and the point where the linker connects to the enzyme, while both the address and enzyme domain are in the avid complex, can be readily determined from, for example, the 3-D structure of the target - adzyme complex. Many cytokine structures are solved (see the Cytokine Web site at http://cmbi.bjmu.edu.cn/cmbidata/cgf/CGF_Database/cytweb/cyt_strucs/index.html). The structure of those other cytokines with sequence homology to cytokines of known structures, as well as the target - adzyme complex may be routinely obtained via molecular modeling.

Once the do value is obtained, Figure 2 of Zhou may be used to find the optimum L for the highest possible p$(d_0)$ value. For example, if it is determined that $d_0$ is about 20 Å, Figure 2 of Zhou indicates that at this do value, the highest possible $p(d_0)$ value is about 20 mM, and that $p(d_0)$ value corresponds to a linker length of about 10-15 amino acids. Note that at do value larger than 20 Å, the maximum $p(d_0)$ value peaks quickly and tapers off very gradually, thus allowing quite a bit of flexibility in chosing a proper linker length. In addition, the method here is rather tolerant of a reletively imprecise estimation of the do value, since in Figure 2 of Zhou, curves for different $d_0$ values tend to converge, especially in long linker length (e.g., more than 40 amino acids) and large do values (30-60 Å). For example, when do is 30 Å, the peak $p(d_0)$ value is about 3-4 mM. When $d_0$ is 40 Å, the peak $p(d_0)$ only decreases to about 1.5 mM, at about the same linker length of around 35 - 40 residues.

This techniques is particularly useful when designing adzymes with optimized balance between its selectivity and potency (see above), since the linker geometry and length have direct impact on *[S]_{eff}* of the adzyme. However, applicants note that in some instances, linker length may be relatively unimportant. As shown in the examples, a mesotrypsin adzyme targeted to TNF-alpha functioned appropriately without regard to the length of the linker.

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a fusion gene sequence (see, for example, Current Protocols in Molecular Biology, eds. Ausubel et al. John Wiley & Sons: 1992).

Fusion proteins can comprise additional sequences, including a leader (or signal peptide) sequence, a portion of an immunoglobulin (e.g., an Fc portion, see below) or other oligomer-forming sequences, as well as sequences encoding highly antigenic moieties, hexahistidine moieties or other elements that provide a means for facile purification or rapid detection of a fusion protein.

To express the fusion protein molecule, it may be desirable to include transcriptional and translational regulatory elements and other non-coding sequences to the fusion gene construct. For instance, regulatory elements including constituitive and inducible promoters, enhancers or inhibitors can be incorporated.

*(ii) Use of Chemical Coupling Agents*

There are a large number of chemical cross-linking agents that are known to those skilled in the art. For the present invention, the preferred cross-linking agents are heterobifunctional cross-linkers, which can be used to link proteins in a stepwise manner. Heterobifunctional cross-linkers provide the ability to design more specific coupling methods for conjugating proteins, thereby reducing the occurrences of unwanted side reactions such as homo-protein polymers. A wide variety of heterobifunctional cross-linkers are known in the art. These include: succinimidyl 4-(N-maleimidomethyl) cyclohexane-1-carboxylate (SMCC), m-Maleimidobenzoyl-N-hydroxysuccinimide ester (MBS); N-succinimidyl (4-iodoacetyl) aminobenzoate (SIAB), succinimidyl 4-(p-maleimidophenyl) butyrate (SMPB), 1-ethyl-3-(3-dimethylaminopropyl) carbodiimide hydrochloride (EDC); 4-succinimidyloxycarbonyl-a-methyl-a-(2-pyridyldithio)-tolune (SMPT), N-succinimidyl 3-(2-pyridyldithio) propionate (SPDP), succinimidyl 6-[(3-(2-pyridyldithio) propionate] hexanoate (LC-SPDP). Those cross-linking agents having N-hydroxysuccinimide moieties can be obtained as the N-hydroxysulfosuccinimide analogs, which generally have greater water solubility. In addition, those cross-linking agents having disulfide bridges within the linking chain can be synthesized instead as the alkyl derivatives so as to reduce the amount of linker cleavage in vivo.

In addition to the heterobifunctional cross-linkers, there exists a number of other cross-linking agents including homobifunctional and photoreactive cross-linkers. Disuccinimidyl suberate (DSS), bismaleimidohexane (BMH) and dimethylpimelimidate-2 HCl (DMP) are examples of usefull homobifunctional cross-linking agents, and bis-[.beta.-(4-azidosalicylamido)ethyl]disulfide (BASED) and N-succinimidyl-6(4'-azido-2'-nitrophenyl-amino)hexanoate (SANPAH) are examples of useful photoreactive cross-linkers for use in this invention. For a review of protein coupling techniques, see Means et al. (1990) Bioconjugate Chemistry 1:2-12. One particularly useful class of heterobifunctional cross-linkers, included above, contain the primary amine reactive group, N-hydroxysuccinimide (NHS), or its water soluble analog N-hydroxysulfosuccinimide (sulfo-NHS). Primary amines (lysine epsilon groups) at alkaline pH's are unprotonated and react by nucleophilic attack on NHS or sulfo-NHS esters. This reaction results in the formation of an amide bond, and release of NHS or sulfo-NHS as a by-product.

Another reactive group useful as part of a heterobifunctional cross-linker is a thiol reactive group. Common thiol reactive groups include maleimides, halogens, and pyridyl disulfides. Maleimides react specifically with free sulfhydryls (cysteine residues) in minutes, under slightly acidic to neutral (pH 6.5-7.5) conditions. Halogens (iodoacetyl functions) react with --SH groups at physiological pH's. Both of these reactive groups result in the formation of stable thioether bonds.

The third component of the heterobifunctional cross-linker is the spacer arm or bridge. The bridge is the structure that connects the two reactive ends. The most apparent attribute of the bridge is its effect on steric hindrance. In some instances, a longer bridge can more easily span the distance necessary to link two complex biomolecules.

Preparing protein-protein conjugates using heterobifunctional reagents is a two-step process involving the amine reaction and the sulfhydryl reaction, and such processes are, in view of this specification, generally well known in the art. See, e.g., Partis et al. (1983) J. Pro. Chem. 2:263); Ellman et al. (1958) Arch. Biochem. Biophys. 74:443; Riddles et al. (1979) Anal. Biochem. 94:75); Blattler et al. (1985) Biochem 24:1517).

*(iii) Multimeric Constructs*

In certain embodiments of the invention, the subject adzyme is a multimeric complex in which the catalytic domain and targeting domain are on separate polypeptide chains. These two domains, when synthesized, can be brought together to form the mature adzyme.

For example, in one embodiment, the adzyme takes the form of an antibody (e.g., Fc fusion) in which the variable regions of the heavy ($V_H$) and light chain ($V_L$) have been replaced with the targeting and catalytic domains (either the targeting or the catalytic domain can replace either the $V_H$ region or the $V_L$ region). For example, soluble proteins comprising an extracellular domain from a membrane-bound protein and an immunoglobulin heavy chain constant region was described by Fanslow et al., J. Immunol. 149:65, 1992 and by Noelle et al., Proc. Nad. Acad. Sci. U.S.A. 89:6550, 1992.

In certain embodiments, an adzyme comprises a first Fc portion that is connected to the appropriate heavy and light chains which may function as a targeting moiety, and a second Fc portion that is fused to a catalytic domain.

Fusion proteins comprising a catalytic domain or a targeting domain may be prepared using nucleic acids encoding polypeptides derived from immunoglobulins. Preparation of fusion proteins comprising heterologous polypeptides fused to various portions of antibody-derived polypeptides (including the Fc domain) has been described, e.g., by Ashkenazi et al., (PNAS USA 88:10535, 1991) and Byrn et al., (Nature 344:677, 1990). In one embodiment of the invention, an adzyme is created by fusing a catalytic domain to a first Fc region of an antibody (*e.g.*, IgG1) and a targeting domain to a second Fc region of an antibody. The Fc polypeptide preferably is fused to the C-terminus of a catalytic or targeting domain. A gene fusion encoding each Fc fusion protein is inserted into an appropriate expression vector. The Fc fusion proteins are expressed in host cells transformed with the recombinant expression vector, and allowed to assemble much like antibody molecules, whereupon interchain disulfide bonds form between Fc polypeptides, yielding the desired adzymes. If fusion proteins are made with both heavy and light chains of an antibody, it is possible to form an adzyme with multiple catalytic and targeting domains.

In certain embodiments, an adzyme comprising one or more immunoglobulin fusion protein may employ an immunoglobulin light chain constant region in association with at least one immunoglobulin heavy chain constant region domain. In another embodiment, an immunoglobulin light chain constant region is associated with at least one immunoglobulin heavy chain constant region domain joined to an immunoglobulin hinge region. In one set of embodiments, an immunoglobulin light chain constant region joined in frame with a polypeptide chain of a non-immunoglobulin polypeptide (e.g., a catalytic domain or polypeptide targeting domain) and is associated with at least one heavy chain constant region. In a preferred set of embodiments a variable region is joined upstream of and in proper reading frame with at least one immunoglobulin heavy chain constant region. In another set of embodiments, an immunoglobulin heavy chain is joined in frame with a polypeptide chain of a non-immunoglobulin polypeptide and is associated with an immunoglobulin light chain constant region. In yet another set of embodiments, a polypeptide chain of a non-immunoglobulin polypeptide dimer or receptor analog is joined to at least one immunoglobulin heavy chain constant region which is joined to an immunoglobulin hinge region and is associated with an immunoglobulin light chain constant region. In a preferred set of embodiments an immunoglobulin variable region is joined upstream of and in proper reading frame with the immunoglobulin light chain constant region.

The term "Fc polypeptide" as used herein includes native and altered forms of polypeptides derived from the Fc region of an antibody. Truncated froms of such polypeptides containing the hinge region that promotes dimerization are also included. One suitable Fc polypeptide, described in PCT application WO 93/10151, is a single chain polypeptide extending from the N-terminal hinge region to the native C-terminus. It may be desirable to use altered forms of Fc polypeptides having improved serum half-life, altered spatial orientation, and the like. Immunoglobulin heavy chain constant region domains include $C_H1$, $C_H2$, $C_H3$, and $C_H4$ of any class of immunoglobulin heavy chain including gamma, alpha, epsilon, mu, and delta classes. A particularly preferred immunoglobulin heavy chain constant region domain is human $C_H1$. Immunoglobulin variable regions include $V_H$, $V_{kappa}$, or $V_{lambda}$ DNA sequences encoding immunoglobulins may be cloned from a variety of genomic or cDNA libraries known in the art. The techniques for isolating such DNA sequences using probe-based methods are conventional techniques and are well known to those skilled in the art. Probes for isolating such DNA sequences may be based on published DNA sequences (see, for example, Hieter et al., Cell 22: 197-207, 1980). Alternatively, the polymerase chain reaction (PCR) method disclosed by Mullis et al. (U.S. Pat. No. 4,683,195) and Mullis (U.S. Pat. No. 4,683,202), may be used. The choice of library and selection of probes for the isolation of such DNA sequences is within the level of ordinary skill in the art.

Host cells for use in preparing immunoglobulin fusions include eukaryotic cells capable of being transformed or transfected with exogenous DNA and grown in culture, such as cultured mammalian and fungal cells. Fungal cells, including species of yeast (e.g., Saccharomvces spp., Schizosaccharomyces spp.), or filamentous fungi (e.g., Aspergillus spp., Neurospora spp.) may be used as host cells within the present invention. Strains of the yeast Saccharomyces cerevisiae are particularly preferred.

In each of the foregoing embodiments, a molecular linker optionally may be interposed between, and covalently join, the rest of the adzyme construct and the dimerization domain.

In another embodiment, various oligomerization domains may be employed to bring together the separately synthesized targeting and catalytic domains.

One class of such oligomerization domain is leucine zipper. WO 94/10308 A1 and its related U.S. Pat. No. 5,716,805 (all incorporated herein by reference) describes the use of leucine zipper oligomerization domains to dimerize/oligomerize two separate heterologous polypeptides. Each of the two separate heterologous polypeptides is synthesized as a fusion protein with a leucine zipper oligomerization domain. In one embodiment, the leucine zipper domain can be removed from the fusion protein, by cleavage with a specific proteolytic enzyme. In another embodiment, a hetero-oligomeric protein is prepared by utilizing leucine zipper domains that preferentially form hetero-oligomers.

Leucine zipper domains were originally identified in several DNA-binding proteins (Landschulz et al., Science 240:1759, 1988). Leucine zipper domain is a term used to refer to a conserved peptide domain present in these (and other) proteins, which is responsible for dimerization of the proteins. The leucine zipper domain (also referred to herein as an oligomerizing, or oligomer-forming, domain) comprises a repetitive heptad repeat, with four or five leucine residues interspersed with other amino acids.

Examples of leucine zipper domains are those found in the yeast transcription factor GCN4 and a heat-stable DNA-binding protein found in rat liver (C/EBP; Landschulz et al., Science 243:1681, 1989). Two nuclear transforming proteins, fos and jun, also exhibit leucine zipper domains, as does the gene product of the murine proto-oncogene, c-myc (Landschulz et al., Science 240:1759, 1988).. The products of the nuclear oncogenes fos and jun comprise leucine zipper domains preferentially form a heterodimer (O'Shea et al., Science 245:646, 1989; Turner and Tjian, Science 243: 1689, 1989). The leucine zipper domain is necessary for biological activity (DNA binding) in these proteins.

The fusogenic proteins of several different viruses, including paramyxovirus, coronavirus, measles virus and many retroviruses, also possess leucine zipper domains (Buckland and Wild, Nature 338:547,1989; Britton, Nature 353: 394, 1991; Delwart and Mosialos, AIDS Research and Human Retroviruses 6:703, 1990). The leucine zipper domains in these fusogenic viral proteins are near the transmembrane region of the proteins; it has been suggested that the leucine zipper domains could contribute to the oligomeric structure of the fusogenic proteins. Oligomerization of fusogenic viral proteins is involved in fusion pore formation (Spruce et al, PNAS 88:3523, 1991). Leucine zipper domains have also been recently report ed to play a role in oligomerization of heat-shock transcription factors (Rabindran et al., Science 259:230, 1993).

Accordingly, in certain embodiments, the dimerization domains of the adzyme components comprise coiled-coil dimerization domains, such as leucine zipper domains. Preferably, the leucine zipper domains include at least four leucine heptads. In one preferred embodiment, the leucine zipper domain is a Fos or Jun leucine zipper domain.

Many other so-called "bundling domains" exist which perform essentially the same function of the above-described leucine zipper domains to bring together the catalytic and target domains. For example, WO 99/10510 A2 describes bundling domains include any domain that induces proteins that contain it to form multimers ("bundles") through protein-protein interactions with each other or with other proteins containing the bundling domain. Examples of these bundling domains include domains such as the lac repressor tetramerization domain, the p53 tetramerization domain, the leucine zipper domain, and domains derived therefrom which retain observable bundling activity. Proteins containing a bundling domain are capable of complexing with one another to form a bundle of the individual protein molecules. Such bundling is "constitutive" in the sense that it does not require the presence of a cross-linking agent (i.e., a cross-linking agent which doesn't itself contain a pertinacious bundling domain) to link the protein molecules.

As described above, bundling domains interact with like domains via protein-protein interactions to induce formation of protein "bundles." Various order oligomers (dimers, trimers, tertramers, etc.) of proteins containing a bundling domain can be formed, depending on the choice of bundling domain.

In one embodiment, incorporation of a tetramerization domain within a fusion protein leads to the constitutive assembly of tetrameric clusters or bundles. The E. coli lactose repressor tetramerization domain (amino acids 46-360; Chakerian et al. (1991) J. Biol. Chem. 266.1371; Alberti et al. (1993) EMBO J. 12:3227; and Lewis et al. (1996) Nature 271: 1247), illustrates this class. Other illustrative tetramerization domains include those derived from residues 322-355 of p53 (Wang et al. (1994) Mol. Cell. Biol. 14:5182; Clore et al. (1994) Science 265: 386) see also U.S. Pat. No. 5,573,925 by Halazonetis.

In yet another embodiment, the catalytic domain and the target domain may each be fused to a "ligand binding domain," which, upon binding to a small molecule, will bring the catalytic domain and the target domain together ("small molecule-mediated oligomerization").

Fusion proteins containing a ligand binding domain for use in practicing this invention can function through one of a variety of molecular mechanisms.

In certain embodiments, the ligand binding domain permits ligand-mediated crosslinking of the fusion protein molecules bearing appropriate ligand binding domains. In these cases, the ligand is at least divalent and functions as a dimerizing agent by binding to the two fusion proteins and forming a cross-linked heterodimeric complex which activates target gene expression. See e.g. WO 94/18317, WO 96/20951, WO 96/06097, WO 97/31898 and WO 96/41865.

In the cross-linking-based dimerization systems the fusion proteins can contain one or more ligand binding domains (in some cases containing two, three, four, or more of such domains) and can further contain one or more additional domains, heterologous with respect to the ligand binding domain, including e.g. a catalytic or target domain of the subject adzyme.

In general, any ligand/ligand binding domain pair may be used in such systems. For example, ligand binding domains may be derived from an immunophilin such as an FKBP, cyclophilin, FRB domain, hormone receptor protein, antibody, etc., so long as a ligand is known or can be identified for the ligand binding domain.

For the most part, the receptor domains will be at least about 50 amino acids, and fewer than about 350 amino acids, usually fewer than 200 amino acids, either as the natural domain or truncated active portion thereof. Preferably the binding domain will be small (<25 kDa, to allow efficient transfection in Viral vectors), monomeric, nonimmunogenic, and should have synthetically accessible, cell permeant, nontoxic ligands as described above.

Preferably the ligand binding domain is for (i.e., binds to) a ligand which is not itself a gene product (i.e., is not a protein), has a molecular weight of less than about 5 kD and preferably less than about 2.5 kD, and optionally is cell permeant. In many cases it will be preferred that the ligand does not have an intrinsic pharmacologic activity or toxicity which interferes with its use as an oligomerization regulator.

The DNA sequence encoding the ligand binding domain can be subjected to mutagenesis for a variety of reasons. The mutagenized ligand binding domain can provide for higher binding affinity, allow for discrimination by a ligand between the mutant and naturally occurring forms of the ligand binding domain, provide opportunities to design ligand-ligand binding domain pairs, or the like. The change in the ligand binding domain can involve directed changes in amino acids known to be involved in ligand binding or with ligand-dependent conformational changes. Alternatively, one may employ random mutagenesis using combinatorial techniques. In either event, the mutant ligand binding domain can be expressed in an appropriate prokaryotic or eukaryotic host and then screened for desired ligand binding or conformational properties.

The ability to employ in vitro mutagenesis or combinatorial modifications of sequences encoding proteins allows for the production of libraries of proteins which can be screened for binding affinity for different ligands. For example, one can randomize a sequence of 1 to 5, 5 to 10, or 10 or more codons, at one or more sites in a DNA sequence encoding a binding protein, make an expression construct and introduce the expression construct into a unicellular microorganism, and develop a library of modified sequences. One can then screen the library for binding affinity of the encoded polypeptides to one or more ligands. The best affinity sequences which are compatible with the cells into which they would be introduced can then be used as the ligand binding domain for a given ligand. The ligand may be evaluated with the desired host cells to determine the level of binding of the ligand to endogenous proteins. A binding profile may be determined for each such ligand which compares ligand binding affinity for the modified ligand binding domain to the affinity for endogenous proteins. Those ligands which have the best binding profile could then be used as the ligand. Phage display techniques, as a non-limiting example, can be used in carrying out the foregoing.

In other embodiments, antibody subunits, e.g. heavy or light chain, particularly fragments, more particularly all or part of the variable region, or single chain antibodies, can be used as the ligand binding domain. Antibodies can be prepared against haptens which are pharmaceutically acceptable and the individual antibody subunits screened for binding affinity. cDNA encoding the antibody subunits can be isolated and modified by deletion of the constant region, portions of the variable region, mutagenesis of the variable region, or the like, to obtain a binding protein domain that has the appropriate affinity for the ligand. In this way, almost any physiologically acceptable hapten can be employed as the ligand. Instead of antibody units, natural receptors can be employed, especially where the binding domain is known. In some embodiments of the invention, a fusion protein comprises more than one ligand binding domain. For example, a DNA binding domain can be linked to 2, 3 or 4 or more ligand binding domains. The presence of multiple ligand binding domains means that ligand-mediated cross-linking can recruit multiple fusion proteins containing transcription activation domains to the DNA binding domain-containing fusion protein.

iv. General Methodologies

In applications of the invention involving the genetic engineering of cells within (or for use within) whole animals, the use of peptide sequence derived from that species is preferred when possible. For instance, for applications involving human therapy, the use of catalytic or targeting domains derived from human proteins may minimize the risk of immunogenic reactions. For example, a single chain antibody to be used as a targeting moiety may preferably be a humanized or human-derived single chain antibody. Likewise, other portions of adzymes, such as Fc portions or oligomerization domains may be matched to the species in which the adzyme is to be used.

E. Miscellaneous Features for Adzymes

*(i) Serum Half-Life*

In certain embodiments of the invention, the subject adzyme can be designed or modified to exibit enhanced or decreased serum half-life. Enhanced serum half-life may be desirable to reduce the frequency of dosing that is required to achieve therapeutic effectiveness. Enhanced serum half-life of adzyme may be additionally desirable, since adzyme advantages over pure binding agents may not be realized immediately, but will be more and more apparent over time. For example, the rate of reaction between an adzyme and a low-abundance (e.g., fempto- or pico-molar) substrate, such as certain extracellular signaling molecules, may occur on a timescale of days to weeks; accordingly, a serum half-life allowing adzyme to persist in the body for days or weeks would be desirable and would decrease the frequency of dosing that is needed. Accordingly, in certain embodiments, the serum half-life of an adzyme is at least one day, and preferably two, three, five, ten, twenty or fifty days or more. On the other side, decreased adzyme serum half-life may be desirable in, for example, acute situations, where swift alteration of a substrate will generally accomplish the desired therapeutic effect, with little added benefit resulting from prolonged adzyme activity. In fact, it may be possible to deliver very high levels of an adzyme with a short half-life, such that a high level of therapeutic effectiveness is rapidly achieved, but the adzyme is quickly cleared from the body so as to reduce side effects that may be associated with high dosages. Examples of acute situations include poisonings with various toxins, where the adzyme neutralizes or otherwise eliminates the toxin, as well as sepsis or other severe fevers, where removal of endogenous pyrogens, such as IL-1 or TNF-$\alpha$, or exogenous pyrogens, such as bacterial lipopolysaccharides, may accomplish the therapeutic purpose.

Serum half-life may be determined by a variety of factors, including degradation, modification to an inactive form and clearance by the kidneys. For example, an effective approach to confer resistance to peptidases acting on the N-terminal or C-terminal residues of a polypeptide is to add chemical groups at the polypeptide termini, such that the modified polypeptide is no longer a substrate for the peptidase. One such chemical modification is glycosylation of the polypeptides at either or both termini. Certain chemical modifications, in particular polyethylene glycols ("pegylation") and N-terminal glycosylation, have been shown to increase the half-life of polypeptides in human serum (Molineux (2003), Pharmacotherapy 8 Pt 2:3S-8S.Powell et al. (1993), Pharma. Res. 10: 1268-1273). Other chemical modifications which enhance serum stability include, but are not limited to, the addition of an N-terminal alkyl group, consisting of a lower alkyl of from 1 to 20 carbons, such as an acetyl group, and/or the addition of a C-terminal amide or substituted amide group.

In certain embodiments, an adzyme may be modified, so as to increase the hydrodynamic volume of the adzyme, thereby, among other things, reducing elimination from the kidneys. For example, modification with an inert polymer, such as polyethylene glycol, tends to decrease elimination through the kidneys. A polymer may be of any effective molecular weight, and may be branched or unbranched. For polyethylene glycol, the preferred molecular weight is between about 1 kDa and about 100 kDa (the term "about" indicating that in preparations of polyethylene glycol, some molecules will weigh more, some less, than the stated molecular weight) for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a therapeutic protein or analog). For example, the polyethylene glycol may have an average molecular weight of about 200, 1000, 5000, 15,000, 30,000 50,000, or 100,000 kDa or more. The polyethylene glycol may have a branched structure. Branched polyethylene glycols are described, for example, in U.S. Pat. No. 5,643,575; Morpurgo et al., Appl. Biochem. Biotechnol. 56:59-72 (1996); Vorobjev et al., Nucleosides Nucleotides 18:2745-2750 (1999); and Caliceti et al., Bioconjug. Chem. 10:638-646 (1999). The polyethylene glycol molecules (or other chemical moieties) may be attached to the adzyme with consideration of effects on catalytic or targeting portions. There are a number of attachment methods available to those skilled in the art, e.g., EP 0 401 384, (coupling PEG to G-CSF), see also Malik et al., Exp. Hematol. 20:1028-1035 (1992) (reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues glutamic acid residues and the C-terminal amino acid residue. Sulfhydryl groups may also be used as a reactive group for attaching the polyethylene glycol molecules. Preferred for therapeutic purposes is attachment at an amino group, such as attachment at the N-terminus or lysine group.

Adzymes may be designed to have a molecular weight of about 50 kilodaltons or greater so as to reduce elimination through the kidneys.

The presence of an N-terminal D-amino acid also increases the serum stability of a polypeptide that otherwise contains L-amino acids, because exopeptidases acting on the N-terminal residue cannot utilize a D-amino acid as a substrate. Similarly, the presence of a C-terminal D-amino acid also stabilizes a polypeptide, because serum exopeptidases acting on the C-terminal residue cannot utilize a D-amino acid as a substrate. With the exception of these terminal modifications, the amino acid sequences of polypeptides with N-terminal and/or C-terminal D-amino acids are usually identical to the sequences of the parent L-amino acid polypeptide.

Substitution of unnatural amino acids for natural amino acids in a subsequence of a polypeptide can confer or enhance desirable attributes including biological activity. Such a substitution can, for example, confer resistance to proteolysis by exopeptidases acting on the N-terminus. The synthesis of polypeptides with unnatural amino acids is routine and known in the art (see, for example, Coller, et al. (1993), cited above).

In another embodiment, adzyme peptides are fused to certain polypeptides to achieve enhanced / increased serum stability or half life. For example, WO 97/34631 A1 describes recombinant vectors encoding immunoglobulin-like domains and portions thereof, such as antibody Fc-hinge fragments, subfragments and mutant domains with expended biological half lives. Such vectors can be used to generate large quantities of fusions with such domains following expression by host cells. These antibody Fc and Fc-hinge domains have the same in vivo stability as intact antibodies. The application also discloses domains engineered to have increased in vivo half lives. These DNA constructs and protein domains can be adapted for use in the instant invention, such as for the production of recombinant adzymes (or adzyme components) with increased stability and longevity for therapeutic and diagnostic uses.

Specifically, WO 97/34631 A1 describes recombinant vectors encoding immunoglobulin-like domains and portions thereof, such as antibody Fc fragments and sub fragments and Fc-hinge domains with extended in vivo half lives. As the invention is exemplified by the production of a variety of immunoglobulin-like domains, including antibody Fc-hinge, Fc, CH2-hinge and CH3 domains; and engineered Fc-hinge domains with extended in vivo half lives, such as, for example, the mutant termed LSF. In addition, other immunoglobulin-like domains may be expressed employing the methods described therein.

Previous studies indicate that the CH2 domain may play an important role in the control of catabolism of antibodies, and sequences in the CH3 domain may be involved (Ellerson et al., 1976, Mueller et al., 1990; Pollock et al., 1990; Kim et al., 1994a: Medesan el al., 1997). The presence of carbohydrate residues on the CH2 domain appears to have a minor if significant effect on the stability, and the extent of the effect is dependent on the isotype (Tao and Morrison, 1989).

Recombinant CH2-hinge, CH3, Fc and Fc-hinge fragments derived from the murine IgG1 and human constant regions have been expressed from host cells. The CH3 domain, Fc fragment and Fc-hinge fragment were all found to be homodimeric proteins. For the Fc and CH3 domain, the dimers are non-covalently linked, and are presumably stabilized by non-covalent interactions. For the Fc-hinge dimer, the fragments are covalently linked by -S-S-bridges between the hinge region cysteines. These domains may also be used to dimerize the adzyme target and catalytic domains.

The immunoglobulin Fc-hinge and Fc fragments, purified following expression in host cells, have the same in vivo stability as a native antibody molecule. Results from previous studies demonstrated that the recombinant aglycosylated Fc-hinge or Fc fragments have similar stability in vivo as the complete glycosylated IgG1 molecule. The recombinant aglycosylated Fc-hinge fragment was found to have a 0 phase similar to that of a complete glycosylated IgG1 inimunoglobulin molecule. In fact the removal of Fc-hinge resulted in a slight decrease in half life (Kim el al., 1995). These results indicate that for the murine IgG1 isotype the presence of carbohydrate residues does not appear to be necessary for in vivo stability, although it may still play a minor role. Previous data obtained using protein chemistry suggested that the CH2 domain is responsible for in vivo stability (Ellerson el al.. 1976) although a recent study indicated that residues in the CH3 domain may also be involved in the catabolism control of the murine IgG2a and IgG2b isotypes (Pollock et al., I 990).

*(ii) Dosing Frequency*

In many instances, an adzyme may be administered by injection or another administration route that may cause some discomfort to a patient, or the adzyme may require the assistance of a physician or other medical professional for safe administration. In such instances, it may be desirable to design an adzyme that is therapeutically effective at dosing frequencies of once per day or less, and preferably the adzyme is effective when administered once per week, once every two weeks, once every four weeks, once every eight weeks or less frequently.

The range of effective dosing frequencies for an adzyme may depend on a variety of characteristics of the adzyme. For example, an adzyme with a shorter serum half-life will tend to be effective for a shorter period of time, leading to a more frequent dosing schedule. Various adzyme characteristics that can extend or decrease serum half-life are described above.

Drug reservoirs in the body may lengthen the time over which an adzyme is effective. Upon dosing, many drugs accumulate in body compartments, such as the fat reserves or various transcellular fluids, from which the drug is then released slowly over a long period of time. Similarly, a drug may be tightly bound by a serum protein, such as albumin or alpha1-glycoprotein and thus retained in the serum in an inactive, protected bound form, from which it may be released slowly over time. Accordingly, an adzyme may be designed to encourage the formation of reservoirs that provide for extended periods of effectiveness of the adzyme. In such embodiments, the adzyme may be administered in a higher initial dose (a "loading dose"), followed by occasional smaller doses ("maintenance doses").

An adzyme may also be formulated and administered so as to have an extended period of effect. For example, an adzyme may be formulated and administered to form a "depot" in the patient that slowly releases the adzyme over time. A depot formulations may be one in which the adzyme is encapsulated in, and released slowly from, microspheres made of biodegradable polymers (e.g., polylactic acid, alginate). Other depot materials include gelfoam sponges and the ProLease® system (Alkermes, Inc., Cambridge, MA).

*(iii) Selectivity*

In many instances, an adzyme will be designed for delivery into a particular milieu. For example, many adzymes for use in humans will be designed for delivery to and/or activity in the blood stream. As described herein, adzymes may be designed for other situations, such as for use in an industrial or environmental setting. In general, it will be desirable to design an adzyme so as to decrease interactions with non-target molecules that inhibit the effectiveness of the adzyme against the target, or, in other words, it will be desirable to design an adzyme that is active against target in the presence of expected levels of other components of the milieu in which the adzyme will be used.

In certain embodiments, an adzyme is designed to be effective against a substrate located in the blood, such as, for example, many extracellular signaling molecules. Such an adzyme may be designed to minimize interactions with other blood components that would interfere with the ability of the adzyme to affect the target. The adzyme may be so designed on the basis of theoretical understanding or on the basis of empirical study, or both. In certain embodiments, an adzyme retains effectiveness against a target in the presence of one or more relatively abundant blood components. An adzyme may be tested for activity against target in the presence of one or more blood components, and particularly abundant blood components. For example, an adzyme may be tested for activity against target in the presence of one or more abundant serum proteins, such as serum albumin (e.g., human serum albumin or other organism-specific albumin), transthyretin ("retinol binding protein"), $\alpha$-1 globulins (e.g., a-1 protease inhibitor [$\alpha$-1 antitrypsin], $\alpha$-1 glycoprotein, high density lipoprotein [HDL]), $\alpha$-2 globulins ($\alpha$-2 macroglobulin, antithrombin III, ceruloplasmin, haptoglobin), $\beta$-globulins (e.g., beta and pre-beta lipoproteins [LDL and VLDL], C3, C-reactive protein, free hemoglobin, plasminogen and transferrin), $\gamma$-globulins (primarily immunoglobulins). In certain embodiments, an adzyme of the invention is active against target in the presence of expected (i.e., physiological, depending on the physiological state of the patient) concentrations of one or more blood components, such as one or more abundant serum proteins. Optionally, the adzyme is active against target in the presence of expected concentrations of an abundant serum protein, and optionally is not significantly affected by concentrations of an abundant serum protein that are one-quarter, one-half, two, five or ten or more times greater than the expected concentration of an abundant serum protein. In a preferred embodiment, the adzyme comprises a catalytic domain that interacts with a polypeptide target that is expected to be found in the blood, and optionally the catalytic domain has protease activity. Other abundant blood components include any of the various cell types, and molecules found on the surfaces thereof. Common blood cell types include red blood cells, platelets, neutrophils, lymphocytes, basophils, eosinophils and monocytes.

*(vi) Resistance to Autocatalysis*

In certain embodiments, the catalytic domain of an adzyme may be able to catalyze a reaction with the adzyme itself, resulting in the alteration of the adzyme. This type of reaction, termed "autocatalysis" may be between a catalytic domain and some other portion of the same adzyme (e.g., a linker, targeting moiety or other part) or between a catalytic domain of one adzyme and a portion of a second adzyme (e.g., the catalytic domain, linker, targeting moiety). The former will tend to be more significant relative to the latter at very low adzyme concentrations, such as may be expected to occur after an adzyme has been deployed in a patient or other setting. The inter-adzyme form of autocatalysis is most likely to occur at higher concentrations, such as during adzyme preparation (e.g., purification from cell cultures and subsequent concentration), storage and in any mixture prepared for administration to a subject (e.g., a dose of adzyme mixed with saline for administration intravenously).

For most types of catalytic domains, autocatalysis will be a relatively unimportant phenomenon, if it occurs at all. For example, catalytic domains that mediate glycosylation, isomerization or phosphorylation may not affect the activity of an adzyme, even if it does undergo autocatalysis. However, in certain situations, a modification of an adzyme could disrupt the ability of the adzyme to act effectively on its target, particularly a modification that occurs in the binding portion of an address moiety or in the active portion of a catalytic domain. Many types of catalytic domains require some type of co-factor (e.g., ATP for a kinase, a sugar for a glycotransferase), and therefore autocatalysis will not occur in the absence of such co-factors. In these circumstances, autocatalysis may be avoided during preparation or storage by ensuring that there is little or no co-factor present in the adzyme preparation.

Catalytic domains that have protease activity or are otherwise are capable of degrading the adzyme are of particular concern. Proteases often do not require any co-factor, and therefore autoproteolytic activity may well occur at any stage of adzyme generation or use. A variety of approaches may be taken to prevent autoproteolysis.

In one embodiment, an adzyme may be designed, or a protease domain selected, such that the protease is active at low levels in the absence of the target. See, for example, the description of contingent adzymes provided herein.

In certain embodiments, protease vulnerable sites may be engineered out of the various portions of an adzyme, such as any polypeptide address domain, catalytic domain or linker. This may be achieved either by altering the sequence of the selected components, or by selecting components in the first place that show resistance to cleavage with the desired protease domain. Trypsin has an internal trypsin vulnerable site and is susceptible to trypsin action for inactivation, however trypsin-resistant trypsin mutants may be generated. Often theoretic protease sensitive sites are present in various domains but are not, in practice, viable protease substrates, perhaps due to folding or other steric hindrances. For example, Applicants have found that a p55(TNFR)-thrombin fusion protein adzyme does not undergo autocatalytic proteolysis, despite the presence of a thrombin cleavage site within the p55(TNFR) polypeptide. Such folding may be adjusted by the presence or absence of agents such as monovalent or divalent cations (e.g., potassium, calcium, zinc, iron) or anions (e.g., phosphates, chloride, iodine), as well as nonionic, zwitterionic and ionic detergents.

In certain embodiments, an address domain, such as a single chain antibody or other scaffold-based address domain, may be arrived at by in vitro RNA selection. In vitro selection allows the selection for protease insensitive address domains and thereby building an address domain that will not be cleaved by the enzyme domain. Similar approaches may be used for linkers, immunoglobulin portions or other polypeptides to be incorporated in an adzyme.

Anther means of limiting auto-proteolysis is to produce the catalytic domain as a zymogen and activate the adzyme just prior to use (e.g., delivery to a patient). A zymogen or pro-protein portion may also be designed to be cleaved upon use (e.g., by a known serum active protease). Cleavage of certain zymogens occurs in the N-terminal direction from the protease domain, meaning that after activation, the protease domain will be separated from the portion of the polypeptide that is N-terminal to the cleavage site. Cleavage of a zymogen that occurs in the N-terminal direction from the protease domain, means that after activation, the protease domain will be separated from the portion of the polypeptide that is N-terminal to the cleavage site. Cleavage of certain zymogens occurs in the C-terminal direction from the protease domain, meaning that after activation, the protease domain will be separated from the portion of the polypeptide that is C-terminal to the cleavage site. Accordingly, a fusion protein comprising a zymogen should be designed such that the protease domain is not separated (unless that is the intent) from the other relevant portion of the fusion protein upon activation.

In further embodiments, reversible competitive inhibitors may be employed. Such inhibitors are preferably selected so as to be readily removable. An inhibitor for use in a pharmaceutical preparation may be selected to have a Ki that allows effective inhibition in the high concentrations of storage and pre-administration, but which readily releases the protease upon dilution in the site of action (e.g., in the patient's body). Preferably, the inhibtor is chosen to be non-toxic or otherwise clinically approved. Inhibitors may also be used during production and purification of adzymes. Many proteases require a metal cofactor, and such proteases can often be reversibly inhibited by formulation with a chelator, such as EDTA, EGTA, BHT, or a polyanion (e.g., polyphosphate).

In a further embodiments, protease vulnerable sites may be post-translationally modified. Protease vulnerable sites could be modified by phosphorylation or methylation or glycoyslation or chemically (in vitro, as opposed to modifications post translationally during production) such that the protease domain can not bind.

As a merely illustrative example, the competitive inhibitor benzamidine has been used to block the action of trypsin in the trypsinogen-p55 anti-TNF adzymes. The benzamidine has increased the yield of adzyme in the transient transfection expression of the trypsinogen adzyme. Benzamadine, boronic acid or other protease inhibitors may be useful for manufacturing adzymes. With respect to the MMP7 catalytic domain, inhibitors such as Thiorphan, Ilomastat, FN 439, Galardin or Marimastat may be employed.

*F. Exemplary Methods for Designing Adzymes*

A significant advantage of adzymes is that they admits of an engineering and design approach that permits the biomolecular engineer to resolve several of the multiple engineering challenges inherent in drug design serially rather than simultaneously. A drug must not only bind the target with high potency, but also it must have one or a combination of medicinal properties. In a given drug discovery/design exercise, the candidate molecule must exhibit various combinations of the following properties: a suitable solubility in blood, no significant inhibition of unintended targets (the higher specificity the better), achieve an effective concentration at the target, pass biological barriers such as the skin, gut, cell walls, or blood brain barrier, have no toxic metabolites, be excreted at a rate permitting achievement of necessary bioavailability without kidney or liver damage, not interfere with commonly prescribed medications, avoid complexation with albumin or other biomolecules or sequestration in tissue compartments, and be synthetically tractable. A single molecular entity simultaneously displaying all necessary combinations of these properties is very hard to find or design.

In contrast, the individual molecular moieties that comprise the adzyme, *e.g.*, the address and the catalytic domain, can be screened individually for the ability to bind to or modify the target of interest, respectively. Candidate structures for these parts can be taken from the ever growing public knowledge of new biological molecules and and engineering efforts supported by increased understanding of their molecular biology and pharmacology. Existing active enzymes can be mutated to give them an address that will confer a new specificity. Nixon et al., in Proc. Natl. Acad. Sci. USA, Biochemistry Vol. 94, p.1069, 1997, have validated the approach of constructing an active enzyme from disparate functional parts of other enzymes. Good candidates for each function may be linked together using various types of linking strategies. For example, they may be inserted into loops, attached via flexible or structured amino acid sequence or other covalent attachments. Candidate constructs are made by choosing amino acid sequence or other structure spaced apart from the binding or catalytic portion of each domain for their ability to non-covalently complex, or via candidate chaperone proteins that complex to both domains. It is contemplated that many experimental constructs will be made in parallel, and that the library of constructs may be screened for desired activity, and active species evolved by mutagenesis or otherwise altered to explore adjacent chemical space for improved properties.

Address domains can be selected using *in vivo* or *in vitro* assays. The address can be tested for the ability to bind to the target of interest using assays for direct binding or assays that measure the activity of the target molecule. Methods that can be used to measure binding of the address to the target molecule include biophysical and biochemical techniques. For example, biophysical methods include fluorescence techniques which rely on intrinsic fluorescence or which rely on the addition of an extrinsic label, *e.g.*, fluorescence energy transfer, fluorescence anisotropy, changes in intrinsic fluorescence of the target molecule or address domain upon binding (see Lakowitz, J. R. (1983) Principles of Fluorescence Spectroscopy, Plenum Press, New York). Surface plasmon resonance (Sjolander, S. and Urbaniczky, C. (1991) Anal. Chem. 63:2338-2345 and Szabo et al. (1995) Curr. Opin. Struct. Biol. 5:699-705) can be used to study biospecific interactions in real time, without labeling any of the interactants (*e.g.*, BIAcore). Changes in the optical phenomenon of surface plasmon resonance can be used as an indication of real-time reactions between biological molecules.

Biochemical techniques that can be used to test the ability of the address domain to bind to the target molecule include techniques such as immunoprecipitation and affinity chromato graphy.

Further, one of both of the molecules can be labeled using a radioisotope, *e.g.*, $^{125}$I, $^{35}$S, $^{14}$C, or $^{3}$H or other detectable label, *e.g.*, an enzyme, and the interaction between the two molecules can be measured by specifically isolating one molecule and measuring the amount of the second molecule that is associated with the first molecule. In the case of a radiolabel, the amount of radio-labeled protein that is isolated can be measured by counting of radio emmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

Address domains (*e.g.*, a target specific peptide, target specific single chain antibody) may be taken from known examples in the literature, preferably from examples of human proteins. Alternatively, address domains may be identified by any of a number of recombinant display techniques, including but not limited to phage display, yeast display, ribosome display, and bacterial display. Methods for preparing and screening libraries of address domains, *e.g.*, peptide or antibody libraries, are well known in the art and include those described in U.S. Patent Nos. 6,156,511; 5,733,731; 5,580,717; 5,498,530; 5,922,545; 5,830,721; 5,811,238; 5,605,793; 5,571,698; 5,223,409; 5,198,346; 5,096,815;

5,403,484; 6,180,336; 5,994,519; 6,172,197; 6,140,471; 5,969,108; 5,872,215; 5,871,907; 5,858,657; 5,837,242; 5,733,743; 5,962,255; 5,565,332; and 5,514,548. Libraries may be functionally selected or screened to identify specific address domains exhibiting the desired properties (*e.g.*, affinity for a target, signal to noise ratio, etc.). A recombinant display technique may also be used to identify candidate address domains. Useful recombinant display techniques include, but are not limited to, phage display (see Hoogenboom et al., Immunol Today 2000 Aug;21(8):371-8), single chain antibody display (see Daugherty et al. (1999) Protein Eng 12(7):613-21; Makeyev et al., FBBS Lett 1999 Feb 12; 444(2-3):177-80), retroviral display (see Kayman et al., J Virol 1999 Mar;73(3):1802-8), bacterial surface display (see Earhart, Methods Enzymol 2000;326:506-16), yeast surface display (see Shusta et al., Curr Opin Biotechnol 1999 Apr; 10(2):117-22), ribosome display (see Schaffitzel et al., J Immunol Methods 1999 Dec 10;231(1-2):119-35), Profusion™ technology (nucleic acid:protein covalent complexes, see e.g., U.S. Patent Nos. 6,207,446; 6,214,553; 6,258,558; 6,261,804; 6,281,344; 6,518,018, which permits the generation and screening of highly diverse polypeptide libraries, including libraries of, e.g., single chain antibodies or $V_H$ or $V_L$ libraries), two-hybrid systems (see, e.g., U.S. Patent Nos. 5,580,736 and 5,955,280), three-hybrid systems, and derivatives thereof. Recombinant display techniques identify address domains capable of binding targets, *e.g.*, proteins (see, for example, Bara et al., Proc Natl Acad Sci U S A 1997 Sep 16;94(19):10063-8; Katz, Biomol Eng 1999 Dec 31;16(1-4):57-65; Han et al., J Biol Chem 2000 May 19;275(20): 14979-84 ; Whaley et al., Nature 2000 Jun 8;405(6787):665-8; Fuh et al., J Biol Chem 2000 Jul 14;275(28):21486-91; Joung et al., Proc Natl Acad Sci U S A 2000 Jun 20.97(13):7382-7; Giannattasio et al., Antimicrob Agents Chemother 2000 Jul;44(7):1961-3).

Catalytic domains can be screened based on their activity. Depending on the specific activity of each molecule being tested, an assay appropriate for that molecule can be used. For example, the assay used to screen the protease activity of the mesotrypsin catalytic domain can be an assay to detect cleavage products generated by the protease, *e.g.*, a chromatography or gel electrophoresis based assay. In an alternative example, the targeted substrate may be labeled and cleavage of the labeled product may produce a detectable product by, for example, a change in fluorescence of the targeted substrate upon cleavage.

It should be noted that the pharmacodynamics (binding and kinetic properties) of the interactions among the molecular address domains, targets, substrates, inhibitors, and enzymatically active sites will often be important properties of candidate constructs embodying the invention. Thus, association and dissociation properties, on-rates, off-rates, and catalytic reaction rates interplay in the various constructs to achieve the desired result. These properties are engineered into the molecules by a combination of rational, structure based design and manufacture of a multiplicity of candidate constructs, or sub-parts thereof, which are screened for appropriate activity, as disclosed herein.

Methods for preparing and screening catalytic domains for the desired activity are well known in the art and described in, for example, U.S. Patent No. 6,383,775 and U.S. Provisional Patent Application Serial No. 60/414,688.

Once the address domain and the catalytic domain have been incorporated into a single molecule a library of adzymes may then be created. The resulting library can be screened for the ability to modify the specific target of interest. An assay for the appropriate biological function can be used to quantitate the amount of modification the catalytic domain carries out. In a preferred embodiment, the catalytic domain is a protease and the assay is one that measures the amount of cleavage product generated by cleavage of the target molecule. It may also be effective to measure biophysical parameters, *e.g.*, $k_{cat}$ or $K_M$, of the select library members. In another embodiment, the assay to screen the library of adzymes can be one which measures the biological activity of the target molecule or a downstream molecule that is regulated by the target molecule.

Once an adzyme, or group of adzymes, has been identified in a selection or screen, its properties may be further enhanced by one or more rounds of mutagenesis and additional selection/screening according to art known methods. Furthermore, a catalytic domain of general utility, such as a protease, may be used in constructs designed for very different purposes.

A library of adzymes comprising combinations of address domains, linkers, and enzymes may be generated using standard molecular biology protocol. Either the address domain or the enzyme domain may be at the N-terminal of the adzyme. The size / length, composition (amino acid sequence) may be varied. Nucleic acids encoding the address domain, the linker, and the enzyme domain can be recombinantly fused and cloned in suitable expression vectors, under the control of operatively linked promoters and transcription regulators. The construct may also include epitope tags to facilitate purification of the recombinant products.

The desired combination of different address domain, linker, and enzyme domain can be generated, for example, by brute force construction of a desired number of candidate adzymes. Each of these adzymes can then be individually tested and compared in one or more of in vivo and/or in vitro functional assays, either for the adzyme itself, or for the target of the adzyme, or both.

Once an adzyme, or group of adzymes, has been identified in a selection or screen, its properties may be further enhanced by one or more rounds of mutagenesis and additional selection/screening according to art known methods. Furthermore, a catalytic domain of general utility, such as a protease, may be used in constructs designed for very different purposes.

To illustrate, U.S. Pat. No. 6,171,820 describes a rapid and facilitated method of producing from a parental template polynucleotide, a set of mutagenized progeny polynucleotides whereby at each original codon position there is produced at least one substitute codon encoding each of the 20 naturally encoded amino acids. Accordingly, the patent also provides a method of producing from a parental template polypeptide, a set of mutagenized progeny polypeptides wherein each of the 20 naturally encoded amino acids is represented at each original amino acid position. The method provided is termed "site-saturation mutagenesis," or simply "saturation mutagenesis," and can be used in combination with other mutagenization processes described above. This method can be adapted to fine-tune / optimize the final chosen combination of address domain, linker, and enzyme domain, so that the adzyme exhibits desired the biological property.

*G. Contigent Adzymes*

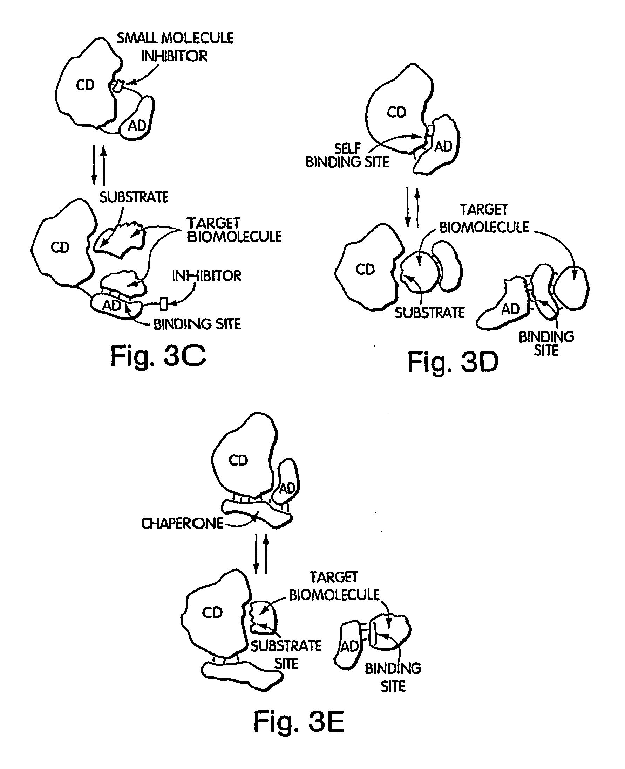

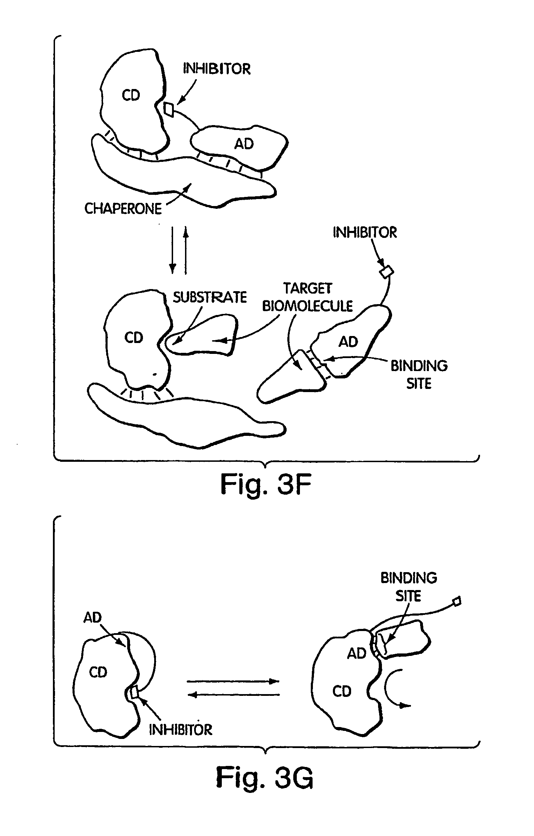

In one important class of adzymes, the activity of the catalytic domain is modulated by the binding of the address to an address binding site (on the target or target associated molecule). Thus, the activity of the catalytic domain may be modulated by target itself, by a target associated molecule, or by part of the adzyme molecule itself. In this class of constructs, the catalytic domain itself is "masked" or sterically hindered, thus mostly inactive, when the address is not bound by an address binding site. Once the address recognizes and binds an address binding site (e.g., when the adzyme reaches its target), such hinderance is released, exposing the active catalytic domain to act on the target. There could be many embodiments of this type of so-called "contingent adzymes." See Figure 3.

*H. Adzymes for Use in Treatment*

The present invention also provides the adzymes of the invention for use in a method of treating a subject suffering from a disease, such as a disease associated with a soluble or solvent accessible molecule. The method includes administering to the subject a therapeutically, prophylactically, or analgesically effective amount of an adzyme of the invention, thereby treating a subject suffering from a disease. Generally, adzymes can be designed and used for treating any disease mediated by a solvent accessible signaling factor in extracellular body fluid or on a cell surface.

A disease associated with a soluble molecule includes a disease, disorder, or condition, which is caused by or associated (*e.g.,* directly or indirectly) with a soluble or membrane bound biomolecule, such as a cytokine or a growth factor or a GPCR. Examples of such diseases include inflammatory diseases, such as asthma, psoriasis, rheumatoid arthritis, osteoarthritis, psoriatic arthritis, inflammatory bowel disease (Crohn's disease, ulcerative colitis), sepsis, vasculitis, and bursitis; autoimmune diseases such as Lupus, Polymyalgia, Rheumatica, Scleroderma, Wegener's granulomatosis, temporal arteritis, cryoglobulinemia, and multiple sclerosis; transplant rejection; osteoporosis; cancer, including solid tumors (*e.g.,* lung, CNS, colon, kidney, and pancreas); Alzheimer's and other neurodegenerative disease; atherosclerosis; viral (*e.g.,* HIV or influenza) infections; chronic viral (*e.g.,* Epstein-Barr, cytomegalovirus, herpes simplex virus) infection; and ataxia telangiectasia.

Adzymes may be used for anti-TNF therapies in place of antibodies, artificial constructs, or small molecules. They can be used to treat Wegner's vasculititus, Psoriasis, ankylosing spondylitis, Psoriatic arthritits, Crohn's and other IBD, and rheumatoid arthritis. They may also be used for routine or rapid intervention in infectious disease cased by bacteria and virus, attacking the infectious agent directly via cell surface proteins or circulating toxins or immune complexes. Examples of particularly promising soluble targets in addition to TNF include IgE, C5, TGFβ, VEGF, and Interlukines such as IL-1.

Adzymes can be used in warm-blooded animals, preferably mammals, including humans. In a preferred embodiment, the subject is a primate. In an even more preferred embodiment, the primate is a human.

As used herein, the term "administering" to a subject includes dispensing, delivering or applying an adzyme of the invention *e.g.,* an adzyme in a pharmaceutical formulation, to a subject by any suitable route for delivery of the composition to the desired location in the subject, including delivery by either the parenteral or oral route, intramuscular injection, subcutaneous/intradermal injection, intravenous injection, buccal administration, transdermal delivery and administration by the rectal, colonic, vaginal, intranasal or respiratory tract route. The catalytic machines of the invention also may be administered by gene therapy approaches wherein nucleotides encoding the constructs are administered to a patient, migrate or are transported to target cells, eater the cells, and are expressed to provide the cells with a therapeutic engineered intelligent machine.

The adzymes of the present invention can be provided alone, or in combination with other agents that modulate a particular pathological process. For example, an adzyme of the present invention can be administered in combination with other known agents useful in the treatment of diseases associated with or caused by a soluble molecule. Known agents that may be used in the methods of the invention can be found in Harrison's Principles of Internal Medicine, Thirteenth Edition, Eds. T.R. Harrison et al. McGraw-Hill N.Y., NY; and the Physicians Desk Reference 50th Edition 1997, Oradell New Jersey, Medical Economics Co.. The adzymes of the invention and the additional agents may be administered to the subject in the same pharmaceutical composition or in different pharmaceutical compositions (at the same time or at different times). In one embodiment, one or more adzymes which are specific for one or more targets, are administered to a subject simultaneously. In another embodiment, the separate domains of the adzymes (*i.e.,* the address domain and the catalytic domain) may be administered to a subject separately. In such an embodiment, the address domain and the catalytic domain assemble *in vivo* to form the adzyme.

The present invention also provides the adzymes of the invention for use in a method of treating a subject suffering from a disease, such as a disease associated with an antigen which fails to elicit appropriate host immune response, including certain tumor antigens. The method includes administering to the subject a therapeutically, prophylactically, or analgesically effective amount of an adzyme of the invention, said adzyme selectively modify a target protein associated with the disease, such that the modified target protein becomes more antigenic and elicits a strong host immune response, leading to the destruction of the modified target protein or cells associated therewith, thereby treating a subject suffering from such a disease.

For example, certain weak antigens may have masked epitopes, thus such antigens usually fail to elicit a sufficient host immune response. By cleaving the intact antigen using adzyme, the previously masked epitopes will be exposed, resulting in stronger immune response against the antigen.

*L Non-medical Use of Adzymes*

The present invention also provides various uses of adzymes in a number of non-medical applications, including but are not limited to, agriculture, environmental protection, food etc.

For example, the subject adzymes can find a wide range of uses in agriculture, including producing animal feed / pet food, grain milling, ethanol production, and food processing.

**Animal Feed / Pet Food** Adzymes may be used to upgrade nutritional quality and removing anti-nutritional factors from feed components, such as barley- and wheat-based feeds. Corn processing co-products such as gluten meal and fiber can also be improved using the subject adzyme. In fact, a number of food safty crisis in recent history (BSE, dioxin scare, etc.) have made it clear that animal feed has to be considered a public hazard to public health and one that can lead to declining public condifence in the safety of food of animal origin.

In one embodiment, a protease may be linked to an address specific for the undesirable nutritional factor present in feed components, thus leading to the degradation /elimination of such component. An added benefit of such adzyme-assisted digestion is that the degraded (inactive) protein factor is now a nutritious source of protein (peptide fragments). For example, Caughey et al. (J. Virol. 2135-2141, Vol 68, No. 4, Apr 1994) reported that the apparent precursor of protease-resistant PrP (responsible for the Prion disease BSE), protease-sensitive PrP, binds to Congo red and heparin, a highly sulfated glycosaminoglycan. Thus adzymes comprising an address domain of congo red or certain sulfated glycans, and an catalytic domain of a protease that can degrade the protease-sensitive PrP, may be used to pretreat certain animal feeds to reduce the risk of prion disease transmission.

In a related embodiment, adzymes of the instant invention may be used with other enzyme as feed additives to improve nutrition and digestibility, reduce waste and lower feeding costs by increasing the solubility of fibers or proteins from grains such as corn, wheat, barley and soy used in typical feeds. The other enzymes that may be used with adzyme include: protease (protein-digesting enzyme), amylase (carbohydrate-digesting enzymes), lipase (fat-digesting enzyme), eellulase (fiber-digesting enzyme), lactase (milk sugar digestive enzyme), invertase, maltase (sugar digestive enzyme), and/or alpha-galactosidase (bean digesting enzyme).

**Fuel Ethanol** Interest in ethanol as a clean-burning fuel is stronger than ever before. Fossil fuels are finite, nonrenewable and cause harm to the environment through pollution and global warming, yet they supply over 80 percent of the world's energy needs. At the present rate of consumption, world oil reserves are expected to deplete in the next 50 to 100 years. Explosive population growth and improved living standards may shorten that timeframe. Ethanol, a chemical distilled from starch crops like com, barley, sorghum and wheat, is both more fuel-efficient and far less polluting than gasoline. The agriculture industry alone produces around 100 billion tons of biomass (unused crops, trees, grasses, and other agricultural "waste" product) worldwide each year, with an energy value five times that of all energy consumed globally. The starch from these grains is converted to fermentable sugars, which are then converted to alcohol by yeast.

While cellulase has been used in bio-fuel production, current estimate for cellulase cost ranges from 30 to 50 cents per gallon of ethanol produced. To be more competitive in price, the objective in bio-fuel production is to reduce cellulase cost to less than 5 cents per gallon of ethanol. This requires a tenfold increase in specific activity or production efficiency or combination thereof. Thus even a few fold increase in cellulase-specific activity (relative to the *Trichoderma reesei* system) would be an important progress in this direction.

Thus in this embodiment, adzymes with cellulase and other enzymes that can digest biomass into simpler suger moieties more suitable for fermentation may be used to facilitate the fuel ethanol production. The address domain may contain more than one binding domains, such as the putative cellulose binding domains of ≈100 amino acids encoded by the cbpA gene of the *Clostridium cellulovorans* cellulose binding protein (CbpA). As mentioned before, the effective $K_d$ of an adzyme with two identical address domains with $K_d$ of about 1 nM would have a much tighter value of about $10^{-15}$ M, and may increase the catalytic efficiency of cellulose digestion.

<u>**Food Processing**</u> Adzymes may be used in the food industry for such purposes as to improve baking, to process proteins more efficiently, to preserve foods, to treat animal hides in the leather industry, to recover silver residue in photographic film processing, and to improve pulp and paper processing. The use of adzymes offers treatment alternatives that are less harsh than traditional chemical processes. The primary benefit offered by is treatments under mild conditions of temperature and pH. Some adzymes useful for these purposes are used for protein hydrolysis and in modification of cellulose and hemicellulose, others are useful to breakdown hydrogen peroxide in waste streams, or for oxygen and glucose removal in food applications.

All these adzymes may be engineered, at the minimum, by including multiple address domains that may enhance the binding specificity and/or affinity, thus increasing overall activity.

For example, adzymes can be used to improve gluten quality in baked goods, enhance the sensory and physical characteristics of breads, and to facilitate the solubility, functionality and nutrition of meat or vegetable proteins in a diverse range of products from infant formula to sports drinks. Adzymes can also be used to more efficiently convert starch to High Fructose Corn Syrup (HFCS), the sweetener widely used in many foods and especially soft drinks.

<u>**Pulp and Paper Industry**</u> Adzyme comprising xylanases may be used for bleach boosting, adzyme comprising cellulases may be used for refining pulp and paper recycling, and adzyme comprising amylases may be used for starch removal and modification.

<u>**In the brewing process,**</u> adzymes may be used to improve process efficiency and the final products. For example, adzymes comprising alpha amylases can be used in the cooking of cereal adjuncts, adzymes comprisin betaglucanases may be used to improve filtration in mashing and maturation, and adzymes comprisin glucoamylase can be used to produce low calorie beers. In addition, adzymes comprisin alpha amylase and glucomylase may be used in the production of potable alcohol.

<u>**Textile Industry**</u> Adzymes are useful in a variety of applications within the cleaning and fabric care industries. The use of adzymes is beneficial because they often replace chemicals or processes that present environmental issues. But naturally occurring enzymes are quite often not available in sufficient quantities or enzymatic activity / efficiency for industrial use.

The faded or worn look of denim that has a high contrast "stonewashed" look was originally achieved by washing denim with pumice stones in large industrial washing machines. In such a process, the lack of abrasion control, damage to the fabric, and wear and tear on the washing machines is considerable. The same effects can be efficiently achieved using adzymes, in a more environmentally friendly manner. Adzymes may be useful in desizing (amylases), denim finishing (cellulases), biofinishing of cotton and cellulosics (cellulases), and hydrogen peroxide elimination (catalases).

<u>**Personal Care Products**</u> Proteases perform various macromolecular maturation or hydrolytic functions within the body. These functions may be enhanced or modified by the application of exogenously supplied adzymes. For example, applying adzymes to the skin's surface may aid in the breakdown of surface oils and removal of dead skin. Thus, with the use of adzymes in skin, hair and oral care applications, it is possible to supplement the body's natural processes which will result in younger looking skin, more beautiful hair and healthier teeth and gums.

<u>**Detergent / Cleaner Products**</u> In other particularly preferred embodiments, the subject adzymes can be used to target and destroy particular preselected molecules whether or not they have a biological activity. Thus, for example, components of various soils or stains (e.g. milk, blood, eggs, grass stains, oil stains, etc.) can be specifically targeted. For example, avidin/egg protein can be specifically targeted by using a biotin as a targeting moiety to specifically directed, e.g. a protease to the site. The stain is degraded/digested and thereby released from the underlying substrate. Such adzymes are particularly useful in various cleaning formulations.

The term "soil" or "stain" refers to the accumulation of foreign material on a substrate of interest (e.g. a textile). The "soil" or "stain" may have no biological activity, but may serve to discolor, and/or degrade the underlying substrate. The "soil" need not be visible to the naked eye. Deposition of foreign materials that, while not visible to the naked eye, but that create odors or support bacterial growth are also considered "soils" in the context of this application. Typical stains or soils include, but are not limited to grass stains, blood stains, milk stains, egg, egg white, and the like.

The adzymes of this invention are useful in a wide variety of contexts where it is desired to degrade a target molecule and/or inhibit the activity of that target molecule. Thus, for example, in ex vivo applications, the catalytic antagonists can be used to specifically target and degrade a particular molecule. Thus, for example, in cleaning operations, the chimeric molecules of this invention can be utilized to specifically target and degrade a component of a soil (e.g. a protein component, a lipid component, etc.). In chemical synthetic processes, or biochemical synthetic processes (e.g. in analytic or industrial preparations, in bioreactors, etc.) to specifically degrade particular preselected molecules. Thus, for example, where it is desired to eliminate a particular enzymatic activity in a bioreactor (e.g. a glycosylation) the catalytic antagonist of this invention comprises, as a targeting moiety, a substrate for the enzyme mediating the activity (e.g. a glycosyltransferase). The enzyme (receptor) in the reactor binds the targeting moiety and the enzymatic component of the chimera (e.g. a hydrolase) degrades the enzyme reducing or eliminating its activity and also freeing itself from the enzyme binding site whereby it is free to attack another target enzyme.

**Silicon Biotechnology** In biological systems, organic molecules exert a remarkable level of control over the nucleation and mineral phase of inorganic materials such as calcium carbonate and silica, and over the assembly of crystallites and other nanoscale building blocks into complex structures required for biological function (Belcher et al., Nature 381, 56-58, 1996; Falini et al., Science 271: 67-69, 1996; Cha, Proc. Natl Acad. Sci. USA 96: 361-365, 1999; Meldrum et al., Proc. R. Soc. Lond. B 251: 238-242, 1993). This ability to direct the assembly of nanoscale components into controlled and sophisticated structures has motivated intense efforts to develop assembly methods that mimic or exploit the recognition capabilities and interactions found in biological systems (Colvin et al., J. Am. Chem. Soc. 144: 5221-5230, 1992; Brust et al., Adv. Mater. 7: 795-797, 1995; Li et al., Chem. Mater. 11: 23-26, 1999; Alivisatos et al., Nature 382: 609-611, 1996; Mirkin et al., Nature 382: 607-609, 1996; Brown, Proc. Natl Acad. Sci. USA 89: 8651-8655, 1992). Brown (Proc. Natl Acad. Sci. USA 89: 8651-8655, 1992; and Nature Biotechnol. 15: 269-272, 1997) describes the successful selection of peptides with limited selectivity for binding to metal surfaces and metal oxide surfaces. In another study, using combinatorial phage-display libraries, Whaley et al. (Nature 405: 665-668, 2000) extend this approach and successfully screened and selected numerous peptides that bind to a range of semiconductor surfaces with high specificity, depending on the crystallographic orientation and composition of the structurally similar materials used. Whaley et al. have extended this peptide recognition and specificity of inorganic crystals to other substrates, including GaN, ZnS, CdS, $Fe_3O_4$, and $CaCO_3$. Such peptides may be used as the address domains of the subject adzymes and specifically place / assemble a variety of functional macromolecules (such as polypeptides) to pre-determined regions of micro-chips (such as protein arrays, etc.).

Such targeted adzymes may be used for targeted, sequential assembly/synthesis of nanomaterials, either inorganic or organic/inorganic hybrid materials. In one embodiment, a first adzyme may be placed on the surface of a first region of a nano-assembly line /container (tank, flow through tube, and other appropriate designs) such that the functional domain - the catalytic domain - on the adzyme may carry out a first step of a series of processes on a reactant / substrate. The reactant can then be passed on to a second adzyme placed on the surface of a second region of a nano-assembly line / container to allow the next step of the process to finish. This process can be repeated for subsequent steps of the processes until all the reactions are done and the final product emerge.

The protein silicatein, which is isolated from the marine sponge *Tethya aurantia,* catalyzes the *in vitro* polymerization of silica and silsesquioxanes from tetraethoxysilane and silica triethoxides, respectively. When tethered to a specific region of the nano-assembly line, the protein may be used to carry out specific steps of a particular nano-assembly.

Alternatively, different adzymes may be attached to different chips, which can be automatically loaded into or taken out of a reaction container to carry out sequential catalytic steps.

Such targeted adzymes may also be used to construct arrays of (identical or different) molecules on silicon chips. In this regard, the subject adzyme technology can be combined with planned chemical assembly of 3-dimensional (3-D) organic / inorganic nanoscale architectures. This approach is based on processes of surface chemical derivatization and controlled self-assembly taking place on organic template scaffolds produced via a hierarchical layer-by-layer self-assembly strategy. For example, arbitrary 2-D patterns can be generated using a novel nano-patterning process, referred to as "Constructive Nanolithography" [1], whereby electrical pulses delivered by a conductive AFM (atomic force microscope) tip induce local electrochemical transformations selectively affecting the top functions of certain highly ordered organosilane monolayers or thicker films selfassembled on silicon. In this patterning process, the AFM tip plays the role of a nano-electrochemical "pen," with which chemical information is inscribed in a nondestructive manner on the top surface of the selected organic film. The patterned film or the product of its further chemical modification is then further utilized as a template capable of guiding the subsequent surface self-assembly of various targeted adzymes, thus creating a gradually evolving self-assembling system in which each self-assembly step is subject to the control provided by a previously assembled template structure. This hierarchical self-assembly approach offers options for the planned assembly of new types of organic-inorganic nanocomposite architectures with variable dimensionality, from 0-D (individual dots) and 1-D (wires), to 3-D (superlattices) structures.

For example, the surface of an array may comprise a first material that cannot be bound by the addresses of any of a number of adzymes later to be attached. Using the above-described technology, a first area of the surface may be altered such that a first address domain of an adzyme may now bind to the altered region. If the binding is saturating, after removing all of the first adzyme, the same process can be repeated for a second region on the surface to expose a second region, such that a second adzyme (may be with a different catalytic domain) may now bind. Since the ATM tip is controlling the exposure of the surface, various patterns can be etched on the surface sequencially, such that different adzymes with different functions may be selectively attached to different areas of the surface in distinct patterns if necessary.

DNA motifs have been used to produce nanoscale patterns in 2D, including a 2D lattice from a junction with sticky ends (see Seeman and Belcher, Proc Natl Acad Sci U S A. 99 Suppl 2: 6451-5; Apr. 30, 2002; Epub 2002 Mar 05). Thus in other embodiments, adzymes with address domains recognizing specific DNA sequences may be attached to such DNA lattice to create patterned adzyme arrays. The address domain can be naturally existing DNA binding domains, or can be selected for specific DNA binding using, for example, phage display or other similar techniques.

*J. Compositions Containing Adzymes*

*(i) Protein Preparations*

Another aspect of the invention pertains to pharmaceutical compositions containing the adzymes of the invention. The pharmaceutical compositions of the invention typically comprise an adzyme of the invention or nucleotides encoding the same for transfection into a target tissue, and a pharmaceutically acceptable carrier. As used herein "pharmaceutically acceptable carrier" includes any and all solvents, dispersion media, coatings, antibacterial and anti-fungal agents, isotonic and absorption delaying agents, and the like that are physiologically compatible. The type of carrier can be selected based upon the intended route of administration. In various embodiments, the carrier is suitable for intravenous, intraperitoneal, subcutaneous, intramuscular, topical, transdermal or oral administration.

Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as manitol, sorbitol, or sodium chloride in the composition. Prolonged absorption of the injectable compositions can be brought about by including in the composition an agent which delays absorption, for example, monostearate salts and gelatin. Moreover, the adzymes can be administered in a time release formulation, for example in a composition which includes a slow release polymer. The adzymes can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are generally known to those skilled in the art.

Sterile injectable solutions can be prepared by incorporating the adzyme in the required amount in an appropriate solvent with one or a combination of ingredients enumerated above, as required, followed by filtered sterilization. Generally, dispersions are prepared by incorporating the adzyme into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Depending on the route of administration, the adzyme may be coated in a material to protect it from the action of enzymes, acids and other natural conditions which may inactivate the agent. For example, the adzyme can be administered to a subject in an appropriate carrier or diluent co-administered with enzyme inhibitors or in an appropriate carrier such as liposomes. Pharmaceutically acceptable diluents include saline and aqueous buffer solutions. Enzyme inhibitors include pancreatic trypsin inhibitor, diisopropylfluoro-phosphate (DEP) and trasylol. Liposomes include water-in-oil-in-water emulsions as well as conventional liposomes (Strejan, et al., (1984) J. Neuroimmunol 7:27). Dispersions can also be prepared in glycerol, liquid polyethylene glycols, and mixtures thereof and in oils. Under ordinary conditions of storage and use, these preparations may contain a preservative to prevent the growth of microorganisms.

The active agent in the composition (*i.e.,* an adzyme of the invention) preferably is formulated in the composition in a therapeutically effective amount. A "therapeutically effective amount" refers to an amount effective, at dosages and for periods of time necessary, to achieve the desired therapeutic result, such as modulation of the activity of a target, to thereby influence the therapeutic course of a particular disease state. A therapeutically effective amount of an adzyme may vary according to factors such as the disease state, age, sex, and weight of the individual, and the ability of the adzyme to elicit a desired response in the individual. Dosage regimens may be adjusted to provide the optimum therapeutic response. A therapeutically effective amount is also one in which any toxic or detrimental effects of the adzyme are outweighed by the therapeutically beneficial effects. In another embodiment, the adzyme is formulated in the composition in a prophylactically effective amount. A "prophylactically effective amount" refers to an amount effective, at dosages and for periods of time necessary, to achieve the desired prophylactic result, for example, modulation of the activity of a target (*e.g.,* TNFα or TNFβ) for prophylactic purposes. Typically, since a prophylactic dose is used in subjects prior to or at an earlier stage of disease, the prophylactically effective amount will be less than the therapeutically effective amount.

The amount of an adzyme in the composition may vary according to factors such as the disease state, age, sex, and weight of the individual. Dosage regimens may be adjusted to provide the optimum therapeutic response. For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

Another aspect of the invention provides aerosols for the delivery of adzymes to the respiratory tract. The respiratory tract includes the upper airways, including the oropharynx and larynx, followed by the lower airways, which include the trachea followed by bifurcations into the bronchi and bronchioli. The upper and lower airways are called the conductive airways. The terminal bronchioli then divide into respiratory bronchioli which then lead to the ultimate respiratory zone, the alveoli, or deep lung.

Herein, administration by inhalation may be oral and/or nasal. Examples of pharmaceutical devices for aerosol delivery include metered dose inhalers (MDIs), dry powder inhalers (DPIs), and air-jet nebulizers. Exemplary nucleic acid delivery systems by inhalation which can be readily adapted for delivery of the subject adzymes are described in, for example, U.S. patents 5,756,353; 5,858,784; and PCT applications WO98/31346; WO98/10796; WO00/27359; WO01/54664; WO02/060412. Other aerosol formulations that may be used are described in U.S. Patents 6,294,153; 6,344,194; 6,071,497, and PCT applications WO02/066078; WO02/053190; WO01/60420; WO00/66206.

The human lungs can remove or rapidly degrade hydrolytically cleavable deposited aerosols over periods ranging from minutes to hours. In the upper airways, ciliated epithelia contribute to the "mucociliary excalator" by which particles are swept from the airways toward the mouth. Pavia, D., "LungMucociliary Clearance," in Aerosols and the Lung: Clinical and Experimental Aspects, Clarke, S. W. and Pavia, D., Eds., Butterworths, London, 1984. In the deep lungs, alveolar macrophages are capable of phagocytosing particles soon after their deposition. Warheit et al. Microscopy Res. Tech., 26: 412-422 (1993); and Brain, J. D., "Physiology and Pathophysiology of Pulmonary Macrophages," in The Reticuloendothelial System, S. M. Reichard and J. Filkins, Eds., Plenum, New. York., pp. 315-327, 1985. The deep lung, or alveoli, are the primary target of inhaled therapeutic aerosols for systemic delivery of adzymes.

In preferred embodiments, particularly where systemic dosing with the adzyme is desired, the aerosoled adzymes are formulated as microparticles. Microparticles having a diameter of between 0.5 and ten microns can penetrate the lungs, passing through most of the natural barriers. A diameter of less than ten microns is required to bypass the throat; a diameter of 0.5 microns or greater is required to avoid being exhaled.

An adzyme of the invention can be formulated into a pharmaceutical composition wherein the compound is the only active agent therein. Alternatively, the pharmaceutical composition can contain additional active agents. For example, two or more adzymes of the invention may be used in combination.

*(ii) Nucleic Acid Compositions*

Another aspect of the invention provides expression vectors for expressing the subject adzyme entities. For instance, expression vectors are contemplated which include a nucleotide sequence encoding a polypeptide adzyme, which coding sequence is operably linked to at least one transcriptional regulatory sequence. Regulatory sequences for directing expression of the instant polypeptide adzyme are art-recognized and are selected by a number of well understood criteria. Exemplary regulatory sequences are described in Goeddel; Gene Expression Technology: Methods in Enzymology, Academic Press, San Diego, CA (1990). For instance, any of a wide variety of expression control sequences that control the expression of a DNA sequence when operatively linked to it may be used in these vectors to express DNA sequences encoding the polypeptide adzymes of this invention. Such useful expression control sequences, include, for example, the early and late promoters of SV40, adenovirus or cytomegalovirus immediate early promoter, the lac system, the trp system, the TAC or TRC system, T7 promoter whose expression is directed by T7 RNA polymerase, the promoter for 3-phosphoglycerate kinase or other glycolytic enzymes, the promoters of acid phosphatase, e.g., Pho5, and the promoters of the yeast α-mating factors and other sequences known to control the expression of genes of prokaryotic or eukaryotic cells or their viruses, and various combinations thereof. It should be understood that the design of the expression vector may depend on such factors as the choice of the target host cell to be transformed. Moreover, the vector's copy number, the ability to control that copy number and the expression of any other protein encoded by the vector, such as antibiotic markers, should also be considered.

As will be apparent, the subject gene constructs can be used to cause expression of the subject polypeptide adzymes in cells propagated in culture, e.g. to produce proteins or polypeptides, including polypeptide adzymes, for purification.

This invention also pertains to a host cell transfected with a recombinant gene in order to express one of the subject polypeptides. The host cell may be any prokaryotic or eukaryotic cell. For example, a polypeptide adzyme of the present invention may be expressed in bacterial cells such as E. coli, insect cells (baculovirus), yeast, or mammalian cells. Other suitable host cells are known to those skilled in the art.

Accordingly, the present invention further pertains to methods of producing the subject polypeptide adzymes. For example, a host cell transfected with an expression vector encoding a protein of interest can be cultured under appropriate conditions to allow expression of the protein to occur. The protein may be secreted, by inclusion of a secretion signal sequence, and isolated from a mixture of cells and medium containing the protein. Alternatively, the protein may be retained cytoplasmically and the cells harvested, lysed and the protein isolated. A cell culture includes host cells, media and other byproducts. Suitable media for cell culture are well known in the art. The proteins can be isolated from cell culture medium, host cells, or both using techniques known in the art for purifying proteins, including ion-exchange chromatography, gel filtration chromatography, ultrafiltration, electrophoresis, and immunoaffinity purification with antibodies specific for particular epitopes of the protein.

Thus, a coding sequence for a polypeptide adzyme of the present invention can be used to produce a recombinant form of the protein via microbial or eukaryotic cellular processes. Ligating the polynucleotide sequence into a gene construct, such as an expression vector, and transforming or transfecting into hosts, either eukaryotic (yeast, avian, insect or mammalian) or prokaryotic (bacterial cells), are standard procedures.

Expression vehicles for production of a recombinant protein include plasmids and other vectors. For instance, suitable vectors for the expression of polypeptide adzymes include plasmids of the types: pBR322-derived plasmids, pEMBL-derived plasmids, pEX-derived plasmids, pBTac-derived plasmids and pUC-derived plasmids for expression in prokaryotic cells, such as E. coli.

A number of vectors exist for the expression of recombinant proteins in yeast. For instance, YEp24, YIp5, YEp51, YEp52, pYES2, and YRp17 are cloning and expression vehicles useful in the introduction of genetic constructs into S. cerevisiae (see, for example, Broach et al., (1983) in Experimental Manipulation of Gene Expression, ed. M. Inouye Academic Press, p. 83). These vectors can replicate in E. coli due the presence of the pBR322 ori, and in S. cerevisiae due to the replication determinant of the yeast 2 micron plasmid. Autotrophic selection or counterselection is often used in yeast. In addition, drug resistance markers such as ampicillin can be used in bacteria.

The preferred mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and transformation of host organisms are well known in the art. For other suitable expression systems for both prokaryotic and eukaryotic cells, as well as general recombinant procedures, see Molecular Cloning: A Laboratory Manual, 2nd Ed., ed. by Sambrook, Fritsch and Maniatis (Cold Spring Harbor Laboratory Press, 1989) Chapters 16 and 17. In some instances, it may be desirable to express the recombinant polypeptide adzymes by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the beta-gal containing pBlueBac III).

In yet other embodiments, the subject expression constructs are derived by insertion of the subject gene into viral vectors including recombinant retroviruses, adenovirus, adeno-associated virus, and herpes simplex virus-1, or recombinant bacterial or eukaryotic plasmids. As described in greater detail below, such embodiments of the subject expression constructs are specifically contemplated for use in various in vivo and ex vivo gene therapy protocols.

Retrovirus vectors and adeno-associated virus vectors are generally understood to be the recombinant gene delivery system of choice for the transfer of exogenous genes in vivo, particularly into humans. These vectors provide efficient delivery of genes into cells, and the transferred nucleic acids are stably integrated into the chromosomal DNA of the host. A major prerequisite for the use of retroviruses is to ensure the safety of their use, particularly with regard to the possibility of the spread of wild-type virus in the cell population. The development of specialized cell lines (termed "packaging cells") which produce only replication-defective retroviruses has increased the utility of retroviruses for gene therapy, and defective retroviruses are well characterized for use in gene transfer for gene therapy purposes (for a review see Miller, A.D. (1990) Blood 76:271). Thus, recombinant retrovirus can be constructed in which part of the retroviral coding sequence (gag, pol, env) has been replaced by nucleic acid encoding a polypeptide adzyme of the present invention, rendering the retrovirus replication defective. The replication defective retrovirus is then packaged into virions which can be used to infect a target cell through the use of a helper virus by standard techniques. Protocols for producing recombinant retroviruses and for infecting cells in vitro or in vivo with such viruses can be found in Current Protocols in Molecular Biology, Ausubel, F.M. et al., (eds.) Greene Publishing Associates, (1989), Sections 9.10-9.14 and other standard laboratory manuals. Examples of suitable retroviruses include pLJ, pZIP, pWE and pEM which are well known to those skilled in the art. Retroviruses have been used to introduce a variety of genes into many different cell types, including neural cells, epithelial cells, endothelial cells, lymphocytes, myoblasts, hepatocytes, bone marrow cells, in vitro and/or in vivo (see for example Eglitis et al., (1985) Science 230:1395-1398; Danos and Mulligan, (1988) PNAS USA 85:6460-6464; Wilson et al., (1988) PNAS USA 85:3014-3018; Armentano et al., (1990) PNAS USA 87: 6141-6145; Huber et al., (1991) PNAS USA 88:8039-8043; Ferry et al., (1991) PNAS USA 88:8377-8381; Chowdhury et al., (1991) Science 254:1802-1805; van Beusechem et al., (1992) PNAS USA 89:7640-7644; Kay et al., (1992) Human Gene Therapy 3:641-647; Dai et al., (1992) PNAS USA 89:10892-10895; Hwu et al., (1993) J. Immunol. 150:4104-4115; U.S. Patent No. 4,868,116; U.S. Patent No. 4,980,286; PCT Application WO 89/07136; PCT Application WO 89/02468; PCT Application WO 89/05345; and PCT Application WO 92/07573).

Furthermore, it has been shown that it is possible to limit the infection spectrum of retroviruses and consequently of retroviral-based vectors, by modifying the viral packaging proteins on the surface of the viral particle (see, for example PCT publications WO93/25234, WO94/06920, and WO94/11524). For instance, strategies for the modification of the infection spectrum of retroviral vectors include: coupling antibodies specific for cell surface antigens to the viral env protein (Roux et al., (1989) PNAS USA 86: 9079-9083; Julan et al., (1992) J. Gen Virol 73:3251-3255; and Goud et al., (1983) Virology 163: 251-254); or coupling cell surface ligands to the viral env proteins (Neda et al., (1991) J. Biol. Chem. 266: 14143-14146). Coupling can be in the form of the chemical cross-linking with a protein or other variety (e.g. lactose to convert the env protein to an asialoglycoprotein), as well as by generating polypeptide adzymes (e.g. single-chain antibody/env polypeptide adzymes). This technique, while useful to limit or otherwise direct the infection to certain tissue types, and can also be used to convert an ecotropic vector in to an amphotropic vector.

Another viral gene delivery system useful in the present invention utilizes adenovirus-derived vectors. The genome of an adenovirus can be manipulated such that it encodes a gene product of interest, but is inactivate in terms of its ability to replicate in a normal lytic viral life cycle (see, for example, Berkner et al., (1988) BioTechniques 6: 616; Rosenfeld et al., (1991) Science 252: 431-434; and Rosenfeld et al., (1992) Cell 68: 143-155). Suitable adenoviral vectors derived from the adenovirus strain Ad type 5 dl324 or other strains of adenovirus (e.g., Ad2, Ad3, Ad7 etc.) are well known to those skilled in the art. Recombinant adenoviruses can be advantageous in certain circumstances in that they are not capable of infecting nondividing cells and can be used to infect a wide variety of cell types, including airway epithelium (Rosenfeld et al., (1992) cited supra), endothelial cells (Lemarchand et al., (1992) PNAS USA 89:6482-6486), hepatocytes (Herz and Gerard, (1993) PNAS USA 90:2812-2816) and muscle cells (Quantin et al., (1992) PNAS USA 89:2581-2584). Furthermore, the virus particle is relatively stable and amenable to purification and concentration, and as above, can be modified so as to affect the spectrum of infectivity. Additionally, introduced adenoviral DNA (and foreign DNA contained therein) is not integrated into the genome of a host cell but remains episomal, thereby avoiding potential problems that can occur as a result of insertional mutagenesis in situations where introduced DNA becomes integrated into the host genome (e.g., retroviral DNA). Moreover, the carrying capacity of the adenoviral genome for foreign DNA is large (up to 8 kilobases) relative to other gene delivery vectors (Berkner et al., supra; Haj-Ahmand and Graham (1986) J. Virol. 57:267). Most replication-defective adenoviral vectors currently in use and therefore favored by the present invention are deleted for all or parts of the viral E1 and E3 genes but retain as much as 80% of the adenoviral genetic material (see, e.g., Jones et al., (1979) Cell 16:683; Berkner et al., supra; and Graham et al., in Methods in Molecular Biology, E.J. Murray, Ed. (Humana, Clifton, NJ, 1991) vol. 7. pp. 109-127). Expression of the inserted chimeric gene can be under control of, for example, the E1A promoter, the major late promoter (MLP) and associated leader sequences, the viral E3 promoter, or exogenously added promoter sequences.

Yet another viral vector system useful for delivery of the subject chimeric genes is the adeno-associated virus (AAV). Adeno-associated virus is a naturally occurring defective virus that requires another virus, such as an adenovirus or a herpes virus, as a helper virus for efficient replication and a productive life cycle. (For a review, see Muzyczka et al., Curr. Topics in Micro. and Immunol. (1992) 158:97-129). It is also one of the few viruses that may integrate its DNA into non-dividing cells, and exhibits a high frequency of stable integration (see for example Flotte et al., (1992) Am. J. Respir. Cell. Mol. Biol. 7:349-356; Samulski et al., (1989) J. Virol. 63:3822-3828; and McLaughlin et al., (1989) J. Virol. 62:1963-1973). Vectors containing as little as 300 base pairs of AAV can be packaged and can integrate. Space for exogenous DNA is limited to about 4.5 kb. An AAV vector such as that described in Tratschin et al., (1985) Mol. Cell. Biol. 5:3251-3260 can be used to introduce DNA into cells. A variety of nucleic acids have been introduced into different cell types using AAV vectors (see for example Hermonat et al., (1984) PNAS USA 81:6466-6470; Tratschin et al., (1985) Mol. Cell. Biol. 4:2072-2081; Wondisford et al., (1988) Mol. Endocrinol. 2:32-39; Tratschin et al., (1984) J. Virol. 51:

611-619; and Flotte et al., (1993) J. Biol. Chem. 268:3781-3790).

Other viral vector systems that may have application in gene therapy have been derived from herpes virus, vaccinia virus, and several RNA viruses. In particular, herpes virus vectors may provide a unique strategy for persistence of the recombinant gene in cells of the central nervous system and ocular tissue (Pepose et al., (1994) Invest Ophthalmol Vis Sci 35:2662-2666).

In addition to viral transfer methods, such as those illustrated above, non-viral methods can also be employed to cause expression of a protein in the tissue of an animal. Most nonviral methods of gene transfer rely on normal mechanisms used by mammalian cells for the uptake and intracellular transport of macromolecules. In preferred embodiments, non-viral gene delivery systems of the present invention rely on endocytic pathways for the uptake of the gene by the targeted cell. Exemplary gene delivery systems of this type include liposomal derived systems, poly-lysine conjugates, and artificial viral envelopes.

In a representative embodiment, a gene encoding an adzyme-containing polypeptide can be entrapped in liposomes bearing positive charges on their surface (e.g., lipofectins) and (optionally) which are tagged with antibodies against cell surface antigens of the target tissue (Mizuno et al., (1992) No Shinkei Geka 20:547-551; PCT publication WO91/06309; Japanese patent 1047381; and European patent publication EP-A-43075). For example, lipofection of neuroglioma cells can be carried out using liposomes tagged with monoclonal antibodies against glioma-associated antigen (Mizuno et al., (1992) Neurol. Med. Chir. 32:873-876).

In yet another illustrative embodiment, the gene delivery system comprises an antibody or cell surface ligand which is cross-linked with a gene targeting moiety such as poly-lysine (see, for example, PCT publications WO93/04701, WO92/22635, WO92/20316, WO92/19749, and WO92/06180). For example, any of the subject gene constructs can be used to transfect specific cells in vivo using a soluble polynucleotide carrier comprising an antibody conjugated to a polycation, e.g. poly-lysine (see U.S. Patent 5,166,320). It will also be appreciated that effective delivery of the subject nucleic acid constructs via -mediated endocytosis can be improved using agents which enhance escape of the gene from the endosomal structures. For instance, whole adenovirus or fusogenic peptides of the influenza HA gene product can be used as part of the delivery system to induce efficient disruption of DNA-containing endosomes (Mulligan et al., (1993) Science 260-926; Wagner et al., (1992) PNAS USA 89:7934; and Christiano et al., (1993) PNAS USA 90:2122).

In clinical settings, the gene delivery systems can be introduced into a patient by any of a number of methods, each of which is familiar in the art.

For instance, a pharmaceutical preparation of the gene delivery system can be introduced systemically, e.g. by intravenous injection, and specific transduction of the construct in the target cells occurs predominantly from specificity of transfection provided by the gene delivery vehicle, cell-type or tissue-type expression due to the transcriptional regulatory sequences controlling expression of the gene, or a combination thereof. In other embodiments, initial delivery of the recombinant gene is more limited with introduction into the animal being quite localized. For example, the gene delivery vehicle can be introduced by catheter (see U.S. Patent 5,328,470) or by stereotactic injection (e.g. Chen et al., (1994) PNAS USA 91: 3054-3057).

The invention is further illustrated by the following examples which should not be construed as limiting.

## EXAMPLES

The following examples are for illustrative purposes only, and should not be considered limiting in any respect.

### Example 1 Chemical Cross-linking of Address and Enzyme Domains

In general, an adzyme can be created in at least two ways: (A) by chemical crosslinking and (B) by recombinant DNA technology.

The cross linking may be performed using techniques well known in the art. For example, in one embodiment, the N-termini (or surface accessible lysines) of one protein domain may be reacted with SPDP, while the N-termini (or surface accessible lysines) of the other protein domain may be reacted with SMCC. Subsequently, the two domains are allowed to react, thus, forming disulfide bridges that join the domains. When linked in the foregoing manner, the estimated distance between the two domains is approximately 14 A.

Glutaraldehyde may also be used to cross link N-terminus of one protein with the C-terminus of the other protein.

These and similar methods well knownin the art of chemical cross-linking can be used to link address (such as the scFv Ab mentioned below) with a mesotrypsin catalytic domain (such as an active mesotrypsin, its zymogen, or its stabilizing mutation).

### Example 2: A Model Adzyme Experimental System

In order to create an adzyme (*e.g.*, a bifunctional protein) that preserves the functions of both domains (address domain and catalytic domain) and confers greater target specificity, applicants designed the following model adzyme experimental system using prethrombin as an enzyme domain and a single-chain antibody specific for the hemagglutinin peptide of influenza virus (HA) [18] as the address domain. Such an adzyme has heightened proteolytic activity on substrates bound by the address domain compared to the proteolytic activity of the enzyme domain alone. Although prethrombin was used in the example as the catalytic domain, mesotrypsin (and its functional fragments, derivatives, variants, stabilizing mutations, homologs, etc.) may use the same or similar single-chain antibody address domains, as shown in some examples below. In addition, the same vector / host systems and construction methods can be readily adapted for use with adzymes with a mesotrypsin catalytic domain (or any other catalytical domains).

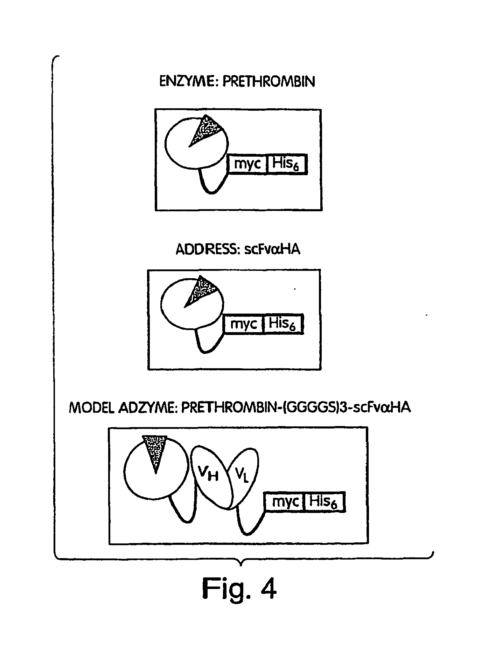

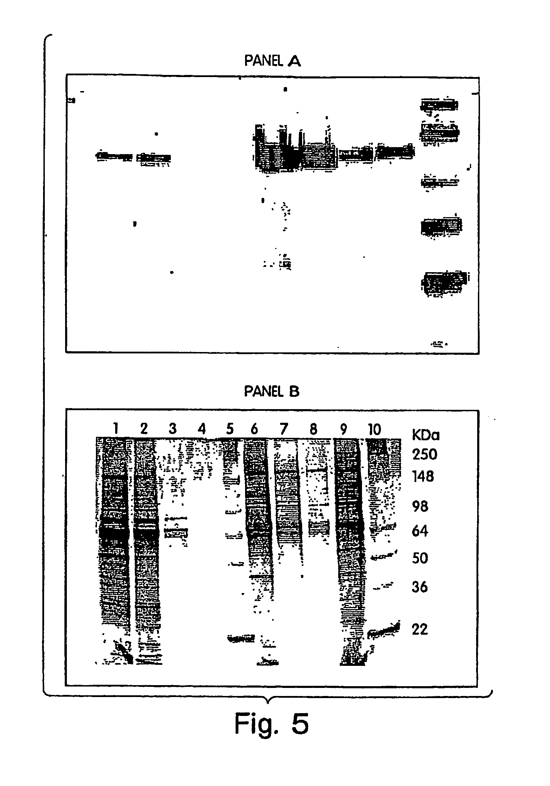

Proteolytic adzymes are expressed and purified as inactive zymogens. Frequently the zymogen has an amino terminal sequence that blocks the catalytic site. Cleavage at a specific activation site removes the blocking peptide and leads to protease activation. To ensure that activation does not uncouple the two domains of the adzyme, the enzyme domain is preferably positioned N-terminal to the address domain. The following examples describe the construction, expression and purification (see below, Figs. 4 & 5) of components that include the address domain alone, the enzyme domain alone and the ADYZME that coupled the address and enzyme domains through a flexible polypeptide linker. Following a partial one-step purification, these recombinant proteins were activated and tested for proteolytic activity against substrates that either contained or lacked a binding site for the address domain. Schematic model adzyme and individual components are shown in Fig. 4.

In Figure 4, all components were assembled in the pSecTag2A vector system (Invitrogen, Carlsbad, CA), which included an N-terminal leader peptide designed to enable secretion from a heterologous expression system and C-terminal tandem myc and $His_6$ tags to enable immunodetection and purification. The address domain was a single chain antibody (scFvαHA) derived from monoclonal antibody mAb26/9, which recognized an influenza virus haemaglutinin (HA) epitope DVPDYA (SEQ ID NO: 13) [18]. The enzyme domain was prethrombin (residues 315 to 622 of human prothrombin; accession no. AAC63054) - a zymogen of thrombin that could be activated using Factor Xa. Address and enzyme domains were connected with a 15 amino acid linker ([GGGGS]$_3$, SEQ ID NO: 14). When tested against a target containing DVPDYA (SEQ ID NO: 13) and a suboptimal thrombin cleavage site (*e.g.,* GGVR, SEQ ID NO: 15), the thrombin domain in the adzyme demonstrates accelerated cleavage because of the higher local concentration of peptide achieved through binding to DVPDYA (SEQ ID NO: 13) by the scFv domain (the address domain).

Both N-terminal and C-terminal fusions of adzymes are created with a variety of tags (myc, $His_6$, V5). Different linker compositions and lengths are used. For example, the following constructs may be created: thrombin-tag-COOH; scFvαHA-tag-COOH; N-thrombin-linker-scFv αHA-tag-COOH; N-scFvαHA-linker-thrombin-tag-COOH; N-scFvαHA-linker-thrombin-linker-scFvαHA-tag-COOH; or constructs with two thrombin units in tandem along with scFv anti-HA.

Prethrombin and the single chain antibody directed against the HA epitope are cloned individually into the HindIII and XhoI sites of the pSecTag2A vector from Invitrogen to generate proteins that will be secreted into the medium for subsequent biochemical characterization. Prethrombin is the inactive form that is activated by Factor Xa or ecarin. Prethrombin-(G$_4$S)$_3$-scHA and scHA-(G$_4$S)$_3$-prethrombin are assembled by overlap /recombinant PCR (using the oligos described in Table X below) and cloned into the pSecTag2A vector as HindIII and XhoI fragments. They will contain myc and $His_6$ as tags at the C-terminus. The slash shows where the cleavage occurs in the signal peptide. The amino acid sequence for Prethrombin-(G$_4$S)$_3$ scFvαHA is:

```
METDTLLLWVLLLWVPGSTG/DAAQPARRAVRSLMTATSEYQTFFNPRTFGSGEADCGLRPL
FEKKSLEDKTERELLESYIDGRIVEGSDAEIGMSPWQVMLFRKSPQELLCGASLISDRWVLT
AAHCLLYPPWDKNFTENDLLVRIGKHSRTRYERNIEKISMLEKIYIHPRYNWRENLDRDIAL
MKLKKPVAFSDYIHPVCLPDRETAASLLQAGYKGRVTGWGNLKETWTANVGKGQPSVLQVVN
LPIVERPVCKDSTRIRITDNMFCAGYKPDEGKRGDACEGDSGGPFVMKSPFNNRWYQMGIVS
WGEGCDRDGKYGFYTHVFRLKKWIQKVIDQFGEGGGGSGGGGSGGGGSMEVQLLESGGDLVK
PGGSLKLSCAASGFTFSTYGMSWVRQTPDKRLEWVATISNGGGYTYYPDSVKGRFTISRDNA
KNTLYLQMSSLKSEDTAMYYCARRERYDENGFAYWGRGTLVTVSAGGGGSGGGGSGGGGSDI
VMSQSPSSLAVSVGEKITMSCKSSQSLFNSGKQKNYLTWYQQKPGQSPKLLIYWASTRESGV
PDRFTGSGSGTDFTLTISSVKAEDLAVYYCQNDYSHPLTFGGGTKLEIKRADAAPTARGGPE
QKLISEEDLNSAVDHHHHHH*(SEQ ID NO: 16).
```

The amino acid sequence for scHA(G$_4$S)$_3$prethrombin as made from pSecTag2 is:

```
METDTLLLWVLLLWVPGSTG/DAAQPARRAVRSLMEVQLLESGGDLVKPGGSLKLSCAASGF
TFSTYGMSWVRQTPDKRLEWVATISNGGGYTYYPDSVKGRFTISRDNAKNTLYLQMSSLKSE
DTAMYYCARRERYDENGFAYWGRGTLVTVSAGGGGSGGGGSGGGGSDIVMSQSPSSLAVSVG
EKITMSCKSSQSLFNSGKQKNYLTWYQQKPGQSPKLLIYWASTRESGVPDRFTGSGSGTDFT
LTISSVKAEDLAVYYCQNDYSHPLTFGGGTKLEIKRADAAPTGGGGSGGGGSGGGGSMTATS
EYQTFFNPRTFGSGEADCGLRPLFEKKSLEDKTERELLESYIDGRIVEGSDAEIGMSPWQVM
LFRKSPQELLCGASLISDRWVLTAAHCLLYPPWDKNFTENDLLVRIGKHSRTRYERNIEKIS
MLEKIYIHPRYNWRENLDRDIALMKLKKPVAFSDYIHPVCLPDRETAASLLQAGYKGRVTGW


GNLKETWTANVGKGQPSVLQVVNLPIVERPVCKDSTRIRITDNMFCAGYKPDEGKRGDACEG
DSGGPFVMKSPFNNRWYQMGIVSWGEGCDRDGKYGFYTHVFRLKKWIQKVIDQFGEARGGPE
QKLISEEDLNSAVDHHHHHH*  (SEQ ID NO: 17).
```

**TABLE X**

| Oligo Name | Alternative name | Sequence (5' to 3') | Length | Tm | Purpose |
|---|---|---|---|---|---|
| B1 | scHAfwdHindIII | CCCGGAAGCTTAatggaggtgcagctgttg (SEQ ID NO: 18) | 30 | 56 | Fwd primer for amplifying ScHA for cloning into pSecTag2A using HindIII. A added after HindIII site to maintain reading frame. |
| B2 | scHArevXhoI | acgccccCTCGAGCagttggtgcagcatcagc (SEQ ID NO: 19) | 31 | 56 | Reverse primer for amplifying scHA for cloning into pSecTag2A using XhoI. C added prior to XhoI site to maintain reading frame. |
| B3 | prethrombinfwdH3 | CCCGGAAGCTTAATGaccgccaccagtgagtac (SEQ ID NO: 20) | 33 | 58 | Fwd primer for amplifying prethrombin into pSecTag2A using |
| B4 | prethrombinrevXhoI | ggccccCTCGAGCctctccaaactgatcaatg (SEQ ID NO: 21) | 31 | 56 | HindIII. A added to keep frame after HindIII. Rev primer to clone prethrombin into XhoI site of pSecTag2A. C |
| B5 | G4ScHAiwd | **Tttggagagggaggcggtgggtctggtgggggcggtagt ggcggaggtgggagcatggaggtgcagctgttg (SEQ ID NO: 22)** | 72 | 56 | added to keep frame. Forward primer to introduce (G4S)3 at 5' end of scHA. |
| B6 | prethrombinG4Srev | **Cacctccatgctcccacctccgccactaccgcccccacca gacccaccgcctccctctccaaactgatcaatg (SEQ ID NO: 23)** | 73 | 54 | Reverse primer to introduce (G4S)3 tag at the 3' end of prethrombin. |
| B7 | G4Sprethrombinfwd | **gcaccaactggaggcggtgggtctggtgggggcggtagt ggcggaggtgggagcATGaccgccaccagtgagtac (SEQ ID NO: 24)** | 75 | 58 | Fwd primer to amplify prethrombin with (G4S)3 at 5' end to create overlap with ScHA. |
| B8 | scHAG4Srev | **ggtggcggtCATgctcccacctccgccactaccgccccc accagacccaccgcctccagttggtgcagcatcagc (SEQ ID NO: 25)** | 75 | 56 | Rev primer to amplify scHA with (G4S)3 at 3' end to create overlap with G4Sprethrombin. |

Substrates tested include: S1, a high affinity epitope (DVPDYA, SEQ ID NO: 13) recognized by scFvαHA linked to the proteolytic target site (HAE-PT: $NH_2$-YPYDVPDYA-(SGSGS)$_4$-GGVR-p-nitroanilide, SEQ ID NO: 26); and S2, the proteolytic target alone (PT: $NH_2$-GGVR-p-nitroanilide, SEQ ID NO: 15). Other synthetic peptide substrates were also made with variable binding and cleaving substrate sequences. The Thrombin cleavage sites were chosen based on the teachings of Backes et al. (2000) Nature Biotechnology 18:187-193. Alternate choices include Ile-Thr-Pro-Arg (SEQ ID NO: 27) as the best cleavage site and Ile-Thr-Leu-Arg (SEQ ID NO: 28) as a poor target.

Cleavage of the peptide bond between the Arg residue in the substrates and the p-nitroanilide by thrombin activity releases free p-nitroaniline (pNA), which has a yellow color visible by spectrophotometric monitoring at 405 nm.

**2.1. Production of model adzyme components: construction, expression, purification and activation.**

Components were constructed in the pSecTag2A vector, expressed transiently in mammalian cells and purified from conditioned media as described below.

Briefly, mammalian expression vector pSecTag2A (Cat. No.V90020; Invitrogen, Carlsbad, CA) was used as the backbone for all constructs. Upstream of the polylinker is a murine Ig κ-chain V-J2-C signal peptide, and downstream are myc and His$_6$ tags, a TAA stop codon and a bovine growth hormone polyadenylation signal. Other notable features of the vector are a cytomegalovirus (CMV) promoter to drive expression of the inserted coding sequence and the selectable markers zeocin and ampicillin. cDNAs corresponding to individual components were generated by PCR and cloned directionally into the polylinker to maintain the reading frame using HindIII at the 5' end and XhoI at the 3' end. The address component (scFvαHA) was amplified from a plasmid template containing the coding sequence of scFvαHA (engeneOS, Waltham, MA); prethrombin was amplified from the full length human cDNA clone (ResGen; Cat. no. FL1001), and; the adzyme was created by overlap PCR designed to insert a 15 amino acid linker (GGGGS)$_3$ (SEQ ID NO: 14) between the N-terminal prethombin domain and the C-terminal address domain. All constructs were sequence confirmed.

Transient transfections were carried out with 2 x 10$^6$ 293T cells cultured in T175 flasks using Fugene (Roche, Indianapolis, IN). Conditioned media from 6 flasks containing the secreted components were harvested when expression reached maximum levels (day 4, 5 or 7-- depending on the construct), clarified and dialyzed against 50 mM $NaH_2PO_4$, 300 mM NaCl, 5 mM imidazole (buffer A) overnight at 4˚C with one change of buffer. For purification, the dialyzed supernatants were incubated for 16 hr at 4˚C with Ni-NTA (Qiagen, CA) resin (0.4 ml resin or 0.8 ml of slurry per 200 ml of the dialyzed supernatant). The resultant slurry was spun at 600 g for 10 mins at 4˚C and the supernatant was removed and saved as a "flowthrough" sample. Then resin containing bound protein was re-suspended in 10ml buffer A, washed 3 times (10 minutes each at 4˚C) and the beads were manually loaded on a 3 ml syringe fitted with 3 mm Whatman filter paper. Three elutions (0.5 - 1 ml each) were performed with 50 mM $NaH_2PO_4$, 300 mM NaCl, 1 M imidazole. Eluted material was dialyzed into phosphate-buffered saline overnight for storage or into Tris-buffered saline + 1 mM $CaCl_2$ buffer for activation with Factor Xa.

As shown in Figure 5, the model adzyme prethrombin-(GGGGS)$_3$-scFvαHA was expressed transiently in 293T cells and conditioned media harvested on day 7. The material was processed and purified as described above. Samples representing equivalent portions of each fraction were loaded onto 4-20% polyacrylamide gels and electrophoresed in Tris-glycine-SDS buffer (Novex). Panel A. Western blot-following electrophoresis the gel was electroblotted to nitrocellulose membranes which were stained with an anti-myc antibody (Invitrogen, Carlsbad, CA). Lane (1) Load; (2) Flow through; (3) Wash 1; (4) Wash 3; (5) Elution 1; (6) Elution 2; (7) Elution 3; (8) Resin boiled in sample loading buffer; (9) Cruz mol. weight marker (Santa Cruz Biotechnology, Santa Cruz, CA). Panel B: Silver-stained gel. Lane (1) starting material; (2) Flow through; (3) Wash 1; (4) Wash 3; (5) molecular weight standard SeeBlue Plus 2; (6) Elution 1; (7) Elution 2; (8) Elution 3; (9) Resin boiled in sample loading buffer; (10) molecular weight standard SeeBlue Plus 2.

An example of an electrophoretic analysis of the model adzyme preparation is shown in Fig. 5. The secreted full-length adzyme was detected with an anti-myc antibody at ~70 kDa (panel A) as expected. Based on the silver-stained gel in this analysis (panel B), the estimated adzyme purity is about 10-20%. The individual address and enzyme components produced in parallel had yields and purity similar to the model adzyme (data not shown).

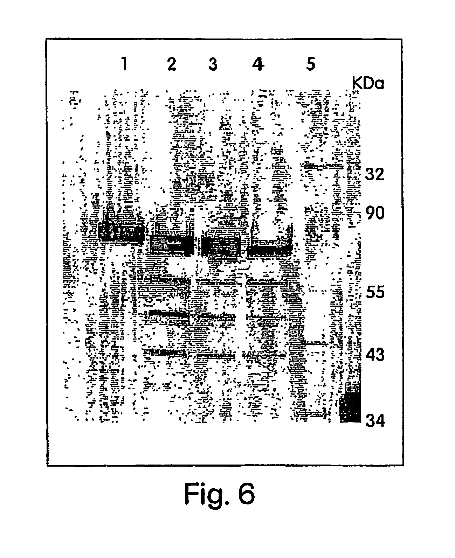

Purified adzyme components containing enzyme domains were activated using Factor Xa, which cleaves prethrombin at Arg 320 thereby releasing a 49-amino acid light chain from the N-terminus and generating the active thrombin heavy chain of 259 amino acids. In the example shown in Fig. 6, the activation process by Western blot indicated that activation using Factor Xa reduced the molecular weight of the model adzyme by ~6 kDa as expected.

Specifically, purified prethrombin and adzyme components were dialyzed at 4˚C overnight against 50 mM Tris pH 8, 0.1M NaCl, 1mM $CaCl_2$, then protein concentrations were determined. Activation was performed using biotinylated Factor Xa (Roche). Applicant adapted the protocol to account for the estimated purity (~10%) of prethrombin to be activated, thus 1μg biotinylated Factor Xa was used per 4.44 μg total protein for 3h at room temperature. Following activation, the biotinylated Factor Xa was removed using streptavidin beads supplied with the kit, and the activated components were analyzed by Western blot, and used for biochemical studies (see below).

As shown in Figure 6, a preparation of our model adzyme (see Fig. 5) was analyzed by Western blot using the anti-myc antibody before and after activation using Factor Xa: partially purified model adzyme dialyzed into TBS (lane 1); Factor Xa activation reaction (lane 2); activation reaction following removal of Factor Xa (lane 3); streptavidin beads used for removal (lane 4); and Cruz molecular weight standards (lane 5, Santa Cruz Biotechnology, CA).

These examples demonstrate that Applicants have developed reliable production methods for preparing and activating recombinant adzyme components. A typical preparation from 2 to 6 T175 flasks yielded 2-3 mg of material recombinant protein. These materials were sufficient for all of the analytical studies on biochemical function described below.

## 2.2. Characterization of adzyme binding and enzymatic activity.

To ensure a meaningful comparison of the address domain, enzyme domain and adzyme properties (see Table 1), Applicants completed a series of control experiments designed to: 1) measure binding to a target epitope; 2) compare activities with well-characterized standards and; 3) normalize the proteolytic activity against control substrates.

Binding to a target epitope. This experiment assessed the binding characteristics of the adzyme address domain. Applicants assessed binding activity of various components using biotinylated peptides in a sandwich ELISA format. Purified components were dialyzed against PBS, captured on plates coated with anti-myc antibody (mAb 9E10; Sigma), then analyzed by ELISA for binding to biotinylated target peptide (NH$_2$-YPYDVPDYAGSGDYKAFD, SEQ ID NO: 29), which contained the high affinity epitope (underline). Bound peptides were quantified using a streptavidin-horseradish peroxidase detection system (Quantablue; Pierce, Rockford, IL). The address domain alone and both the activated and zymogen forms of the adzyme bound comparable levels of the peptide per mole. However the enzyme domain alone failed to bind measurable amounts of the peptide, as expected.

Model adzyme thrombolytic activity. Characterization of the proteolytic activity of the model adzyme helps to determine if either the address domain or the polypeptide linker affected its enzymatic properties.

Applicants compared the activities of the model adzyme against a commercially available thrombin preparation (Sigma, St Louis, Mo) on standard fluoro or colorimetric derivatives of the thrombin tripeptide substrate-tosyl-gly-pro-arg-(p-nitroaniline, pNA or amino methyl coumarin AMC, Sigma). Activity was monitored over a 5 min. time course in a cuvette-based fluorometric assay that measured released fluorophore AMC (excitation at 383 nm, emission at 455 nm) in a Perkin Elmer LS55 fluorescence spectrophotometer. Based on a standard curve for free AMC, data obtained in terms of arbitrary fluorescence units vs time were converted into molecules of substrate hydrolyzed per unit time. Reaction velocities were determined over a range of substrate concentrations (0-50 $\mu$M) and $K_M$ values for the tripeptide substrate and were determined using a Line-Weaver-Burke plot. From these studies, it was confirmed that commercially available human thrombin and the activated model adzyme had comparable $K_M$ values for this standard substrate, 4.2 $\mu$M and 3.9 $\mu$M, respectively, which were in good agreement with literature values.

Second, Applicants determined the specificity constants ($k_{cat}$ / $K_M$) of thrombin for the substrates S 1 and S2. Both substrates contain a thrombin cleavage site, and substrate S 1 also includes the high affinity epitope recognized by the anti-HA single chain antibody. A significant difference in thrombin selectivity for either S1 (HAE-PT) or S2 (PT) would require the selection of an alternative control substrate. Applicants measured the proteolytic activity of a standard human thrombin preparation (Sigma) at two different concentrations (0.0033 NIH Units/ml and 0.01NIH Units/ml) against a concentration range between 3 $\mu$M to 25 $\mu$M of fluorometric derivatives of the substrates S 1 and S2. Applicants followed the same protocol that was utilized to determine $K_M$ values for the tosyl-GPR-AMC substrate (see above). Values for $K_M$ and $V_{max}$ were calculated from Line-Weaver-Burke plots. Active and total enzyme concentration ($E_{total}$) was determined from active site titration with D-Phe-Pro-Arg-ChloroMethylKetone (D-FPR-CMK), an irreversible active site inhibitor. These experiments provided the data for a calculation of the absolute enzyme concentration ($E_{total}$) in 0.0033 NIH Units/ml and 0.01NIH Units/ml of Sigma thrombin proteolytic activity. From these data, Applicants calculated $k_{cat}$ = $V_{max}$ / $E_{total}$, then derived the specificity constants $k_{cat}$ / $K_M$ for the substrates as 8.9 $\mu$M$^{-1}$sec$^{-1}$ and 10.3 $\mu$M$^{-1}$sec$^{-1}$ for S1 and S2, respectively. The close match of these values indicated that thrombin was acting at either substrate with equivalent specificity and proteolytic activity. Thus, the high affinity epitope has no effect on thrombin activity.

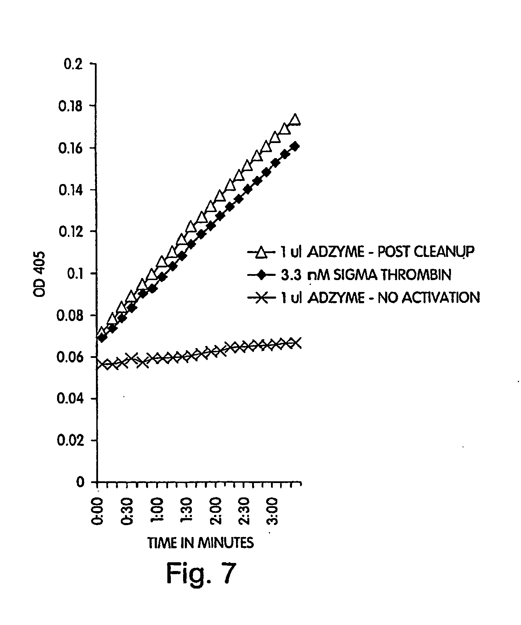

Normalization of proteolytic activity. Applicants needed to quantify the enzymatic activity of the model thrombin-(GGGGS)$_3$-scFv$\alpha$HA adzyme with reference to the standard human thrombin. The commercially available tripeptide tosyl-GPR-pNA (Sigma), which lacked the high affinity HA binding site was used as substrate. Cleavage of the peptide bond following the Arg residue releases released the chromophore p-nitroaniline (pNA) which is visible at 405 nm. Applicants determined the relative proteolytic activity, in units of thrombin activity per ml, of adzyme components before and after activation with Factor Xa. Factor Xa has no activity on the commercial substrate. Data from one such experiment are shown below in Fig. 7. This allowed normalization based on enzymatic activity of the adzyme preparation and comparison of equivalent activities for adzyme and native commercial thrombin against substrate S1 and S2.

Specifically, as shown in Figure 7, proteolytic activity was determined in a plate format using varying amounts of test components against a commercially available enzyme standard (3.3 nM human alpha thrombin, Sigma) by monitoring the release of pNA absorbance at 405 nm in a Spectramax plate reader (Molecular Devices). Based on a standard curve for free p-nitroaniline, data obtained in terms of absorbance units vs. time were converted into molecules of substrate hydrolyzed per molecule of enzyme per unit time.

Results of this experiment showed that this model thrombin-(GGGGS)$_3$-scFvαHA adzyme preparation: 1) had no detectable activity prior to activation and; 2) could be normalized against a standard thrombin preparation-in this case 5 μl/ml of the activated model adzyme was equivalent to 3.3 nM (0.1 NIH U/ml) of thrombin. Active site titration of activated samples with D-FPR-CMK provided independent verification of the normalization. Hence, the proteolytic activity for adzyme preparations were normalized relative to the thrombin standard.

In summary, these control experiments have shown that: 1) the address domain-mediated binding to the high affinity epitope and linkage of an enzyme domain did not interfere with binding activity; 2) the activated model thrombin-(GGGGS)$_3$-scFvαHA adzyme had a $K_M$ value comparable to thrombin for a standard thrombin substrate; 3) thrombin had equivalent specificity for substrates S1 and S2; 4) activation using Factor Xa was required to obtain detectable proteolytic activity; and 5) Applicants were able to normalize the proteolytic activities of adzyme preparations relative to a commercial thrombin standard. This series of control experiments have provided the basis for testing and comparing the adzyme and isolated components on substrates that contained or lacked a high affinity epitope for the address domain.

### 2.3. Test of Adzyme Function.

Applicants have designed an adzyme, thrombin-(GGGGS)$_3$-scFvαHA, comprising a prethrombin enzyme domain linked by a 15 amino acid polypeptide to a single chain antibody to the HA epitope as the address domain. Thrombin does not bind or cleave the HA epitope but binds its targeted substrate site GGVR (SEQ ID NO: 15), whether in the context of S1 or S2, with the same affinity. The activated thrombin component of the thrombin-scFvαHA adzyme also binds the GGVR (SEQ ID NO: 15) of S1 with the same affinity; however the adzyme concept predicts that thrombin coupled to the anti-HA antibody will bind to substrates containing the HA epitope with the typical higher affinities of antibodies and may affect the adzyme reaction rate. It is predicted that the adzyme could have heightened enzymatic activity compared to thrombin.

In the reaction velocity experiments using the substrates S1 and S2 with either thrombin or thrombin-(GGGGS)$_3$-scFvαHA adzyme; it is predicted that: 1) the address domain alone (A) would be inactive (-) on both substrates; 2) the enzyme alone (B) and the adzyme (D) would have equivalent (+) proteolytic activity on substrate S2, the thrombin cleavage site alone; 3) the adzyme would be more active (+++) against substrate S 1 (S1 has both the high affinity epitope and the thrombin cleavage site) than against substrate S2 or the enzyme alone against either substrate (+); and 5) a stoichiometric mixture (C) of the unlinked address domain and enzyme domain would be equivalent to the enzyme domain alone on both substrates (+) (see Table 1) and less than the adzyme.

Table 1: Model thrombin-(GGGGS)$_3$-scFvαHA adzyme and components tested against linear peptide substrates

|  |  | Substrate | |
| --- | --- | --- | --- |
| Test component |  | S 1: HAE-PT | S2:PT |
| A | scFvαHA | - | - |
| B | Thrombin | + | + |
| C | A+B | + | + |
| D | Thrombin-(GGGGS)$_3$-scFvαHA | +++ | + |

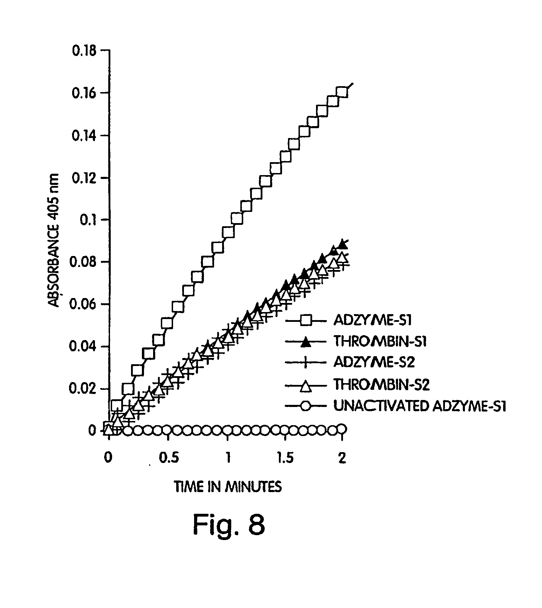

<u>Adzyme activity is driven by the address domain.</u> The proteolytic activities of the model adzyme (D) to thrombin alone (B) were compared on substrates that either contained (on S1) or lacked (on S2) a high affinity epitope for the address domain. Results of this experiment are shown below in Fig. 8.

Specifically, in Figure 8, proteolytic release of pNA from substrates S1 and S2 was followed by monitoring absorbance at 405 nm over a two minute time course in a quartz cuvette. Reactions were carried out in thrombin running buffer (50mM Tris-HCl pH 8, 0.1M NaCl, 0.1 % polyethylene glycol 8000) containing matched active enzyme concentrations (3.3 nM) as determined in normalization experiments (see Fig. 6). Reactions were initiated with the addition of substrate to 25 μM.

Equivalent activities of the activated thrombin-(GGGGS)$_3$-scFvαHA adzyme and activated commercial thrombin, as determined with the toysl-GPR-pNA substrate and hence normalized, were tested against S1 and S2. As shown in Figure 8, the reaction rate for both the adzyme and thrombin are the same on the S2 substrate which contains just the thrombin cleavage site as expected, since both the adzyme preparations had been normalized to thrombin. However, as predicted, the model adzyme showed increased activity towards substrate S 1 which contained a high affinity epitope in addition to the thrombin cleavage site. There is a 2X increase in reaction rate. The presence of this high affinity epitope on the substrate did not alter the activity of the thrombin alone. In the absence of activation the adzyme did not show detectable proteolytic activity. Thus the enhanced activity of thrombin-(GGGGS)$_3$-scFv$\alpha$HA adzyme is driven by the presence of an address domain that directed the enzyme activity to the substrate through binding a high affinity epitope.

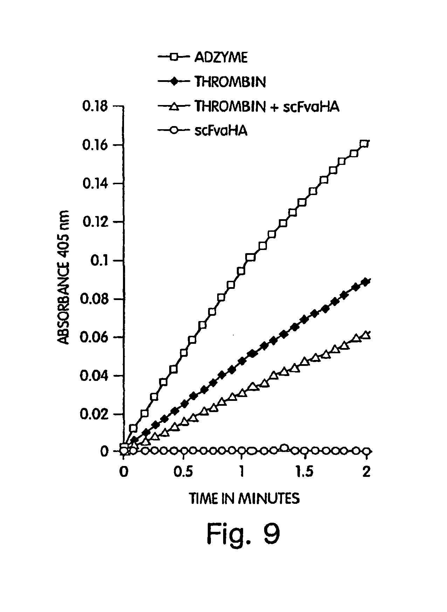

<u>Enhanced adzyme activity requires linkage of the address and enzyme domains.</u> To determine if the enhanced adzyme activity requires linkage of the address and enzyme domain on the same polypeptide chain (D), or whether a stoichiometric mixture of the address domain and thrombin (C) perform equally well, Applicants compared these two proteolytic activities on substrate S1, which contained a high affinity epitope for the address domain. Data from this comparison are shown in Fig. 9.

Specifically, in Figure 9, purified address domain scFv$\alpha$HA was used at 3.3 nM (concentration estimated based on Bradford assay and estimated percent purity from a Coomassie Blue stained gel).

The results of the experiment clearly show that mixing the individual address domain and enzyme thrombin together did not produce the accelerated rate of proteolysis observed with the model adzyme. Interestingly, applicants noted that the mixture was slightly less active than thrombin. Perhaps the unlinked address domain interfered slightly with access to the site of proteolysis by thrombin. Further, the address domain alone showed no detectable activity. Thus linkage of the address and enzyme domains produced a cooperative benefit in proteolytic rate over a stoichiometric mixture of the separated domains.

These studies have supported and validated the predicted adzyme function. The model adzyme design has preserved the functions of the individual components AND produced a cooperative advantage over the stoichiometric mixture. The technology can be equally applied to produce a proteolytic adzyme specific for a clinically relevant target protein, such as TNF-$\alpha$ or IL-1, using, for example, one of the mesotrypsins described herein as the catalytic domain.

## Example 3. Adzymes that Selectively Inactivates the Bioactivity of TNF-$\alpha$

This example describes the construction and optimization of adzymes that selectively inactivate the bioactivity of TNF$\alpha$. Although mesotrypsin is not one of the described catalytical domains in this example, the same design principles (espacially for linkers and address domains), the various bio-assays, and the same vector / expression systems may be used with minor adaptation for mesotrypsin-based adzymes.

To illustrate, ninty-six (96) adzyme structures for selective catalytic inactivation of TNF$\alpha$ are designed, and at least half are constructed using standard molecular biology techniques. These adzyme structures include combinations of just two enzyme catalytic domains, three address domains and sixteen linkers (including zero linker).

Specifically, the enzymes are: cationic trypsin and MMP7; the addresses are: Sp55, Sp55_2.6, and scFv; the linkers are: linkers with 0, 10, 20, 30, 40, or 50 amino acids (corresponding to repeating units of GGGGS), FcIgG1 (knob mutation), FcIgG1 (hole mutation), FcIgG2 (knob mutation), FcIgG2 (hole mutation), FcIgG3 (knob mutation), FcIgG3 (hole mutation), FcIgG2-(G$_4$S)$_2$ hole mutation, FcIgG2-(G$_4$S)$_4$ hole mutation, FcIgG2-(G$_4$S)$_3$ hole mutation, FcIgG2-(G$_4$S)$_4$ hole mutation. The knob and hole mutations refer to the paired mutations (S354C:T366'W/Y349C:T366S: L368'A:Y407'V) in CH3 domains that had been identified as giving rise to predominantly heterodimeric bispecific antibodies (Merchant et al. Nature Biotechnology, 1998, 16, p. 677-681).

Six of the adzymes are then produced, purified, and tested for bioactivity. One or more of these adzymes fulfills the essential criteria of a useful adzyme - preserve the function of individual components and yet produce a cooperative advantage through a polypeptide linkage of the two domains. Specifically, the adzyme(s) inactivates TNF$\alpha$ more effectively than either the address or enzyme alone, or a stoichiometric mixture of the individual domains.

Applicants have constructed, expressed and performed initial characterization of a series of three TNF$\alpha$-targeted adzyme proteases, consisting of an address domain selected from soluble TNF receptor(s) linked to the catalytic domain of human cationic trypsin. The produced adzymes have been analyzed to quantify binding and proteolytic activities.

<u>3.1. Design of TNF$\alpha$-specific Adzymes</u>

Three components - the enzyme, the linker and the address domain - work together effectively to produce a catalytic antagonist of TNF$\alpha$. The enzyme domains are preferably positioned at the N-terminus in this particular example, although in other adzyme designs, the enzyme domain may be C-terminal or even internal to the fusion protein. The enzyme domain here is encoded as a zymogen and has proteolytic activity capable of inactivating TNF$\alpha$. The address domains will bind TNF$\alpha$ with a high degree of selectivity, and the linkers will produce functional coupling of enzyme and address domains to support cooperativity in catalytic inactivation of TNF$\alpha$.

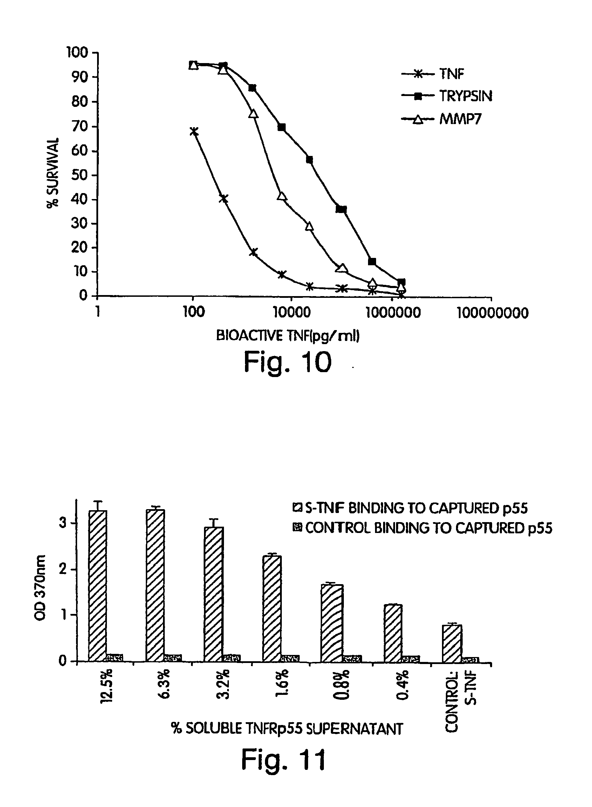

***a. Selection of the enzyme domains*** A survey of the literature and public domain databases (MEROPS: http://www.merops.sanger.ac.uk) for proteases that are commercially available, expressible as zymogens, and expected to cleave and inactivate TNF$\alpha$ [19-24] led to the selection of twenty candidate proteases, which were then tested for inactivation of TNFα using a TNF cytotoxicity assay. Specifically, TNF activation of functional TNFα receptor TNFR-1 [10, 25] leads to apoptotic cell death, which can be quantified in a cell-based assay [26]. This assay served as the basis to screen the 20 proteases for inactivation of TNFα bioactivity (see below, Fig. 10, Table 2).

Specifically, in Figure 10, L929 mouse connective tissue fibroblasts (ATCC catalog # CCL-1) were used to bioassay cell death induced by TNFα with the CellTiter 96® AQueous One Solution Cell Proliferation Assay system from Promega (Madison, WI). This system provides a colorimetric assay method for determining the number of viable cells. Briefly, for each test protease, a solution of 5 μM. TNFα was digested overnight at 37˚C, then bioactivity was determined for eight serial dilutions of the digestion solution. Data are mean values of triplicate determinations at each dilution of TNFα. Examples of TNFα inactivation by trypsin and MMP7 are shown in the figure. Results from the tests on all twenty proteases are summarized in Table 2.

More specifically, 10,000 L929 cells per well were seeded in 96 well plates and cultured in DMEM + 10% FBS overnight in a humidified C02 incubator. Actinomycin D was added to all wells (final concentration 1 μg/mL) and a standard TNFα survival curve was generated by adding human TNFα (RDI, Flanders, NJ) to achieve final concentrations in the wells ranging from 100 pg/ml - 1 μg/ml. Protease digestion samples of TNFα were similarly diluted and added to parallel rows of wells. Triplicate determinations were done for each dilution of TNFα. Following an overnight incubation in a humidified $CO_2$ incubator 20 μl of pre-mixed MTS/PES was added to each well and incubation continued for 2 - 4 hours at 37˚C. Metabolically active viable cells reduced the assay reagent (MTS/PES includes a tetrazolium compound) into a formazan product that was soluble in tissue culture media. Absorbance was read at 490 nm in a plate reader after 4 hr to determine the number of viable cells. Complete details of the protocol were provided in Promega Technical Bulletin No. 245.

**Table 2: Proteases tested for inactivation of TNFα.**

| Proteases that inactivated TNFα | Proteases that did not inactivate TNF | | |
|---|---|---|---|
| MT1-MMP (0.86) | Furin | Urokinase | Plasmin |
| MMP12 (0.65) | Cathepsin G | Enterokinase | Kallikrein5 |
| Tryptase (0.62) | HIV Protease | TACE | ADAMTS4 |
| MT2-MMP (0.5) | ADAM10 | MMP3 | MT5-MMP |
| ELASTASE (1.45) | | | |
| MMP7 (1.22) | | | |
| CHYMOTRYPSIN (2.74) | | | |
| TRYPSIN (2.3) | | | |

TNFα was digested with test proteases in overnight incubations at 37˚C, then analyzed for bioactivity as described in Fig. 10. Twelve proteases had no activity against TNFα; eight had varying levels of activity. Numbers in parentheses reflect log reduction in TNFα activity calculated at the 50% survival level from inactivation curves similar to the ones shown in Fig. 10.

The survival curve for standard TNFα shows a steep reduction in survival from 100 pg/ml to 10 ng/ml (Fig. 10). In the presence of ~600 pg/ml TNFα reference standard only 10% of the cells survive. This is in contrast to 40% and 70% survival for the equivalent dilution of TNFα digested with MMP7 or trypsin, respectively. The curve for dilutions of trypsin-digested TNFα showed a consistent shift to the right, indicating that the bioactivity of TNFα was reduced more than two logs compared to the TNFα reference standard. Similar studies were done with all of the enzymes listed in Table 2, including MMP7 (Fig. 10). Chymotrypsin was the most active protease against TNFα (2.74 log reduction in TNFα bioactivity). However it also showed significant auto-degradation (not shown), which may be improved by eliminating autocleavage sites in the enzyme (see above). All of these enzymes are candidates for the enzyme component of anti-TNF adzymes.

***b. Selection of the address domains.*** Address domains will preferably bind TNFα with high specificity, high affinity and will preferably be resistant to proteolytic cleavage by the catalytic domain. Quantitative models of how binding domains cooperate [27] and our experience with the thrombin model adzyme (above) suggested a range of binding affinities suitable for TNFα-specific adzymes. Address domains will be derived from two independent sources that bind TNFα with $K_{affinity}$ values in the nM range -- the TNFR-1 p55 extracellular domain and a single chain antibody to TNFα obtained from Genetastix (San Jose, CA) or generated in house from standard display technologies.

The sp55 address domains were constructed from the full-length human ectodomain of TNFR-1, and its binding to TNFα was characterized. Briefly, human TNFR-1 encoded by the CD120A gene (accession no. NM_001065; IMAGE clone 4131360, Invitrogen, Carlsbad, CA) was used as the template to amplify residues 30-211 in the TNFR-1 ecto-domain (protein accession no. P19438) [28] to construct a full-length sp55. Alternative address domains that might be evaluated may include subdomains of sTNFR-1, such as sp55Δ4 (residues 22-167) [29] or sp55 domain 2.6 (residues 41-150) [30]. These subdomains are smaller than the full ecto-domain, and hence might have reduced sensitivity to proteolytic degradation. Since a significant function of the address domain is to bind the target with high affinity, sp55 binding to TNFα was quantified using an indirect ELISA format to validate the presence of a functional address domain (Fig. 11). Briefly, in Figure 11, address domains were expressed transiently in 293T cells and captured on Ni-NTA coated wells. Binding to TNFα was quantified using the S-Tag™ system (Novagen, Madison, WI). The S-Tag™ system is a protein tagging and detection system based on the interaction of the 15 amino acid S-Tag peptide with ribonuclease S-protein, which is conjugated with horseradish peroxidase (HRP). Applicants constructed, expressed and purified a human TNFα fusion protein that included an N-terminal S-Tag™, then used this reagent (S-TNF) to quantify binding activity of the sp55 address domains (vertical stripes). Background (control) binding of TNFα that lacks the S-tag is shown in the hatched boxes.

More specifically in Figure 11, conditioned media, harvested and clarified by centrifugation, was diluted 1:10 into buffer (0.5 % BSA Fraction V, 0.05 % Tween-20 in 1 X PBS pH 7.4). Expressed proteins were captured on Ni-NTA coated wells (HisSorb plates, Catalog # 35061, Qiagen) for 1h at room temperature with shaking and washed four times in 0.05 % Tween-20 in 1 X PBS to remove un-bound materials. Binding to TNFα was determined by adding 100 μL of S-TNF (or control TNFα) at 1 μg/mL in assay buffer per well, followed by incubation for 1 hr at room temperature with shaking.

Plates were washed 4 times in 0.05 % Tween-20 in 1 X PBS, then S-protein HRP (1:2000 in assay buffer at 100 μL/ well, Novagen, Madison, WI) was added and incubated for 1 hr further at room temperature with shaking. A final wash step in 0.05 % Tween-20 in 1 X PBS was done 4 times to remove the S-protein-HRP, then 100 μL HRP substrate tetramethylbenzidine (TMB; Sigma T 4444, St. Louis, MO) was added per well. Color was allowed to develop for 5 - 45 minutes, then absorbance read at 370 nm in a Spectromax plate reader (Molecular Devices).

Figure 11 shows a three-fold elevation in S-TNF binding (vertical stripes) compared to non-specific binding in control samples (control: S-TNF; conditioned media from mock transfected cells). Binding appeared to saturate at 6-12 % of conditioned media in the assay, and the dilution series showed that binding was proportional to the amount of expressed sp55 added. TNFα that lacked the S-tag was not detected with S-protein-HRP (hatched boxes). These results showed that the expressed sp55 address domain can bind TNFα.

As an alternative to using sp55 as an address domain, one anti-TNFα scFV antibody will be selected from a set of eighteen that were obtained from Genetastix (San Jose, CA). These scFV antibodies were identified by Genetastix through use of their proprietary technology (www.genetastix.com) as having TNFα binding activity. Briefly, a human scFv cDNA library was produced from polyA RNA of human spleen, lymph nodes and peripheral blood lymphocytes through amplification of $V_H$ and $V_L$ sequences that were assembled in frame with a GAL4 activation domain (AD). The 18 scFvs were identified as binding human TNFα-lexA DNA binding domain when co-expressed intracellularly in yeast. The Genetastix scFvs expression vectors were obtained in the form of bacterial periplasmic expression vector pET25B (Novagen, Madison, WI). Standard recombinant DNA methods were used to subclone the scFv coding sequences into the pSecTag2A vector. The constructs were then sequenced to verify the structures. These scFv anti-TNFα antibodies is expressed and purified as described for the previous adzyme components, then analyzed for binding to TNFα. An indirect ELISA is used for TNFα based on the S-Tag™ system (see above, Fig. 11) to identify one of the 18 scFvs that shows high affinity binding to TNFα for use as an address domain. The selection of a specific scFv is based on a ranking of their relative binding strengths of the various structures. Further quantitative determinations of binding affinities for TNFα may be included once a prototype adzyme has been identified.

***c. Selection of the linkers*** A significant function of a linker is to connect a catalytic domain and an address domain in a fusion protein to yield cooperative function. The linker lengths can be experimentally investigated. Applicants found that a triple-repeat (or "3-repeat") of the flexible pentapeptide GGGGS (SEQ ID NO: 43) enabled a functional linkage of the enzyme and address domains. This linker can range in length from 23.60 Å in α-helical conformation to 50.72 Å as an extended chain. The initial adzymes have been built with 0 amino acids as linker (to minimize intramolecular digestion, 3 amino acids (AAA) and 20 amino acids (4 repeats of $G_4S$). Additional linker lengths may include 2 repeats of $G_4S$ (10 amino acids), 6 repeats of $G_4S$ (30 amino acids), 8 repeats of $G_4S$ (40 amino acids) and 10 repeats of $G_4S$ (50 amino acids), etc. The effect of various linker lengths (0-60 amino acids) on a mesotrypsin-based adzyme activity is demonstrated in an example below.

|  | Extended form | α-helical form |
|---|---|---|
| $(GGGGS)_2$ (SEQ ID NO: 30) | 32.02 Å | 15.96 Å |
| $(GGGGS)_4$ (SEQ ID NO: 31) | 64.04 Å | 31.92 Å |
| $(GGGGS)_6$ (SEQ ID NO: 32) | 96.06 Å | 47.88 Å |
| $(GGGGS)_8$ (SEQ ID NO: 33) | 128.08 Å | 63.84 Å |
| $(GGGGS)_{10}$ (SEQ ID NO: 34) | 160.1 Å | 79.8 Å |

***d. Adzyme Structures*** There are currently no reports in the literature for heterologous expression of trypsin in mammalian cells. Thus, it might be prudent to express the zymogen form that could be activated by enterokinase. Trypsinogen was thus cloned to be in frame with the leader sequence and N-terminal to the linker and address domain and in frame with the tandem myc-His$_6$ tags at the C-terminus.

| | |
|---|---|
| N-murine Igκ leader sequence-trypsinogen-0aa-sp55-myc-His6 | tgn-0-sp55 |
| N-murine Igκ leader sequence-trypsinogen-AAA-sp55-myc-His6 | tgn-3-sp55 |
| N-murine Igκ leader sequence-(G$_4$S)$_4$-trypsinogen-20aa-sp55-myc-His6 | tgn-20-sp55 |

***e. Self- or auto-proteolysis of the adzyme by the catalytic domain*** For those adzymes that employ a protease as a catalytic domain, it will generally be preferable to generate an adzyme that is resistant to self- or auto-proteolysis, which may affect the integrity and activity of the address domain, the catalytic domain or the linker.

Accordingly, potential address domains may be tested for their susceptibility to protease attack. If the set of potential proteases and address domains is sufficiently large then there are likely to be combinations in which the protease attacks the target but not the address domain. Thus it may be advantageous to generate a relatively large library of potential adzymes, and screen among these candidate adzymes for the optimal combination of address domain, linker, and enzyme domain. Single chain antibodies, due to their beta sheet structure, may be more resistant by nature to protease action. Once selected, the linkage arrangement of the address and enzyme domain can be used to minimize auto-proteolysis. Increasing the rigidity of the linker, limiting the degrees of freedom of each adzyme domain or applying a linker domain that orients the address and enzyme toward target but away from each other is possible. Additionally, address domains may be designed on the basis of evolved protein scaffolds, such as that of the single chain antibody, and such scaffolds may be re-engineered at vulnerable conserved positions to remove protease sensitive sites by mutagenesis. Alternatively or in combination, protease sites within an address or linker region may be selected against by using, for example, display evolutionary techniques.

Additionally, certain enzymes can undergo autolysis within the enzyme domain. For example, trypsin undergoes autolysis at R122. The autolysis site can be mutated to prevent autolysis (for example, R122H is a mutation in the human trypsin I gene which leads to inactivation of the autolysis pathway and thus overexpression of active trypsin leading to hereditary pancreatitis [31]). Protease domains can be expressed as zymogens to minimize the level of auto-proteolysis and maintain the adzyme in an inactive form. Adzymes will be activated immediately prior to application, or adzymes could be stored with an inhibitor that blocks the catalytic site that can be diluted away to render the adzyme active.

## 3.2. Production of adzymes

Recombinant adzymes may be generated using the pSecTag2A vector system or any other equivalently functional system for transient expression in mammalian cells. The adzymes can be purified, for example, from conditioned media by binding the His$_6$ tags to a nickel resin. Additional technical details are described in example section 3.1.a., above. All adzyme constructs generated in this section have been sequence confirmed.

***a. Adzyme construction*** In this particular example, the enzyme domain is a zymogen of human trypsin, although similar constructs using human MMP7 are also obtained. Human trypsin I (cationic trypsin) is encoded by PRSS1 gene (Accession #NM_002769). The catalytic domain and part of the propeptide of trypsinogen I is amplified (residues 16-247) from IMAGE clones 3950350 and 394971 (Invitrogen, Carlsbad, CA) and cloned into pSecTag2A. Human MMP7 (accession no. BC003635) residues 18-267, encoding the activation peptide (18-94) and catalytic domain (95-267) is amplified from IMAGE clone 3545760 (Open Biosystems, Huntsville, AL) and cloned into pSecTag2A (data not shown).

Also in this particular example, the address domain used is sp55, although other address domains such as scFV anti-TNFα antibody may also be used (both selected from a set of 18 potential candidates). All of these constructs when completed are verified by DNA sequencing.

The amino acid sequence of trypsinogen (tgn) is:

```
METDTLLLWVLLLWVPGSTG↓DIAPFDDDDKIVGGYNCEENSVPYQVSLNSGYHFCGGSLIN

EQWVVSAGHCYKSRIQVRLGEHNIEVLEGNEQFINAAKIIRHPQYDRKTLNNDIMLIKLSSR

AVINARVSTISLPTAPPATGTKCLISGWGNTASSGADYPDELQCLDAPVLSQAKCEASYPGK

ITSNMFCVGFLEGGKDSCQGDSGGPVVCNGQLQGVVSWGDGCAQKNKPGVYTKVYNYVKWIK

NTIAANSTRGGPEQKLISEEDLNSAVDHHHHHH*  (SEQ ID NO: 35)
```

The amino acid sequence of trypsinogen-0aa-sp55 (tgn-0-sp55) as expressed from pSecTag2A is:

METDTLLLWVLLLWVPGSTG↓DIAPFDDDDKIVGGYNCEENSVPYQVSLNSGYHFCGGSLIN
EQWVVSAGHCYKSRIQVRLGEHNIEVLEGNEQFINAAKIIRHPQYDRKTLNNDIMLIKLSSR
AVINARVSTISLPTAPPATGTKCLISGWGNTASSGADYPDELQCLDAPVLSQAKCEASYPGK
ITSNMFCVGFLEGGKDSCQGDSGGPVVCNGQLQGVVSWGDGCAQKNKPGVYTKVYNYVKWIK
NTIAANSLVPHLGDREKRDSVCPQGKYIHPQNNSICCTKCHKGTYLYNDCPGPGQDTDCREC
ESGSFTASENHLRHCLSCSKCRKEMGQVEISSCTVDRDTVCGCRKNQYRHYWSENLFQCFNC
SLCLNGTVHLSCQEKQNTVCTCHAGFFLRENECVSCSNCKKSLECTKLCLPQIENVKGTEDS
GTTRGGPEQKLISEEDLNSAVDHHHHHH* (SEQ ID NO: 36)

The amino acid sequence of trypsinogen-3aa-sp55 (tgn-3-sp55) as expressed from pSecTag2A is:

METDTLLLWVLLLWVPGSTG↓DIAPFDDDDKIVGGYNCEENSVPYQVSLNSGYHFCGGSLIN
EQWVVSAGHCYKSRIQVRLGEHNIEVLEGNEQFINAAKIIRHPQYDRKTLNNDIMLIKLSSR
AVINARVSTISLPTAPPATGTKCLISGWGNTASSGADYPDELQCLDAPVLSQAKCEASYPGK
ITSNMFCVGFLEGGKDSCQGDSGGPVVCNGQLQGVVSWGDGCAQKNKPGVYTKVYNYVKWIK
NTIAANSAAALVPHLGDREKRDSVCPQGKYIHPQNNSICCTKCHKGTYLYNDCPGPGQDTDC
RECESGSFTASENHLRHCLSCSKCRKEMGQVEISSCTVDRDTVCGCRKNQYRHYWSENLFQC
FNCSLCLNGTVHLSCQEKQNTVCTCHAGFFLRENECVSCSNCKKSLECTKLCLPQIENVKGT
EDSGTTRGGPEQKLISEEDLNSAVDHHHHHH* (SEQ ID NO: 37)

The amino acid sequence of trypsinogen-20aa-sp55 (tgn-20-sp55) as expressed from pSecTag2A is:

METDTLLLWVLLLWVPGSTG↓DIAPFDDDDKIVGGYNCEENSVPYQVSLNSGYHFCGGSLIN
EQWVVSAGHCYKSRIQVRLGEHNIEVLEGNEQFINAAKIIRHPQYDRKTLNNDIMLIKLSSR
AVINARVSTISLPTAPPATGTKCLISGWGNTASSGADYPDELQCLDAPVLSQAKCEASYPGK
ITSNMFCVGFLEGGKDSCQGDSGGPVVCNGQLQGVVSWGDGCAQKNKPGVYTKVYNYVKWIK
NTIAANSAAAGGGGSGGGGSGGGGSGGGGSRLVPHLGDREKRDSVCPQGKYIHPQNNSICCT
KCHKGTYLYNDCPGPGQDTDCRECESGSFTASENHLRHCLSCSKCRKEMGQVEISSCTVDRD
TVCGCRKNQYRHYWSENLFQCFNCSLCLNGTVHLSCQEKQNTVCTCHAGFFLRENECVSCSN
CKKSLECTKLCLPQIENVKGTEDSGTTRGGPEQKLISEEDLNSAVDHHHHHH* (SEQ ID
NO: 38)

In addition, sp55 was also cloned into pSecTag in similar fashion. The amino acid sequence of sp55 as expressed from pSecTag2A is:

```
METDTLLLWVLLLWVPGSTG↓DAAQPARRAVRSLVPHLGDREKRDSVCPQGKYIHPQNNSIC

CTKCHKGTYLYNDCPGPGQDTDCRECESGSFTASENHLRHCLSCSKCRKEMGQVEISSCTVD

RDTVCGCRKNQYRHYWSENLFQCFNCSLCLNGTVHLSCQEKQNTVCTCHAGFFLRENECVSC

SNCKKSLECTKLCLPQIENVKGTEDSGTTRGGPEQKLISEEDLNSAVDHHHHHH*        (SEQ

ID NO: 39)
```

The adzymes are constructed from the individual enzyme and address domains connected via the three different linkers using an overlap PCR method; as was done for the model thrombin adzyme (see previous examples). The constructs have been verified by DNA sequencing.

**b. adzyme Expression** Transient expression in 293T cells are carried out in T175 flasks. Benzamidine, a small molecule competitive inhibitor of trypsin activity with a $K_i$ of 18 $\mu$M, is added to a final concentration of 1 mM to stabilize trypsinogen and trypsinogen adzyme expression. Conditioned media is harvested at 24 hour intervals, or allowed to accumulate upto 72 hrs.

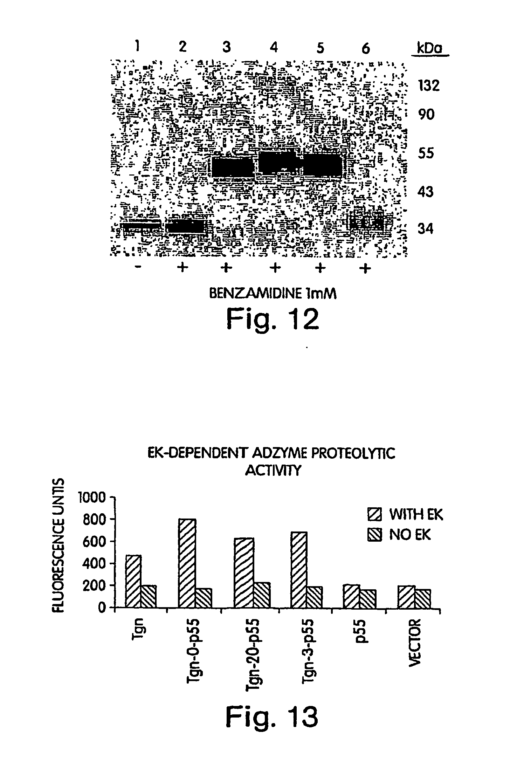

An example of representative expression as analyzed by Western blotting with anti-myc antibody is shown below in Figure 12. The increased intensity of anti-myc signal in lane 2 demonstrates the stabilizing effect of the small molecule trypsin inhibitor, benzamidine. Adzymes containing 0, 3 and 20 amino acids as the linker are expressed at similar levels (lanes 3-5) and are also stabilized by the presence of benzamidine. The myc reactive band is of the expected size of approximately 51 kDa. Finally, sp55 is also produced in comparable amounts to trypsinogen and expression is not affected by the presence of benzamidine.

In brief, equal volumes of conditioned media after accumulation of secreted protein for 24 hours post transfection were electrophoresed on 4-20% TGS (Novex) gels, electroblotted to nitrocellulose membrane and stained with anti-myc antibody.

**c. adzyme Purification** In one embodiment, $His_6$-nickel methodology is the preferred method of purification. This method is rapid, simple and available in either column format for large batches or in a 96 well format for parallel assay testing. However, many other alternative methods of purification can be used (see Example 2, section 2.1). For example, one option could be benzamidine sepharose column chromatography (Pharmacia, NJ), which incorporates a protease inhibitor into the resin. Standard characterization of purified proteins will include Western analysis with anti-myc antibodies and silver-stained gels to assess purity and recovery of the adzyme preparations. The produced adzymes may be further analyzed to quantify binding and proteolytic activities.

The following protocol is merely one of the preferred purification methods for AdZyme / enzyme purification.

Briefly, pooled conditioned media (CM) is pH-adjusted to ~ 2 pH units above the pI of the protein of interest, and is filtered through a 0.2 $\mu$M filter prior to loading on a Q Sephrarose Fast Flow HiTrap Column (Amersham Biosciences part # 17-5156-01). The anion exchange chromatography can be used as a concentration / buffer exchange step to remove media components that may interfere with subsequent affinity chromatography. The column is equilibrated with 20mM Tris, 5mM NaCl pH (of adjusted CM) + 1mM Benzamidine, loaded at 5 ml/min, and washed with 20 mM Tris pH 8.0 + 1 mM Benzamidine, followed by 20 mM Tris, 10 mM NaCl pH 8.0 + 1 mM Benzamidine.

Bound proteins can be eluted with 20 mM Tris, 500 mM NaCl pH 8.0 + 1 mM Benzamidine. The column can be stripped with 20 mM Tris, 1.0 M NaCl, pH 8.0 + 1 mM Benzamindine for further use.

The eluted fractions can be pooled for HisTrap column.

To purify $His_6$-tagged protein, a HisTrap HP Ni Sepharose (Amersham Biosciences part # 17-5247-01) is loaded with the eluate from the Q column at a flow rate of 1 ml/min. The column is equilibrated with PBS pH 7.4 + 1 mM benzamidine prior to the load. Following washes of increasing stringency (Wash 2: PBS + 0.5 M NaCl pH 7.4 + 1 mM Benzamidine, Wash 3: PBS, 0.475 M NaCl, 50 mM Imidazole pH 7.4 + 1 mM Benzamidine), the $His_6$ tagged protein is eluted with PBS, 0.25 M NaCl, 500 mM Imidazole pH 7.4 + 1 mM Benzamidine.

Scale of columns determined by volume of conditioned media being processed. Generally, a 15 ml Q column may be used (3 X 5 ml HiTrap) for 2.0 L of CM. For HisTrap chromatography, a 1-2 ml column may be used, depending on the volume of CM processed at the Q column step. Typical yield for AdZymes purified using this protocol is around 0.8 - 1.2 mg/L of CM.

**d. Recombinant protein determination.** In one embodiment, adzymes are constructed with a carboxy terminal tandem myc-$His_6$ tags. An ELISA method is developed to detect the c-myc tag for quantitating recombinant proteins bound to Ni-NTA on surfaces. This helps to normalize the amount of adzyme used in any biochemical analyses and bioassays.

The following method can be used to quantify heterologously expressed proteins containing tandem myc and

His$_6$ tags using a sandwich ELISA approach. In summary, diluted conditioned medium containing recombinant proteins are incubated in wells of Ni-NTA coated HisSorb microtiter plates (catalog no. 35061 Qiagen, Valencia, CA) and then reacted with anti-myc-HRP (catalog no. R951-25, Invitrogen, Carlsbad, CA). Bound recombinant material is then detected by incubation with a chromogenic substrate. A standard curve was established in parallel with purified recombinant sp55 (independently quantified using a commercially available ELISA (catalog no. QLA98, Oncogene Research Products, Madison, WI) containing tandem myc His$_6$ tag allowing quantification of captured material. Conditioned media from mock transfected cells served as a negative control.

In brief, conditioned media from transfections was diluted directly into assay buffer (0.5 % BSA Fraction V, 0.05 % Tween-20 in 1 X PBS pH 7.4) to a final volume of 100 μL /well. Known amounts of the standard, sp55, was serially diluted in similar fashion in assay buffer. Binding of the His$_6$ tag of the recombinant proteins to the Ni-NTA surface was allowed to proceed at room temperature for half an hour with slow shaking. Anti-myc-HRP was then added to all wells at a final dilution of 1:1500 such that the final volume in the wells was 150 μL. Binding was allowed to proceed for two hours at room temperature with slow shaking. Following the binding of anti-myc to the His$_6$-captured proteins, the wells were washed 6 times with wash buffer (PBS containing 0.05% Tween 20) and blotted dry. Then, the chromogenic substrate TMB (Sigma Catalog #T-4444) was added to each of the wells to a final volume of 100 μL. The increase in absorbance at 370 nm was monitored by a microtiter plate UV/VIS reader (Molecular Devices SPECTRAmax 384 Plus). All samples are assayed in duplicate.

Using this method of quantification, average yield of trypsinogen adzymes were estimated at 1 μg/mL.

**3.3. Biochemical analysis of TNFα-specific adzymes.**

This section describes methods to quantify binding and proteolytic activities of adzymes made against TNFα.

*a. Adzyme binding* Adzyme address domain functionality, e.g., binding to TNFα, is quantified by the TNFα binding assay described above and by the ability of the address domain to independently inhibit TNFα activity in the L929 assay prior to activation. Adzymes with the ecto-domain of p55 have been tested with recombinant p55 as parallel controls. The adzyme proteins exhibit specific binding characteristics (amount of TNF bound per mole of protein) and binding affinities similar to the address domains alone.

Alternatively, the following method can be used to establish the presence of a functional TNFα address domain within recombinantly expressed Adzymes by means of a modified ELISA-like assay. In summary, wells of a microtiter plate are precoated with TNFα and then reacted with diluted conditioned medium containing candidate adzymes. The detection of adzymes that express a functional, high-affinity TNFα binding domain (and hence, are retained on the microtiter plate following washing of the microtiter plate) is effected by subsequent capture of a chromogenic enzyme conjugate that is specific for a detection tag within the adzyme and control constructs, followed by addition of a chromogenic substrate. The inclusion of control wells in which the capture and detection of adzymes is not expected to be present, and parallel evaluation of similar constructs that do not encode the detection tag or TNFα-specific address domain provides evidence that the expressed adzymes contain a functional TNFα-specific address domain that binds specifically to the immobilized TNFα. Typically, one or more reversible or irreversible protease inhibitors may also be included in assay buffers to prevent autocatalysis or proteolytic activity of the adzyme, thereby restricting degradation of the adzyme and/or assay reagents.

In an illustrative example, an assay for human trypsinogen-containing adzymes specific for TNFα is described. Wells of a microtiter plate (Nunc-ImmunoModule, MaxiSorp Surface) were precoated with 100 μL/well of recombinant human TNFα (RDI Catalog #RDI-301X) at a concentration of 1 μg/mL diluted into phosphate-buffered saline (PBS), pH 7.2. An equal number of wells received 100 μL of PBS alone. The microtiter plate was then incubated at 4°C overnight (approximately 16 hours). The liquid from the wells was removed and the microtiter plate was washed twice with wash buffer (PBS containing 0.05% Tween 20). All wells of the microtiter plate were blocked by addition of 200 μL/well of block/diluent buffer (PBS containing 0.05% Tween 20 and 0.05% bovine serum albumin [BSA; Fraction V, RIA & ELISA-grade, Calbiochem Catalog #125593]). The microtiter plate was incubated at room temperature for 2 hours with slow shaking. The block solution was removed from the wells and 100 μL/well of conditioned medium from transient adzyme transfections in 293T cells diluted 1:10 into block/diluent buffer containing 1 mM benzamidine (Sigma Catalog #B-6506) was added to TNFα-containing wells and to wells that do not contain TNFα. The plate was incubated for 1 hour at room temperature with slow shaking. Following removal of liquid, the wells of the microtiter plate were washed four times with wash buffer. Wells then received anti-myc antibody conjugated to horseradish peroxidase (anti-myc-HRP; Invitrogen Catalog #46-0709) diluted 1:2000 in block/diluent buffer containing 1 mM benzamidine. The microtiter plate was incubated for 1 hour at room temperature with slow shaking. Following removal of liquid and washing as in the above, 100 μL/well of substrate (TMB, Sigma Catalog #T-4444) was added to each of the wells. The increase in absorbance at 370 nm was monitored by a microtiter plate UV/VIS reader (Molecular Devices SPECTRAmax 384 Plus).

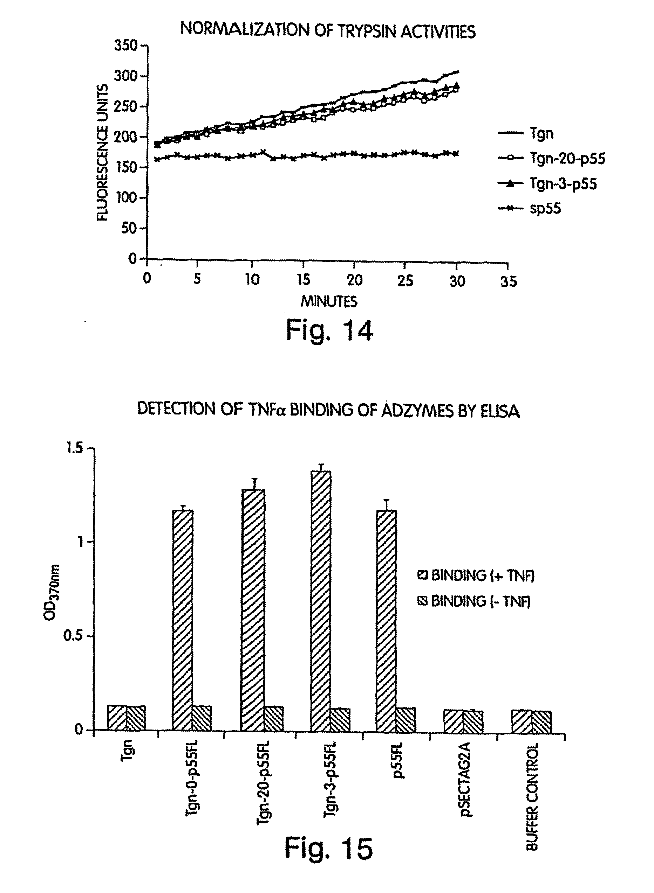

The results shown in Figure 15 are from a representative experiment and reveal the mean OD and standard deviation for samples and experimental controls evaluated in triplicate on a single microtiter plate. As illustrated, only Adzyme constructs and a control protein that are able to bind to immobilized TNFα and that also contain the c-myc antibody sequence generate a positive signal above background at 370 nm. Included in this category is the trypsinogen-p55FL adzymes containing no linker (Tgn-0-p55FL) as well as those containing linkers of 3 amino acids (Tgn-3-p55FL) and 20 amino acids (Tgn-20-p55FL). As expected, a positive control sample containing the p55FL-myc-his construct (p55L) also binds and produces a positive signal above background. A construct consisting of Trypsinogen-myc-his did not bind above background presumably due to significantly lower affinity for TNFα in the absence of a high affinity address domain (p55FL). Similarly, the conditioned medium from a transfection vector control (pSECTAG2A) did not demonstrate a positive signal above background. Background, non-specific binding of the anti-myc antibody to wells that contain or do not contain TNFα was negligible as revealed by "Buffer Control."

It should be understood that, although the present illustrative example detects binding to TNFα, this assay format is generic to any of the target molecules. One advantage of the assay described here is the inclusion of a reversible protease inhibitor in cell culture, during the expression of the adzymes and in assay buffers, to prevent inadvertent autoactivation /proteolytic breakdown of the adzyme and/or activation by endogenous proteases. This can be used as a general solution to expression of zymogens and/or active proteases. Importantly, one or more protease inhibitors can also be included in assay buffers for the purposes of protein quantitation and confirmation of target specificity (as shown in this example). This general approach alleviates concerns regarding handling of autocatalytically-prone and/or active adzymes.

***b. adzyme activation.*** Activation of the adzyme enzyme domain is carried out by incubating at 37˚C following the manufacturer's recommendations. The progress may be monitored by SDS-PAGE and Western blotting (e.g., see Fig. 7). Enterokinase (Novagen, Madison, WI) was used for activation of trypsinogen. For an in vitro TNFα assay, enterokinase need not be removed post activation, since it has been determined that enterokinase has no proteolytic activity towards TNFα and no effect in the L929 bioassay.

Applicants have developed a method for carrying out on-plate capture, activation and proteolytic assays for recombinantly produced enzymes or adzymes containing a His$_6$ tag. In summary, diluted conditioned medium containing recombinant proteins are incubated in wells of Ni-NTA coated HisSorb microtiter plates, then treated with enterokinase and presented with suitable peptide substrates. The peptide substrate used in the current example is tosyl-GPR-AMC (Catalog no. 444228, Sigma, St. Louis, MO) which has been described previously. Proteolysis of the peptide bond between the Arg residue in the substrate and the AMC leads to the release of free fluorescent AMC (excitation 383nm, emission 455 nm). Inclusion of conditioned media from sp55 or vector transfections provide important negative controls for the levels of adventitious protease expression in transfected cells and substrate background and hydrolysis under assay conditions.

In brief, conditioned medium containing recombinant proteins was diluted directly into assay buffer (0.5 % BSA Fraction V, 0.05 % Tween-20 in 1 X PBS pH 7.4) to a final volume of 100 μL / well. Typically, 5-25% of conditioned medium per well yielded good linear response. Binding of the His$_6$ tag of the recombinant proteins to the Ni-NTA surface was allowed to proceed at room temperature for two hours with slow shaking. Following the binding of anti-myc to the His$_6$-captured proteins, the wells were washed 6 times with wash buffer (PBS containing 0.05% Tween 20 or PBST, 200 μL per wash) and blotted dry. This step also accomplishes the removal of benzamidine which would otherwise interfere with subsequent steps in the assay. Activation of zymogen is achieved by the addition of 1 U of enterokinase (EK, Catalog no. 69066, Novagen, Madison, WI) in a final volume of 100 uL of PBST. Activation was carried out for 1 hour at 37˚C. A parallel set of samples received no enterokinase but underwent similar incubation. Finally, the wells were washed 6 times with PBST prior to the addition of trypsin digestion buffer (100 mM Tris pH 8, 5 mM CaCl$_2$) containing 10 μM tosyl-GPR-AMC. Proteolytic activity was followed by monitoring the fluorescence at 455 nm following excitation at 383 nm using a Gemini EM microplate spectrofluorometer (Molecular Devices, CA). Figure 13 shows a snapshot of representative experiments where the fluorescence detected at the end of 2 hours of incubation is compared for the different recombinant proteins. There is negligible proteolytic activity in the absence of enterokinase activation of captured recombinant trypsinogen and trypsinogen adzymes (striped bars). In this assay format, conditioned media from sp55 and vector transfections do not contain detectable amounts of proteases which could give rise to artifacts as evidenced by the background levels of fluorescence. However, following enterokinase treatment tryspinogen and the adzymes (tgn-0-p55, tgn-20-p55, tgn-3-p55) exhibit significant amounts of proteolysis as evidenced by the 4-7 fold higher levels of fluorescence as compared to the no activation controls.

On the other hand, MMP7 is activated with organomercurial compound p-aminophenylmercuric acetate (APMA, Calbiochem 164610) and APMA can be (and will be) removed according to instructions provided by the supplier.

***c. Proteolysis assay using synthetic peptide substrates.*** The adzyme catalytic domain's proteolytic activity post activation was determined with synthetic linear peptide substrates as described above. Proteolytic activity was determined in a plate format as described above using varying amounts of adzymes and substrates against a commercially available enzyme standard. Substrate (tosyl-GPR-AMC) cleavage was monitored by the release of the fluorogen AMC. Data from a representative experiment is shown below in Figure 14, where conditioned media from transfections (24 hours post transfection) were bound to Ni-NTA plates, activated on plate, and assayed for proteolytic activity with a fixed concentration (10 $\mu$M) of substrate (tosyl-GPR-AMC).

The assay for MMP7 proteolytic activity may use a fluorogenic substrate (dinitrophenyl-RPLALWRS; Calbiochem Cat. No. 444228).

Data from the biochemical analyses of adzymes can be used to normalize the concentration and proteolytic activity of adzyme preparations for assessment of bioactivity.

### 3.4. Testing adzymes for bioactivity.

To determine the bioactivity and selectivity of adzymes against TNF$\alpha$, adzymes will be used to inactivate TNF$\alpha$ and bioactivity will be quantified in a TNF$\alpha$-induced L929 cell death bioassay. Selectivity can be determined by comparing adzyme inactivation of TNF$\alpha$ alone and mixed with human serum albumin (HSA). The soluble TNF$\alpha$ receptor p55 may serve as a stoichiometric blocker of TNF$\alpha$.

The L929 bioassay is a stringent test for biologically active TNF$\alpha$. Assays are done using preparations of all twelve adzymes, plus the four individual address and enzyme domains singly and in combinations. In each case, normalized quantities of purified adzymes (as assessed above) will be mixed with TNF$\alpha$ alone or TNF$\alpha$ plus HSA and incubated at 37˚C for 4 hr and overnight. The overnight digestion represents the standard protocol. Preliminary results may be followed by time course studies as needed. Residual activity may be assayed by the L929 bioassay.

It is expected that the enzyme domain alone will inactivate TNF$\alpha$ and shift the survival curve to the right by 2 logs for the trypsin domain (Fig. 10, Table 2). In contrast, an effective adzyme will be expected to effect a larger rightward shift and/or do so at much lower concentrations or more rapidly (e.g, 4hr as opposed to overnight). A 10-fold enhancement in the inactivation of TNF$\alpha$ (a shift in the inactivation curve one log unit to the right) is a convincing demonstration of the potential of adzymes as catalytic protein antagonists. Furthermore address domains alone should only minimally inactivate (by stoichiometric binding) TNF$\alpha$, and mixtures of the address and enzyme domains should fare no better than the enzyme domains alone. The bioactivity of all adzymes may be ranked at matched molar concentrations, and the selectivity of those that inactivate TNF$\alpha$ can be analyzed.

Selectivity can be demonstrated in a mixing experiment (e.g., see Davis et al, 2003)adzymes will be used to digest TNF$\alpha$ alone and TNF$\alpha$ plus HSA, and the digests will be analyzed in the bioassay (see Fig. 10). Human serum albumin is the most logical choice for this mixing experiment. It is present in serum at high concentration and most likely to pose a challenge to the selective action of a TNF$\alpha$-specific adzyme. Initial tests of all adzymes can be done using a 10-fold molar excess of HSA over TNF$\alpha$. Adzymes that are not selective are expected to show reduced bioactivity in the presence of the competing substrate. However selective adzymes should retain full bioactivity in the presence of excess HSA. Adzymes that pass this first test can be compared further by repeating the analysis in the presence of a higher concentration of HSA in the mixture. Once again, adzymes can be ranked according to how much bioactivity is retained in the presence of HSA. Several rounds of competition should reveal structures that are both bioactive and selective catalytic antagonists of TNF$\alpha$.

### Example 4. Using Kinetic Modeling to Study the Adzyme System

Kinetic theory was applied to the reaction network of a direct adzyme, shown in (Eq-2), to develop a mathematical model of adzyme performance. Such a model can be used to design and optimize the parameters of an adzyme, and to predict important functional properties of the adzyme such as the amount of substrate that it can inactivate.

In this example, a simulation of the total amount of inactivation of a substrate by three different drugs was performed with the objective of comparing the potency of the adzyme to the potency of its constituent domains individually. The three drugs were:

1. An address with $k_{on}$ = $10^6$ M$^{-1}$s$^{-1}$ and $k_{off}$ = $10^{-3}$ s$^{-1}$ (K$_D$ = 1 nM)
2. An enzyme with $k_{on}$ = $10^3$ M$^{-1}$s$^{-1}$, $k_{off}$ = $10^{-3}$ s$^{-1}$, and $k_{cat}$ = 1 s$^{-1}$ (K$_M$ = $10^{-3}$ M)
3. A direct adzyme with the properties of the address and enzyme above, and $[S]_{eff}$ = $10^{-6}$ M.

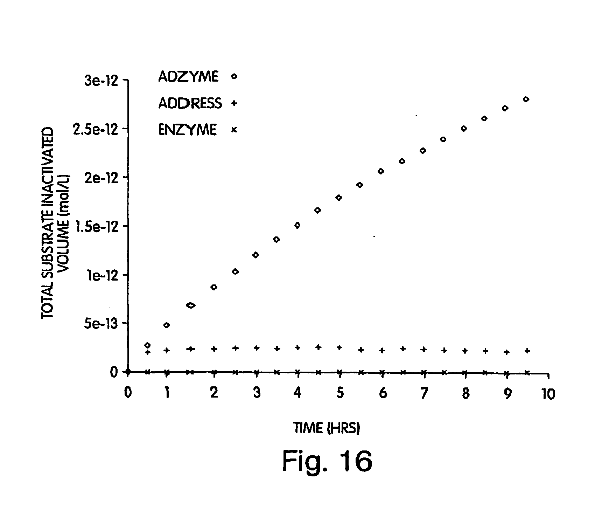

The initial concentrations of the drugs were 50 pM and the initial concentration of target substrate was 5 pM. The total amount of substrate inactivated by each of these three drugs is shown in Figure 16.

Specifically, Figure 16 illustrates kinetic model results comparing the performance of an adzyme, an address, and an enzyme. The results indicate that the adzyme inactivates significantly more substrate than either the address or the enzyme alone.

For example, the enzyme is too weak by itself to inactivate a substrate at such low (pM) concentrations.

Consequently, the total amount of substrate inactivation by the enzyme is not significantly different from zero. The address rapidly binds and inactivates some substrate, but because the concentration of substrate is much less than the $K_D$ of the address, binding quickly becomes equilibrium limited and the address can only inactivate about 0.25 pM, or 5 %, of the total substrate. The adzyme can rapidly bind and inactivate substrate like the address, but it can also convert the adzyme-substrate complex into product, removing the equilibrium limitation.

This example shows that the model adzyme combines address and enzyme functionality in a synergistic way. Its potency is significantly higher than the sum of the address and the enzyme alone.

**Example 5. Construction, Expression, & Purification of Mesotrypsin-TNF receptor I**

To provide an illustrative example of a working adzyme, an active fragment of mesotrypsin was linked through a short linker sequence to the TNF receptor I fragment sp55 to create a functional adzyme.

Mesotrypsin (Accession no. NM_002771 & NP_002762) was expressed with its native leader sequence, and tagged at its C-terminus with the myc and His$_6$ tags. The coding sequence of mesotrypsin was cloned into the expression vector, pDEST40 (Invitrogen, Carlsbad, CA), such that expression was driven by the CMV promoter. Mesotrypsin_ $(G_4S)_7$_p55_2.6 was assembled by overlap PCR such that a flexible linker of 35 amino acids (Gly$_4$Ser repeated 7 times) was introduced between the N-terminal mesotryspin (residues 1-247) and the C-terminal truncated sp55 (residues 41-150) or TNF receptor I (this truncation is referred to as sp55_2.6 and has been described previously in the application). Finally, the coding sequence of the adzyme was also tagged C-terminally with the myc and His6 tags, followed by a TGA stop codon and the BGH polyadenylation signal. All constructs were sequence confirmed. Mesotrypsin is expressed in both constructs as an inactive zymogen. The propeptide is removed by enterokinase cleavage, leading to the formation of active mesotrypsin.

The amino acid sequence for mesotrypsinogen as made from pDEST40 is:

```
MNPFLILAFVGAAVAVPFDDDDK/IVGGYTCEENSLPYQVSLNSGSHFCGGSLISEQWVVSA
AHCYKTRIQVRLGEHNIKVLEGNEQFINAAKIIRHPKYNRDTLDNDIMLIKLSSPAVINARV
STISLPTAPPAAGTECLISGWGNTLSFGADYPDELKCLDAPVLTQAECKASYPGKITNSMFC
VGFLEGGKDSCQRDSGGPVVCNGQLQGVVSWGHGCAWKNRPGVYTKVYNYVDWIKDTIAANS
EQKLISEEDLNSAVDHHHHHH (SEQ ID NO: 40)
```

The amino acid sequence for mesotrypsinogen_35aa_p55_2.6 as made from pDEST40 is:

```
MNPFLILAFVGAAVAVPFDDDDK/IVGGYTCEENSLPYQVSLNSGSHFCGGSLISEQWVVSA
AHCYKTRIQVRLGEHNIKVLEGNEQFINAAKIIRHPKYNRDTLDNDIMLIKLSSPAVINARV
STISLPTAPPAAGTECLISGWGNTLSFGADYPDELKCLDAPVLTQAECKASYPGKITNSMFC
VGFLEGGKDSCQRDSGGPVVCNGQLQGVVSWGHGCAWKNRPGVYTKVYNYVDWIKDTIAANS
GGGGSGGGGSGGGGSGGGGSGGGGSGGGGSGGGGSPGSTGDDSVCPQGKYIHPQNNSICCT

KCHKGTYLYNDCPGPGQDTDCRECESGSFTASENHLRHCLSCSKCRKEMGQVEISSCTVDRD
TVCGCRKNQYRHYWSENLFQCFNCSLCLTRGGPEQKLISEEDLNSAVDHHHHHH (SEQ ID
NO: 41)
```

The slash ("/") shows the site of enterokinase cleavage.

Transient transfections were carried out in 293T cells (Genhunter, Nashville, TN) using Lipofectamine 2000 (Invitrogen, Carlsbad, CA). About $1.2 \times 10^6$ cells per T175 flask were transfected with 6.6 µg of DNA as per the manufacturer's instructions. The day after transfection, the media was supplemented with benzamidine (Sigma, St. Louis, MO) at a final concentration of 1 mM. Benzamidine is a reversible small molecule inhibitor of serine proteases with micromolar Ki. In particular, the Ki of benzamidine for mesotrypsin is 0.22 $\mu$M (Szmola et al. Human mesotrypsin is a unique digestive protease specialized for the degradation of trypsin inhibitors. J Biol Chem. 278(49):48580-9, 2003). Harvesting of the adzyme from the conditioned media (CM) of transiently transfected cells were carried out every 48-72 hours for a total of 6 harvests per transfection. Pooled CM (typically 600 mL) was clarified by centrifugation and concentrated via Amicon 80 centrifugal devices (Millipore, Bedford, MA), and then dialyzed overnight at 4˚C against PBS pH 7.4 containing 1 mM benzamidine with two changes of buffer. The concentrated dialyzed CM is loaded onto a 5 mL HiTrap chelating column (Pharmacia, Piscataway, NJ). The column was washed with 10 column volumes of PBS with 1 M NaCl and 1 mM benzamidine, then with 10 column volumes of PBS with 1 M NaCl, 20 mM imidazole and 1 mM benzamidine. Recombinant protein was eluted with 5 column volumes of PBS with 1 M NaCl, 0.5 M imidazole, 1 mM benzamidine. The nickel column eluate was dialyzed overnight at 4˚C against 20 mM Tris pH 8.0 with 1 mM benzamidine and then loaded onto a HiTrap-Q 1 mL anion exchange column. The column was then washed with 10 column volumes of 20 mM Tris pH 8.0 with 1 mM benzamidine. The bound protein was eluted in a 50 mL gradient of 0-500 mM NaCl in 20 mM Tris pH 8.0 containing 1 mM benzamidine.

To screen fractions for protease activity, 2 $\mu$L of each fraction was added to 98 $\mu$L of trypsin digestion buffer (100 mM Tris pH 8.0, 5 mM CaCl$_2$, 0.05% Tween-20) and activated with 0.1 $\mu$L EK (1.7 U/$\mu$L) from Novagen (Madison, WI) for 1 hr at 37˚C. Substrate (tosyl-GPR-AMC or t-GPR-AMC) was then added to a final concentration of 50 $\mu$M, and proteolytic activity was monitored by the generation of fluorescence from free AMC (excitation 350 nm, emission 450 nm) using a Gemini plate reader (Molecular Devices, Sunnyvale, CA). Fractions exhibiting high level of proteolytic activity were screened by Western blotting with anti-myc and size exclusion chromatography HPLC (SEC-HPLC) to monitor solution phase behavior. Fractions with high proteolytic activity and monomeric behavior in solution were pooled and checked for binding to TNF by SEC-HPLC.

Active site titration was performed on activated mesotrypsin and mesotrypsin_35aa_p55_2.6 with a non-fluorescent substrate 4-methylumbelliferyl-p-guanidinobenzoate (MUGB). This compound binds to the active center of serine proteases and the nucleophilic attack of the catalytic Ser residue liberates the highly fluorescent product 4-methylumbelliferone (MU, excitation 350 nm, emission 450 nm). The concentration of mesotrypsin was determined to be 500 nM and the concentration of mesotrypsin_35aa_p55_2.6 was determined to be 86 nM.

## Example 6. Comparison of Adzyme (mesotrypsin-TNF Receptor I) and Enzyme (mesotrypsin) Activities

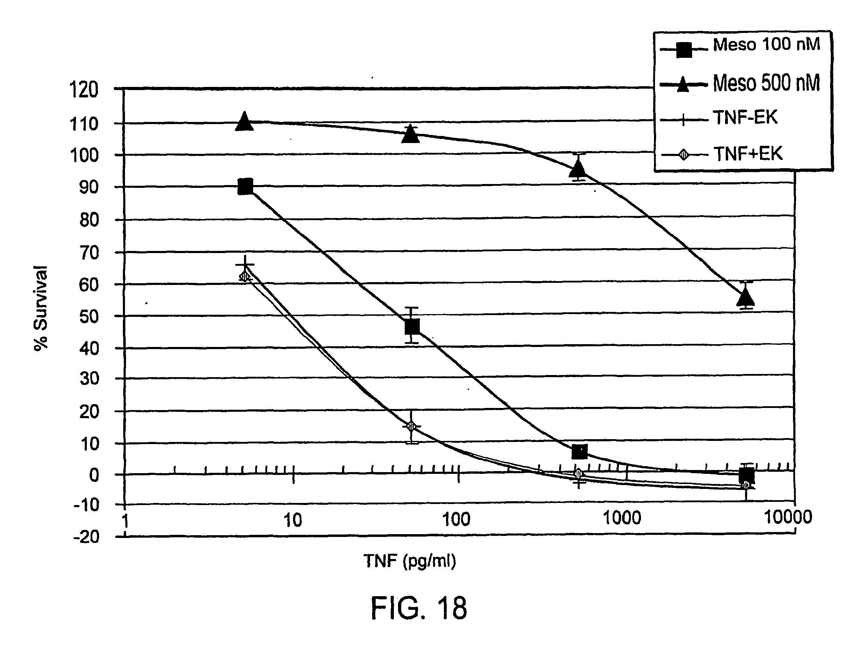

Mesotrypsin is a relatively weak protease compared to other trypsin isoforms. It had been demonstrated that a molar excess of mesotrypsin is needed to inactivate TNF in the L929 bioassay as shown in Figure 18.

Specifically, in this set of experiments, mesotrypsinogen was activated with enterokinase (EK) at a final concentration of either 100 or 500 nM. Substrate (target) TNF was included in the reactions at a final concentration of 100 nM. As controls, identical concentration of TNF was incubated in trypsin digestion buffer (100 mM Tris pH 8.0, 5 mM CaCl$_2$, 0.05% Tween-20) with or without the activating enterokinase (1.1 U EK/ 100 $\mu$L 100 nM TNF). All reactions were allowed to proceed overnight at 37˚C. Aliquots were removed to verify proteolytic activity post activation using the synthetic substrate t-GPR-AMC as described above. The TNF digestion reactions were serially diluted and applied to L929 cells in a simplified 4-point dilution series overnight. Bioactive TNF retains the ability to induce apoptosis in L929 cells, while cleaved TNF loses that activity. Thus L929 cell survival, as measured by the formation of a formazan product the next day (as described previously), can be used to quantify the amount of remaining TNF bioactivity in each reaction.

Figure 18 indicated that, at equimolar ratios, mesotrypsin achieved only marginal inactivation of TNF in solution. A molar excess of mesotrypsin is required to achieve substantial inactivation (greater than 1 log) of TNF.

In contrast, the following series of experiments demonstrated that the corresponding adzyme exhibited greater specificity than the enzyme, and thus was able to inactivate TNF at lower molar ratios than required by the enzyme mesotrypsin. The activated adzyme was also more potent than the stoichiometric binder, sp55-2.6, which is present in the unactivated adzyme.

First of all, to generate active enzyme and adzyme, EK activation was carried out for one hour, using either mesotrypsin diluted to 86 nM, or mesotrypsin_35aa-p55_2.6 at 86 nM (1.7 U of EK per 100 pL of enzymatic species). Mock activation reactions (without EK activation) for both enzyme and adzyme at similar concentrations were also performed as controls. After one hour of activation (or mock activation), enzyme and adzyme were serially diluted 1:2 and 1:4, before TNF was added to each reaction to a final concentration of 100 nM. TNF digestion was then allowed to proceed overnight at 37˚C. Identical amounts of TNF (100 nM) were incubated, at the absence of enzyme and adzyme, with or without EK to serve as negative controls for the enzyme and adzyme reactions. Proteolytic activities of all reactions towards the synthetic substrate t-GPR-AMC were monitored at the start and the end of TNF digestion. Overnight TNF digestion reactions were diluted and applied to L929 cells. Digestions were also subjected to Western blot analysis with an anti-TNF antibody (Abcam, UK) and an anti-trypsin antibody (Abcam, UK).

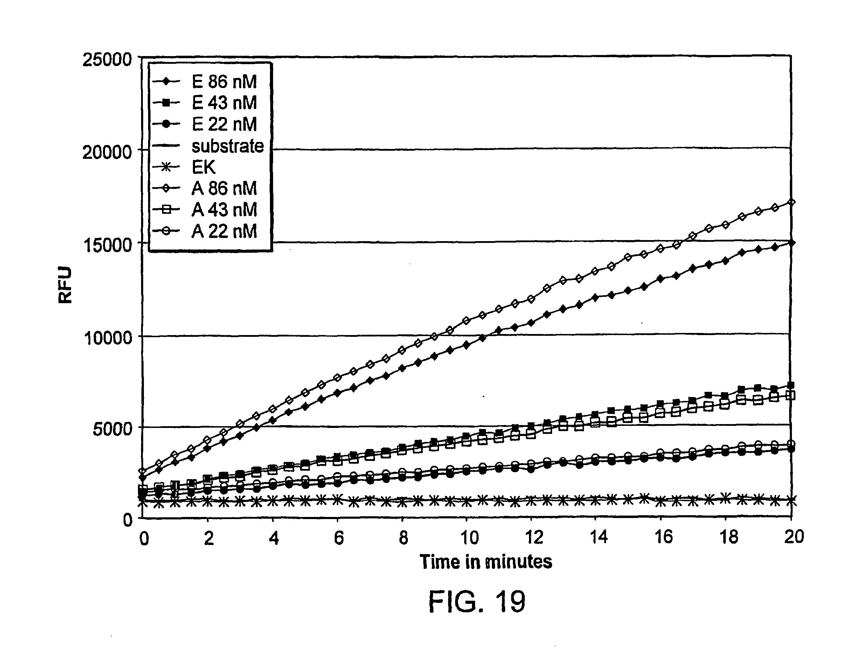

Figure 19 shows largely well-normalized proteolytic activities of enzyme and adzyme towards the synthetic peptide t-GPR-AMC (which fits into the active site of the protease). This demonstrated that the inherent catalytic properties of mesotrypsin are preserved in the context of the mesotrypsin_35aa_p55 adzyme, since the enzyme and adzyme have very similar activities. Under all three experimental concentrations of adzyme / enzyme tested, enzyme and adzyme have well normalized activities. Mock activation reactions showed no proteolytic activity for either enzyme or adzyme (data not shown).

Adzyme is More Selective than Enzyme

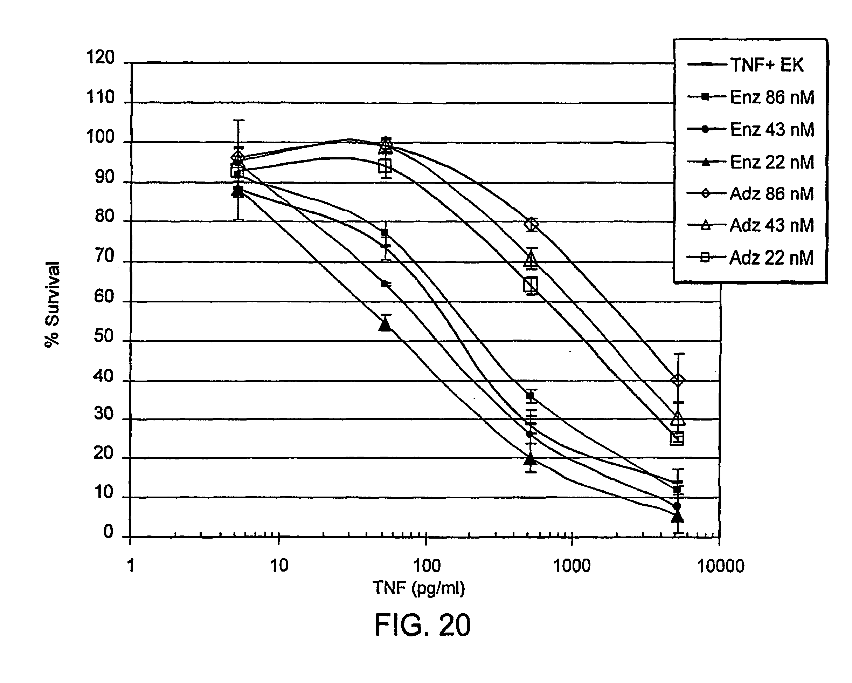

Compared to identical concentration of enzyme (mesotrypsin), adzyme (meso_35aa_p55_2.6) achieves greater than 1 log (more than 10-fold) inactivation of the bioactivity of the target protein TNF, at all 3 concentrations tested (compare open symbols with the corresponding solid symbols in Figure 20). In contrast, at these concentrations, the enzyme mesotrypsin, is either inactive or marginally active towards TNF. This difference in activity between adzyme and enzyme is not due to the inherent differences in proteolytic activities, as already demonstrated in Figure 19. While not wishing to be bound by any particular theory, the adzyme is likely able to preferentially bind TNF by virtue of its address domain, sp55_2.6, thus bringing the bound TNF in close proximity to the mesotrypsin catalytic domain, and allowing proteolysis to proceed efficiently. The proteolysis by mesotrypsin alone is inefficient at these experimental conditions (TNF concentration is below the $K_M$). TNF incubated overnight with EK serves as the experimental control for bioactivity of TNF under our experimental conditions.

The Adzyme is More Potent than the Stoichiometric Binder

It is possible that the loss in bioactivity in the adzyme-TNF reactions arises from neutralization of TNF, rather than proteolytic cleavage of TNF. However, this is unlikely since we have previously established that a 3 log (1000-fold) excess of stoichiometric binder is required to neutralize TNF bioactivity.

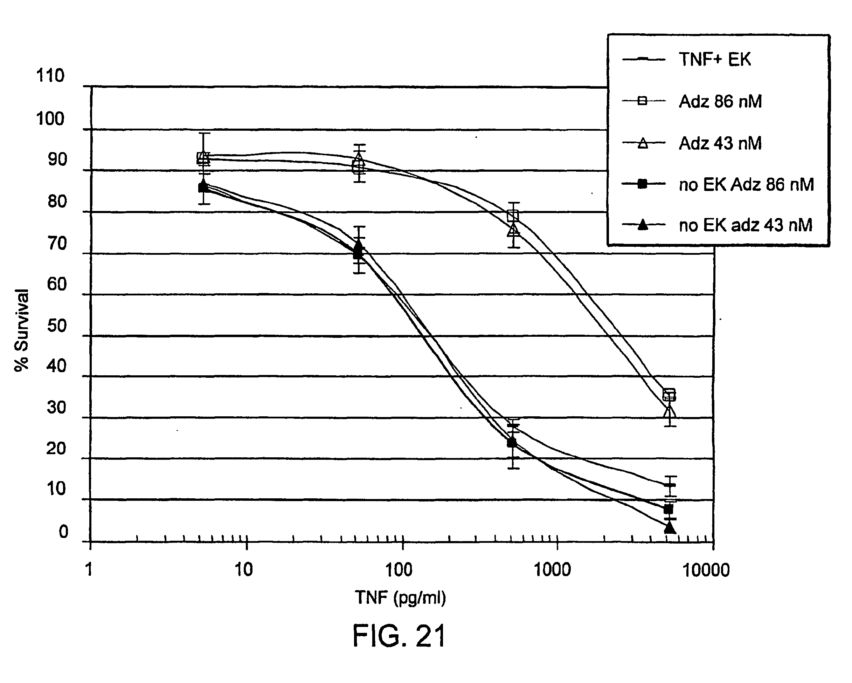

To conclusively rule out any effect of TNF neutralization, we examined the bioactivity of TNF in unactivated adzyme reactions. As described above, unactivated adzyme largely functions as a stoichiometric binder by virtue of the presence of its sp55 domain. As shown in Figure 21, in the absence of EK activation, TNF incubated with adzyme remains fully bioactive, as seen in the near complete superimposition of the two curves representing unactivated adzymes (closed symbols) with that of the negative control (TNF incubated with EK alone, "TNF+EK" in Figure 21). Meanwhile, both concentrations of activated adzymes (open symbols) are very effective at destroying TNF bioactivity. Thus the loss of TNF bioactivity in the activated adzyme reactions arises from proteolytic cleavage of TNF by the adzyme, not by pure stoichiometric binding of adzyme to TNF.

Figure 22 is a Western blot image using anti-TNF antibody, showing cleavage of TNF by different concentrations of activated adzymes after overnight incubation, but not by enzyme (mesotrypsin) to an appreciatable degree.

**Example 7. Construction of Additional Mesotrypsin-based Adzymes**

Several additional mesotrypsin-based adzymes are described below. Although specific mesotrypsin - linker - address domain combinations have to be used in this example, it should be understood that, absence of obvious concerns, all mesotrypsin versions can be freely combined with any other linkers and any other address domains of the invention. Thus the specific examples below are merely for illustartive purpose only, and are by no means limiting in any respect.

Mesotrypsin_sc7 comprises a mesotrypsin catalytic domain linked (through a linker) to a modified single-chain mouse monoclonal antibody as address domain. It was assembled by overlap PCR, such that a flexible linker of 35 amino acids (seven repeats of the Gly4Ser sequence), followed by the amino acid sequence PGSTGD (SEQ ID NO: 47) were introduced between the N-terminal mesotryspin (residues 1- 247) and the coding sequence of the single chain amplified from the mRNA of the mouse monoclonal antibody hybridoma (6402-3). In its various forms, the coding sequence of this adzyme may also contain a C-terminal tandem His6, lumio and myc tags; or myc His6 tags; or his6 tag alone. The tags are followed by a TGA stop codon and the BGH polyadenylation signal.

All constructs were sequence-confirmed, and alteration of the tags did not appear to affect adzyme function (data not shown). The mesotrypsin catalytic domain was expressed as an inactive zymogen. The propeptide can be cleaved by enterokinase, leading to the formation of active mesotrypsin. In the following sequences, "/" shows the site of enterokinase cleavage. The signal peptide is shown with underline.

The amino acid sequence for mesotrypsin_sc7 (pCT0138) as made from pDEST40 is:

MNPFLILAFVGAAVAVPFDDDDK/IVGGYTCEENSLPYQVSLNSGSHFCGGSLISEQWVVSA
AHCYKTRIQVRLGEHNIKVLEGNEQFINAAKIIRHPKYNRDTLDNDIMLIKLSSPAVINARV
STISLPTAPPAAGTECLISGWGNTLSFGADYPDELKCLDAPVLTQAECKASYPGKITNSMFC
VGFLEGGKDSCQRDSGGPVVCNGQLQGVVSWGHGCAWKNRPGVYTKVYNYVDWIKDTIAANS
GGGGSGGGGSGGGGSGGGGSGGGGSGGGGSGGGGSPGSTGDEVKLQESGPGLVKPSQSLSL
TCTVTGSSITSDYAWNWIRQFPGNKLEWMGYITYSGNTNYNPSLKSRISVTRDTSKNQFFLQ
LNSVTTEDTATYYCARSLLYYNFDFSWGQGTTVTVSSGGGGSGGGGSGGGGSDILMTQSPPS
MYASLGERVTITCKASQDISSYLIWFQQKPGKSPKTLIYRANRLVAGVPSRFSGSGSGQDYS
LTIRRLVYEDMGIYYCLQYDEFPYTFGGGTKLEIKGGPEQKLISEEDLNSAVDHHHHHH
(SEQ ID NO: 48)

The amino acid sequence for mesotrypsin_sc7 (pCT216) as made from pDEST40 is

MNPFLILAFVGAAVAVPFDDDDK/IVGGYTCEENSLPYQVSLNSGSHFCGGSLISEQWVVSA
AHCYKTRIQVRLGEHNIKVLEGNEQFINAAKIIRHPKYNRDTLDNDIMLIKLSSPAVINARV
STISLPTAPPAAGTECLISGWGNTLSFGADYPDELKCLDAPVLTQAECKASYPGKITNSMFC

VGFLEGGKDSCQRDSGGPVVCNGQLQGVVSWGHGCAWKNRPGVYTKVYNYVDWIKDTIAANS
GGGGSGGGGSGGGGSGGGGSGGGGSGGGGSGGGGSPGSTGDEVKLQESGPGLVKPSQSLSL
TCTVTGSSITSDYAWNWIRQFPGNKLEWMGYITYSGNTNYNPSLKSRISVTRDTSKNQFFLQ
LNSVTTEDTATYYCARSLLYYNFDFSWGQGTTVTVSSGGGGSGGGGSGGGGSDILMTQSPPS
MYASLGERVTITCKASQDISSYLIWFQQKPGKSPKTLIYRANRLVAGVPSRFSGSGSGQDYS
LTIRRLVYEDMGIYYCLQYDEFPYTFGGGTKLEIKGGPHHHHHHCCPGCCEQKLISEEDL
(SEQ ID NO: 49)

The amino acid sequence for meso0sc7 (pCT298) as made from pDEST40 is

<u>MNPFLILAFVGAAVA</u>VPFDDDDKIVGGYTCEENSLPYQVSLNSGSHFCGGSLISEQWVVSAA

HCYKTRIQVRLGEHNIKVLEGNEQFINAAKIIRHPKYNRDTLDNDIMLIKLSSPAVINARVS

TISLPTAPPAAGTECLISGWGNTLSFGADYPDELKCLDAPVLTQAECKASYPGKITNSMFCV

GFLEGGKDSCQRDSGGPVVCNGQLQGVVSWGHGCAWKNRPGVYTKVYNYVDWIKDTIAANSE

VKLQESGPGLVKPSQSLSLTCTVTGSSITSDYAWNWIRQFPGNKLEWMGYITYSGNTNYNPS

LKSRISVTRDTSKNQFFLQLNSVTTEDTATYYCARSLLYYNFDFSWGQGTTVTVSSGGGGSG

GGGSGGGGSDILMTQSPPSMYASLGERVTITCKASQDISSYLIWFQQKPGKSPKTLIYRANR

LVAGVPSRFSGSGSGQDYSLTIRRLVYEDMGIYYCLQYDEFPYTFGGGTKLEIKGGPHHHHH

HCCPGCCEQKLISEEDL　　(SEQ ID NO: 50)

The amino acid sequence for meso30sc7 (pCT299) as made from pDEST40 is

<u>MNPFLILAFVGAAVA</u>VPFDDDDKIVGGYTCEENSLPYQVSLNSGSHFCGGSLISEQWVVSAA

HCYKTRIQVRLGEHNIKVLEGNEQFINAAKIIRHPKYNRDTLDNDIMLIKLSSPAVINARVS

TISLPTAPPAAGTECLISGWGNTLSFGADYPDELKCLDAPVLTQAECKASYPGKITNSMFCV

GFLEGGKDSCQRDSGGPVVCNGQLQGVVSWGHGCAWKNRPGVYTKVYNYVDWIKDTIAANSG

SGSGSGSGSGSGSGSGSGSGSGSGSGSGSEVKLQESGPGLVKPSQSLSLTCTVTGSSITSDY

AWNWIRQFPGNKLEWMGYITYSGNTNYNPSLKSRISVTRDTSKNQFFLQLNSVTTEDTATYY

CARSLLYYNFDFSWGQGTTVTVSSGGGGSGGGGSGGGGSDILMTQSPPSMYASLGERVTITC

KASQDISSYLIWFQQKPGKSPKTLIYRANRLVAGVPSRFSGSGSGQDYSLTIRRLVYEDMGI

YYCLQYDEFPYTFGGGTKLEIKGGPHHHHHHCCPGCCEQKLISEEDL　　　(SEQ　ID　NO:

51)

The amino acid sequence for meso60sc7 (pCT292) as made from pDEST40 is

<u>MNPFLILAFVGAAVA</u>VPFDDDDKIVGGYTCEENSLPYQVSLNSGSHFCGGSLISEQWVVSAA

HCYKTRIQVRLGEHNIKVLEGNEQFINAAKIIRHPKYNRDTLDNDIMLIKLSSPAVINARVS

TISLPTAPPAAGTECLISGWGNTLSFGADYPDELKCLDAPVLTQAECKASYPGKITNSMFCV

```
GFLEGGKDSCQRDSGGPVVCNGQLQGVVSWGHGCAWKNRPGVYTKVYNYVDWIKDTIAANSG

SGSGSGSGSGSGSGSGSGSGSGSGSGSGSGSGSGSGSGSGSGSGSGSGSGSGSGSGSGSEVK

LQESGPGLVKPSQSLSLTCTVTGSSITSDYAWNWIRQFPGNKLEWMGYITYSGNTNYNPSLK

SRISVTRDTSKNQFFLQLNSVTTEDTAAYYCARSLLYYNFDFSWGQGTTVTVSSGGGGSGGG

GSGGGGSDILMTQSPPSMYASLGERVTITCKASQDISSYLIWFQQKPGKSPKTLIYRANRLV

AGVPSRFSGSGSGQDYSLTIRRLVYEDMGIYYCLQYDEFPYTFGGGTKLEIKGGPHHHHHHC

CPGCCEQKLISEEDL (SEQ ID NO: 52)
```

Adzyme Activation

In general, zymogens were diluted to 1-2 $\mu$M prior to activation. In a typical protocol, about 1000 pmoles of zymogen is activated with 0.02 U porcine enterokinase (EK, Sigma) for 2 hours at 37˚C, followed by active site titration to determine the amount of activated enzymatic species. These were then diluted into reactions containing TNF (100 nM) under the conditions specified.

Mesotrypsinogen and meso_Sc7 (pCT138) were activated under identical conditions. Following active site titration, the enzyme and adzyme were diluted to 10 nM in reactions containing 100 nM TNF (enzyme / substrate: ~1: 10). Similar reactions were set up with unactivated enzyme and meso_Sc7. Identical amounts of TNF (100 nM) were incubated with and without EK to serve as controls for the enzyme and adzyme reactions. An additional control was activated mesotrypsin coincubated with an equivalent amount of Sc7 and 100 nM TNF. Digestion was allowed to proceed overnight at 37˚C. Aliquots of the reactions at the start and end of the TNF digestion were analyzed with synthetic peptide substrates as described previously (data not shown).

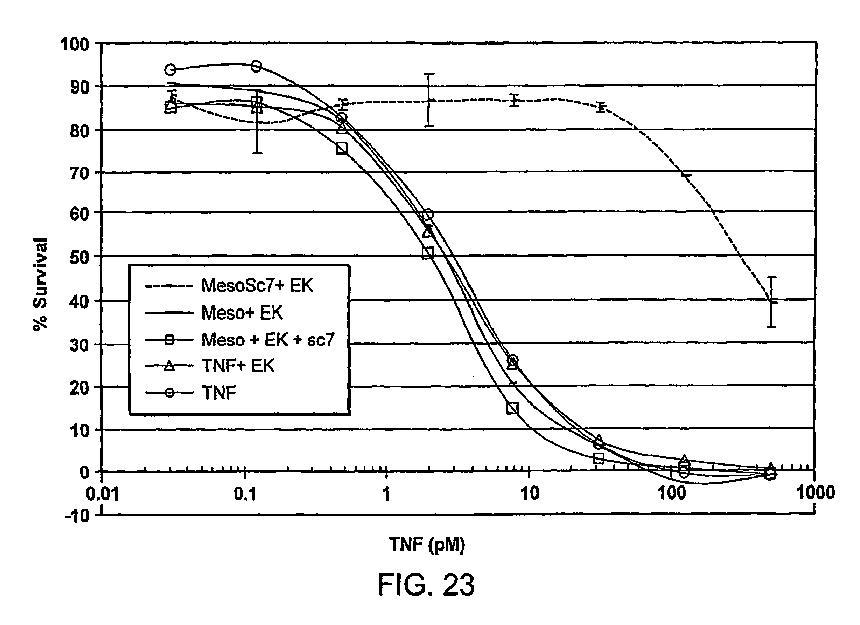

The bioactivity of TNF after the overnight digestions was assayed using the viability of L929 cells (as described previously) and the data are shown in Figure 23.

Figure 23 shows that Meso_Sc7 is a potent AdZyme, achieving TNF inactivation over 2 logs when incubated with TNF at the substoichiometric 1:10 ratio (broken line). Consistent with previous observations, mesotrypsin cannot inactivate TNF at substoichiometric ratios (solid line). The enzyme domain and the address domain need to be present as a cotranslational fusion, since mixing the two components together does not lead to inactivation of TNF (square symbols). The lack of activity of the enzyme and the equimolar mixture of the enzyme and single chain address domain is evident from the superposition of the bioassay curves with those of TNF alone (circle symbols). EK, the protease used to activate TNF, does not affect the bioactivity of TNF under our reaction conditions (triangles).

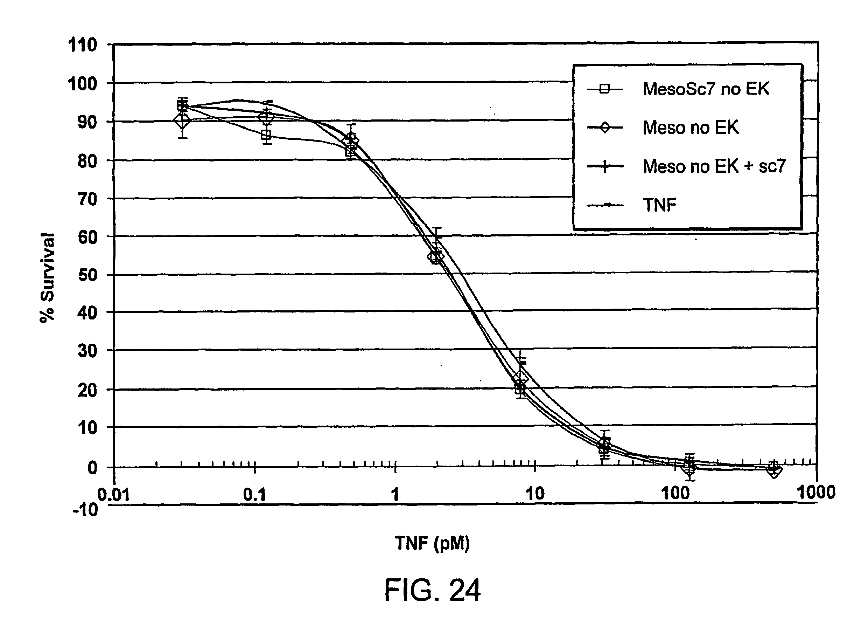

Figure 24 illustrates the requirement of EK activation for Meso_Sc7 to exibit AdZyme behavior, i.e. inactivation of TNF at the substoichiometric ratio of 1:10. The loss of bioactive TNF observed in Figure 23 cannot arise from neutralization of TNF by the address domain alone, since neither unactivated adzyme or Sc7 alone have any effect on TNF.

**Example 8. Effect of Linker Length on Activities of Mesotrypsin-based Adzymes**

To test the effect of varying the length of the linker sequences on the activity of these mesotrypsin-based adzymes, four adzyme constructs with linker lengths varying from 0 amino acid (no linker) to 60 amino acids were prepared, and their activities were measured in the TNF inactivation assay described above.

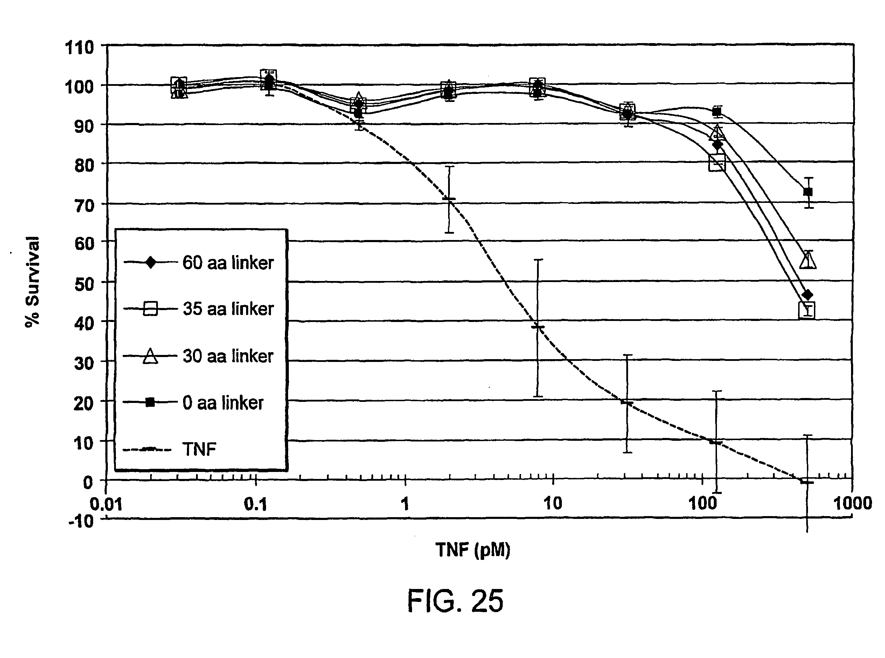

Specifically, the linker length was varied from 0 amino acids (i.e. a direct fusion of mesotrypsin with the Sc7 sequence) to 60 amino acids using the dipeptide GS as the repeat. pCT298, pCT299, pCT216 and pCT292 were activated under standard conditions and incubated with 100 nM TNF at a 1:10 ratio overnight. As shown in Fig 25, all four of the AdZymes (including meso_Sc7 or pCT216 shown here in square symbols) demonstrate a 2 log inactivation of TNF under these conditions.

This example indicates that in mesotrpsin-based adzymes, linker length does not appear to be critical for adzyme function. Adzymes with identical catalytic and address domains, but wide variation of linker lengths (from no linker to 60-residue linker) possess substantially the the same adzyme activity as measured in the TNF inactivation assay.

Thus is one embodiment, the mesotrypsin-based adzyme has a catalytic domain directly linked to an address domain with no linker.

In another embodiment, the mesotrypsin-based adzyme has a linker sequence linking the catalytic domain and the address domain, wherein the linker is at least about 10, 20, 30, 40, 50, 60, 70, 80, 90, or 100 or more amino acids.

**Example 9. Adzyme Stabilization**

Adzyme stability may be partially compromised because of autoproteolysis, especially when the catalytic domain is a protease. Therefore, eliminating one or more of the potential autoproteolytic sites, especially those sites that do not appear to be essential for the catalytic function of the catalytic domain, is expected to substantially improve the overall stability of the adzyme, while substantially conserve the desired catalytic activity. Under certain circumstances, a trade-off between catalytic activity and overall stability may be desirable. This approach not only applies to adzyme comprising such stabilized version of the catalytic domain, but also stand alone enzymes comprising the same catalytic domain.

The following example demonstrates the concept of improving adzyme / enzyme stability in general. Mesotrypsin was used as such an example.

One way to identify potential autoproteolytic sites is to analyze autoproteolytic break-down products. We performed N-terminal analysis of break-down products of mesotrypsin-based adzymes following their activation. This led to the identification of Lys193 as a major vulnerable site in the catalytic domain of mesotrypsin. Replacement of the Lys residue with a non-Lys and non-Arg residue such as Alanine is expected to remove the possibility of autoproteolysis. Ala was tested as an example. Empirical testing may be used for other sustitutions to determine if the resultant mutant was still proteolytically active.

In addition, we compared the amino acid sequences of mesotrypsin and cationic trypsin to identify non-conserved Lys or Arg residues in mesotrypsin. We identified Lys 98 and Lys 159 in mesotrypsin, which were both replaced with Gln in cationic trypsin. These two mutations in mesotrypsin were expected to be tolerated (without interfering with the catalytic function). The conserved Arg 122 may also be a potential autoproteolysis site.

To test the effect of eliminating these potentially destabilizing residues, K98Q, R122H, K159Q and K193A were introduced by PCR mutagenesis into the mesotrypsin domain of the meso_Sc7 adzyme (pCT216). Further mutagenesis was done to convert the tandem his-lumio-myc tags to a single His6 tag.

The amino acid sequence of this stabilized version of meso_Sc7 is:


MNPFLILAFVGAAVAVPFDDDDK/IVGGYTCEENSLPYQVSLNSGSHFCGGSLISEQWVVSA

AHCYKTRIQVRLGEHNIKVLEGNEQFINAAKIIRHPQYNRDTLDNDIMLIKLSSPAVINAHV

STISLPTAPPAAGTECLISGWGNTLSFGADYPDELQCLDAPVLTQAECKASYPGKITNSMFC

VGFLEGGADSCQRDSGGPVVCNGQLQGVVSWGHGCAWKNRPGVYTKVYNYVDWIKDTIAANS

GGGGSGGGGSGGGGSGGGGSGGGGSGGGGSGGGGSPGSTGDEVKLQESGPGLVKPSQSLSL

TCTVTGSSITSDYAWNWIRQFPGNKLEWMGYITYSGNTNYNPSLKSRISVTRDTSKNQFFLQ


LNSVTTEDTATYYCARSLLYYNFDFSWGQGTTVTVSSGGGGSGGGGSGGGGSDILMTQSPPS

MYASLGERVTITCKASQDISSYLIWFQQKPGKSPKTLIYRANRLVAGVPSRFSGSGSGQDYS

LTIRRLVYEDMGIYYCLQYDEFPYTFGGGTKLEIKGGPHHHHHH    (SEQ ID NO: 53)


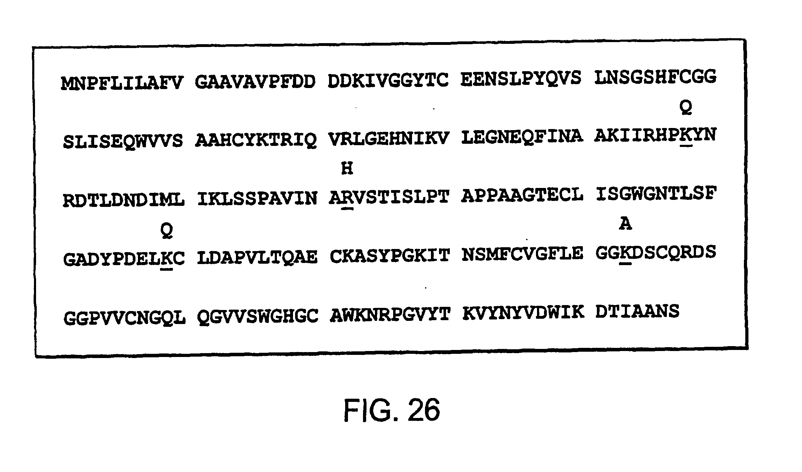

Figure 26 shows the coding sequence of mesotrypsin with the stabilizing mutations. Activity of this stabilized version of meso_Sc7 was compared with the original Adyme pCT138 by activating overnight under identical conditions. Based on the starting mass, the adzymes were diluted to 1:25 with respect to 100 nM TNF, and TNF proteolysis was allowed to proceed overnight.

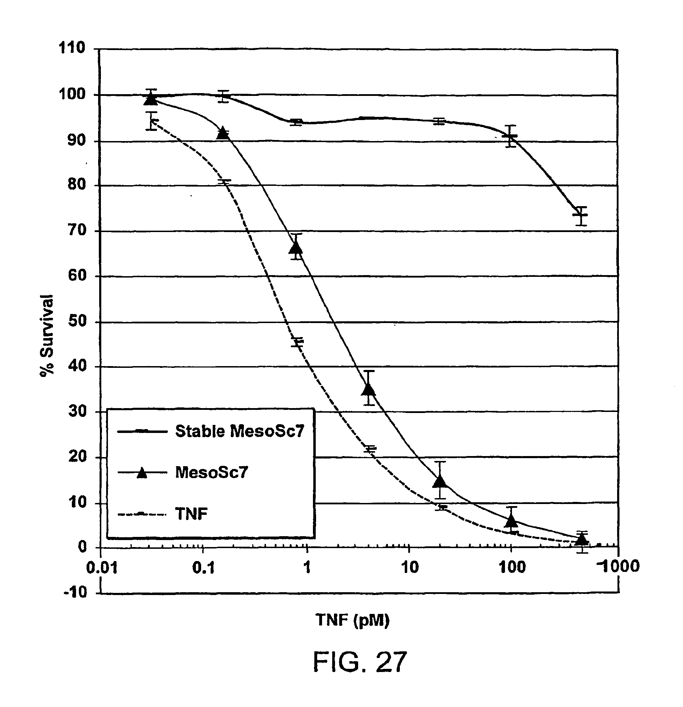

As shown in Figure 27, the stable adzyme (solid line) exhibited significantly superior properties as shown by the greater than 2 log inactivation of TNF. Under these conditions, pCT138 (filled triangles) was considerably less potent, likely because autoproteolysis had led to significant loss of activity leading to insufficient proteolysis of TNF.

The stable AdZyme was also evaluated by performing active site titration over a 10 day period. The results were shown below:

|  | 1 day | 2 days | 3 days | 6 days | 10 days |
|---|---|---|---|---|---|
| Stable meso_Sc7 | 37% | 30% | 50% | 38% | 37% |

EP 1 730 198 B1

(continued)

| | 1 day | 2 days | 3 days | 6 days | 10 days |
|---|---|---|---|---|---|
| Meso_Sc7 (pCT138) | 10% | 1.5% | 6% | 3% | 5% |

## LITERATURE CITED

1. YG Kim, J.C., S Chandrasegaran, Hybrid restriction enzymes-zinc finger fusions to Fok I cleavage domain. Proc Natl Acad Sci U S A, 1996. 93: p. 1156-1160.

2. Zhou, P., Bogacki R, McReynolds L., Howley PM, Harnessing the Ubiquitination Machinery to Target the Degradation of Specific Cellular Proteins. Molecular Cell, 2000. 6: p. 751-756.

3. Bode, C., Runge M.S., Branscomb E.E., Newell J.B., Matsueda G.R. and Haber E. Anitbody-directed Fibrinolysis. The Journal of Biological Chemistry, 1989. 264(January 15): p. 944-948.

4. Runge, M.S.B., Christoph; Matsueda, Gary R.; Haber, Edgar, Antibody-Enhanced Thrombolysis: Targeting of Tissue Plasminogen Activator in vivo. Proceedings of the National Academy of Sciences of the United States of America, 1987. 84(21): p. 7659-7662.

5. Davis BG, S., RF, Hodgspm DRW, Ullman A, Khumtaveeporn K, Estell DA, Sanford K, Bott RR, Jones JB, Selective protein degradation by ligand-targeted enzymes: towards the creation of catalytic antagonists. ChemBioChem, 2003. 4: p. 531-540.

6. Zhou, H.-X., Quantitative Account of the Enhanced Affinity of Two Linked scFvs Specific for Different Epitopes on the Same Antigen. J. Mol. Biol. 2003: p. 1-8.

7. Choy EH, P.G., Cytokine pathways and joint inflammation in rheumatoid arthritis. N Engl J Med., 2001. 344(12): p. 907-16.

8. Feldmann M, M.R., Anti-TNF alpha ther apy of rheumatoid arthritis: what have we learned? Annu Rev Immunol., 2001. 19: p. 163-96.

9. Feldman, M., Development of anti-TNF therapy for rheumatoid arthritis. Nature Publishing Group, 2002. 2.

10. Idriss, H.T.N., James H., TNF$\alpha$ and the TNF Receptor Superfamily: Structure-Function Relationship(s). Microscopy Research and Technique, 2000: p. 184-195.

11. Bodmer, J.-L., Schneider P., Tschopp J., The molecular architecture of the TNF superfamily. Trends in Biochemical Sciences, 2002. 27(1): p. 19-.

12. Pennica D, K.W., Fendly BM, Shire SJ, Raab HE, Borchardt PE, Lewis M, Goeddel DV., Characterization of a recombinant extracellular domain of the type 1 tumor necrosis factor receptor: evidence for tumor necrosis factor-alpha induced receptor aggregation. Biochemistry, 1992.31(4): p. 1134-41.

13. Nophar, Y., Brakebusch C., Englemann H., Zwang R, Aderka D., Holtman H., Wallach D. Soluble forms of tumor necrosis factor receptors (TNF-Rs). The cDNA for the type I TNF-R, cloned using amino acid sequence data of its soluble form, encodes both the cell surface and a soluble form of the receptor. EMBO J, 1990. 9(10): p. 3269-78.

14. Maini RN, Z.N., Rheumatoid arthritis, in Rheumatology, D.P. Klippel JH, Editor. 1994, Mosby: London. p. 3.1-3.14.8.

15. Warris, A., A. Bjorneklett, and P. Gaustad, Invasive pulmonary aspergillosis associated with infliximab therapy. N Engl J Med, 2001. 344(14): p. 1099-100.

16. Keane, J., Gershon S., Wise R.P., mirabilke-Levens E., Kasznica J., Schwieterman W.D., Siegel J.N., Braun M.M. Tuberculosis associated with infliximab, a tumor necrosis factor alpha-neutralizing agent. N Engl J Med, 2001. 345(15): p. 1098-104.

17. Williams, R.O., M. Feldmann, and R.N. Maini, Anti-tumor necrosis factor ameliorates joint disease in murine collagen-induced arthritis. Proc Natl Acad Sci U S A, 1992. 89(20): p. 9784-8.

18. Churchill ME, S.E., Pinilla C, Appel JR, Houghten RA, Kono DH, Balderas RS, Fieser GG, Schulze-Gahmen U, Wilson IA., Crystal structure of a peptide complex of anti-influenza peptide antibody Fab 26/9. Comparison of two different antibodies bound to the same peptide antigen. J Mol Biol., 1994. 241(4): p. 534-56.

19. Calkins CC, P.K., Potempa J, Travis J., Inactivation of tumor necrosis factor-alpha by proteinases (gingipains) from the periodontal pathogen, Porphyromonas gingivalis. Implications of immune evasion. J Biol Chem, 1998. 273 (12): p. 6611-4.

20. Nakamura K, K.M., Proteolysis of human tumor necrosis factor (TNF) by endo- and exopeptidases: process of proteolysis and formation of active fragments. Biol Pharm Bull:, 1996. 19(5): p. 672-7.

21. Narhi LO, RM., Hunt P, Arakawa T., The limited proteolysis of tumor necrosis factor-alpha. J Protein Chem, 1989. 8(5): p. 669-77.

22. Kim YJ, C.S., Kim JS, Shin NK, Jeong W, Shin HC, Oh BH, Hahn JH., Determination of the limited trypsinolysis pathways of tumor necrosis factor-alpha and its mutant by electrospray ionization mass spectrometry. Anal Biochem., 1999. 267(2): p. 279-86.

23. Magni F, C.F., Marazzini L, Colombo R, Sacchi A, Corti A, Kienle MG., Biotinylation sites of tumor necrosis factor-alpha determined by liquid chromatography-mass spectrometry. Anal Biochem., 2001. 298(2): p. 181-8.

24. van Kessel KP, v.S.J., Verhoef J., Inactivation of recombinant human tumor necrosis factor-alpha by proteolytic enzymes released from stimulated human neutrophils. J Immunol., 1991. 147(11): p. 3862-8.

25. Locksley RM, K.N., Lenardo MJ., The TNF and TNF receptor superfamilies: integrating mammalian biology. Cell, 2001. 104(4): p. 487-501.

26. Humphreys, D.T. and M.R. Wilson, Modes of L929 cell death induced by TNF-alpha and other cytotoxic agents. Cytokine, 1999. 11(10): p. 773-82.

27. Zhao, X.M., L; Song, K; Oliver, P; Chin, SY; Simon, H; Schurr, JR; Zhang, Z; Thoppil, D; Lee, S; Nelson, S; Kolls, JK, Acute Alcohol Inhibits TNF-alpha Processing in Human Monocytes by Inhibiting TNF/TNF-alpha-Converting Enzyme Interactions in the Cell Membrane. 2003: p. 2923-2931.

28. Marsters SA, F.A., Simpson NJ, Fendly BM, Ashkenazi A., Identification of cysteine-rich domains of the type 1 tumor necrosis factor receptor involved in ligand binding. J Biol Chem., 1992. 267(9): p. 5747-50.

29. Chen PC, D.G., Chen MJ., Mapping the domain(s) critical for the binding of human tumor necrosis factor-alpha to its two receptors. J Biol Chem., 1995. 270(6): p. 2874-8.

30. Rosenberg JJ, M.S., Seely JE, Kinstler O, Gaines GC, Fukuzuka K, Rose J, Kohno T, Boyle WJ, Nelson A, Kieft GL, Marshall WS, Feige U, Gasser J, St Clair J, Frazier J, Abouhamze A, Moldawer LL, Edwards CK 3rd., Development of a novel, nonimmunogenic, soluble human TNF receptor type I (sTNFR-I) construct in the baboon. J Appl Physiol., 2001. 91(5): p. 2213-23.

31. Whitcomb, D.C.,Gorry M.C., Preston R.A., Furey W., Sossenheimer M.J., Ulrich C.D., Martin S.P., Gates L.K., Amann S.T., Toskes P.P., Liddle R., McGrath K., Uomo G., Post J.C., Ehrlich G.D.., Hereditary pancreatitis is caused by a mutation in the cationic trypsinogen gene. Nat Genet, 1996. 14(2): p. 141-5.

**Equivalents**

Those skilled in the art will recognize, or be able to ascertain using no more than routine experimentation, many equivalents to the specific embodiments of the invention described herein. Such equivalents are intended to be encompassed by the following claims.

**Claims**

1. A fusion protein comprising:

   (a) a mesotrypsin catalytic domain that catalyzes the proteolysis of a target substrate; and
   (b) a targeting moiety that binds to the target substrate.

2. The fusion protein of claim 1, wherein the fusion protein is a cotranslational fusion protein encoded by a recombinant nucleic acid.

3. The fusion protein of claim 1, wherein the targeting moiety is linked directly to the mesotrypsin catalytic domain without linker.

4. The fusion protein of claim 1, wherein the targeting moiety is linked to the mesotrypsin catalytic domain by an unstructured peptide.

5. The fusion protein of claim 4, wherein the unstructured peptide comprises a plurality of Glycine and Serine amino acids, and has a length of between 15 and 50 amino acids.

6. The fusion protein of claim 4, wherein the unstructured peptide comprises one or more repeats of $Ser_4Gly$ or $SerGly_4$ or $GlySer_4$ or $Gly_4Ser$ flexible polypeptide linker.

7. The fusion protein of claim 1, wherein the mesotrypsin catalytic domain is positioned N terminal to the targeting moiety.

8. The fusion protein of claim 1, wherein the mesotrypsin catalytic domain comprises a polypeptide having an amino acid sequence that is at least 85% identical to an amino acid sequence of a human mesotrypsin polypeptide.

9. The fusion protein of claim 8, wherein the mesotrypsin catalytic domain comprises an amino acid sequence of SEQ ID NO: 45.

10. The fusion protein of claim 8, wherein the mesotrypsin catalytic domain comprises an amino acid sequence that differs from SEQ ID NO: 45 only in one or more conservative amino acid changes.

11. The fusion protein of claim 1, wherein the targeting moiety comprises an antibody or an antigen binding site thereof.

12. The fusion protein of claim 1, wherein the targeting moiety is selected from the group consisting of a monoclonal antibody, an Fab and $F(ab)_2$, an scFv, a diabody, a heavy chain variable region and a light chain variable region.

13. The fusion protein of claim 1, wherein the targeting moiety comprises an immunoglobulin-like scaffold.

14. The fusion protein of claim 13, wherein the immunoglobulin-like scaffold is an FN3 scaffold.

15. The fusion protein of claim 1, wherein the targeting moiety comprises a scaffold selected from the group consisting of: anticalins, affibodies, knottins, tendamistad, GST P1-1/A1-1, lectins, transferrin, tetranectins, and trimeric binding proteins.

16. The fusion protein of claim 1, wherein the fusion protein is resistant to autocatalyzed proteolysis.

17. The fusion protein of claim 16, wherein the fusion protein is resistant to autocatalyzed proteolysis at a concentration that is about equal to the concentration of the fusion protein in a solution to be administered to a subject.

18. The fusion protein of claim 1, wherein said substrate is a receptor ligand, and said targeting moiety includes a ligand binding domain of a cognate receptor of said ligand.

19. The fusion protein of claim 1, wherein said substrate is a polypeptide hormone, a growth factor and/or a cytokine.

20. The fusion protein of claim 1, wherein the mesotrypsin catalytic domain is derived from a stabilized mesotrypsin, said stabilized mesotrypsin comprises one or more amino acid sequence changes at potential autoproteolytic sites, wherein said changes do not diminish the catalytic activity of said mesotrypsin.

21. The fusion protein of claim 20, wherein said potential autoproteolytic sites comprise Arg or Lys.

22. The fusion protein of claim 21, wherein said potential autoproteolytic sites comprise one or more of: K98, R122, K159 and K193.

23. The fusion protein of claim 22, wherein said changes comprise one or more of: K98Q, R122H, K159Q and K193A.

24. The fusion protein of claim 20, wherein the apparent activity of the stabilized mesotrypsin is at least about 3-fold higher than wild-type mesotrypsin.

25. The fusion protein of claim 20, which retains substantially the same activity over a period of at least about 2 days, preferably about 3, 4, 5, 6, 10 or more days.

26. The fusion protein of any one of claims 1 to 7, wherein the targeting moiety is based on an FN3 scaffold.

27. A fusion protein for inhibiting the activity of a substrate polypeptide, the fusion protein being an immunoglobulin fusion complex comprising: a first fusion protein bound to a second fusion protein, wherein the first fusion protein comprises a constant portion of an immunoglobulin heavy chain and a mesotrypsin catalytic domain that catalyzes the proteolytic cleavage of at least one peptide bond of the substrate polypeptide, and wherein the second fusion protein comprises a constant portion of an immunoglobulin heavy chain and a targeting domain that binds with an address site on said substrate polypeptide, wherein said targeting domain and said protease domain are discrete and heterologous with respect to each other.

28. A polynucleotide encoding the fusion protein of any one of claims 1 to 27.

29. A vector comprising the polynucleotide of claim 28.

30. A host cell comprising the fusion protein of any one of claims 1 to 27, the polynucleotide of claim 28, or the vector of claim 29.

31. A pharmaceutical preparation comprising the fusion protein of any one of claims 1 to 27 and a pharmaceutically effective carrier.

32. The pharmaceutical preparation of claim 31, formulated such that autocatalytic proteolysis of the fusion protein is inhibited.

33. The pharmaceutical preparation of claim 32, further comprising a reversible inhibitor of said protease domain.


**Patentansprüche**

1. Fusionsprotein, umfassend:

   (a) eine katalytische Mesotrypsin-Domäne, die die Proteolyse eines Target-Substrats katalysiert; und
   (b) einen targetierenden Teil, der an das Target-Substrat bindet.

2. Fusionsprotein nach Anspruch 1, worin das Fusionsprotein ein cotranslationales Fusionsprotein ist, das durch eine rekombinante Nukleinsäure kodiert ist.

3. Fusionsprotein nach Anspruch 1, worin der targetierende Teil direkt mit der katalytischen Mesotrypsin-Domäne ohne Linker verknüpft ist.

4. Fusionsprotein nach Anspruch 1, worin der targetierende Teil mit der katalytischen Mesotrypsin-Domäne durch ein unstrukturiertes Peptid verknüpft ist.

5. Fusionsprotein nach Anspruch 4, worin das unstrukturierte Peptid eine Vielzahl von Glycin- und Serin-Aminosäuren umfasst, und eine Länge zwischen 15 und 50 Aminosäuren aufweist.

6. Fusionsprotein nach Anspruch 4, worin das unstrukturierte Peptid eine oder mehr Wiederholung(en) eines flexiblen $Ser_4Gly$- oder $SerGly_4$- oder $GlySer_4$- oder $Gly_4Ser$-Polypeptid-Linkers umfasst.

7. Fusionsprotein nach Anspruch 1, worin die katalytische Mesotrypsin-Domäne N-terminal zum targetierenden Teil positioniert ist.

8. Fusionsprotein nach Anspruch 1, worin die katalytische Mesotrypsin-Domäne ein Polypeptid mit einer Aminosäuresequenz umfasst, die mindestens zu 85 % mit einer Aminosäuresequenz von einem humanen Mesotrypsin-Polypeptid identisch ist.

9. Fusionsprotein nach Anspruch 8, worin die katalytische Mesotrypsin-Domäne eine Aminosäuresequenz von SEQ ID NO: 45 umfasst.

10. Fusionsprotein nach Anspruch 8, worin die katalytische Mesotrypsin-Domäne eine Aminosäuresequenz umfasst, die sich von SEQ ID NO: 45 nur in einer oder mehr konservativen Aminosäureänderung(en) unterscheidet.

11. Fusionsprotein nach Anspruch 1, worin der targetierende Teil einen Antikörper oder eine Antigenbindungsstelle davon umfasst.

12. Fusionsprotein nach Anspruch 1, worin der targetierende Teil aus der Gruppe bestehend aus einem monoklonalen Antikörper, einem Fab und $F(ab)_2$, einem scFv, einem Diakörper, einer variablen Schwerketten-Region und einer variablen Leichtketten-Region ausgewählt ist.

13. Fusionsprotein nach Anspruch 1, worin der targetierende Teil ein immunglobulinähnliches Gerüst umfasst.

14. Fusionsprotein nach Anspruch 13, worin das immunoglobulinähnliche Gerüst ein FN3-Gerüst ist.

15. Fusionsprotein nach Anspruch 1, worin der targetierende Teil ein Gerüst umfasst, das aus der Gruppe bestehend aus Antikalinen, Affibodies, Knottinen, Tendamistad, GST P1-1/A1-1, Lektinen, Transferrin, Tetranektinen, und trimeren Bindungsproteinen ausgewählt ist.

16. Fusionsprotein nach Anspruch 1, worin das Fusionsprotein gegen eine autokatalysierte Proteolyse resistent ist.

17. Fusionsprotein nach Anspruch 16, worin das Fusionsprotein gegen eine autokatalysierte Proteolyse in einer Konzentration resistent ist, die in einer an einen Patienten zu verabreichenden Lösung ungefähr gleich der Konzentration des Fusionsproteins ist.

18. Fusionsprotein nach Anspruch 1, worin das Substrat ein Rezeptorligand ist und der targetierende Teil eine Ligandenbindungsdomäne von einem kognaten Rezeptor des Liganden einschließt.

19. Fusionsprotein nach Anspruch 1, worin das Substrat ein Polypeptidhormon, ein Wachstumsfaktor und/oder ein Zytokin ist.

20. Fusionsprotein nach Anspruch 1, worin sich die katalytische Mesotrypsin-Domäne von einem stabilisierten Mesotrypsin ableitet, wobei das stabilisierte Mesotrypsin eine oder mehr Aminosäuresequenzänderungen an potenziell autoproteolytischen Stellen umfasst, worin die Änderungen die katalytische Aktivität des Mesotrypsins nicht verringern.

21. Fusionsprotein nach Anspruch 20, worin die potenziell autoproteolytischen Stellen Arg oder Lys umfassen.

**22.** Fusionsprotein nach Anspruch 21, worin die potenziell autoproteolytischen Stellen eines oder mehr von K98, R122, K159 und K193 umfassen.

**23.** Fusionsprotein nach Anspruch 22, worin die Änderungen eines oder mehr von K98Q, R122H, K159Q und K193A umfassen.

**24.** Fusionsprotein nach Anspruch 20, worin die apparente Aktivität des stabilisierten Mesotrypsins mindestens um das ca. 3fache höher ist als die des Wildtyp-Mesotrypsins.

**25.** Fusionsprotein nach Anspruch 20, welches über eine Zeitdauer von mindestens ca. 2 Tagen, bevorzugt ca. 3, 4, 5, 6, 10 oder mehr Tagen, im Wesentlichen die gleiche Aktivität beibehält.

**26.** Fusionsprotein nach einem der Ansprüche 1 bis 7, worin der targetierende Teil auf einem FN3-Gerüst basiert.

**27.** Fusionsprotein zur Inhibition der Aktivität eines Substrat-Polypeptids, wobei das Fusionsprotein ein Immunglobulin-Fusionskomplex ist, umfassend: ein erstes Fusionsprotein, das an ein zweites Fusionsprotein gebunden ist, worin das erste Fusionsprotein einen konstanten Anteil einer Immunglobulin-Schwerkette und eine die proteolytische Spaltung von mindestens einer Peptidbindung des Substratpolypeptids katalysierende katalytische Mesotrypsin-Domäne umfasst und worin das zweite Fusionsprotein einen konstanten Anteil einer Immunglobulin-Schwerkette und eine mit einer "Adressen"-Stelle an das Substratpolypeptid bindende targetierende Domäne umfasst, worin die targetierende Domäne und die Proteasedomäne bezogen aufeinander diskret und heterolog sind.

**28.** Polynukleotid, das für das Fusionsprotein nach einem der Ansprüche 1 bis 27 kodiert.

**29.** Vektor, der das Polynukleotid nach Anspruch 28 umfasst.

**30.** Wirtszelle, die das Fusionsprotein nach einem der Ansprüche 1 bis 27, das Polynukleotid nach Anspruch 28 oder den Vektor nach Anspruch 29 umfasst.

**31.** Pharmazeutische Zubereitung, die das Fusionsprotein nach einem der Ansprüche 1 bis 27 und einen pharmazeutisch wirksamen Träger umfasst.

**32.** Pharmazeutische Zubereitung nach Anspruch 31, die dergestalt formuliert ist, dass die autokatalytische Proteolyse des Fusionsproteins inhibiert wird.

**33.** Pharmazeutische Zubereitung nach Anspruch 32, die ferner einen reversiblen Inhibitor der Proteasedomäne umfasst.


**Revendications**

**1.** Protéine de fusion comprenant:

(a) un domaine catalytique de méso trypsine qui catalyse la protéolyse d'un substrat cible; et
(b) un fragment de ciblage qui se lie au substrat cible.

**2.** La protéine de fusion de la revendication 1, dans laquelle la protéine de fusion est une protéine de fusion de co-traduction codée par un acide nucléique recombinant.

**3.** La protéine de fusion de la revendication 1, dans laquelle le fragment de ciblage est lié directement au domaine catalytique de mésotrypsine sans lieur.

**4.** La protéine de fusion de la revendication 1, dans laquelle le fragment de ciblage est lié au domaine catalytique de mésotrypsine par un peptide non structuré.

**5.** La protéine de fusion de la revendication 4, dans laquelle le peptide non structuré comprend une pluralité d'acides aminés glycine et sérine et fait entre 15 et 50 acides aminés de long.

**6.** La protéine de fusion de la revendication 4, dans laquelle le peptide non structuré comprend une ou plusieurs répétitions de lieur polypeptique flexible $Ser_4Gly$ ou $SerGly_4$ ou $GlySer_4$ ou $Gly_4Ser$.

**7.** La protéine de fusion de la revendication 1, dans laquelle le domaine catalytique de mésotrypsine est en position N-terminale du fragment de ciblage.

**8.** La protéine de fusion de la revendication 1, dans laquelle le domaine catalytique de mésotrypsine comprend un polypeptide ayant une séquence d'acides aminés qui est au moins identique à 85% à une séquence d'acides aminés d'un polypeptide mésotrypsine humain.

**9.** La protéine de fusion de la revendication 8, dans laquelle le domaine catalytique de mésotrypsine comprend une séquence d'acides aminés de la SEQ ID NO:45.

**10.** La protéine de fusion de la revendication 8, dans laquelle le domaine catalytique de mésotrypsine comprend une séquence d'acides aminés qui diffère de la SEQ ID NO:45 de seulement un ou plusieurs changements conservateurs d'acides aminés.

**11.** La protéine de fusion de la revendication 1, dans laquelle le fragment de ciblage comprend un anticorps ou un site de liaison à un antigène de celui-ci.

**12.** La protéine de fusion de la revendication 1, dans laquelle le fragment de ciblage est sélectionné parmi le groupe consistant en un anticorps monoclonal, un Fab et $F(ab)_2$, un scFv, un diacorps, une région variable de chaîne lourde et une région variable de chaîne légère.

**13.** La protéine de fusion de la revendication 1, dans laquelle le fragment de ciblage comprend un échafaudage de type immunoglobuline.

**14.** La protéine de fusion de la revendication 13, dans laquelle l'échafaudage de type immunoglobuline est un échafaudage FN3.

**15.** La protéine de fusion de la revendication 1, dans laquelle le fragment de ciblage comprend un échafaudage sélectionné parmi le groupe consistant en: anticalines, ligands d'affinité Affibody, knottines, tendamistad, GST P1-1/A1-1, lectines, transferrine, tétranectines et protéines de liaison trimères.

**16.** La protéine de fusion de la revendication 1, dans laquelle la protéine de fusion est résistante à la protéolyse autocatalysée.

**17.** La protéine de fusion de la revendication 16, dans laquelle la protéine de fusion est résistante à la protéolyse autocatalysée à une concentration qui est à peu près égale à la concentration de la protéine de fusion dans une solution à administrer à un sujet.

**18.** La protéine de fusion de la revendication 1, dans laquelle ledit substrat est un récepteur/ligand et ledit fragment de ciblage comprend un domaine de liaison de ligand d'un récepteur correspondant dudit ligand.

**19.** La protéine de fusion de la revendication 1, dans laquelle ledit substrat est une hormone polypeptidique, un facteur de croissance et/ou une cytokine.

**20.** La protéine de fusion de la revendication 1, dans laquelle ledit domaine catalytique de mésotrypsine provient d'une mésotrypsine stabilisée, ladite mésotrypsine stabilisée comprend un ou plusieurs changements de séquences d'acides aminés à des sites autoprotéolytiques potentiels, où lesdits changements ne diminuent pas l'activité catalytique de ladite mésotrypsine.

**21.** La protéine de fusion de la revendication 20, dans laquelle lesdites sites autoprotéolytiques potentiels comprennent Arg ou Lys.

**22.** La protéine de fusion de la revendication 21, dans laquelle lesdites sites autoprotéolytiques potentiels comprennent un ou plusieurs d'entre: K98, R122, K159 et K193.

**23.** La protéine de fusion de la revendication 22, dans laquelle lesdits changements comprennent un ou plusieurs d'entre: K98Q, R122H, K159Q et K193A.

**24.** La protéine de fusion de la revendication 20, dans laquelle l'activité apparente de la mésotrypsine stabilisée est au moins trois fois plus élevée que celle de la mésotrypsine de type sauvage.

**25.** La protéine de fusion de la revendication 20, laquelle conserve sensiblement la même activité pendant une période d'environ 2 jours au moins, de préférence d'environ 3, 4, 5, 6, 10 jours ou plus.

**26.** La protéine de fusion de l'une quelconque des revendications 1 à 7, dans laquelle le fragment de ciblage est basé sur un échafaudage FN3.

**27.** Protéine de fusion destinée à inhiber l'activité d'un polypeptide substrat, la protéine de fusion étant un complexe de fusion d'immunoglobuline comprenant: une première protéine de fusion liée à une deuxième protéine de fusion, où la première protéine de fusion comprend une partie constante d'une chaîne lourde d'immunoglobuline et un domaine catalytique de mésotrypsine qui catalyse le clivage protéolytique d'au moins une liaison peptidique du polypeptide substrat et où la deuxième protéine de fusion comprend une partie constante d'une chaîne lourde d'immunoglobuline et un domaine de ciblage qui se lie à un site d'adresse sur ledit polypeptide substrat, où ledit domaine de ciblage et ledit domaine protéase sont discrets et hétérologues l'un par rapport à l'autre.

**28.** Polynucléotide codant la protéine de fusion de l'une quelconque des revendications 1 à 27.

**29.** Vecteur comprenant le polynucléotide de la revendication 28.

**30.** Cellule hôte comprenant la protéine de fusion de l'une quelconque des revendications 1 à 27, le polynucléotide de la revendication 28 ou le vecteur de la revendication 29.

**31.** Préparation pharmaceutique comprenant la protéine de fusion de l'une quelconque des revendications 1 à 27 et un excipient pharmaceutiquement efficace.

**32.** La préparation pharmaceutique de la revendication 31, formulée de telle manière que la protéolyse autocatalytique de la protéine de fusion est inhibée.

**33.** La préparation pharmaceutique de la revendication 32, comprenant en outre un inhibiteur réversible dudit domaine protéase.

Fig. 1

Fig. 2A

Fig. 2B

Fig. 2C

Fig. 2D

Fig. 2E

Fig. 2F

Fig. 2G

Fig. 2H

Fig. 2I

Fig. 2J

Fig. 3A

Fig. 3B

Fig. 3C

Fig. 3D

Fig. 3E

Fig. 3F

Fig. 3G

Fig. 4

PANEL A

PANEL B

Fig. 5

Fig. 6

Fig. 7

Fig. 8

Fig. 9

Fig. 10

Fig. 11

BENZAMIDINE 1mM

## Fig. 12

EK-DEPENDENT ADZYME PROTEOLYTIC ACTIVITY

## Fig. 13

NORMALIZATION OF TRYPSIN ACTIVITIES

Fig. 14

DETECTION OF TNFα BINDING OF ADZYMES BY ELISA

Fig. 15

Fig. 16

There is a tradeoff between potency and selectivity as the strength of
the enzyme domain is changed
(at constant $k_{off}^{AS}$)

This tradeoff implies selecting $k_{cat}^{ES}/K_M^{ES} = k_{off}^{AS}/[S]_{eff}$ for a "balanced" design.

FIG. 17

EP 1 730 198 B1

FIG. 18

EP 1 730 198 B1

FIG. 19

EP 1 730 198 B1

FIG. 20

EP 1 730 198 B1

FIG. 21

FIG. 22

FIG. 23

FIG. 24

EP 1 730 198 B1

FIG. 25

EP 1 730 198 B1

```
MNPFLILAFV GAAVAVPFDD DDKIVGGYTC EENSLPYQVS LNSGSHFCGG
                                                     Q
SLISEQWVVS AAHCYKTRIQ VRLGEHNIKV LEGNEQFINA AKIIRHPKYN
                          H
RDTLDNDIML IKLSSPAVIN ARVSTISLPT APPAAGTECL ISGWGNTLSF
    Q                                            A
GADYPDELKC LDAPVLTQAE CKASYPGKIT NSMFCVGFLE GGKDSCQRDS

GGPVVCNGQL QGVVSWGHGC AWKNRPGVYT KVYNYVDWIK DTIAANS
```

FIG. 26

FIG. 27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02097042 A
- US 5888762 A
- US 6015787 A
- US 5846743 A
- US 5747641 A
- US 5804604 A
- WO 9852614 A
- WO 0029427 A
- WO 9929721 A
- US 6673901 B
- US 6462189 B
- WO 0232925 A
- US 5831012 A
- US 6534628 B
- US 6740734 B
- WO 04039841 A2
- US 6258558 B
- US 5837500 A
- US 5843701 A
- US 5843653 A
- EP 45665 A
- US 8702968 W
- US 8903587 W
- US 9007335 W
- US 5091513 A
- US 5258498 A
- WO 9310151 A
- US 4683195 A, Mullis
- US 4683202 A, Mullis
- WO 9410308 A1
- US 5716805 A
- WO 9910510 A2
- US 5573925 A
- WO 9418317 A
- WO 9620951 A
- WO 9606097 A
- WO 9731898 A
- WO 9641865 A
- US 5643575 A
- EP 0401384 A
- WO 9734631 A1
- US 6156511 A
- US 5733731 A
- US 5580717 A
- US 5498530 A
- US 5922545 A
- US 5830721 A
- US 5811238 A
- US 5605793 A
- US 5571698 A
- US 5223409 A
- US 5198346 A
- US 5096815 A
- US 5403484 A
- US 6180336 A
- US 5994519 A
- US 6172197 A
- US 6140471 A
- US 5969108 A
- US 5872215 A
- US 5871907 A
- US 5858657 A
- US 5837242 A
- US 5733743 A
- US 5962255 A
- US 5565332 A
- US 5514548 A
- US 6207446 B
- US 6214553 B
- US 6261804 B
- US 6281344 B
- US 6518018 B
- US 5580736 A
- US 5955280 A
- US 6383775 B
- US 414688 P
- US 6171820 B
- US 5756353 A
- US 5858784 A
- WO 9831346 A
- WO 9810796 A
- WO 0027359 A
- WO 0154664 A
- WO 02060412 A
- US 6294153 B
- US 6344194 B
- US 6071497 A
- WO 02066078 A
- WO 02053190 A
- WO 0160420 A
- WO 0066206 A
- US 4868116 A
- US 4980286 A
- WO 8907136 A
- WO 8902468 A
- WO 8905345 A
- WO 9207573 A
- WO 9325234 A

- WO 9406920 A
- WO 9411524 A
- WO 9106309 A
- JP 1047381 A
- EP 43075 A
- WO 9304701 A

- WO 9222635 A
- WO 9220316 A
- WO 9219749 A
- WO 9206180 A
- US 5166320 A
- US 5328470 A

**Non-patent literature cited in the description**

- **Walajtys-Rode ; Elizbieta.** *Kosmos,* 1995, vol. 44, 451-464
- *KOSMOS,* 1995, vol. 124, 199735a
- **Cornelio N.M. Nyaruhucha ; Makoto Kito ; Shin-Ichi Fukuoka.** *The Journal of Biological Chemistry,* 1997, vol. 272, 10573-10578
- **Yamamoto R. et al.** *Protein Engineering,* 1989, vol. 2 (7), 553-8
- **Rawlings, N.D. ; Barrett, A.J.** *Biochem. J.,* 1993, vol. 290, 205-218
- **Schechter ; Berger.** *Biochem. Biophys. Res. Commun.,* 1967, vol. 27, 157-162
- **Zhou.** *J. Mol. Biol,* 2003, vol. 329, 1-8
- **Legendre D. et al.** *Nature Biotechnology,* 1999, vol. 17, 67-72
- **Legendre D. et al.** *Protein Science,* 2002, vol. 11, 1506-1518
- **Soumillion P. ; Fastrez J.** *Current Opinion in Biotechnology,* 2001, vol. 12, 387-394
- **Derossi et al.** *J. Biol. Chem,* 1994, vol. 269, 10444-10450
- **Lindgren et al.** *Trends Pharmacol. Sci.,* 2000, vol. 21, 99-103
- **Ho et al.** *Cancer Research,* 2001, vol. 61, 474-477
- **Tuerk.** *Science,* 1990, vol. 249, 505-510
- **Klug.** *Mol Biol Reports,* 1994, vol. 20, 97-107
- **Morris et al.** *PNAS,* 1998, vol. 95, 2902-2907
- **Derossi et al.** *J Biol Chem,* 1994, vol. 269, 10444-10450
- **Perez et al.** *J Cell Sci,* 1992, vol. 102, 717-722
- **Derossi et al.** *J Biol Chem,* 1996, vol. 271, 18188-18193
- **Kuppuswamy et al.** *Nucl. Acids Res.,* 1989, vol. 17, 3551-3561
- **Frankel ; Pabo.** *Cell,* 1989, vol. 55, 1189-1193
- **Green ; Loewenstein.** *Cell,* 1989, vol. 55, 1179-1188
- **Ruben et al.** *J. Virol.,* 1989, vol. 63, 1-8
- **T. Higashijima et al.** *J. Biol. Chem.,* 1990, vol. 265, 14176
- **Subbarao et al.** *Biochemistry,* 1987, vol. 26, 2964
- **Raport, C.J. et al.** *J. Biol. Chem.,* 1996, vol. 271, 17161
- **Choe, H. et al.** *Cell,* 1996, vol. 85, 1135

- **Cocchi, F. et al.** *Science,* 1995, vol. 270, 1811
- **Yokota, T. et al.** *Proc. Natl. Acad. Sci. USA,* 1986, vol. 83, 5894
- **Kitamura et al.** *J. Cell Physiol.,* 1989, vol. 140, 323
- **McKenzie et al.** *PNAS USA,* 1993, vol. 90, 3735-3739
- **Guo et al.** *Di Yi Jun Yi Da Xue Xue Bao,* 2002, vol. 22 (7), 597
- **Kombluth et al.** *J. Immunol.,* 1986, vol. 137, 2585-2591
- **Dinarello.** *Int. Rev. Immunol.,* 1998, vol. 16, 457-499
- **Smith ; Waterman.** *Adv. Appl. Math.,* 1991, vol. 2, 482
- **Needleman ; Wunsch.** *J. Mol. Biol.,* 1970, vol. 48, 443
- **Pearson ; Lipman.** *Proc. Natl. Acad. Sci. (U.S.A.),* 1988, vol. 85, 2444
- **Fauchere, J.** *Adv. Drug Res.,* 1986, vol. 15, 29
- **Veber ; Freidinger.** *TINS,* 1985, 392
- **Evans et al.** *J. Med. Chem,* 1987, vol. 30, 1229
- **Spatola, A.F.** Chemistry and Biochemistry of Amino Acids, Peptides, and Proteins. Marcel Dekker, 1983, 267
- **Szatola, A. F.** *Peptide Backbone Modifications,* March 1983, vol. 1 (3
- **Morley, J. S.** *Trends Pharm Sci,* 1980, 463-468
- **Hudson, D. et al.** *Int J Pent Prot Res,* 1979, vol. 14, 177-185
- **Spatola, A. F. et al.** *Life Sci,* 1986, vol. 38, 1243-1249
- **Hann, M. M.** *J Chem Soc Perkin Trans,* 1982, vol. I, 307-314
- **Almquist, R. G. et al.** *J Med Chem,* 1980, vol. 23, 1392-1398
- **Jennings-White, C. et al.** *Tetrahedron Lett,* 1982, vol. 23, 2533
- **Holladay, M. W. et al.** *Tetrahedron Lett,* 1983, vol. 24, 4401-4404
- **Hruby, V. J.** *Life Sci,* 1982, vol. 31, 189-199
- **Rizo ; Gierasch.** *Ann. Rev. Biochem.,* 1992, vol. 61, 387

- **Maniatis et al.** Molecular Cloning: A Laboratory Manual. Cold Spring Harbor, 1989
- Guide to Molecular Cloning Techniques. **Berger ; Kimmel.** Methods in Enzymology. Academic Press, Inc, 1987, vol. 152
- **Merrifield, J.** *J. Am. Chem. Soc.,* 1969, vol. 91, 501
- **Chaiken I. M.** *CRC Crit. Rev. Biochem.,* 1981, vol. 11, 255
- **Kaiser et al.** *Science,* 1989, vol. 243, 187
- **Merrifield, B.** *Science,* 1986, vol. 232, 342
- **Kent, S. B. H.** *Ann. Rev. Biochem.,* 1988, vol. 57, 957
- **Offord, R. E.** Semisynthetic Proteins. Wiley Publishing, 1980
- **Goodman, M. ; Chorev, M.** *Accounts of Chem. Res.,* 1979, vol. 12, 423
- **Sambrook et al.** Molecular Cloning: A Laboratory Manual. Cold Spring Harbor Laboratory Press, 1989
- **Alexandrov N.N. et al.** *Bioinformatics,* 1998, vol. 14, 206-11
- **Labesse G et al.** *Proteins,* 1997, vol. 1, 38-42
- **Xu, Y. et al.** *Protein Eng.,* 1999, vol. 12, 899-907
- **Russel A. J. et al.** *Proteins,* 2002, vol. 47, 496-505
- **Reva, B. et al.** *Proteins,* 2002, vol. 47, 180-93
- **Traunecker et al.** *Nature,* 1989, vol. 339, 68
- **Namen et al.** *Nature,* 1988, vol. 333, 571
- **Huston et al.** *PNAS,* 1988, vol. 85, 4879
- **Zhou.** *J. Mol. Biol.,* 2003, vol. 329, 1-8
- **Zhou.** *Biochemistry,* 2001, vol. 40, 15069-15073
- Current Protocols in Molecular Biology. John Wiley & Sons, 1992
- **Means et al.** *Bioconjugate Chemistry,* 1990, vol. 1, 2-12
- **Partis et al.** *J. Pro. Chem.,* 1983, vol. 2, 263
- **Ellman et al.** *Arch. Biochem. Biophys,* 1958, vol. 74, 443
- **Riddles et al.** *Anal. Biochem,* 1979, vol. 94, 75
- **Blattler et al.** *Biochem,* 1985, vol. 24, 1517
- **Fanslow et al.** *J. Immunol.,* 1992, vol. 149, 65
- **Noelle et al.** *Proc. Nad. Acad. Sci. U.S.A.,* 1992, vol. 89, 6550
- **Ashkenazi et al.** *PNAS USA,* 1991, vol. 88, 10535
- **Byrn et al.** *Nature,* 1990, vol. 344, 677
- **Hieter et al.** *Cell,* 1980, vol. 22, 197-207
- **Landschulz et al.** *Science,* 1988, vol. 240, 1759
- **Landschulz et al.** *Science,* 1989, vol. 243, 1681
- **O'Shea et al.** *Science,* 1989, vol. 245, 646
- **Turner ; Tjian.** *Science,* 1989, vol. 243, 1689
- **Buckland ; Wild.** *Nature,* 1989, vol. 338, 547
- **Britton.** *Nature,* 1991, vol. 353, 394
- **Delwart ; Mosialos.** *AIDS Research and Human Retroviruses,* 1990, vol. 6, 703
- **Spruce et al.** *PNAS,* 1991, vol. 88, 3523
- **Rabindran et al.** *Science,* 1993, vol. 259, 230
- **Chakerian et al.** *J. Biol. Chem.,* 1991, vol. 266, 1371
- **Alberti et al.** *EMBO J.,* 1993, vol. 12, 3227
- **Lewis et al.** *Nature,* 1996, vol. 271, 1247
- **Wang et al.** *Mol. Cell. Biol.,* 1994, vol. 14, 5182
- **Clore et al.** *Science,* 1994, vol. 265, 386
- **Molineux.** *Pharmacotherapy,* 2003, vol. 8, 3S-8S
- **Powell et al.** *Pharma. Res.,* 1993, vol. 10, 1268-1273
- **Morpurgo et al.** *Appl. Biochem. Biotechnol.,* 1996, vol. 56, 59-72
- **Vorobjev et al.** *Nucleosides Nucleotides,* 1999, vol. 18, 2745-2750
- **Caliceti et al.** *Bioconjug. Chem.,* 1999, vol. 10, 638-646
- **Malik et al.** *Exp. Hematol.,* 1992, vol. 20, 1028-1035
- **Nixon et al.** Proc. Natl. Acad. Sci. USA. *Biochemistry,* 1997, vol. 94, 1069
- **Lakowitz, J. R.** Principles of Fluorescence Spectroscopy. Plenum Press, 1983
- **Sjolander, S. ; Urbaniczky, C.** *Anal. Chem.,* 1991, vol. 63, 2338-2345
- **Szabo et al.** *Curr. Opin. Struct. Biol.,* 1995, vol. 5, 699-705
- **Hoogenboom et al.** *Immunol Today,* August 2000, vol. 21 (8), 371-8
- **Daugherty et al.** *Protein Eng,* 1999, vol. 12 (7), 613-21
- **Makeyev et al.** *FBBS Lett,* 12 February 1999, vol. 444 (2-3), 177-80
- **Kayman et al.** *J Virol,* March 1999, vol. 73 (3), 1802-8
- **Earhart.** *Methods Enzymol,* 2000, vol. 326, 506-16
- **Shusta et al.** *Curr Opin Biotechnol,* April 1999, vol. 10 (2), 117-22
- **Schaffitzel et al.** *J Immunol Methods,* 10 December 1999, vol. 231 (1-2), 119-35
- **Bara et al.** *Proc Natl Acad Sci U S A,* 16 September 1997, vol. 94 (19), 10063-8
- **Katz.** *Biomol Eng,* 31 December 1999, vol. 16 (1-4), 57-65
- **Han et al.** *J Biol Chem,* 19 May 2000, vol. 275 (20), 14979-84
- **Whaley et al.** *Nature,* 08 June 2000, vol. 405 (6787), 665-8
- **Fuh et al.** *J Biol Chem,* 14 July 2000, vol. 275 (28), 21486-91
- **Joung et al.** *Proc Natl Acad Sci U S A,* 20 June 2000, vol. 97 (13), 7382-7

- **Giannattasio et al.** *Antimicrob Agents Chemother,* July 2000, vol. 44 (7), 1961-3
- Harrison's Principles of Internal Medicine. Physicians Desk Reference. McGraw-Hill, 1997
- **Caughey et al.** *J. Virol.,* April 1994, vol. 68 (4), 2135-2141
- **Belcher et al.** *Nature,* 1996, vol. 381, 56-58
- **Falini et al.** *Science,* 1996, vol. 271, 67-69
- **Cha.** *Proc. Natl Acad. Sci. USA,* 1999, vol. 96, 361-365
- **Meldrum et al.** *Proc. R. Soc. Lond. B,* 1993, vol. 251, 238-242
- **Colvin et al.** *J. Am. Chem. Soc.,* 1992, vol. 144, 5221-5230
- **Brust et al.** *Adv. Mater.,* 1995, vol. 7, 795-797
- **Li et al.** *Chem. Mater.,* 1999, vol. 11, 23-26
- **Alivisatos et al.** *Nature,* 1996, vol. 382, 609-611
- **Mirkin et al.** *Nature,* 1996, vol. 382, 607-609
- **Brown.** *Proc. Natl Acad. Sci. USA,* 1992, vol. 89, 8651-8655
- *Nature Biotechnol.,* 1997, vol. 15, 269-272
- **Whaley et al.** *Nature,* 2000, vol. 405, 665-668
- **Seeman ; Belcher.** *Proc Natl Acad Sci U S A.,* 05 March 2002, vol. 99 (2), 6451-5
- **Strejan et al.** *J. Neuroimmunol,* 1984, vol. 7, 27
- LungMucociliary Clearance. **Pavia, D.** Aerosols and the Lung: Clinical and Experimental Aspects. 1984
- **Warheit et al.** *Microscopy Res. Tech.,* 1993, vol. 26, 412-422
- Physiology and Pathophysiology of Pulmonary Macrophages. **Brain, J. D.** The Reticuloendothelial System. Plenum, 1985, 315-327
- **Goeddel.** Gene Expression Technology: Methods in Enzymology. Academic Press, 1990
- **Broach et al.** Experimental Manipulation of Gene Expression. Academic Press, 1983, 83
- **Sambrook ; Fritsch ; Maniatis.** Molecular Cloning: A Laboratory Manual. Cold Spring Harbor Laboratory Press, 1989
- **Miller, A.D.** *Blood,* 1990, vol. 76, 271
- Current Protocols in Molecular Biology. Greene Publishing Associates, 1989, 9.10-9.14
- **Eglitis et al.** *Science,* 1985, vol. 230, 1395-1398
- **Danos ; Mulligan.** *PNAS USA,* 1988, vol. 85, 6460-6464
- **Wilson et al.** *PNAS USA,* 1988, vol. 85, 3014-3018
- **Armentano et al.** *PNAS USA,* 1990, vol. 87, 6141-6145
- **Huber et al.** *PNAS USA,* 1991, vol. 88, 8039-8043
- **Ferry et al.** *PNAS USA,* 1991, vol. 88, 8377-8381
- **Chowdhury et al.** *Science,* 1991, vol. 254, 1802-1805
- **van Beusechem et al.** *PNAS USA,* 1992, vol. 89, 7640-7644
- **Kay et al.** *Human Gene Therapy,* 1992, vol. 3, 641-647
- **Dai et al.** *PNAS USA,* 1992, vol. 89, 10892-10895
- **Hwu et al.** *J. Immunol.,* 1993, vol. 150, 4104-4115
- **Roux et al.** *PNAS USA,* 1989, vol. 86, 9079-9083
- **Julan et al.** *J. Gen Virol,* 1992, vol. 73, 3251-3255
- **Goud et al.** *Virology,* 1983, vol. 163, 251-254
- **Neda et al.** *J. Biol. Chem.,* 1991, vol. 266, 14143-14146
- **Berkner et al.** *BioTechniques,* 1988, vol. 6, 616
- **Rosenfeld et al.** *Science,* 1991, vol. 252, 431-434
- **Rosenfeld et al.** *Cell,* 1992, vol. 68, 143-155
- **Lemarchand et al.** *PNAS USA,* 1992, vol. 89, 6482-6486
- **Herz ; Gerard.** *PNAS USA,* 1993, vol. 90, 2812-2816
- **Quantin et al.** *PNAS USA,* 1992, vol. 89, 2581-2584
- **Haj-Ahmand ; Graham.** *J. Virol.,* 1986, vol. 57, 267
- **Jones et al.** *Cell,* 1979, vol. 16, 683
- **Graham et al.** Methods in Molecular Biology. 1991, vol. 7, 109-127
- **Muzyczka et al.** *Curr. Topics in Micro. and Immunol.,* 1992, vol. 158, 97-129
- **Flotte et al.** *Am. J. Respir. Cell. Mol. Biol.,* 1992, vol. 7, 349-356
- **Samulski et al.** *J. Virol.,* 1989, vol. 63, 3822-3828
- **McLaughlin et al.** *J. Virol.,* 1989, vol. 62, 1963-1973
- **Tratschin et al.** *Mol. Cell. Biol.,* 1985, vol. 5, 3251-3260
- **Hermonat et al.** *PNAS USA,* 1984, vol. 81, 6466-6470
- **Tratschin et al.** *Mol. Cell. Biol.,* 1985, vol. 4, 2072-2081
- **Wondisford et al.** *Mol. Endocrinol.,* 1988, vol. 2, 32-39
- **Tratschin et al.** *J. Virol.,* 1984, vol. 51, 611-619
- **Flotte et al.** *J. Biol. Chem.,* 1993, vol. 268, 3781-3790
- **Pepose et al.** *Invest Ophthalmol Vis Sci,* 1994, vol. 35, 2662-2666
- **Mizuno et al.** *No Shinkei Geka,* 1992, vol. 20, 547-551

- **Mizuno et al.** *Neurol. Med. Chir.,* 1992, vol. 32, 873-876
- **Mulligan et al.** *Science,* 1993, 260-926
- **Wagner et al.** *PNAS USA,* 1992, vol. 89, 7934
- **Christiano et al.** *PNAS USA,* 1993, vol. 90, 2122
- **Chen et al.** *PNAS USA,* 1994, vol. 91, 3054-3057
- **Backes et al.** *Nature Biotechnology,* 2000, vol. 18, 187-193
- **Merchant et al.** *Nature Biotechnology,* 1998, vol. 16, 677-681
- **Szmola et al.** Human mesotrypsin is a unique digestive protease specialized for the degradation of trypsin inhibitors. *J Biol Chem.,* 2003, vol. 278 (49), 48580-9
- **YG Kim, J.C. ; S Chandrasegaran.** Hybrid restriction enzymes-zinc finger fusions to Fok I cleavage domain. *Proc Natl Acad Sci U S A,* 1996, vol. 93, 1156-1160
- **Zhou, P. ; Bogacki R ; McReynolds L. ; Howley PM.** Harnessing the Ubiquitination Machinery to Target the Degradation of Specific Cellular Proteins. *Molecular Cell,* 2000, vol. 6, 751-756
- **Bode, C. ; Runge M.S. ; Branscomb E.E. ; Newell J.B. ; Matsueda G.R. ; Haber E.** Anitbody-directed Fibrinolysis. *The Journal of Biological Chemistry,* 15 January 1989, vol. 264, 944-948
- **Runge, M.S.B., Christoph ; Matsueda, Gary R.** Haber, Edgar, Antibody-Enhanced Thrombolysis: Targeting of Tissue Plasminogen Activator in vivo. *Proceedings of the National Academy of Sciences of the United States of America,* 1987, vol. 84 (21), 7659-7662
- **Davis BG, S., RF ; Hodgspm DRW ; Ullman A ; Khumtaveeporn K ; Estell DA ; Sanford K ; Bott RR ; Jones JB.** Selective protein degradation by ligand-targeted enzymes: towards the creation of catalytic antagonists. *ChemBioChem,* 2003, vol. 4, 531-540
- **Zhou, H.-X.** Quantitative Account of the Enhanced Affinity of Two Linked scFvs Specific for Different Epitopes on the Same Antigen. *J. Mol. Biol.,* 2003, 1-8
- **Choy EH, P.G.** Cytokine pathways and joint inflammation in rheumatoid arthritis. *N Engl J Med.,* 2001, vol. 344 (12), 907-16
- **Feldmann M, M.R.** Anti-TNF alpha ther apy of rheumatoid arthritis: what have we learned?. *Annu Rev Immunol.,* 2001, vol. 19, 163-96
- **Feldman, M.** Development of anti-TNF therapy for rheumatoid arthritis. Nature Publishing Group, 2002, 2
- **Idriss, H.T.N. ; James H.** TNF$\alpha$ and the TNF Receptor Superfamily: Structure-Function Relationship(s). *Microscopy Research and Technique,* 2000, 184-195
- **Bodmer, J.-L. ; Schneider P. ; Tschopp J.** The molecular architecture of the TNF superfamily. *Trends in Biochemical Sciences,* 2002, vol. 27 (1), 19
- **Pennica D, K.W. ; Fendly BM ; Shire SJ ; Raab HE ; Borchardt PE ; Lewis M ; Goeddel DV.** Characterization of a recombinant extracellular domain of the type 1 tumor necrosis factor receptor: evidence for tumor necrosis factor-alpha induced receptor aggregation. *Biochemistry,* 1992, vol. 31 (4), 1134-41
- **Nophar, Y. ; Brakebusch C. ; Englemann H. ; Zwang R ; Aderka D. ; Holtman H. ; Wallach D.** Soluble forms of tumor necrosis factor receptors (TNF-Rs). The cDNA for the type I TNF-R, cloned using amino acid sequence data of its soluble form, encodes both the cell surface and a soluble form of the receptor. *EMBO J,* 1990, vol. 9 (10), 3269-78
- **Maini RN, Z.N.** Rheumatoid arthritis, in Rheumatology. 1994, 3.1-3.14.8
- **Warris, A. ; A. Bjorneklett ; P. Gaustad.** Invasive pulmonary aspergillosis associated with infliximab therapy. *N Engl J Med,* 2001, vol. 344 (14), 1099-100
- **Keane, J. ; Gershon S. ; Wise R.P. ; mirabilke-Levens E. ; Kasznica J. ; Schwieterman W.D. ; Siegel J.N. ; Braun M.M.** Tuberculosis associated with infliximab, a tumor necrosis factor alpha-neutralizing agent. *N Engl J Med,* 2001, vol. 345 (15), 1098-104
- **Williams, R.O. ; M. Feldmann ; R.N. Maini.** Anti-tumor necrosis factor ameliorates joint disease in murine collagen-induced arthritis. *Proc Natl Acad Sci U S A,* 1992, vol. 89 (20), 9784-8
- **Churchill ME, S.E. ; Pinilla C ; Appel JR ; Houghten RA ; Kono DH ; Balderas RS ; Fieser GG ; Schulze-Gahmen U ; Wilson IA.** Crystal structure of a peptide complex of anti-influenza peptide antibody Fab 26/9. Comparison of two different antibodies bound to the same peptide antigen. *J Mol Biol.,* 1994, vol. 241 (4), 534-56
- **Calkins CC, P.K. ; Potempa J ; Travis J.** Inactivation of tumor necrosis factor-alpha by proteinases (gingipains) from the periodontal pathogen, Porphyromonas gingivalis. Implications of immune evasion. *J Biol Chem,* 1998, vol. 273 (12), 6611-4
- **Nakamura K, K.M.** Proteolysis of human tumor necrosis factor (TNF) by endo- and exopeptidases: process of proteolysis and formation of active fragments. *Biol Pharm Bull,* 1996, vol. 19 (5), 672-7
- **Narhi LO, RM. ; Hunt P ; Arakawa T.** The limited proteolysis of tumor necrosis factor-alpha. *J Protein Chem,* 1989, vol. 8 (5), 669-77

- **Kim YJ, C.S. ; Kim JS ; Shin NK ; Jeong W ; Shin HC ; Oh BH ; Hahn JH.** Determination of the limited trypsinolysis pathways of tumor necrosis factor-alpha and its mutant by electrospray ionization mass spectrometry. *Anal Biochem.,* 1999, vol. 267 (2), 279-86
- **Magni F, C.F. ; Marazzini L ; Colombo R ; Sacchi A ; Corti A ; Kienle MG.** Biotinylation sites of tumor necrosis factor-alpha determined by liquid chromatography-mass spectrometry. *Anal Biochem.,* 2001, vol. 298 (2), 181-8
- **van Kessel KP, v.S.J. ; Verhoef J.** Inactivation of recombinant human tumor necrosis factor-alpha by proteolytic enzymes released from stimulated human neutrophils. *J Immunol.,* 1991, vol. 147 (11), 3862-8
- **Locksley RM, K.N. ; Lenardo MJ.** The TNF and TNF receptor superfamilies: integrating mammalian biology. *Cell,* 2001, vol. 104 (4), 487-501
- **Humphreys, D.T. ; M.R. Wilson.** Modes of L929 cell death induced by TNF-alpha and other cytotoxic agents. *Cytokine,* 1999, vol. 11 (10), 773-82
- **Zhao, X.M., L ; Song, K ; Oliver, P ; Chin, SY ; Simon, H ; Schurr, JR ; Zhang, Z ; Thoppil, D ; Lee, S ; Nelson, S.** *Acute Alcohol Inhibits TNF-alpha Processing in Human Monocytes by Inhibiting TNF/TNF-alpha-Converting Enzyme Interactions in the Cell Membrane.,* 2003, 2923-2931
- **Marsters SA, F.A. ; Simpson NJ ; Fendly BM ; Ashkenazi A.** Identification of cysteine-rich domains of the type 1 tumor necrosis factor receptor involved in ligand binding. *J Biol Chem.,* 1992, vol. 267 (9), 5747-50
- **Chen PC, D.G. ; Chen MJ.** Mapping the domain(s) critical for the binding of human tumor necrosis factor-alpha to its two receptors. *J Biol Chem.,* 1995, vol. 270 (6), 2874-8
- **Rosenberg JJ, M.S. ; Seely JE ; Kinstler O ; Gaines GC ; Fukuzuka K ; Rose J ; Kohno T ; Boyle WJ ; Nelson A ; Kieft GL.** Development of a novel, nonimmunogenic, soluble human TNF receptor type I (sTNFR-I) construct in the baboon. *J Appl Physiol.,* 2001, vol. 91 (5), 2213-23
- **Whitcomb, D.C. ; Gorry M.C. ; Preston R.A. ; Furey W. ; Sossenheimer M.J. ; Ulrich C.D. ; Martin S.P. ; Gates L.K. ; Amann S.T. ; Toskes P.P.** Hereditary pancreatitis is caused by a mutation in the cationic trypsinogen gene. *Nat Genet,* 1996, vol. 14 (2), 141-5